(19) Europäisches Patentamt
European Patent Office
Office européen des brevets

(11) EP 3 875 489 A1

(12) **EUROPEAN PATENT APPLICATION**
published in accordance with Art. 153(4) EPC

(43) Date of publication:
**08.09.2021 Bulletin 2021/36**

(21) Application number: **19877895.3**

(22) Date of filing: **31.10.2019**

(51) Int Cl.:
*C07K 16/46* (2006.01) *C12N 15/13* (2006.01)
*A61K 39/395* (2006.01) *A61P 35/00* (2006.01)

(86) International application number:
**PCT/CN2019/114818**

(87) International publication number:
**WO 2020/088608 (07.05.2020 Gazette 2020/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.11.2018 CN 201811294887**

(71) Applicant: **Ampsource Biopharma Shanghai Inc.**
**Shanghai 201318 (CN)**

(72) Inventors:
- **LI, Qiang**
  **Shanghai 201318 (CN)**
- **MA, Xinlu**
  **Shanghai 201318 (CN)**
- **JIA, Shixiang**
  **Shanghai 201318 (CN)**
- **YAN, Yuan**
  **Shanghai 201318 (CN)**
- **ZHANG, Yuhua**
  **Shanghai 201318 (CN)**
- **ZHOU, Li**
  **Shanghai 201318 (CN)**
- **SUN, Rilong**
  **Shanghai 201318 (CN)**
- **CUI, Xueyuan**
  **Shanghai 201318 (CN)**
- **YU, Lingju**
  **Shanghai 201318 (CN)**
- **YAN, Yujie**
  **Shanghai 201318 (CN)**
- **JIN, Yingying**
  **Shanghai 201318 (CN)**
- **XIONG, Yao**
  **Shanghai 201318 (CN)**
- **LI, Yuanli**
  **Shanghai 201318 (CN)**
- **CHEN, Si**
  **Shanghai 201318 (CN)**
- **LIU, Xuemei**
  **Shanghai 201318 (CN)**
- **DIAO, Jiasheng**
  **Shanghai 201318 (CN)**

(74) Representative: **Prinz & Partner mbB**
**Patent- und Rechtsanwälte**
**Leipziger Platz 15**
**10117 Berlin (DE)**

(54) **HOMODIMERIC BISPECIFIC ANTIBODY, PREPARATION METHOD THEREFOR AND USE THEREOF**

(57) Provided is a tetravalent homodimeric bispecific antibody molecule simultaneously targeting an immune effector cell antigen CD3 and a tumor-associated antigen, wherein the bispecific antibody molecule contains, in order from N-terminus to C-terminus, a first single chain Fv, a second single chain Fv and a Fc fragment; wherein the first single chain Fv can specifically bind to the tumor-associated antigen, the second single chain Fv can specifically bind to CD3, and the first and the second single chain Fvs are connected by a linker peptide, while the second single chain Fv and the Fc fragment are directly connected or connected by a linker peptide; and the Fc fragment does not have effector functions such as CDC, ADCC and ADCP.

EP 3 875 489 A1

SEQUENCE LISTING

<110> AMPSOURCE BIOPHARMA SHANGHAI INC.

<120> HOMODIMERIC BISPECIFIC ANTIBODY, PREPARATION METHOD THEREFOR AND USE THEREOF

<130> A4657EP

<140> PCT/CN2019/114818
<141> 2019-10-31

<150> CN201811294887.4
<151> 2018-11-01

<160> 286

<170> PatentIn version 3.5

<210> 1
<211> 744
<212> PRT
<213> Artificial sequence

<220>
<223> bispecific antibody AB7K5 amino acid sequence

<400> 1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140
```

```
Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
            180                 185                 190

Val Pro Ser Arg Phe Ser Gly Ser Arg Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Asp Lys Thr His Thr Ser Pro Pro Ser Pro Ala Pro Glu Ala
                245                 250                 255

Ala Gly Gly Pro Ser Val Phe Leu Phe Pro Pro Lys Pro Lys Asp Gln
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Ala Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Ser Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Arg Cys His Val Lys Gly Phe Tyr Pro Ser Asp Ile Ala
```

385 390 395 400

Val Glu Trp Glu Ser Asn Gly Gln Pro Glu Asn Asn Tyr Lys Thr Thr
405 410 415

Pro Pro Val Leu Asp Ser Asp Gly Ser Phe Phe Leu Tyr Ser Thr Leu
420 425 430

Thr Val Asp Lys Ser Arg Trp Gln Gln Gly Asn Val Phe Ser Cys Ser
435 440 445

Val Leu His Glu Ala Leu His Asn His Tyr Thr Gln Lys Ser Leu Ser
450 455 460

Leu Ser Pro Gly Gly Gly Gly Gly Gly Gly Ser Gly Gly Gly Gly Ser
465 470 475 480

Gly Gly Gly Gly Ser Ser Ser Ser Ser Lys Ala Pro Pro Pro Ser Glu
485 490 495

Val Gln Leu Leu Glu Ser Gly Gly Gly Leu Val Gln Pro Gly Gly Ser
500 505 510

Leu Lys Leu Ser Cys Ala Ala Ser Gly Phe Thr Phe Asn Thr Tyr Ala
515 520 525

Met Asn Trp Val Arg Gln Ala Pro Gly Lys Gly Leu Glu Trp Val Ala
530 535 540

Arg Ile Arg Ser Lys Tyr Asn Asn Tyr Ala Thr Tyr Tyr Ala Asp Ser
545 550 555 560

Val Lys Asp Arg Phe Thr Ile Ser Arg Asp Asp Ser Lys Asn Thr Ala
565 570 575

Tyr Leu Gln Met Asn Asn Leu Lys Thr Glu Asp Thr Ala Val Tyr Tyr
580 585 590

Cys Val Arg His Gly Asn Phe Gly Asn Ser Tyr Val Ser Trp Phe Ala
595 600 605

Tyr Trp Gly Gln Gly Thr Leu Val Thr Val Ser Ser Gly Gly Gly Gly
610 615 620

Ser Gly Gly Gly Gly Ser Gly Gly Gly Gly Ser Glu Leu Val Val Thr
625 630 635 640

Gln Glu Pro Ser Leu Thr Val Ser Pro Gly Gly Thr Val Thr Leu Thr
645 650 655

Cys Arg Ser Ser Thr Gly Ala Val Thr Thr Ser Asn Tyr Ala Asn Trp
660 665 670

Val Gln Gln Lys Pro Gly Gln Ala Pro Arg Gly Leu Ile Gly Gly Thr
675 680 685

Asn Lys Arg Ala Pro Gly Thr Pro Ala Arg Phe Ser Gly Ser Leu Leu
690 695 700

Gly Gly Lys Ala Ala Leu Thr Leu Ser Gly Val Gln Pro Glu Asp Glu
705 710 715 720

Ala Glu Tyr Tyr Cys Ala Leu Trp Tyr Ser Asn Leu Trp Val Phe Gly
725 730 735

Gly Gly Thr Lys Leu Thr Val Leu
740

<210> 2
<211> 744
<212> PRT
<213> Artificial sequence

<220>
<223> bispecific antibody AB7K6 amino acid sequence

<400> 2

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln

```
            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
            180                 185                 190

Val Pro Ser Arg Phe Ser Gly Ser Arg Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
                325                 330                 335

Asp Arg Phe Thr Ile Ser Arg Asp Asp Ser Lys Asn Thr Ala Tyr Leu
            340                 345                 350
```

Gln Met Asn Asn Leu Lys Thr Glu Asp Thr Ala Val Tyr Tyr Cys Val
355 360 365

Arg His Gly Asn Phe Gly Asn Ser Tyr Val Ser Trp Phe Ala Tyr Trp
370 375 380

Gly Gln Gly Thr Leu Val Thr Val Ser Ser Gly Gly Gly Gly Ser Gly
385 390 395 400

Gly Gly Gly Ser Gly Gly Gly Gly Ser Glu Leu Val Val Thr Gln Glu
405 410 415

Pro Ser Leu Thr Val Ser Pro Gly Gly Thr Val Thr Leu Thr Cys Arg
420 425 430

Ser Ser Thr Gly Ala Val Thr Thr Ser Asn Tyr Ala Asn Trp Val Gln
435 440 445

Gln Lys Pro Gly Gln Ala Pro Arg Gly Leu Ile Gly Gly Thr Asn Lys
450 455 460

Arg Ala Pro Gly Thr Pro Ala Arg Phe Ser Gly Ser Leu Leu Gly Gly
465 470 475 480

Lys Ala Ala Leu Thr Leu Ser Gly Val Gln Pro Glu Asp Glu Ala Glu
485 490 495

Tyr Tyr Cys Ala Leu Trp Tyr Ser Asn Leu Trp Val Phe Gly Gly Gly
500 505 510

Thr Lys Leu Thr Val Leu Asp Lys Thr His Thr Ser Pro Pro Ser Pro
515 520 525

Ala Pro Glu Ala Ala Gly Gly Pro Ser Val Phe Leu Phe Pro Pro Lys
530 535 540

Pro Lys Asp Gln Leu Met Ile Ser Arg Thr Pro Glu Val Thr Cys Val
545 550 555 560

Val Val Asp Val Ser His Glu Asp Pro Glu Val Lys Phe Asn Trp Tyr
565 570 575

Val Asp Gly Val Glu Val His Asn Ala Lys Thr Lys Pro Arg Glu Glu
580 585 590

Gln Tyr Ala Ser Thr Tyr Arg Val Val Ser Val Leu Thr Val Leu His
595 600 605

Gln Asp Trp Leu Asn Gly Lys Glu Tyr Lys Cys Lys Val Ser Asn Lys
610 615 620

Ala Leu Pro Ala Ser Ile Glu Lys Thr Ile Ser Lys Ala Lys Gly Gln
625 630 635 640

Pro Arg Glu Pro Gln Val Tyr Thr Leu Pro Pro Ser Arg Asp Glu Leu
645 650 655

Thr Lys Asn Gln Val Ser Leu Arg Cys His Val Lys Gly Phe Tyr Pro
660 665 670

Ser Asp Ile Ala Val Glu Trp Glu Ser Asn Gly Gln Pro Glu Asn Asn
675 680 685

Tyr Lys Thr Thr Pro Pro Val Leu Asp Ser Asp Gly Ser Phe Phe Leu
690 695 700

Tyr Ser Thr Leu Thr Val Asp Lys Ser Arg Trp Gln Gln Gly Asn Val
705 710 715 720

Phe Ser Cys Ser Val Leu His Glu Ala Leu His Asn His Tyr Thr Gln
725 730 735

Lys Ser Leu Ser Leu Ser Pro Gly
740

<210> 3
<211> 526
<212> PRT
<213> Artificial sequence

<220>
<223> bispecific antibody AB7K8 amino acid sequence

<400> 3

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys Arg Ala
145                 150                 155                 160

Ser Gln Asp Val Asn Thr Ala Val Ala Trp Tyr Gln Gln Lys Pro Gly
                165                 170                 175

Lys Ala Pro Lys Leu Leu Ile Tyr Ser Ala Ser Phe Leu Tyr Ser Gly
            180                 185                 190

Val Pro Ser Arg Phe Ser Gly Ser Arg Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln His Tyr Thr Thr Pro Pro Thr Phe Gly Gln Gly Thr Lys Val Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
325 330 335

Asp Arg Phe Thr Ile Ser Arg Asp Asp Ser Lys Asn Thr Ala Tyr Leu
340 345 350

Gln Met Asn Asn Leu Lys Thr Glu Asp Thr Ala Val Tyr Tyr Cys Val
355 360 365

Arg His Gly Asn Phe Gly Asn Ser Tyr Val Ser Trp Phe Ala Tyr Trp
370 375 380

Gly Gln Gly Thr Leu Val Thr Val Ser Ser Gly Gly Gly Gly Ser Gly
385 390 395 400

Gly Gly Gly Ser Gly Gly Gly Gly Ser Glu Leu Val Val Thr Gln Glu
405 410 415

Pro Ser Leu Thr Val Ser Pro Gly Gly Thr Val Thr Leu Thr Cys Arg
420 425 430

Ser Ser Thr Gly Ala Val Thr Thr Ser Asn Tyr Ala Asn Trp Val Gln
435 440 445

Gln Lys Pro Gly Gln Ala Pro Arg Gly Leu Ile Gly Gly Thr Asn Lys
450 455 460

Arg Ala Pro Gly Thr Pro Ala Arg Phe Ser Gly Ser Leu Leu Gly Gly
465 470 475 480

Lys Ala Ala Leu Thr Leu Ser Gly Val Gln Pro Glu Asp Glu Ala Glu
485 490 495

Tyr Tyr Cys Ala Leu Trp Tyr Ser Asn Leu Trp Val Phe Gly Gly Gly
500 505 510

Thr Lys Leu Thr Val Leu His His His His His His His His
515 520 525

<210> 4
<211> 744
<212> PRT
<213> Artificial sequence

<220>
<223> bispecific antibody AB7K heavy chain amino acid sequence

<400> 4

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255
```

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys Gly Ser Gly Gly Gly Gly Ser Gly Gly Gly Gly Ser Gly Gly
450 455 460

Gly Gly Ser Ser Ser Ser Ser Lys Ala Pro Pro Pro Ser Leu Pro Ser
465 470 475 480

Pro Ser Arg Leu Pro Gly Pro Ser Asp Thr Pro Ile Leu Pro Gln Glu
485 490 495

Val Gln Leu Leu Glu Ser Gly Gly Gly Leu Val Gln Pro Gly Gly Ser
500 505 510

Leu Lys Leu Ser Cys Ala Ala Ser Gly Phe Thr Phe Asn Thr Tyr Ala
515 520 525

Met Asn Trp Val Arg Gln Ala Pro Gly Lys Gly Leu Glu Trp Val Ala
530 535 540

Arg Ile Arg Ser Lys Tyr Asn Asn Tyr Ala Thr Tyr Tyr Ala Asp Ser
545 550 555 560

Val Lys Asp Arg Phe Thr Ile Ser Arg Asp Asp Ser Lys Asn Thr Ala
565 570 575

Tyr Leu Gln Met Asn Asn Leu Lys Thr Glu Asp Thr Ala Val Tyr Tyr
580 585 590

Cys Val Arg His Gly Asn Phe Gly Asn Ser Tyr Val Ser Trp Phe Ala
595 600 605

Tyr Trp Gly Gln Gly Thr Leu Val Thr Val Ser Ser Gly Gly Gly Gly
610 615 620

Ser Gly Gly Gly Gly Ser Gly Gly Gly Gly Ser Glu Leu Val Val Thr
625 630 635 640

Gln Glu Pro Ser Leu Thr Val Ser Pro Gly Gly Thr Val Thr Leu Thr
645 650 655

Cys Arg Ser Ser Thr Gly Ala Val Thr Thr Ser Asn Tyr Ala Asn Trp
660 665 670

Val Gln Gln Lys Pro Gly Gln Ala Pro Arg Gly Leu Ile Gly Gly Thr
675 680 685

Asn Lys Arg Ala Pro Gly Thr Pro Ala Arg Phe Ser Gly Ser Leu Leu
690 695 700

Gly Gly Lys Ala Ala Leu Thr Leu Ser Gly Val Gln Pro Glu Asp Glu
705 710 715 720

Ala Glu Tyr Tyr Cys Ala Leu Trp Tyr Ser Asn Leu Trp Val Phe Gly
725 730 735

Gly Gly Thr Lys Leu Thr Val Leu
740

<210> 5

<211> 214
<212> PRT
<213> Artificial sequence

<220>
<223> bispecific antibody AB7K light chain amino acid sequence

<400> 5

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 6
<211> 449
<212> PRT
<213> Artificial sequence

<220>
<223> bispecific antibody AB7K4 heavy chain amino acid sequence

<400> 6

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

```
Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Gln Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Ala Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Ser Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Leu His
            420                 425                 430

Glu Ala Leu His Asn His Tyr Thr Gln Lys Ser Leu Ser Leu Ser Pro
        435                 440                 445

Gly
```

<210> 7
<211> 490
<212> PRT
<213> Artificial sequence

<220>
<223> bispecific antibody AB7K4 light chain amino acid sequence

<400> 7

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Gly Gly Ser Gly Gly Gly
    210                 215                 220

Gly Ser Gly Gly Gly Gly Ser Ser Ser Ser Ser Lys Ala Pro Pro Pro
225                 230                 235                 240

Ser Glu Val Gln Leu Leu Glu Ser Gly Gly Gly Leu Val Gln Pro Gly
                245                 250                 255

Gly Ser Leu Lys Leu Ser Cys Ala Ala Ser Gly Phe Thr Phe Asn Thr
            260                 265                 270

Tyr Ala Met Asn Trp Val Arg Gln Ala Pro Gly Lys Gly Leu Glu Trp
        275                 280                 285

Val Ala Arg Ile Arg Ser Lys Tyr Asn Asn Tyr Ala Thr Tyr Tyr Ala
    290                 295                 300

Asp Ser Val Lys Asp Arg Phe Thr Ile Ser Arg Asp Asp Ser Lys Asn
305                 310                 315                 320

Thr Ala Tyr Leu Gln Met Asn Asn Leu Lys Thr Glu Asp Thr Ala Val
                325                 330                 335

Tyr Tyr Cys Val Arg His Gly Asn Phe Gly Asn Ser Tyr Val Ser Trp
            340                 345                 350

Phe Ala Tyr Trp Gly Gln Gly Thr Leu Val Thr Val Ser Ser Gly Gly
        355                 360                 365

Gly Gly Ser Gly Gly Gly Gly Ser Gly Gly Gly Gly Ser Glu Leu Val
    370                 375                 380

Val Thr Gln Glu Pro Ser Leu Thr Val Ser Pro Gly Gly Thr Val Thr
385                 390                 395                 400

Leu Thr Cys Arg Ser Ser Thr Gly Ala Val Thr Thr Ser Asn Tyr Ala
                405                 410                 415

Asn Trp Val Gln Gln Lys Pro Gly Gln Ala Pro Arg Gly Leu Ile Gly
            420                 425                 430

Gly Thr Asn Lys Arg Ala Pro Gly Thr Pro Ala Arg Phe Ser Gly Ser
        435                 440                 445

Leu Leu Gly Gly Lys Ala Ala Leu Thr Leu Ser Gly Val Gln Pro Glu
    450                 455                 460

Asp Glu Ala Glu Tyr Tyr Cys Ala Leu Trp Tyr Ser Asn Leu Trp Val
465 470 475 480

Phe Gly Gly Gly Thr Lys Leu Thr Val Leu
485 490

<210> 8
<211> 744
<212> PRT
<213> Artificial sequence

<220>
<223> bispecific antibody AB7K7 amino acid sequence

<400> 8

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser Gly Gly Gly Gly Ser Gly Gly Gly
115 120 125

Gly Ser Gly Gly Gly Gly Ser Asp Ile Gln Met Thr Gln Ser Pro Ser
130 135 140

Ser Leu Ser Ala Ser Val Gly Asp Arg Val Thr Ile Thr Cys Arg Ala
145 150 155 160

Ser Gln Asp Val Asn Thr Ala Val Ala Trp Tyr Gln Gln Lys Pro Gly
165 170 175

Lys Ala Pro Lys Leu Leu Ile Tyr Ser Ala Ser Phe Leu Tyr Ser Gly
180 185 190

Val Pro Ser Arg Phe Ser Gly Ser Arg Ser Gly Thr Asp Phe Thr Leu
195 200 205

Thr Ile Ser Ser Leu Gln Pro Glu Asp Phe Ala Thr Tyr Tyr Cys Gln
210 215 220

Gln His Tyr Thr Thr Pro Pro Thr Phe Gly Gln Gly Thr Lys Val Glu
225 230 235 240

Ile Lys Gly Gly Gly Gly Gly Gly Ser Gly Gly Gly Gly Ser Gly Gly
245 250 255

Gly Gly Ser Ser Ser Ser Ser Lys Ala Pro Pro Pro Ser Glu Val Gln
260 265 270

Leu Leu Glu Ser Gly Gly Gly Leu Val Gln Pro Gly Gly Ser Leu Lys
275 280 285

Leu Ser Cys Ala Ala Ser Gly Phe Thr Phe Asn Thr Tyr Ala Met Asn
290 295 300

Trp Val Arg Gln Ala Pro Gly Lys Gly Leu Glu Trp Val Ala Arg Ile
305 310 315 320

Arg Ser Lys Tyr Asn Asn Tyr Ala Thr Tyr Tyr Ala Asp Ser Val Lys
325 330 335

Asp Arg Phe Thr Ile Ser Arg Asp Asp Ser Lys Asn Thr Ala Tyr Leu
340 345 350

Gln Met Asn Asn Leu Lys Thr Glu Asp Thr Ala Val Tyr Tyr Cys Val
355 360 365

Arg His Gly Asn Phe Gly Asn Ser Tyr Val Ser Trp Phe Ala Tyr Trp
370 375 380

Gly Gln Gly Thr Leu Val Thr Val Ser Ser Gly Gly Gly Gly Ser Gly
385 390 395 400

Gly Gly Gly Ser Gly Gly Gly Gly Ser Glu Leu Val Val Thr Gln Glu
405 410 415

Pro Ser Leu Thr Val Ser Pro Gly Gly Thr Val Thr Leu Thr Cys Arg
420 425 430

Ser Ser Thr Gly Ala Val Thr Thr Ser Asn Tyr Ala Asn Trp Val Gln
435 440 445

Gln Lys Pro Gly Gln Ala Pro Arg Gly Leu Ile Gly Gly Thr Asn Lys
450 455 460

Arg Ala Pro Gly Thr Pro Ala Arg Phe Ser Gly Ser Leu Leu Gly Gly
465 470 475 480

Lys Ala Ala Leu Thr Leu Ser Gly Val Gln Pro Glu Asp Glu Ala Glu
485 490 495

Tyr Tyr Cys Ala Leu Trp Tyr Ser Asn Leu Trp Val Phe Gly Gly Gly
500 505 510

Thr Lys Leu Thr Val Leu Asp Lys Thr His Thr Cys Pro Pro Cys Pro
515 520 525

Ala Pro Glu Ala Ala Gly Gly Pro Ser Val Phe Leu Phe Pro Pro Lys
530 535 540

Pro Lys Asp Gln Leu Met Ile Ser Arg Thr Pro Glu Val Thr Cys Val
545 550 555 560

Val Val Asp Val Ser His Glu Asp Pro Glu Val Lys Phe Asn Trp Tyr
565 570 575

Val Asp Gly Val Glu Val His Asn Ala Lys Thr Lys Pro Arg Glu Glu
580 585 590

Gln Tyr Ala Ser Thr Tyr Arg Val Val Ser Val Leu Thr Val Leu His
595 600 605

Gln Asp Trp Leu Asn Gly Lys Glu Tyr Lys Cys Lys Val Ser Asn Lys
610 615 620

Ala Leu Pro Ala Ser Ile Glu Lys Thr Ile Ser Lys Ala Lys Gly Gln
625 630 635 640

Pro Arg Glu Pro Gln Val Tyr Thr Leu Pro Pro Ser Arg Asp Glu Leu
645 650 655

Thr Lys Asn Gln Val Ser Leu Thr Cys Leu Val Lys Gly Phe Tyr Pro
660 665 670

Ser Asp Ile Ala Val Glu Trp Glu Ser Asn Gly Gln Pro Glu Asn Asn

675 680 685

Tyr Lys Thr Thr Pro Pro Val Leu Asp Ser Asp Gly Ser Phe Phe Leu
690 695 700

Tyr Ser Lys Leu Thr Val Asp Lys Ser Arg Trp Gln Gln Gly Asn Val
705 710 715 720

Phe Ser Cys Ser Val Leu His Glu Ala Leu His Asn His Tyr Thr Gln
725 730 735

Lys Ser Leu Ser Leu Ser Pro Gly
740

<210> 9
<211> 5
<212> PRT
<213> Artificial sequence

<220>
<223> anti-CD19 antibody 1 HCDR1 amino acid sequence

<400> 9

Ser Tyr Trp Met Asn
1 5

<210> 10
<211> 17
<212> PRT
<213> Artificial sequence

<220>
<223> anti-CD19 antibody 1 HCDR2 amino acid sequence

<400> 10

Gln Ile Trp Pro Gly Asp Gly Asp Thr Asn Tyr Asn Gly Lys Phe Lys
1 5 10 15

Gly

<210> 11
<211> 15
<212> PRT
<213> Artificial sequence

<220>
<223> anti-CD19 antibody 1 HCDR3 amino acid sequence

<400> 11

Arg Glu Thr Thr Thr Val Gly Arg Tyr Tyr Tyr Ala Met Asp Tyr
1 5 10 15

<210> 12
<211> 15
<212> PRT
<213> Artificial sequence

<220>
<223> anti-CD19 antibody 1 LCDR1 amino acid sequence

<400> 12

Lys Ala Ser Gln Ser Val Asp Tyr Asp Gly Asp Ser Tyr Leu Asn
1               5                   10                  15

<210> 13
<211> 7
<212> PRT
<213> Artificial sequence

<220>
<223> anti-CD19 antibody 1 LCDR2 amino acid sequence

<400> 13

Asp Ala Ser Asn Leu Val Ser
1               5

<210> 14
<211> 9
<212> PRT
<213> Artificial sequence

<220>
<223> anti-CD19 antibody 1 LCDR3 amino acid sequence

<400> 14

Gln Gln Ser Thr Glu Asp Pro Trp Thr
1               5

<210> 15
<211> 124
<212> PRT
<213> Artificial sequence

<220>
<223> anti-CD19 antibody 1 VH amino acid sequence

<400> 15

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Ser Tyr
            20                  25                  30

Trp Met Asn Trp Val Lys Gln Arg Pro Gly Gln Gly Leu Glu Trp Ile

35 40 45

Gly Gln Ile Trp Pro Gly Asp Gly Asp Thr Asn Tyr Asn Gly Lys Phe
50 55 60

Lys Gly Lys Ala Thr Leu Thr Ala Asp Glu Ser Ser Ser Thr Ala Tyr
65 70 75 80

Met Gln Leu Ser Ser Leu Ala Ser Glu Asp Ser Ala Val Tyr Phe Cys
85 90 95

Ala Arg Arg Glu Thr Thr Thr Val Gly Arg Tyr Tyr Tyr Ala Met Asp
100 105 110

Tyr Trp Gly Gln Gly Thr Thr Val Thr Val Ser Ser
115 120

<210> 16
<211> 111
<212> PRT
<213> Artificial sequence

<220>
<223> anti-CD19 antibody 1 VL amino acid sequence

<400> 16

Asp Ile Gln Leu Thr Gln Ser Pro Ala Ser Leu Ala Val Ser Leu Gly
1 5 10 15

Gln Arg Ala Thr Ile Ser Cys Lys Ala Ser Gln Ser Val Asp Tyr Asp
20 25 30

Gly Asp Ser Tyr Leu Asn Trp Tyr Gln Gln Ile Pro Gly Gln Pro Pro
35 40 45

Lys Leu Leu Ile Tyr Asp Ala Ser Asn Leu Val Ser Gly Ile Pro Pro
50 55 60

Arg Phe Ser Gly Ser Gly Ser Gly Thr Asp Phe Thr Leu Asn Ile His
65 70 75 80

Pro Val Glu Lys Val Asp Ala Ala Thr Tyr His Cys Gln Gln Ser Thr
85 90 95

Glu Asp Pro Trp Thr Phe Gly Gly Gly Thr Lys Leu Glu Ile Lys
100 105 110

<210> 17
<211> 5

<212> PRT
<213> Artificial sequence

<220>
<223> anti-CD19 antibody 2 HCDR1 amino acid sequence

<400> 17

Ser Asn Trp Met His
1 5

<210> 18
<211> 17
<212> PRT
<213> Artificial sequence

<220>
<223> anti-CD19 antibody 2 HCDR2 amino acid sequence

<400> 18

Glu Ile Asp Pro Ser Asp Ser Tyr Thr Asn Tyr Asn Gln Asn Phe Gln
1 5 10 15

Gly

<210> 19
<211> 11
<212> PRT
<213> Artificial sequence

<220>
<223> anti-CD19 antibody 2 HCDR3 amino acid sequence

<400> 19

Gly Ser Asn Pro Tyr Tyr Tyr Ala Met Asp Tyr
1 5 10

<210> 20
<211> 10
<212> PRT
<213> Artificial sequence

<220>
<223> anti-CD19 antibody 2 LCDR1 amino acid sequence

<400> 20

Ser Ala Ser Ser Gly Val Asn Tyr Met His
1 5 10

<210> 21
<211> 7
<212> PRT
<213> Artificial sequence

<220>
<223> anti-CD19 antibody 2 LCDR2 amino acid sequence

<400> 21

Asp Thr Ser Lys Leu Ala Ser
1 5

<210> 22
<211> 7
<212> PRT
<213> Artificial sequence

<220>
<223> anti-CD19 antibody 2 LCDR3 amino acid sequence

<400> 22

His Gln Arg Gly Ser Tyr Thr
1 5

<210> 23
<211> 120
<212> PRT
<213> Artificial sequence

<220>
<223> anti-CD19 antibody 2 VH amino acid sequence

<400> 23

Gln Val Gln Leu Val Gln Pro Gly Ala Glu Val Val Lys Pro Gly Ala
1 5 10 15

Ser Val Lys Leu Ser Cys Lys Thr Ser Gly Tyr Thr Phe Thr Ser Asn
20 25 30

Trp Met His Trp Val Lys Gln Ala Pro Gly Gln Gly Leu Glu Trp Ile
35 40 45

Gly Glu Ile Asp Pro Ser Asp Ser Tyr Thr Asn Tyr Asn Gln Asn Phe
50 55 60

Gln Gly Lys Ala Lys Leu Thr Val Asp Lys Ser Thr Ser Thr Ala Tyr
65 70 75 80

Met Glu Val Ser Ser Leu Arg Ser Asp Asp Thr Ala Val Tyr Tyr Cys
85 90 95

Ala Arg Gly Ser Asn Pro Tyr Tyr Tyr Ala Met Asp Tyr Trp Gly Gln
100 105 110

Gly Thr Ser Val Thr Val Ser Ser
115 120

<210> 24
<211> 104
<212> PRT
<213> Artificial sequence

<220>
<223> anti-CD19 antibody 2 VL amino acid sequence

<400> 24

Glu Ile Val Leu Thr Gln Ser Pro Ala Ile Met Ser Ala Ser Pro Gly
1               5                   10                  15

Glu Arg Val Thr Met Thr Cys Ser Ala Ser Ser Gly Val Asn Tyr Met
            20                  25                  30

His Trp Tyr Gln Gln Lys Pro Gly Thr Ser Pro Arg Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Asp Tyr Ser Leu Thr Ile Ser Ser Met Glu Pro Glu
65                  70                  75                  80

Asp Ala Ala Thr Tyr Tyr Cys His Gln Arg Gly Ser Tyr Thr Phe Gly
                85                  90                  95

Gly Gly Thr Lys Leu Glu Ile Lys
            100

<210> 25
<211> 7
<212> PRT
<213> Artificial sequence

<220>
<223> anti-CD19 antibody 3 HCDR1 amino acid sequence

<400> 25

Thr Ser Gly Met Gly Val Gly
1               5

<210> 26
<211> 16
<212> PRT
<213> Artificial sequence

<220>
<223> anti-CD19 antibody 3 HCDR2 amino acid sequence

<400> 26

His Ile Trp Trp Asp Asp Asp Lys Arg Tyr Asn Pro Ala Leu Lys Ser
1 5 10 15

<210> 27
<211> 10
<212> PRT
<213> Artificial sequence

<220>
<223> anti-CD19 antibody 3 HCDR3 amino acid sequence

<400> 27

Met Glu Leu Trp Ser Tyr Tyr Phe Asp Tyr
1 5 10

<210> 28
<211> 10
<212> PRT
<213> Artificial sequence

<220>
<223> anti-CD19 antibody 3 LCDR1 amino acid sequence

<400> 28

Ser Ala Ser Ser Ser Val Ser Tyr Met His
1 5 10

<210> 29
<211> 7
<212> PRT
<213> Artificial sequence

<220>
<223> anti-CD19 antibody 3 LCDR2 amino acid sequence

<400> 29

Asp Thr Ser Lys Leu Ala Ser
1 5

<210> 30
<211> 9
<212> PRT
<213> Artificial sequence

<220>
<223> anti-CD19 antibody 3 LCDR3 amino acid sequence

<400> 30

Phe Gln Gly Ser Val Tyr Pro Phe Thr
1 5

<210> 31
<211> 120

<212> PRT
<213> Artificial sequence

<220>
<223> anti-CD19 antibody 3 VH amino acid sequence

<400> 31

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Thr Ser
            20                  25                  30

Gly Met Gly Val Gly Trp Ile Arg Gln His Pro Gly Lys Gly Leu Glu
        35                  40                  45

Trp Ile Gly His Ile Trp Trp Asp Asp Asp Lys Arg Tyr Asn Pro Ala
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Met Glu Leu Trp Ser Tyr Tyr Phe Asp Tyr Trp Gly Gln
            100                 105                 110

Gly Thr Leu Val Thr Val Ser Ser
        115                 120
```

<210> 32
<211> 106
<212> PRT
<213> Artificial sequence

<220>
<223> anti-CD19 antibody 3 VL amino acid sequence

<400> 32

```
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
```

50 55 60

Gly Ser Gly Thr Asp Phe Thr Leu Thr Ile Ser Ser Leu Glu Pro Glu
65 70 75 80

Asp Val Ala Val Tyr Tyr Cys Phe Gln Gly Ser Val Tyr Pro Phe Thr
85 90 95

Phe Gly Gln Gly Thr Lys Leu Glu Ile Lys
100 105

<210> 33
<211> 5
<212> PRT
<213> Artificial sequence

<220>
<223> anti-CD19 antibody 4 HCDR1 amino acid sequence

<400> 33

Ser Ser Trp Met Asn
1 5

<210> 34
<211> 17
<212> PRT
<213> Artificial sequence

<220>
<223> anti-CD19 antibody 4 HCDR2 amino acid sequence

<400> 34

Arg Ile Tyr Pro Gly Asp Gly Asp Thr Asn Tyr Asn Val Lys Phe Lys
1 5 10 15

Gly

<210> 35
<211> 12
<212> PRT
<213> Artificial sequence

<220>
<223> anti-CD19 antibody 4 HCDR3 amino acid sequence

<400> 35

Ser Gly Phe Ile Thr Thr Val Arg Asp Phe Asp Tyr
1 5 10

<210> 36
<211> 15

<212> PRT
<213> Artificial sequence

<220>
<223> anti-CD19 antibody 4 LCDR1 amino acid sequence

<400> 36

Arg Ala Ser Glu Ser Val Asp Thr Phe Gly Ile Ser Phe Met Asn
1 5 10 15

<210> 37
<211> 7
<212> PRT
<213> Artificial sequence

<220>
<223> anti-CD19 antibody 4 LCDR2 amino acid sequence

<400> 37

Glu Ala Ser Asn Gln Gly Ser
1 5

<210> 38
<211> 9
<212> PRT
<213> Artificial sequence

<220>
<223> anti-CD19 antibody 4 LCDR3 amino acid sequence

<400> 38

Gln Gln Ser Lys Glu Val Pro Phe Thr
1 5

<210> 39
<211> 121
<212> PRT
<213> Artificial sequence

<220>
<223> anti-CD19 antibody 4 VH amino acid sequence

<400> 39

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Ser
20 25 30

Trp Met Asn Trp Val Arg Gln Ala Pro Gly Lys Gly Leu Glu Trp Val
35 40 45

Gly Arg Ile Tyr Pro Gly Asp Gly Asp Thr Asn Tyr Asn Val Lys Phe

50 55 60

Lys Gly Arg Phe Thr Ile Ser Arg Asp Asp Ser Lys Asn Ser Leu Tyr
65 70 75 80

Leu Gln Met Asn Ser Leu Lys Thr Glu Asp Thr Ala Val Tyr Tyr Cys
85 90 95

Ala Arg Ser Gly Phe Ile Thr Thr Val Arg Asp Phe Asp Tyr Trp Gly
100 105 110

Gln Gly Thr Leu Val Thr Val Ser Ser
115 120

<210> 40
<211> 111
<212> PRT
<213> Artificial sequence

<220>
<223> anti-CD19 antibody 4 VL amino acid sequence

<400> 40

Glu Ile Val Leu Thr Gln Ser Pro Asp Phe Gln Ser Val Thr Pro Lys
1 5 10 15

Glu Lys Val Thr Ile Thr Cys Arg Ala Ser Glu Ser Val Asp Thr Phe
20 25 30

Gly Ile Ser Phe Met Asn Trp Phe Gln Gln Lys Pro Asp Gln Ser Pro
35 40 45

Lys Leu Leu Ile His Glu Ala Ser Asn Gln Gly Ser Gly Val Pro Ser
50 55 60

Arg Phe Ser Gly Ser Gly Ser Gly Thr Asp Phe Thr Leu Thr Ile Asn
65 70 75 80

Ser Leu Glu Ala Glu Asp Ala Ala Thr Tyr Tyr Cys Gln Gln Ser Lys
85 90 95

Glu Val Pro Phe Thr Phe Gly Gly Gly Thr Lys Val Glu Ile Lys
100 105 110

<210> 41
<211> 5
<212> PRT
<213> Artificial sequence

<220>

<223> anti-CD20 antibody 1 HCDR1 amino acid sequence

<400> 41

```
Ser Tyr Asn Met His
1               5
```

<210> 42
<211> 17
<212> PRT
<213> Artificial sequence

<220>
<223> anti-CD20 antibody 1 HCDR2 amino acid sequence

<400> 42

```
Ala Ile Tyr Pro Gly Asn Gly Asp Thr Ser Tyr Asn Gln Lys Phe Lys
1               5                   10                  15

Gly
```

<210> 43
<211> 12
<212> PRT
<213> Artificial sequence

<220>
<223> anti-CD20 antibody 1 HCDR3 amino acid sequence

<400> 43

```
Ser Thr Tyr Tyr Gly Gly Asp Trp Tyr Phe Asn Val
1               5                   10
```

<210> 44
<211> 10
<212> PRT
<213> Artificial sequence

<220>
<223> anti-CD20 antibody 1 LCDR1 amino acid sequence

<400> 44

```
Arg Ala Ser Ser Ser Val Ser Tyr Ile His
1               5                   10
```

<210> 45
<211> 7
<212> PRT
<213> Artificial sequence

<220>
<223> anti-CD20 antibody 1 LCDR2 amino acid sequence

<400> 45

Ala Thr Ser Asn Leu Ala Ser
1               5

<210>  46
<211>  9
<212>  PRT
<213>  Artificial sequence

<220>
<223>  anti-CD20 antibody 1 LCDR3 amino acid sequence

<400>  46

Gln Gln Trp Thr Ser Asn Pro Pro Thr
1               5

<210>  47
<211>  121
<212>  PRT
<213>  Artificial sequence

<220>
<223>  anti-CD20 antibody 1 VH amino acid sequence

<400>  47

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ala
        115                 120

<210>  48
<211>  106

<212> PRT
<213> Artificial sequence

<220>
<223> anti-CD20 antibody 1 VL amino acid sequence

<400> 48

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Ile
20 25 30

His Trp Phe Gln Gln Lys Pro Gly Ser Ser Pro Lys Pro Trp Ile Tyr
35 40 45

Ala Thr Ser Asn Leu Ala Ser Gly Val Pro Val Arg Phe Ser Gly Ser
50 55 60

Gly Ser Gly Thr Ser Tyr Ser Leu Thr Ile Ser Arg Val Glu Ala Glu
65 70 75 80

Asp Ala Ala Thr Tyr Tyr Cys Gln Gln Trp Thr Ser Asn Pro Pro Thr
85 90 95

Phe Gly Gly Gly Thr Lys Leu Glu Ile Lys
100 105

<210> 49
<211> 5
<212> PRT
<213> Artificial sequence

<220>
<223> anti-CD20 antibody 2 HCDR1 amino acid sequence

<400> 49

Asn Tyr Tyr Ile His
1 5

<210> 50
<211> 17
<212> PRT
<213> Artificial sequence

<220>
<223> anti-CD20 antibody 2 HCDR2 amino acid sequence

<400> 50

Trp Ile Tyr Pro Gly Asp Gly Asn Thr Lys Tyr Asn Glu Lys Phe Lys
1 5 10 15

Gly

<210> 51
<211> 10
<212> PRT
<213> Artificial sequence

<220>
<223> anti-CD20 antibody 2 HCDR3 amino acid sequence

<400> 51

Asp Ser Tyr Ser Asn Tyr Tyr Phe Asp Tyr
1 5 10

<210> 52
<211> 10
<212> PRT
<213> Artificial sequence

<220>
<223> anti-CD20 antibody 2 LCDR1 amino acid sequence

<400> 52

Arg Ala Ser Ser Ser Val Ser Tyr Met His
1 5 10

<210> 53
<211> 7
<212> PRT
<213> Artificial sequence

<220>
<223> anti-CD20 antibody 2 LCDR2 amino acid sequence

<400> 53

Ala Pro Ser Asn Leu Ala Ser
1 5

<210> 54
<211> 9
<212> PRT
<213> Artificial sequence

<220>
<223> anti-CD20 antibody 2 LCDR3 amino acid sequence

<400> 54

Gln Gln Trp Ser Phe Asn Pro Pro Thr
1 5

<210> 55
<211> 119

<212> PRT
<213> Artificial sequence

<220>
<223> anti-CD20 antibody 2 VH amino acid sequence

<400> 55

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Asn Thr Lys Tyr Asn Glu Lys Phe
    50                  55                  60

Lys Gly Arg Ala Thr Leu Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Ser Tyr Ser Asn Tyr Tyr Phe Asp Tyr Trp Gly Gln Gly
            100                 105                 110

Thr Leu Val Thr Val Ser Ser
        115

<210> 56
<211> 106
<212> PRT
<213> Artificial sequence

<220>
<223> anti-CD20 antibody 2 VL amino acid sequence

<400> 56

Asp Ile Gln Met Thr Gln Ser Pro Ser Ser Leu Ser Ala Ser Val Gly
1               5                   10                  15

Asp Arg Val Thr Ile Thr Cys Arg Ala Ser Ser Ser Val Ser Tyr Met
            20                  25                  30

His Trp Tyr Gln Gln Lys Pro Gly Lys Ala Pro Lys Pro Leu Ile Tyr
        35                  40                  45

Ala Pro Ser Asn Leu Ala Ser Gly Val Pro Ser Arg Phe Ser Gly Ser

50 55 60

Gly Ser Gly Thr Asp Phe Thr Leu Thr Ile Ser Ser Leu Gln Pro Glu
65 70 75 80

Asp Phe Ala Thr Tyr Tyr Cys Gln Gln Trp Ser Phe Asn Pro Pro Thr
85 90 95

Phe Gly Gln Gly Thr Lys Val Glu Ile Lys
100 105

<210> 57
<211> 5
<212> PRT
<213> Artificial sequence

<220>
<223> anti-CD20 antibody 3 HCDR1 amino acid sequence

<400> 57

Tyr Ser Trp Ile Asn
1 5

<210> 58
<211> 17
<212> PRT
<213> Artificial sequence

<220>
<223> anti-CD20 antibody 3 HCDR2 amino acid sequence

<400> 58

Arg Ile Phe Pro Gly Asp Gly Asp Thr Asp Tyr Asn Gly Lys Phe Lys
1 5 10 15

Gly

<210> 59
<211> 10
<212> PRT
<213> Artificial sequence

<220>
<223> anti-CD20 antibody 3 HCDR3 amino acid sequence

<400> 59

Asn Val Phe Asp Gly Tyr Trp Leu Val Tyr
1 5 10

<210> 60
<211> 16

<212> PRT
<213> Artificial sequence

<220>
<223> anti-CD20 antibody 3 LCDR1 amino acid sequence

<400> 60

Arg Ser Ser Lys Ser Leu Leu His Ser Asn Gly Ile Thr Tyr Leu Tyr
1 5 10 15

<210> 61
<211> 7
<212> PRT
<213> Artificial sequence

<220>
<223> anti-CD20 antibody 3 LCDR2 amino acid sequence

<400> 61

Gln Met Ser Asn Leu Val Ser
1 5

<210> 62
<211> 9
<212> PRT
<213> Artificial sequence

<220>
<223> anti-CD20 antibody 3 LCDR3 amino acid sequence

<400> 62

Ala Gln Asn Leu Glu Leu Pro Tyr Thr
1 5

<210> 63
<211> 119
<212> PRT
<213> Artificial sequence

<220>
<223> anti-CD20 antibody 3 VH amino acid sequence

<400> 63

Gln Val Gln Leu Val Gln Ser Gly Ala Glu Val Lys Lys Pro Gly Ser
1 5 10 15

Ser Val Lys Val Ser Cys Lys Ala Ser Gly Tyr Ala Phe Ser Tyr Ser
20 25 30

Trp Ile Asn Trp Val Arg Gln Ala Pro Gly Gln Gly Leu Glu Trp Met
35 40 45

Gly Arg Ile Phe Pro Gly Asp Gly Asp Thr Asp Tyr Asn Gly Lys Phe

50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asn Val Phe Asp Gly Tyr Trp Leu Val Tyr Trp Gly Gln Gly
100 105 110

Thr Leu Val Thr Val Ser Ser
115

<210> 64
<211> 112
<212> PRT
<213> Artificial sequence

<220>
<223> anti-CD20 antibody 3 VL amino acid sequence

<400> 64

Asp Ile Val Met Thr Gln Thr Pro Leu Ser Leu Pro Val Thr Pro Gly
1 5 10 15

Glu Pro Ala Ser Ile Ser Cys Arg Ser Ser Lys Ser Leu Leu His Ser
20 25 30

Asn Gly Ile Thr Tyr Leu Tyr Trp Tyr Leu Gln Lys Pro Gly Gln Ser
35 40 45

Pro Gln Leu Leu Ile Tyr Gln Met Ser Asn Leu Val Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ala Gln Asn
85 90 95

Leu Glu Leu Pro Tyr Thr Phe Gly Gly Gly Thr Lys Val Glu Ile Lys
100 105 110

<210> 65
<211> 5
<212> PRT
<213> Artificial sequence

<220>

<223> anti-CD20 antibody 4 HCDR1 amino acid sequence

<400> 65

Asp Tyr Ala Met His
1 5

<210> 66
<211> 17
<212> PRT
<213> Artificial sequence

<220>
<223> anti-CD20 antibody 4 HCDR2 amino acid sequence

<400> 66

Thr Ile Ser Trp Asn Ser Gly Ser Ile Gly Tyr Ala Asp Ser Val Lys
1 5 10 15

Gly

<210> 67
<211> 13
<212> PRT
<213> Artificial sequence

<220>
<223> anti-CD20 antibody 4 HCDR3 amino acid sequence

<400> 67

Asp Ile Gln Tyr Gly Asn Tyr Tyr Tyr Gly Met Asp Val
1 5 10

<210> 68
<211> 11
<212> PRT
<213> Artificial sequence

<220>
<223> anti-CD20 antibody 4 LCDR1 amino acid sequence

<400> 68

Arg Ala Ser Gln Ser Val Ser Ser Tyr Leu Ala
1 5 10

<210> 69
<211> 7
<212> PRT
<213> Artificial sequence

<220>
<223> anti-CD20 antibody 4 LCDR2 amino acid sequence

<400> 69

Asp Ala Ser Asn Arg Ala Thr
1               5

<210>  70
<211>  9
<212>  PRT
<213>  Artificial sequence

<220>
<223>  anti-CD20 antibody 4 LCDR3 amino acid sequence

<400>  70

Gln Gln Arg Ser Asn Trp Pro Ile Thr
1               5

<210>  71
<211>  122
<212>  PRT
<213>  Artificial sequence

<220>
<223>  anti-CD20 antibody 4 VH amino acid sequence

<400>  71

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser
        115                 120

<210>  72
<211>  107

<212> PRT
<213> Artificial sequence

<220>
<223> anti-CD20 antibody 4 VL amino acid sequence

<400> 72

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Ile
85 90 95

Thr Phe Gly Gln Gly Thr Arg Leu Glu Ile Lys
100 105

<210> 73
<211> 5
<212> PRT
<213> Artificial sequence

<220>
<223> anti-CD22 antibody 1 HCDR1 amino acid sequence

<400> 73

Arg Ser Trp Met Asn
1 5

<210> 74
<211> 17
<212> PRT
<213> Artificial sequence

<220>
<223> anti-CD22 antibody 1 HCDR2 amino acid sequence

<400> 74

Arg Ile Tyr Pro Gly Asp Gly Asp Thr Asn Tyr Ser Gly Lys Phe Lys
1 5 10 15

Gly

<210> 75
<211> 11
<212> PRT
<213> Artificial sequence

<220>
<223> anti-CD22 antibody 1 HCDR3 amino acid sequence

<400> 75

Asp Gly Ser Ser Trp Asp Trp Tyr Phe Asp Val
1 5 10

<210> 76
<211> 16
<212> PRT
<213> Artificial sequence

<220>
<223> anti-CD22 antibody 1 LCDR1 amino acid sequence

<400> 76

Arg Ser Ser Gln Ser Ile Val His Ser Val Gly Asn Thr Phe Leu Glu
1 5 10 15

<210> 77
<211> 7
<212> PRT
<213> Artificial sequence

<220>
<223> anti-CD22 antibody 1 LCDR2 amino acid sequence

<400> 77

Lys Val Ser Asn Arg Phe Ser
1 5

<210> 78
<211> 9
<212> PRT
<213> Artificial sequence

<220>
<223> anti-CD22 antibody 1 LCDR3 amino acid sequence

<400> 78

Phe Gln Gly Ser Gln Phe Pro Tyr Thr
1 5

<210> 79
<211> 120

<212> PRT
<213> Artificial sequence

<220>
<223> anti-CD22 antibody 1 VH amino acid sequence

<400> 79

Glu Val Gln Leu Val Glu Ser Gly Gly Gly Leu Val Gln Pro Gly Gly
1 5 10 15

Ser Leu Arg Leu Ser Cys Ala Ala Ser Gly Tyr Glu Phe Ser Arg Ser
20 25 30

Trp Met Asn Trp Val Arg Gln Ala Pro Gly Lys Gly Leu Glu Trp Val
35 40 45

Gly Arg Ile Tyr Pro Gly Asp Gly Asp Thr Asn Tyr Ser Gly Lys Phe
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ala Arg Asp Gly Ser Ser Trp Asp Trp Tyr Phe Asp Val Trp Gly Gln
100 105 110

Gly Thr Leu Val Thr Val Ser Ser
115 120

<210> 80
<211> 112
<212> PRT
<213> Artificial sequence

<220>
<223> anti-CD22 antibody 1 VL amino acid sequence

<400> 80

Asp Ile Gln Met Thr Gln Ser Pro Ser Ser Leu Ser Ala Ser Val Gly
1 5 10 15

Asp Arg Val Thr Ile Thr Cys Arg Ser Ser Gln Ser Ile Val His Ser
20 25 30

Val Gly Asn Thr Phe Leu Glu Trp Tyr Gln Gln Lys Pro Gly Lys Ala
35 40 45

Pro Lys Leu Leu Ile Tyr Lys Val Ser Asn Arg Phe Ser Gly Val Pro

50 55 60

Ser Arg Phe Ser Gly Ser Gly Ser Gly Thr Asp Phe Thr Leu Thr Ile
65 70 75 80

Ser Ser Leu Gln Pro Glu Asp Phe Ala Thr Tyr Tyr Cys Phe Gln Gly
85 90 95

Ser Gln Phe Pro Tyr Thr Phe Gly Gln Gly Thr Lys Val Glu Ile Lys
100 105 110

<210> 81
<211> 5
<212> PRT
<213> Artificial sequence

<220>
<223> anti-CD22 antibody 2 HCDR1 amino acid sequence

<400> 81

Ile Tyr Asp Met Ser
1 5

<210> 82
<211> 17
<212> PRT
<213> Artificial sequence

<220>
<223> anti-CD22 antibody 2 HCDR2 amino acid sequence

<400> 82

Tyr Ile Ser Ser Gly Gly Gly Thr Thr Tyr Tyr Pro Asp Thr Val Lys
1 5 10 15

Gly

<210> 83
<211> 14
<212> PRT
<213> Artificial sequence

<220>
<223> anti-CD22 antibody 2 HCDR3 amino acid sequence

<400> 83

His Ser Gly Tyr Gly Thr His Trp Gly Val Leu Phe Ala Tyr
1 5 10

<210> 84
<211> 11

<212> PRT
<213> Artificial sequence

<220>
<223> anti-CD22 antibody 2 LCDR1 amino acid sequence

<400> 84

Arg Ala Ser Gln Asp Ile Ser Asn Tyr Leu Asn
1               5                   10

<210> 85
<211> 7
<212> PRT
<213> Artificial sequence

<220>
<223> anti-CD22 antibody 2 LCDR2 amino acid sequence

<400> 85

Tyr Thr Ser Ile Leu His Ser
1               5

<210> 86
<211> 9
<212> PRT
<213> Artificial sequence

<220>
<223> anti-CD22 antibody 2 LCDR3 amino acid sequence

<400> 86

Gln Gln Gly Asn Thr Leu Pro Trp Thr
1               5

<210> 87
<211> 123
<212> PRT
<213> Artificial sequence

<220>
<223> anti-CD22 antibody 2 VH amino acid sequence

<400> 87

Glu Val Gln Leu Val Glu Ser Ala Ser Thr Gly Gly Gly Leu Val Lys
1               5                   10                  15

Pro Gly Gly Ser Leu Lys Leu Ser Cys Ala Ala Ser Gly Phe Ala Phe
            20                  25                  30

Ser Ile Tyr Asp Met Ser Trp Val Arg Gln Thr Pro Glu Lys Cys Leu
        35                  40                  45

Glu Trp Val Ala Tyr Ile Ser Ser Gly Gly Gly Thr Thr Tyr Tyr Pro

50 55 60

Asp Thr Val Lys Gly Arg Phe Thr Ile Ser Arg Asp Asn Ala Lys Asn
65 70 75 80

Thr Leu Tyr Leu Gln Met Ser Ser Leu Lys Ser Glu Asp Thr Ala Met
85 90 95

Tyr Tyr Cys Ala Arg His Ser Gly Tyr Gly Thr His Trp Gly Val Leu
100 105 110

Phe Ala Tyr Trp Gly Gln Gly Thr Leu Val Thr
115 120

<210> 88
<211> 107
<212> PRT
<213> Artificial sequence

<220>
<223> anti-CD22 antibody 2 VL amino acid sequence

<400> 88

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Ser Asn Tyr
20 25 30

Leu Asn Trp Tyr Gln Gln Lys Pro Asp Gly Thr Val Lys Leu Leu Ile
35 40 45

Tyr Tyr Thr Ser Ile Leu His Ser Gly Val Pro Ser Arg Phe Ser Gly
50 55 60

Ser Gly Ser Gly Thr Asp Tyr Ser Leu Thr Ile Ser Asn Leu Glu Gln
65 70 75 80

Glu Asp Phe Ala Thr Tyr Phe Cys Gln Gln Gly Asn Thr Leu Pro Trp
85 90 95

Thr Phe Gly Cys Gly Thr Lys Leu Glu Ile Lys
100 105

<210> 89
<211> 5
<212> PRT
<213> Artificial sequence

<220>

<223>  anti-CD30 antibody 1 HCDR1 amino acid sequence

<400>  89

Asp Tyr Tyr Ile Thr
1               5

<210>  90
<211>  17
<212>  PRT
<213>  Artificial sequence

<220>
<223>  anti-CD30 antibody 1 HCDR2 amino acid sequence

<400>  90

Trp Ile Tyr Pro Gly Ser Gly Asn Thr Lys Tyr Asn Glu Lys Phe Lys
1               5                   10                  15

Gly

<210>  91
<211>  8
<212>  PRT
<213>  Artificial sequence

<220>
<223>  anti-CD30 antibody 1 HCDR3 amino acid sequence

<400>  91

Tyr Gly Asn Tyr Trp Phe Ala Tyr
1               5

<210>  92
<211>  15
<212>  PRT
<213>  Artificial sequence

<220>
<223>  anti-CD30 antibody 1 LCDR1 amino acid sequence

<400>  92

Lys Ala Ser Gln Ser Val Asp Phe Asp Gly Asp Ser Tyr Met Asn
1               5                   10                  15

<210>  93
<211>  7
<212>  PRT
<213>  Artificial sequence

<220>
<223>  anti-CD30 antibody 1 LCDR2 amino acid sequence

<400>  93

Ala Ala Ser Asn Leu Glu Ser
1               5

<210> 94
<211> 9
<212> PRT
<213> Artificial sequence

<220>
<223> anti-CD30 antibody 1 LCDR3 amino acid sequence

<400> 94

Gln Gln Ser Asn Glu Asp Pro Trp Thr
1               5

<210> 95
<211> 117
<212> PRT
<213> Artificial sequence

<220>
<223> anti-CD30 antibody 1 VH amino acid sequence

<400> 95

Gln Ile Gln Leu Gln Gln Ser Gly Pro Glu Val Val Lys Pro Gly Ala
1               5                   10                  15

Ser Val Lys Ile Ser Cys Lys Ala Ser Gly Tyr Thr Phe Thr Asp Tyr
            20                  25                  30

Tyr Ile Thr Trp Val Lys Gln Lys Pro Gly Gln Gly Leu Glu Trp Ile
        35                  40                  45

Gly Trp Ile Tyr Pro Gly Ser Gly Asn Thr Lys Tyr Asn Glu Lys Phe
    50                  55                  60

Lys Gly Lys Ala Thr Leu Thr Val Asp Thr Ser Ser Ser Thr Ala Phe
65                  70                  75                  80

Met Gln Leu Ser Ser Leu Thr Ser Glu Asp Thr Ala Val Tyr Phe Cys
                85                  90                  95

Ala Asn Tyr Gly Asn Tyr Trp Phe Ala Tyr Trp Gly Gln Gly Thr Gln
            100                 105                 110

Val Thr Val Ser Ala
        115

<210> 96
<211> 111

<212> PRT
<213> Artificial sequence

<220>
<223> anti-CD30 antibody 1 VL amino acid sequence

<400> 96

Asp Ile Val Leu Thr Gln Ser Pro Ala Ser Leu Ala Val Ser Leu Gly
1 5 10 15

Gln Arg Ala Thr Ile Ser Cys Lys Ala Ser Gln Ser Val Asp Phe Asp
20 25 30

Gly Asp Ser Tyr Met Asn Trp Tyr Gln Gln Lys Pro Gly Gln Pro Pro
35 40 45

Lys Val Leu Ile Tyr Ala Ala Ser Asn Leu Glu Ser Gly Ile Pro Ala
50 55 60

Arg Phe Ser Gly Ser Gly Ser Gly Thr Asp Phe Thr Leu Asn Ile His
65 70 75 80

Pro Val Glu Glu Glu Asp Ala Ala Thr Tyr Tyr Cys Gln Gln Ser Asn
85 90 95

Glu Asp Pro Trp Thr Phe Gly Gly Gly Thr Lys Leu Glu Ile Lys
100 105 110

<210> 97
<211> 5
<212> PRT
<213> Artificial sequence

<220>
<223> anti-CD30 antibody 2 HCDR1 amino acid sequence

<400> 97

Ala Tyr Tyr Trp Ser
1 5

<210> 98
<211> 16
<212> PRT
<213> Artificial sequence

<220>
<223> anti-CD30 antibody 2 HCDR2 amino acid sequence

<400> 98

Asp Ile Asn His Gly Gly Gly Thr Asn Tyr Asn Pro Ser Leu Lys Ser
1 5 10 15

<210> 99
<211> 4
<212> PRT
<213> Artificial sequence

<220>
<223> anti-CD30 antibody 2 HCDR3 amino acid sequence

<400> 99

Leu Thr Ala Tyr
1

<210> 100
<211> 11
<212> PRT
<213> Artificial sequence

<220>
<223> anti-CD30 antibody 2 LCDR1 amino acid sequence

<400> 100

Arg Ala Ser Gln Gly Ile Ser Ser Trp Leu Thr
1               5                   10

<210> 101
<211> 7
<212> PRT
<213> Artificial sequence

<220>
<223> anti-CD30 antibody 2 LCDR2 amino acid sequence

<400> 101

Ala Ala Ser Ser Leu Gln Ser
1               5

<210> 102
<211> 9
<212> PRT
<213> Artificial sequence

<220>
<223> anti-CD30 antibody 2 LCDR3 amino acid sequence

<400> 102

Gln Gln Tyr Asp Ser Tyr Pro Ile Thr
1               5

<210> 103
<211> 112
<212> PRT
<213> Artificial sequence

<220>

<223>  anti-CD30 antibody 2 VH amino acid sequence

<400>  103

```
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Ala Tyr
            20                  25                  30

Tyr Trp Ser Trp Ile Arg Gln Pro Pro Gly Lys Gly Leu Glu Trp Ile
        35                  40                  45

Gly Asp Ile Asn His Gly Gly Gly Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Asn Ser Val Thr Ala Ala Asp Thr Ala Val Tyr Tyr Cys Ala
                85                  90                  95

Ser Leu Thr Ala Tyr Trp Gly Gln Gly Ser Leu Val Thr Val Ser Ser
            100                 105                 110
```

<210>  104
<211>  107
<212>  PRT
<213>  Artificial sequence

<220>
<223>  anti-CD30 antibody 2 VL amino acid sequence

<400>  104

```
Asp Ile Gln Met Thr Gln Ser Pro Thr Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Thr Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asp Ser Tyr Pro Ile
```

85 90 95

Thr Phe Gly Gln Gly Thr Arg Leu Glu Ile Lys
100 105

<210> 105
<211> 5
<212> PRT
<213> Artificial sequence

<220>
<223> anti-EpCAM antibody 1 HCDR1 amino acid sequence

<400> 105

Ser Tyr Gly Met His
1 5

<210> 106
<211> 17
<212> PRT
<213> Artificial sequence

<220>
<223> anti-EpCAM antibody 1 HCDR2 amino acid sequence

<400> 106

Val Ile Ser Tyr Asp Gly Ser Asn Lys Tyr Tyr Ala Asp Ser Val Lys
1 5 10 15

Gly

<210> 107
<211> 18
<212> PRT
<213> Artificial sequence

<220>
<223> anti-EpCAM antibody 1 HCDR3 amino acid sequence

<400> 107

Asp Met Gly Trp Gly Ser Gly Trp Arg Pro Tyr Tyr Tyr Tyr Gly Met
1 5 10 15

Asp Val

<210> 108
<211> 11
<212> PRT
<213> Artificial sequence

<220>

<223> anti-EpCAM antibody 1 LCDR1 amino acid sequence

<400> 108

Arg Thr Ser Gln Ser Ile Ser Ser Tyr Leu Asn
1               5                   10

<210> 109
<211> 7
<212> PRT
<213> Artificial sequence

<220>
<223> anti-EpCAM antibody 1 LCDR2 amino acid sequence

<400> 109

Trp Ala Ser Thr Arg Glu Ser
1               5

<210> 110
<211> 9
<212> PRT
<213> Artificial sequence

<220>
<223> anti-EpCAM antibody 1 LCDR3 amino acid sequence

<400> 110

Gln Gln Ser Tyr Asp Ile Pro Tyr Thr
1               5

<210> 111
<211> 127
<212> PRT
<213> Artificial sequence

<220>
<223> anti-EpCAM antibody 1 VH amino acid sequence

<400> 111

Glu Val Gln Leu Leu Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr

65 70 75 80

Leu Gln Met Asn Ser Leu Arg Ala Glu Asp Thr Ala Val Tyr Tyr Cys
85 90 95

Ala Lys Asp Met Gly Trp Gly Ser Gly Trp Arg Pro Tyr Tyr Tyr Tyr
100 105 110

Gly Met Asp Val Trp Gly Gln Gly Thr Thr Val Thr Val Ser Ser
115 120 125

<210> 112
<211> 107
<212> PRT
<213> Artificial sequence

<220>
<223> anti-EpCAM antibody 1 VL amino acid sequence

<400> 112

Glu Leu Gln Met Thr Gln Ser Pro Ser Ser Leu Ser Ala Ser Val Gly
1 5 10 15

Asp Arg Val Thr Ile Thr Cys Arg Thr Ser Gln Ser Ile Ser Ser Tyr
20 25 30

Leu Asn Trp Tyr Gln Gln Lys Pro Gly Gln Pro Pro Lys Leu Leu Ile
35 40 45

Tyr Trp Ala Ser Thr Arg Glu Ser Gly Val Pro Asp Arg Phe Ser Gly
50 55 60

Ser Gly Ser Gly Thr Asp Phe Thr Leu Thr Ile Ser Ser Leu Gln Pro
65 70 75 80

Glu Asp Ser Ala Thr Tyr Tyr Cys Gln Gln Ser Tyr Asp Ile Pro Tyr
85 90 95

Thr Phe Gly Gln Gly Thr Lys Leu Glu Ile Lys
100 105

<210> 113
<211> 5
<212> PRT
<213> Artificial sequence

<220>
<223> anti-EpCAM antibody 2 HCDR1 amino acid sequence

<400> 113

Asn Tyr Gly Met Asn
1               5

<210>  114
<211>  17
<212>  PRT
<213>  Artificial sequence

<220>
<223>  anti-EpCAM antibody 2 HCDR2 amino acid sequence

<400>  114

Trp Ile Asn Thr Tyr Thr Gly Glu Ser Thr Tyr Ala Asp Ser Phe Lys
1               5                   10                  15

Gly

<210>  115
<211>  7
<212>  PRT
<213>  Artificial sequence

<220>
<223>  anti-EpCAM antibody 2 HCDR3 amino acid sequence

<400>  115

Phe Ala Ile Lys Gly Asp Tyr
1               5

<210>  116
<211>  16
<212>  PRT
<213>  Artificial sequence

<220>
<223>  anti-EpCAM antibody 2 LCDR1 amino acid sequence

<400>  116

Arg Ser Thr Lys Ser Leu Leu His Ser Asn Gly Ile Thr Tyr Leu Tyr
1               5                   10                  15

<210>  117
<211>  7
<212>  PRT
<213>  Artificial sequence

<220>
<223>  anti-EpCAM antibody 2 LCDR2 amino acid sequence

<400>  117

Gln Met Ser Asn Leu Ala Ser
1               5

<210> 118
<211> 9
<212> PRT
<213> Artificial sequence

<220>
<223> anti-EpCAM antibody 2 LCDR3 amino acid sequence

<400> 118

Ala Gln Asn Leu Glu Ile Pro Arg Thr
1               5

<210> 119
<211> 116
<212> PRT
<213> Artificial sequence

<220>
<223> anti-EpCAM antibody 2 VH amino acid sequence

<400> 119

Glu Val Gln Leu Val Gln Ser Gly Pro Gly Leu Val Gln Pro Gly Gly
1               5                   10                  15

Ser Val Arg Ile Ser Cys Ala Ala Ser Gly Tyr Thr Phe Thr Asn Tyr
            20                  25                  30

Gly Met Asn Trp Val Lys Gln Ala Pro Gly Lys Gly Leu Glu Trp Met
        35                  40                  45

Gly Trp Ile Asn Thr Tyr Thr Gly Glu Ser Thr Tyr Ala Asp Ser Phe
    50                  55                  60

Lys Gly Arg Phe Thr Phe Ser Leu Asp Thr Ser Ala Ser Ala Ala Tyr
65                  70                  75                  80

Leu Gln Ile Asn Ser Leu Arg Ala Glu Asp Thr Ala Val Tyr Tyr Cys
                85                  90                  95

Ala Arg Phe Ala Ile Lys Gly Asp Tyr Trp Gly Gln Gly Thr Leu Leu
            100                 105                 110

Thr Val Ser Ser
        115

<210> 120
<211> 112
<212> PRT
<213> Artificial sequence

<220>

<223> anti-EpCAM antibody 2 VL amino acid sequence

<400> 120

Asp Ile Gln Met Thr Gln Ser Pro Ser Ser Leu Ser Ala Ser Val Gly
1 5 10 15

Asp Arg Val Thr Ile Thr Cys Arg Ser Thr Lys Ser Leu Leu His Ser
20 25 30

Asn Gly Ile Thr Tyr Leu Tyr Trp Tyr Gln Gln Lys Pro Gly Lys Ala
35 40 45

Pro Lys Leu Leu Ile Tyr Gln Met Ser Asn Leu Ala Ser Gly Val Pro
50 55 60

Ser Arg Phe Ser Ser Ser Gly Ser Gly Thr Asp Phe Thr Leu Thr Ile
65 70 75 80

Ser Ser Leu Gln Pro Glu Asp Phe Ala Thr Tyr Tyr Cys Ala Gln Asn
85 90 95

Leu Glu Ile Pro Arg Thr Phe Gly Gln Gly Thr Lys Val Glu Leu Lys
100 105 110

<210> 121
<211> 5
<212> PRT
<213> Artificial sequence

<220>
<223> anti-CEA antibody 1 HCDR1 amino acid sequence

<400> 121

Asp Thr Tyr Met His
1 5

<210> 122
<211> 17
<212> PRT
<213> Artificial sequence

<220>
<223> anti-CEA antibody 1 HCDR2 amino acid sequence

<400> 122

Arg Ile Asp Pro Ala Asn Gly Asn Ser Lys Tyr Ala Asp Ser Val Lys
1 5 10 15

Gly

<210> 123
<211> 12
<212> PRT
<213> Artificial sequence

<220>
<223> anti-CEA antibody 1 HCDR3 amino acid sequence

<400> 123

Phe Gly Tyr Tyr Val Ser Asp Tyr Ala Met Ala Tyr
1 5 10

<210> 124
<211> 15
<212> PRT
<213> Artificial sequence

<220>
<223> anti-CEA antibody 1 LCDR1 amino acid sequence

<400> 124

Arg Ala Gly Glu Ser Val Asp Ile Phe Gly Val Gly Phe Leu His
1 5 10 15

<210> 125
<211> 7
<212> PRT
<213> Artificial sequence

<220>
<223> anti-CEA antibody 1 LCDR2 amino acid sequence

<400> 125

Arg Ala Ser Asn Leu Glu Ser
1 5

<210> 126
<211> 9
<212> PRT
<213> Artificial sequence

<220>
<223> anti-CEA antibody 1 LCDR3 amino acid sequence

<400> 126

Gln Gln Thr Asn Glu Asp Pro Tyr Thr
1 5

<210> 127
<211> 121
<212> PRT
<213> Artificial sequence

<220>

<223>  anti-CEA antibody 1 VH amino acid sequence

<400>  127

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Met His Trp Val Arg Gln Ala Pro Gly Lys Gly Leu Glu Trp Val
        35                  40                  45

Ala Arg Ile Asp Pro Ala Asn Gly Asn Ser Lys Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser
        115                 120

<210>  128
<211>  111
<212>  PRT
<213>  Artificial sequence

<220>
<223>  anti-CEA antibody 1 VL amino acid sequence

<400>  128

Asp Ile Gln Leu Thr Gln Ser Pro Ser Ser Leu Ser Ala Ser Val Gly
1               5                   10                  15

Asp Arg Val Thr Ile Thr Cys Arg Ala Gly Glu Ser Val Asp Ile Phe
            20                  25                  30

Gly Val Gly Phe Leu His Trp Tyr Gln Gln Lys Pro Gly Lys Ala Pro
        35                  40                  45

Lys Leu Leu Ile Tyr Arg Ala Ser Asn Leu Glu Ser Gly Val Pro Ser
    50                  55                  60

Arg Phe Ser Gly Ser Gly Ser Arg Thr Asp Phe Thr Leu Thr Ile Ser

65 70 75 80

Ser Leu Gln Pro Glu Asp Phe Ala Thr Tyr Tyr Cys Gln Gln Thr Asn
85 90 95

Glu Asp Pro Tyr Thr Phe Gly Gln Gly Thr Lys Val Glu Ile Lys
100 105 110

<210> 129
<211> 5
<212> PRT
<213> Artificial sequence

<220>
<223> anti-CEA antibody 2 HCDR1 amino acid sequence

<400> 129

Thr Tyr Trp Met Ser
1 5

<210> 130
<211> 17
<212> PRT
<213> Artificial sequence

<220>
<223> anti-CEA antibody 2 HCDR2 amino acid sequence

<400> 130

Glu Ile His Pro Asp Ser Ser Thr Ile Asn Tyr Ala Pro Ser Leu Lys
1 5 10 15

Asp

<210> 131
<211> 10
<212> PRT
<213> Artificial sequence

<220>
<223> anti-CEA antibody 2 HCDR3 amino acid sequence

<400> 131

Leu Tyr Phe Gly Phe Pro Trp Phe Ala Tyr
1 5 10

<210> 132
<211> 11
<212> PRT
<213> Artificial sequence

<220>

<223>  anti-CEA antibody 2 LCDR1 amino acid sequence

<400>  132

Lys Ala Ser Gln Asp Val Gly Thr Ser Val Ala
1               5                   10

<210>  133
<211>  7
<212>  PRT
<213>  Artificial sequence

<220>
<223>  anti-CEA antibody 2 LCDR2 amino acid sequence

<400>  133

Trp Thr Ser Thr Arg His Thr
1               5

<210>  134
<211>  8
<212>  PRT
<213>  Artificial sequence

<220>
<223>  anti-CEA antibody 2 LCDR3 amino acid sequence

<400>  134

Gln Gln Tyr Ser Leu Tyr Arg Ser
1               5

<210>  135
<211>  119
<212>  PRT
<213>  Artificial sequence

<220>
<223>  anti-CEA antibody 2 VH amino acid sequence

<400>  135

Glu Val Gln Leu Val Glu Ser Gly Gly Gly Val Val Gln Pro Gly Arg
1               5                   10                  15

Ser Leu Arg Leu Ser Cys Ser Ala Ser Gly Phe Asp Phe Thr Thr Tyr
            20                  25                  30

Trp Met Ser Trp Val Arg Gln Ala Pro Gly Lys Gly Leu Glu Trp Ile
        35                  40                  45

Gly Glu Ile His Pro Asp Ser Ser Thr Ile Asn Tyr Ala Pro Ser Leu
    50                  55                  60

Lys Asp Arg Phe Thr Ile Ser Arg Asp Asn Ala Lys Asn Thr Leu Phe

65 70 75 80

Leu Gln Met Asp Ser Leu Arg Pro Glu Asp Thr Gly Val Tyr Phe Cys
85 90 95

Ala Ser Leu Tyr Phe Gly Phe Pro Trp Phe Ala Tyr Trp Gly Gln Gly
100 105 110

Thr Pro Val Thr Val Ser Ser
115

<210> 136
<211> 106
<212> PRT
<213> Artificial sequence

<220>
<223> anti-CEA antibody 2 VL amino acid sequence

<400> 136

Asp Ile Gln Leu Thr Gln Ser Pro Ser Ser Leu Ser Ala Ser Val Gly
1 5 10 15

Asp Arg Val Thr Ile Thr Cys Lys Ala Ser Gln Asp Val Gly Thr Ser
20 25 30

Val Ala Trp Tyr Gln Gln Lys Pro Gly Lys Ala Pro Lys Leu Leu Ile
35 40 45

Tyr Trp Thr Ser Thr Arg His Thr Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Tyr Ser Leu Tyr Arg Ser
85 90 95

Phe Gly Gln Gly Thr Lys Val Glu Ile Lys
100 105

<210> 137
<211> 5
<212> PRT
<213> Artificial sequence

<220>
<223> anti-CEA antibody 3 HCDR1 amino acid sequence

<400> 137

Glu Phe Gly Met Asn
1               5

<210>  138
<211>  17
<212>  PRT
<213>  Artificial sequence

<220>
<223>  anti-CEA antibody 3 HCDR2 amino acid sequence

<400>  138

Trp Ile Asn Thr Lys Thr Gly Glu Ala Thr Tyr Val Glu Glu Phe Lys
1               5                   10                  15

Gly

<210>  139
<211>  12
<212>  PRT
<213>  Artificial sequence

<220>
<223>  anti-CEA antibody 3 HCDR3 amino acid sequence

<400>  139

Trp Asp Phe Ala Tyr Tyr Val Glu Ala Met Asp Tyr
1               5                   10

<210>  140
<211>  11
<212>  PRT
<213>  Artificial sequence

<220>
<223>  anti-CEA antibody 3 LCDR1 amino acid sequence

<400>  140

Lys Ala Ser Ala Ala Val Gly Thr Tyr Val Ala
1               5                   10

<210>  141
<211>  7
<212>  PRT
<213>  Artificial sequence

<220>
<223>  anti-CEA antibody 3 LCDR2 amino acid sequence

<400>  141

Ser Ala Ser Tyr Arg Lys Arg
1               5

<210> 142
<211> 10
<212> PRT
<213> Artificial sequence

<220>
<223> anti-CEA antibody 3 LCDR3 amino acid sequence

<400> 142

His Gln Tyr Tyr Thr Tyr Pro Leu Phe Thr
1 5 10

<210> 143
<211> 121
<212> PRT
<213> Artificial sequence

<220>
<223> anti-CEA antibody 3 VH amino acid sequence

<400> 143

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Glu Phe
20 25 30

Gly Met Asn Trp Val Arg Gln Ala Pro Gly Gln Gly Leu Glu Trp Met
35 40 45

Gly Trp Ile Asn Thr Lys Thr Gly Glu Ala Thr Tyr Val Glu Glu Phe
50 55 60

Lys Gly Arg Val Thr Phe Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Trp Asp Phe Ala Tyr Tyr Val Glu Ala Met Asp Tyr Trp Gly
100 105 110

Gln Gly Thr Thr Val Thr Val Ser Ser
115 120

<210> 144
<211> 108
<212> PRT
<213> Artificial sequence

<220>

<223> anti-CEA antibody 3 VL amino acid sequence

<400> 144

Asp Ile Gln Met Thr Gln Ser Pro Ser Ser Leu Ser Ala Ser Val Gly
1               5                   10                  15

Asp Arg Val Thr Ile Thr Cys Lys Ala Ser Ala Ala Val Gly Thr Tyr
            20                  25                  30

Val Ala Trp Tyr Gln Gln Lys Pro Gly Lys Ala Pro Lys Leu Leu Ile
        35                  40                  45

Tyr Ser Ala Ser Tyr Arg Lys Arg Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys His Gln Tyr Tyr Thr Tyr Pro Leu
                85                  90                  95

Phe Thr Phe Gly Gln Gly Thr Lys Leu Glu Ile Lys
            100                 105

<210> 145
<211> 5
<212> PRT
<213> Artificial sequence

<220>
<223> anti-Her2 antibody 1 HCDR1 amino acid sequence

<400> 145

Asp Thr Tyr Ile His
1               5

<210> 146
<211> 17
<212> PRT
<213> Artificial sequence

<220>
<223> anti-Her2 antibody 1 HCDR2 amino acid sequence

<400> 146

Arg Ile Tyr Pro Thr Asn Gly Tyr Thr Arg Tyr Ala Asp Ser Val Lys
1               5                   10                  15

Gly

<210> 147
<211> 11
<212> PRT
<213> Artificial sequence

<220>
<223> anti-Her2 antibody 1 HCDR3 amino acid sequence

<400> 147

Trp Gly Gly Asp Gly Phe Tyr Ala Met Asp Tyr
1 5 10

<210> 148
<211> 11
<212> PRT
<213> Artificial sequence

<220>
<223> anti-Her2 antibody 1 LCDR1 amino acid sequence

<400> 148

Arg Ala Ser Gln Asp Val Asn Thr Ala Val Ala
1 5 10

<210> 149
<211> 7
<212> PRT
<213> Artificial sequence

<220>
<223> anti-Her2 antibody 1 LCDR2 amino acid sequence

<400> 149

Ser Ala Ser Phe Leu Tyr Ser
1 5

<210> 150
<211> 9
<212> PRT
<213> Artificial sequence

<220>
<223> anti-Her2 antibody 1 LCDR3 amino acid sequence

<400> 150

Gln Gln His Tyr Thr Thr Pro Pro Thr
1 5

<210> 151
<211> 120
<212> PRT
<213> Artificial sequence

<220>

<223>  anti-Her2 antibody 1 VH amino acid sequence

<400>  151

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120

<210>  152
<211>  107
<212>  PRT
<213>  Artificial sequence

<220>
<223>  anti-Her2 antibody 1 VL amino acid sequence

<400>  152

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro

65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys
100 105

<210> 153
<211> 5
<212> PRT
<213> Artificial sequence

<220>
<223> anti-Her2 antibody 2 HCDR1 amino acid sequence

<400> 153

Asp Tyr Thr Met Asp
1 5

<210> 154
<211> 17
<212> PRT
<213> Artificial sequence

<220>
<223> anti-Her2 antibody 2 HCDR2 amino acid sequence

<400> 154

Asp Val Asn Pro Asn Ser Gly Gly Ser Ile Tyr Asn Gln Arg Phe Lys
1 5 10 15

Gly

<210> 155
<211> 10
<212> PRT
<213> Artificial sequence

<220>
<223> anti-Her2 antibody 2 HCDR3 amino acid sequence

<400> 155

Asn Leu Gly Pro Ser Phe Tyr Phe Asp Tyr
1 5 10

<210> 156
<211> 11
<212> PRT
<213> Artificial sequence

<220>

<223>  anti-Her2 antibody 2 LCDR1 amino acid sequence

<400>  156

Lys Ala Ser Gln Asp Val Ser Ile Gly Val Ala
1               5                   10

<210>  157
<211>  7
<212>  PRT
<213>  Artificial sequence

<220>
<223>  anti-Her2 antibody 2 LCDR2 amino acid sequence

<400>  157

Ser Ala Ser Tyr Arg Tyr Thr
1               5

<210>  158
<211>  9
<212>  PRT
<213>  Artificial sequence

<220>
<223>  anti-Her2 antibody 2 LCDR3 amino acid sequence

<400>  158

Gln Gln Tyr Tyr Ile Tyr Pro Tyr Thr
1               5

<210>  159
<211>  119
<212>  PRT
<213>  Artificial sequence

<220>
<223>  anti-Her2 antibody 2 VH amino acid sequence

<400>  159

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr

65 70 75 80

Leu Gln Met Asn Ser Leu Arg Ala Glu Asp Thr Ala Val Tyr Tyr Cys
85 90 95

Ala Arg Asn Leu Gly Pro Ser Phe Tyr Phe Asp Tyr Trp Gly Gln Gly
100 105 110

Thr Leu Val Thr Val Ser Ser
115

<210> 160
<211> 107
<212> PRT
<213> Artificial sequence

<220>
<223> anti-Her2 antibody 2 VL amino acid sequence

<400> 160

Asp Ile Gln Met Thr Gln Ser Pro Ser Ser Leu Ser Ala Ser Val Gly
1 5 10 15

Asp Arg Val Thr Ile Thr Cys Lys Ala Ser Gln Asp Val Ser Ile Gly
20 25 30

Val Ala Trp Tyr Gln Gln Lys Pro Gly Lys Ala Pro Lys Leu Leu Ile
35 40 45

Tyr Ser Ala Ser Tyr Arg Tyr Thr Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Tyr Ile Tyr Pro Tyr
85 90 95

Thr Phe Gly Gln Gly Thr Lys Val Glu Ile Lys
100 105

<210> 161
<211> 5
<212> PRT
<213> Artificial sequence

<220>
<223> anti-Her2 antibody 3 HCDR1 amino acid sequence

<400> 161

Asp Thr Tyr Ile His
1               5

<210> 162
<211> 17
<212> PRT
<213> Artificial sequence

<220>
<223> anti-Her2 antibody 3 HCDR2 amino acid sequence

<400> 162

Arg Ile Tyr Pro Thr Asn Gly Tyr Thr Arg Tyr Asp Pro Lys Phe Gln
1               5                   10                  15

Asp

<210> 163
<211> 11
<212> PRT
<213> Artificial sequence

<220>
<223> anti-Her2 antibody 3 HCDR3 amino acid sequence

<400> 163

Trp Gly Gly Asp Gly Phe Tyr Ala Met Asp Tyr
1               5                   10

<210> 164
<211> 11
<212> PRT
<213> Artificial sequence

<220>
<223> anti-Her2 antibody 3 LCDR1 amino acid sequence

<400> 164

Lys Ala Ser Gln Asp Val Asn Thr Ala Val Ala
1               5                   10

<210> 165
<211> 7
<212> PRT
<213> Artificial sequence

<220>
<223> anti-Her2 antibody 3 LCDR2 amino acid sequence

<400> 165

Ser Ala Ser Phe Arg Tyr Thr
1               5

<210> 166
<211> 9
<212> PRT
<213> Artificial sequence

<220>
<223> anti-Her2 antibody 3 LCDR3 amino acid sequence

<400> 166

Gln Gln His Tyr Thr Thr Pro Pro Thr
1               5

<210> 167
<211> 120
<212> PRT
<213> Artificial sequence

<220>
<223> anti-Her2 antibody 3 VH amino acid sequence

<400> 167

Gln Val Gln Leu Gln Gln Ser Gly Pro Glu Leu Val Lys Pro Gly Ala
1               5                   10                  15

Ser Leu Lys Leu Ser Cys Thr Ala Ser Gly Phe Asn Ile Lys Asp Thr
            20                  25                  30

Tyr Ile His Trp Val Lys Gln Arg Pro Glu Gln Gly Leu Glu Trp Ile
        35                  40                  45

Gly Arg Ile Tyr Pro Thr Asn Gly Tyr Thr Arg Tyr Asp Pro Lys Phe
    50                  55                  60

Gln Asp Lys Ala Thr Ile Thr Ala Asp Thr Ser Ser Asn Thr Ala Tyr
65                  70                  75                  80

Leu Gln Val Ser Arg Leu Thr Ser Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Ala Ser Val Thr Val Ser Ser
        115                 120

<210> 168
<211> 107
<212> PRT
<213> Artificial sequence

<220>

<223>  anti-Her2 antibody 3 VL amino acid sequence

<400>  168

Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Asn Thr Ala
            20                  25                  30

Val Ala Trp Tyr Gln Gln Lys Pro Gly His Ser Pro Lys Leu Leu Ile
        35                  40                  45

Tyr Ser Ala Ser Phe Arg Tyr Thr Gly Val Pro Asp Arg Phe Thr Gly
    50                  55                  60

Ser Arg Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Thr Thr Pro Pro
                85                  90                  95

Thr Phe Gly Gly Gly Thr Lys Val Glu Ile Lys
            100                 105

<210>  169
<211>  5
<212>  PRT
<213>  Artificial sequence

<220>
<223>  anti-EGFR antibody 1 HCDR1 amino acid sequence

<400>  169

Asn Tyr Gly Val His
1               5

<210>  170
<211>  16
<212>  PRT
<213>  Artificial sequence

<220>
<223>  anti-EGFR antibody 1 HCDR2 amino acid sequence

<400>  170

Val Ile Trp Ser Gly Gly Asn Thr Asp Tyr Asn Thr Pro Phe Thr Ser
1               5                   10                  15

<210>  171
<211>  11
<212>  PRT

<213> Artificial sequence

<220>
<223> anti-EGFR antibody 1 HCDR3 amino acid sequence

<400> 171

Ala Leu Thr Tyr Tyr Asp Tyr Glu Phe Ala Tyr
1 5 10

<210> 172
<211> 11
<212> PRT
<213> Artificial sequence

<220>
<223> anti-EGFR antibody 1 LCDR1 amino acid sequence

<400> 172

Arg Ala Ser Gln Ser Ile Gly Thr Asn Ile His
1 5 10

<210> 173
<211> 7
<212> PRT
<213> Artificial sequence

<220>
<223> anti-EGFR antibody 1 LCDR2 amino acid sequence

<400> 173

Tyr Ala Ser Glu Ser Ile Ser
1 5

<210> 174
<211> 9
<212> PRT
<213> Artificial sequence

<220>
<223> anti-EGFR antibody 1 LCDR3 amino acid sequence

<400> 174

Gln Gln Asn Asn Asn Trp Pro Thr Thr
1 5

<210> 175
<211> 119
<212> PRT
<213> Artificial sequence

<220>
<223> anti-EGFR antibody 1 VH amino acid sequence

<400> 175

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ala
        115

<210> 176
<211> 107
<212> PRT
<213> Artificial sequence

<220>
<223> anti-EGFR antibody 1 VL amino acid sequence

<400> 176

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr

85 90 95

Thr Phe Gly Ala Gly Thr Lys Leu Glu Leu Lys
100 105

<210> 177
<211> 7
<212> PRT
<213> Artificial sequence

<220>
<223> anti-EGFR antibody 2 HCDR1 amino acid sequence

<400> 177

Ser Gly Asp Tyr Tyr Trp Ser
1 5

<210> 178
<211> 16
<212> PRT
<213> Artificial sequence

<220>
<223> anti-EGFR antibody 2 HCDR2 amino acid sequence

<400> 178

Tyr Ile Tyr Tyr Ser Gly Ser Thr Asp Tyr Asn Pro Ser Leu Lys Ser
1 5 10 15

<210> 179
<211> 11
<212> PRT
<213> Artificial sequence

<220>
<223> anti-EGFR antibody 2 HCDR3 amino acid sequence

<400> 179

Val Ser Ile Phe Gly Val Gly Thr Phe Asp Tyr
1 5 10

<210> 180
<211> 11
<212> PRT
<213> Artificial sequence

<220>
<223> anti-EGFR antibody 2 LCDR1 amino acid sequence

<400> 180

Arg Ala Ser Gln Ser Val Ser Ser Tyr Leu Ala
1 5 10

<210> 181
<211> 7
<212> PRT
<213> Artificial sequence

<220>
<223> anti-EGFR antibody 2 LCDR2 amino acid sequence

<400> 181

Asp Ala Ser Asn Arg Ala Thr
1               5

<210> 182
<211> 9
<212> PRT
<213> Artificial sequence

<220>
<223> anti-EGFR antibody 2 LCDR3 amino acid sequence

<400> 182

His Gln Tyr Gly Ser Thr Pro Leu Thr
1               5

<210> 183
<211> 121
<212> PRT
<213> Artificial sequence

<220>
<223> anti-EGFR antibody 2 VH amino acid sequence

<400> 183

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Asp Tyr Tyr Trp Ser Trp Ile Arg Gln Pro Pro Gly Lys Gly Leu Glu
        35                  40                  45

Trp Ile Gly Tyr Ile Tyr Tyr Ser Gly Ser Thr Asp Tyr Asn Pro Ser
    50                  55                  60

Leu Lys Ser Arg Val Thr Met Ser Val Asp Thr Ser Lys Asn Gln Phe
65                  70                  75                  80

Ser Leu Lys Val Asn Ser Val Thr Ala Ala Asp Thr Ala Val Tyr Tyr
                85                  90                  95

Cys Ala Arg Val Ser Ile Phe Gly Val Gly Thr Phe Asp Tyr Trp Gly

100 105 110

Gln Gly Thr Leu Val Thr Val Ser Ser
115 120

<210> 184
<211> 107
<212> PRT
<213> Artificial sequence

<220>
<223> anti-EGFR antibody 2 VL amino acid sequence

<400> 184

Glu Ile Val Met Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys His Gln Tyr Gly Ser Thr Pro Leu
85 90 95

Thr Phe Gly Gly Gly Thr Lys Ala Glu Ile Lys
100 105

<210> 185
<211> 5
<212> PRT
<213> Artificial sequence

<220>
<223> anti-EGFR antibody 3 HCDR1 amino acid sequence

<400> 185

Asn Tyr Tyr Ile Tyr
1 5

<210> 186
<211> 17
<212> PRT
<213> Artificial sequence

<220>
<223> anti-EGFR antibody 3 HCDR2 amino acid sequence

<400> 186

Gly Ile Asn Pro Thr Ser Gly Gly Ser Asn Phe Asn Glu Lys Phe Lys
1 5 10 15

Thr

<210> 187
<211> 14
<212> PRT
<213> Artificial sequence

<220>
<223> anti-EGFR antibody 3 HCDR3 amino acid sequence

<400> 187

Gln Gly Leu Trp Phe Asp Ser Asp Gly Arg Gly Phe Asp Phe
1 5 10

<210> 188
<211> 16
<212> PRT
<213> Artificial sequence

<220>
<223> anti-EGFR antibody 3 LCDR1 amino acid sequence

<400> 188

Arg Ser Ser Gln Asn Ile Val His Ser Asn Gly Asn Thr Tyr Leu Asp
1 5 10 15

<210> 189
<211> 7
<212> PRT
<213> Artificial sequence

<220>
<223> anti-EGFR antibody 3 LCDR2 amino acid sequence

<400> 189

Lys Val Ser Asn Arg Phe Ser
1 5

<210> 190
<211> 9
<212> PRT
<213> Artificial sequence

<220>
<223> anti-EGFR antibody 3 LCDR3 amino acid sequence

<400> 190

Phe Gln Tyr Ser His Val Pro Trp Thr
1 5

<210> 191
<211> 123
<212> PRT
<213> Artificial sequence

<220>
<223> anti-EGFR antibody 3 VH amino acid sequence

<400> 191

Gln Val Gln Leu Gln Gln Ser Gly Ala Glu Val Lys Lys Pro Gly Ser
1 5 10 15

Ser Val Lys Val Ser Cys Lys Ala Ser Gly Tyr Thr Phe Thr Asn Tyr
20 25 30

Tyr Ile Tyr Trp Val Arg Gln Ala Pro Gly Gln Gly Leu Glu Trp Ile
35 40 45

Gly Gly Ile Asn Pro Thr Ser Gly Gly Ser Asn Phe Asn Glu Lys Phe
50 55 60

Lys Thr Arg Val Thr Ile Thr Val Asp Glu Ser Thr Asn Thr Ala Tyr
65 70 75 80

Met Glu Leu Ser Ser Leu Arg Ser Glu Asp Thr Ala Phe Tyr Phe Cys
85 90 95

Ala Arg Gln Gly Leu Trp Phe Asp Ser Asp Gly Arg Gly Phe Asp Phe
100 105 110

Trp Gly Gln Gly Ser Thr Val Thr Val Ser Ser
115 120

<210> 192
<211> 112
<212> PRT
<213> Artificial sequence

<220>
<223> anti-EGFR antibody 3 VL amino acid sequence

<400> 192

Asp Ile Gln Met Thr Gln Ser Pro Ser Ser Leu Ser Ala Ser Val Gly
1 5 10 15

Asp Arg Val Thr Ile Thr Cys Arg Ser Ser Gln Asn Ile Val His Ser
20 25 30

Asn Gly Asn Thr Tyr Leu Asp Trp Tyr Gln Gln Thr Pro Gly Lys Ala
35 40 45

Pro Lys Leu Leu Ile Tyr Lys Val Ser Asn Arg Phe Ser Gly Val Pro
50 55 60

Ser Arg Phe Ser Gly Ser Gly Ser Gly Thr Asp Phe Thr Phe Thr Ile
65 70 75 80

Ser Ser Leu Gln Pro Glu Asp Ile Ala Thr Tyr Tyr Cys Phe Gln Tyr
85 90 95

Ser His Val Pro Trp Thr Phe Gly Gln Gly Thr Lys Leu Gln Ile Thr
100 105 110

<210> 193
<211> 5
<212> PRT
<213> Artificial sequence

<220>
<223> anti-GPC-3 antibody HCDR1 amino acid sequence

<400> 193

Asp Tyr Glu Met His
1 5

<210> 194
<211> 17
<212> PRT
<213> Artificial sequence

<220>
<223> anti-GPC-3 antibody HCDR2 amino acid sequence

<400> 194

Ala Ile Asp Pro Gln Thr Gly Asn Thr Ala Phe Asn Gln Lys Phe Lys
1 5 10 15

Gly

<210> 195
<211> 6
<212> PRT
<213> Artificial sequence

<220>
<223> anti-GPC-3 antibody HCDR3 amino acid sequence

<400> 195

Phe Tyr Ser Leu Thr Tyr
1 5

<210> 196
<211> 16
<212> PRT
<213> Artificial sequence

<220>
<223> anti-GPC-3 antibody LCDR1 amino acid sequence

<400> 196

Arg Ser Ser Gln Ser Ile Val His Ser Asn Gly Asn Thr Tyr Leu Gln
1 5 10 15

<210> 197
<211> 7
<212> PRT
<213> Artificial sequence

<220>
<223> anti-GPC-3 antibody LCDR2 amino acid sequence

<400> 197

Lys Val Ser Asn Arg Phe Ser
1 5

<210> 198
<211> 9
<212> PRT
<213> Artificial sequence

<220>
<223> anti-GPC-3 antibody LCDR3 amino acid sequence

<400> 198

Phe Gln Gly Ser His Phe Pro Tyr Ala
1 5

<210> 199
<211> 115
<212> PRT
<213> Artificial sequence

<220>
<223> anti-GPC-3 antibody VH amino acid sequence

<400> 199

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Lys Gln Ala Pro Gly Gln Gly Leu Glu Trp Ile
35 40 45

Gly Ala Ile Asp Pro Gln Thr Gly Asn Thr Ala Phe Asn Gln Lys Phe
50 55 60

Lys Gly Arg Val Thr Leu Thr Arg Asp Lys Ser Ser Ser Thr Val Tyr
65 70 75 80

Met Glu Leu Ser Ser Leu Arg Ser Glu Asp Thr Ala Val Tyr Tyr Cys
85 90 95

Thr Arg Phe Tyr Ser Leu Thr Tyr Trp Gly Gln Gly Thr Leu Val Thr
100 105 110

Val Ser Ser
115

<210> 200
<211> 112
<212> PRT
<213> Artificial sequence

<220>
<223> anti-GPC-3 antibody VL amino acid sequence

<400> 200

Asp Val Leu Met Thr Gln Ser Pro Leu Ser Leu Pro Val Thr Leu Gly
1 5 10 15

Gln Pro Ala Ser Ile Ser Cys Arg Ser Ser Gln Ser Ile Val His Ser
20 25 30

Asn Gly Asn Thr Tyr Leu Gln Trp Tyr Leu Gln Arg Pro Gly Gln Ser
35 40 45

Pro Lys Leu Leu Ile Tyr Lys Val Ser Asn Arg Phe Ser Gly Val Pro
50 55 60

Asp Arg Phe Ser Gly Ser Gly Ser Gly Thr Tyr Phe Thr Leu Lys Ile
65 70 75 80

Ser Arg Val Glu Ala Glu Asp Val Gly Val Tyr Tyr Cys Phe Gln Gly
85 90 95

Ser His Phe Pro Tyr Ala Phe Gly Gly Gly Thr Lys Val Glu Ile Lys

100 105 110

<210> 201
<211> 5
<212> PRT
<213> Artificial sequence

<220>
<223> anti-Mesothelin antibody HCDR1 amino acid sequence

<400> 201

Ile Tyr Gly Met His
1 5

<210> 202
<211> 17
<212> PRT
<213> Artificial sequence

<220>
<223> anti-Mesothelin antibody HCDR2 amino acid sequence

<400> 202

Val Ile Trp Tyr Asp Gly Ser His Glu Tyr Tyr Ala Asp Ser Val Lys
1 5 10 15

Gly

<210> 203
<211> 13
<212> PRT
<213> Artificial sequence

<220>
<223> anti-Mesothelin antibody HCDR3 amino acid sequence

<400> 203

Asp Gly Asp Tyr Tyr Asp Ser Gly Ser Pro Leu Asp Tyr
1 5 10

<210> 204
<211> 11
<212> PRT
<213> Artificial sequence

<220>
<223> anti-Mesothelin antibody LCDR1 amino acid sequence

<400> 204

Arg Ala Ser Gln Ser Val Ser Ser Tyr Leu Ala
1 5 10

<210> 205
<211> 7
<212> PRT
<213> Artificial sequence

<220>
<223> anti-Mesothelin antibody LCDR2 amino acid sequence

<400> 205

Asp Ala Ser Asn Arg Ala Thr
1               5

<210> 206
<211> 9
<212> PRT
<213> Artificial sequence

<220>
<223> anti-Mesothelin antibody LCDR3 amino acid sequence

<400> 206

Gln Gln Arg Ser Asn Trp Pro Leu Thr
1               5

<210> 207
<211> 122
<212> PRT
<213> Artificial sequence

<220>
<223> anti-Mesothelin antibody VH amino acid sequence

<400> 207

Gln Val Tyr Leu Val Glu Ser Gly Gly Gly Val Val Gln Pro Gly Arg
1               5                   10                  15

Ser Leu Arg Leu Ser Cys Ala Ala Ser Gly Ile Thr Phe Ser Ile Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser His Glu Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Leu Met Asn Ser Leu Arg Ala Glu Asp Thr Ala Val Tyr Tyr Cys
                85                  90                  95

Ala Arg Asp Gly Asp Tyr Tyr Asp Ser Gly Ser Pro Leu Asp Tyr Trp

100 105 110

Gly Gln Gly Thr Leu Val Thr Val Ser Ser
115 120

<210> 208
<211> 107
<212> PRT
<213> Artificial sequence

<220>
<223> anti-Mesothelin antibody VL amino acid sequence

<400> 208

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Leu
85 90 95

Thr Phe Gly Gly Gly Thr Lys Val Glu Ile Lys
100 105

<210> 209
<211> 5
<212> PRT
<213> Artificial sequence

<220>
<223> anti-Mucin1 antibody 1 HCDR1 amino acid sequence

<400> 209

Ser Tyr Val Leu His
1 5

<210> 210
<211> 17
<212> PRT
<213> Artificial sequence

<220>
<223> anti-Mucin1 antibody 1 HCDR2 amino acid sequence

<400> 210

Tyr Ile Asn Pro Tyr Asn Asp Gly Thr Gln Tyr Asn Glu Lys Phe Lys
1 5 10 15

Gly

<210> 211
<211> 10
<212> PRT
<213> Artificial sequence

<220>
<223> anti-Mucin1 antibody 1 HCDR3 amino acid sequence

<400> 211

Gly Phe Gly Gly Ser Tyr Gly Phe Ala Tyr
1 5 10

<210> 212
<211> 12
<212> PRT
<213> Artificial sequence

<220>
<223> anti-Mucin1 antibody 1 LCDR1 amino acid sequence

<400> 212

Ser Ala Ser Ser Ser Val Ser Ser Ser Tyr Leu Tyr
1 5 10

<210> 213
<211> 7
<212> PRT
<213> Artificial sequence

<220>
<223> anti-Mucin1 antibody 1 LCDR2 amino acid sequence

<400> 213

Ser Thr Ser Asn Leu Ala Ser
1 5

<210> 214
<211> 9
<212> PRT
<213> Artificial sequence

<220>
<223> anti-Mucin1 antibody 1 LCDR3 amino acid sequence

<400> 214

His Gln Trp Asn Arg Tyr Pro Tyr Thr
1               5

<210> 215
<211> 119
<212> PRT
<213> Artificial sequence

<220>
<223> anti-Mucin1 antibody 1 VH amino acid sequence

<400> 215

Gln Val Gln Leu Gln Gln Ser Gly Ala Glu Val Lys Lys Pro Gly Ala
1               5                   10                  15

Ser Val Lys Val Ser Cys Glu Ala Ser Gly Tyr Thr Phe Pro Ser Tyr
            20                  25                  30

Val Leu His Trp Val Lys Gln Ala Pro Gly Gln Gly Leu Glu Trp Ile
        35                  40                  45

Gly Tyr Ile Asn Pro Tyr Asn Asp Gly Thr Gln Tyr Asn Glu Lys Phe
    50                  55                  60

Lys Gly Lys Ala Thr Leu Thr Arg Asp Thr Ser Ile Asn Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Phe Gly Gly Ser Tyr Gly Phe Ala Tyr Trp Gly Gln Gly
            100                 105                 110

Thr Leu Val Thr Val Ser Ser
        115

<210> 216
<211> 108
<212> PRT
<213> Artificial sequence

<220>
<223> anti-Mucin1 antibody 1 VL amino acid sequence

<400> 216

Asp Ile Gln Leu Thr Gln Ser Pro Ser Ser Leu Ser Ala Ser Val Gly
1               5                   10                  15

Asp Arg Val Thr Met Thr Cys Ser Ala Ser Ser Ser Val Ser Ser Ser
20 25 30

Tyr Leu Tyr Trp Tyr Gln Gln Lys Pro Gly Lys Ala Pro Lys Leu Trp
35 40 45

Ile Tyr Ser Thr Ser Asn Leu Ala Ser Gly Val Pro Ala Arg Phe Ser
50 55 60

Gly Ser Gly Ser Gly Thr Asp Phe Thr Leu Thr Ile Ser Ser Leu Gln
65 70 75 80

Pro Glu Asp Ser Ala Ser Tyr Phe Cys His Gln Trp Asn Arg Tyr Pro
85 90 95

Tyr Thr Phe Gly Gly Gly Thr Arg Leu Glu Ile Lys
100 105

<210> 217
<211> 5
<212> PRT
<213> Artificial sequence

<220>
<223> anti-Mucin1 antibody 2 HCDR1 amino acid sequence

<400> 217

Asn Tyr Trp Met Asn
1 5

<210> 218
<211> 19
<212> PRT
<213> Artificial sequence

<220>
<223> anti-Mucin1 antibody 2 HCDR2 amino acid sequence

<400> 218

Glu Ile Arg Leu Lys Ser Asn Asn Tyr Thr Thr His Tyr Ala Glu Ser
1 5 10 15

Val Lys Gly

<210> 219
<211> 6
<212> PRT
<213> Artificial sequence

<220>
<223> anti-Mucin1 antibody 2 HCDR3 amino acid sequence

<400> 219

His Tyr Tyr Phe Asp Tyr
1 5

<210> 220
<211> 16
<212> PRT
<213> Artificial sequence

<220>
<223> anti-Mucin1 antibody 2 LCDR1 amino acid sequence

<400> 220

Arg Ser Ser Lys Ser Leu Leu His Ser Asn Gly Ile Thr Tyr Phe Phe
1 5 10 15

<210> 221
<211> 7
<212> PRT
<213> Artificial sequence

<220>
<223> anti-Mucin1 antibody 2 LCDR2 amino acid sequence

<400> 221

Gln Met Ser Asn Leu Ala Ser
1 5

<210> 222
<211> 9
<212> PRT
<213> Artificial sequence

<220>
<223> anti-Mucin1 antibody 2 LCDR3 amino acid sequence

<400> 222

Ala Gln Asn Leu Glu Leu Pro Pro Thr
1 5

<210> 223
<211> 117
<212> PRT
<213> Artificial sequence

<220>
<223> anti-Mucin1 antibody 2 VH amino acid sequence

<400> 223

Glu Val Gln Leu Val Glu Ser Gly Gly Gly Leu Val Gln Pro Gly Gly
1 5 10 15

Ser Met Arg Leu Ser Cys Val Ala Ser Gly Phe Pro Phe Ser Asn Tyr
20 25 30

Trp Met Asn Trp Val Arg Gln Ala Pro Gly Lys Gly Leu Glu Trp Val
35 40 45

Gly Glu Ile Arg Leu Lys Ser Asn Asn Tyr Thr Thr His Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Thr Arg His Tyr Tyr Phe Asp Tyr Trp Gly Gln Gly Thr Leu
100 105 110

Val Thr Val Ser Ser
115

<210> 224
<211> 112
<212> PRT
<213> Artificial sequence

<220>
<223> anti-Mucin1 antibody 2 VL amino acid sequence

<400> 224

Asp Ile Val Met Thr Gln Ser Pro Leu Ser Asn Pro Val Thr Pro Gly
1 5 10 15

Glu Pro Ala Ser Ile Ser Cys Arg Ser Ser Lys Ser Leu Leu His Ser
20 25 30

Asn Gly Ile Thr Tyr Phe Phe Trp Tyr Leu Gln Lys Pro Gly Gln Ser
35 40 45

Pro Gln Leu Leu Ile Tyr Gln Met Ser Asn Leu Ala Ser Gly Val Pro
50 55 60

Asp Arg Phe Ser Gly Ser Gly Ser Gly Thr Asp Phe Thr Leu Arg Ile
65 70 75 80

Ser Arg Val Glu Ala Glu Asp Val Gly Val Tyr Tyr Cys Ala Gln Asn
85 90 95

Leu Glu Leu Pro Pro Thr Phe Gly Gln Gly Thr Lys Val Glu Ile Lys

100 105 110

<210> 225
<211> 5
<212> PRT
<213> Artificial sequence

<220>
<223> anti-CA125 antibody HCDR1 amino acid sequence

<400> 225

Ser Tyr Ala Met Ser
1 5

<210> 226
<211> 17
<212> PRT
<213> Artificial sequence

<220>
<223> anti-CA125 antibody HCDR2 amino acid sequence

<400> 226

Thr Ile Ser Ser Ala Gly Gly Tyr Ile Phe Tyr Ser Asp Ser Val Gln
1 5 10 15

Gly

<210> 227
<211> 14
<212> PRT
<213> Artificial sequence

<220>
<223> anti-CA125 antibody HCDR3 amino acid sequence

<400> 227

Gln Gly Phe Gly Asn Tyr Gly Asp Tyr Tyr Ala Met Asp Tyr
1 5 10

<210> 228
<211> 17
<212> PRT
<213> Artificial sequence

<220>
<223> anti-CA125 antibody LCDR1 amino acid sequence

<400> 228

Lys Ser Ser Gln Ser Leu Leu Asn Ser Arg Thr Arg Lys Asn Gln Leu
1 5 10 15

Ala

<210> 229
<211> 7
<212> PRT
<213> Artificial sequence

<220>
<223> anti-CA125 antibody LCDR2 amino acid sequence

<400> 229

Trp Ala Ser Thr Arg Gln Ser
1 5

<210> 230
<211> 8
<212> PRT
<213> Artificial sequence

<220>
<223> anti-CA125 antibody LCDR3 amino acid sequence

<400> 230

Gln Gln Ser Tyr Asn Leu Leu Thr
1 5

<210> 231
<211> 122
<212> PRT
<213> Artificial sequence

<220>
<223> anti-CA125 antibody VH amino acid sequence

<400> 231

Val Lys Leu Gln Glu Ser Gly Gly Gly Phe Val Lys Pro Gly Gly Ser
1 5 10 15

Leu Lys Val Ser Cys Ala Ala Ser Gly Phe Thr Phe Ser Ser Tyr Ala
20 25 30

Met Ser Trp Val Arg Leu Ser Pro Glu Met Arg Leu Glu Trp Val Ala
35 40 45

Thr Ile Ser Ser Ala Gly Gly Tyr Ile Phe Tyr Ser Asp Ser Val Gln
50 55 60

Gly Arg Phe Thr Ile Ser Arg Asp Asn Ala Lys Asn Thr Leu His Leu
65 70 75 80

Gln Met Gly Ser Leu Arg Ser Gly Asp Thr Ala Met Tyr Tyr Cys Ala

85 90 95

Arg Gln Gly Phe Gly Asn Tyr Gly Asp Tyr Tyr Ala Met Asp Tyr Trp
100 105 110

Gly Gln Gly Thr Thr Val Thr Val Ser Ser
115 120

<210> 232
<211> 112
<212> PRT
<213> Artificial sequence

<220>
<223> anti-CA125 antibody VL amino acid sequence

<400> 232

Asp Ile Glu Leu Thr Gln Ser Pro Ser Ser Leu Ala Val Ser Ala Gly
1 5 10 15

Glu Lys Val Thr Met Ser Cys Lys Ser Ser Gln Ser Leu Leu Asn Ser
20 25 30

Arg Thr Arg Lys Asn Gln Leu Ala Trp Tyr Gln Gln Lys Pro Gly Gln
35 40 45

Ser Pro Glu Leu Leu Ile Tyr Trp Ala Ser Thr Arg Gln Ser Gly Val
50 55 60

Pro Asp Arg Phe Thr Gly Ser Gly Ser Gly Thr Asp Phe Thr Leu Thr
65 70 75 80

Ile Ser Ser Val Gln Ala Glu Asp Leu Ala Val Tyr Tyr Cys Gln Gln
85 90 95

Ser Tyr Asn Leu Leu Thr Phe Gly Pro Gly Thr Lys Leu Glu Val Lys
100 105 110

<210> 233
<211> 5
<212> PRT
<213> Artificial sequence

<220>
<223> anti-BCMA antibody HCDR1 amino acid sequence

<400> 233

Asn Tyr Trp Met His
1 5

<210> 234
<211> 17
<212> PRT
<213> Artificial sequence

<220>
<223> anti-BCMA antibody HCDR2 amino acid sequence

<400> 234

Ala Thr Tyr Arg Gly His Ser Asp Thr Tyr Tyr Asn Gln Lys Phe Lys
1 5 10 15

Gly

<210> 235
<211> 12
<212> PRT
<213> Artificial sequence

<220>
<223> anti-BCMA antibody HCDR3 amino acid sequence

<400> 235

Gly Ala Ile Tyr Asp Gly Tyr Asp Val Leu Asp Asn
1 5 10

<210> 236
<211> 11
<212> PRT
<213> Artificial sequence

<220>
<223> anti-BCMA antibody LCDR1 amino acid sequence

<400> 236

Ser Ala Ser Gln Asp Ile Ser Asn Tyr Leu Asn
1 5 10

<210> 237
<211> 7
<212> PRT
<213> Artificial sequence

<220>
<223> anti-BCMA antibody LCDR2 amino acid sequence

<400> 237

Tyr Thr Ser Asn Leu His Ser
1 5

<210> 238
<211> 9
<212> PRT

<213> Artificial sequence

<220>
<223> anti-BCMA antibody LCDR3 amino acid sequence

<400> 238

Gln Gln Tyr Arg Lys Leu Pro Trp Thr
1 5

<210> 239
<211> 121
<212> PRT
<213> Artificial sequence

<220>
<223> anti-BCMA antibody VH amino acid sequence

<400> 239

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Asn Tyr
20 25 30

Trp Met His Trp Val Arg Gln Ala Pro Gly Gln Gly Leu Glu Trp Met
35 40 45

Gly Ala Thr Tyr Arg Gly His Ser Asp Thr Tyr Tyr Asn Gln Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Ala Ile Tyr Asp Gly Tyr Asp Val Leu Asp Asn Trp Gly
100 105 110

Gln Gly Thr Leu Val Thr Val Ser Ser
115 120

<210> 240
<211> 107
<212> PRT
<213> Artificial sequence

<220>
<223> anti-BCMA antibody VL amino acid sequence

<400> 240

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys
            100                 105

<210> 241
<211> 5
<212> PRT
<213> Artificial sequence

<220>
<223> anti-CD3 antibody 3 HCDR1 amino acid sequence

<400> 241

Thr Tyr Ala Met Asn
1               5

<210> 242
<211> 19
<212> PRT
<213> Artificial sequence

<220>
<223> anti-CD3 antibody 3 HCDR2 amino acid sequence

<400> 242

Arg Ile Arg Ser Lys Tyr Asn Asn Tyr Ala Thr Tyr Tyr Ala Asp Ser
1               5                   10                  15

Val Lys Asp

<210> 243
<211> 14
<212> PRT

<213> Artificial sequence

<220>
<223> anti-CD3 antibody 3 HCDR3 amino acid sequence

<400> 243

His Gly Asn Phe Gly Asn Ser Tyr Val Ser Trp Phe Ala Tyr
1 5 10

<210> 244
<211> 14
<212> PRT
<213> Artificial sequence

<220>
<223> anti-CD3 antibody 3 LCDR1 amino acid sequence

<400> 244

Arg Ser Ser Thr Gly Ala Val Thr Thr Ser Asn Tyr Ala Asn
1 5 10

<210> 245
<211> 7
<212> PRT
<213> Artificial sequence

<220>
<223> anti-CD3 antibody 3 LCDR2 amino acid sequence

<400> 245

Gly Thr Asn Lys Arg Ala Pro
1 5

<210> 246
<211> 9
<212> PRT
<213> Artificial sequence

<220>
<223> anti-CD3 antibody 3 LCDR3 amino acid sequence

<400> 246

Ala Leu Trp Tyr Ser Asn Leu Trp Val
1 5

<210> 247
<211> 125
<212> PRT
<213> Artificial sequence

<220>
<223> anti-CD3 antibody 3 VH amino acid sequence

<400> 247

Glu Val Gln Leu Leu Glu Ser Gly Gly Gly Leu Val Gln Pro Gly Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210> 248
<211> 109
<212> PRT
<213> Artificial sequence

<220>
<223> anti-CD3 antibody 3 VL amino acid sequence

<400> 248

Glu Leu Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn

85 90 95

Leu Trp Val Phe Gly Gly Gly Thr Lys Leu Thr Val Leu
100 105

<210> 249
<211> 5
<212> PRT
<213> Artificial sequence

<220>
<223> anti-CD3 antibody 4 HCDR1 amino acid sequence

<400> 249

Lys Tyr Ala Met Asn
1 5

<210> 250
<211> 19
<212> PRT
<213> Artificial sequence

<220>
<223> anti-CD3 antibody 4 HCDR2 amino acid sequence

<400> 250

Arg Ile Arg Ser Lys Tyr Asn Asn Tyr Ala Thr Tyr Tyr Ala Asp Ser
1 5 10 15

Val Lys Asp

<210> 251
<211> 14
<212> PRT
<213> Artificial sequence

<220>
<223> anti-CD3 antibody 4 HCDR3 amino acid sequence

<400> 251

His Gly Asn Phe Gly Asn Ser Tyr Ile Ser Tyr Trp Ala Tyr
1 5 10

<210> 252
<211> 14
<212> PRT
<213> Artificial sequence

<220>
<223> anti-CD3 antibody 4 LCDR1 amino acid sequence

<400> 252

Gly Ser Ser Thr Gly Ala Val Thr Ser Gly Tyr Tyr Pro Asn
1 5 10

<210> 253
<211> 7
<212> PRT
<213> Artificial sequence

<220>
<223> anti-CD3 antibody 4 LCDR2 amino acid sequence

<400> 253

Gly Thr Lys Phe Leu Ala Pro
1 5

<210> 254
<211> 9
<212> PRT
<213> Artificial sequence

<220>
<223> anti-CD3 antibody 4 LCDR3 amino acid sequence

<400> 254

Ala Leu Trp Tyr Ser Asn Arg Trp Val
1 5

<210> 255
<211> 125
<212> PRT
<213> Artificial sequence

<220>
<223> anti-CD3 antibody 4 VH amino acid sequence

<400> 255

Glu Val Gln Leu Leu Glu Ser Gly Gly Gly Leu Val Gln Pro Gly Gly
1 5 10 15

Ser Leu Lys Leu Ser Cys Ala Ala Ser Gly Phe Thr Phe Asn Lys Tyr
20 25 30

Ala Met Asn Trp Val Arg Gln Ala Pro Gly Lys Gly Leu Glu Trp Val
35 40 45

Ala Arg Ile Arg Ser Lys Tyr Asn Asn Tyr Ala Thr Tyr Tyr Ala Asp
50 55 60

Ser Val Lys Asp Arg Phe Thr Ile Ser Arg Asp Asp Ser Lys Asn Thr
65 70 75 80

Ala Tyr Leu Gln Met Asn Asn Leu Lys Thr Glu Asp Thr Ala Val Tyr

                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210>  256
<211>  109
<212>  PRT
<213>  Artificial sequence

<220>
<223>  anti-CD3 antibody 4 VL amino acid sequence

<400>  256

Glu Leu Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Tyr Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Arg Trp Val Phe Gly Gly Gly Thr Lys Leu Thr Val Leu
            100                 105

<210>  257
<211>  28
<212>  PRT
<213>  Homo sapiens

<400>  257

Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25

<210> 258
<211> 27
<212> PRT
<213> Artificial sequence

<220>
<223> G2(GGGGS)3CTP1 peptide linker

<400> 258

Gly Gly Gly Gly Gly Gly Ser Gly Gly Gly Gly Ser Gly Gly Gly Gly
1 5 10 15

Ser Ser Ser Ser Ser Lys Ala Pro Pro Pro Ser
20 25

<210> 259
<211> 25
<212> PRT
<213> Artificial sequence

<220>
<223> (GGGGS)3CTP1 peptide linker

<400> 259

Gly Gly Gly Gly Ser Gly Gly Gly Gly Ser Gly Gly Gly Gly Ser Ser
1 5 10 15

Ser Ser Ser Lys Ala Pro Pro Pro Ser
20 25

<210> 260
<211> 22
<212> PRT
<213> Artificial sequence

<220>
<223> GS(GGGGS)2CTP1 peptide linker

<400> 260

Gly Ser Gly Gly Gly Gly Ser Gly Gly Gly Gly Ser Ser Ser Ser Ser
1 5 10 15

Lys Ala Pro Pro Pro Ser
20

<210> 261
<211> 33
<212> PRT
<213> Artificial sequence

<220>
<223> (GGGGS)1CTP4 peptide linker

<400> 261

Gly Gly Gly Gly Ser Ser Ser Ser Ser Lys Ala Pro Pro Pro Ser Leu
1 5 10 15

Pro Ser Pro Ser Arg Leu Pro Gly Pro Ser Asp Thr Pro Ile Leu Pro
20 25 30

Gln

<210> 262
<211> 227
<212> PRT
<213> Artificial sequence

<220>
<223> IgG1 Fc(L234A,L235A) mutant constant region amino acid sequence

<400> 262

Asp Lys Thr His Thr Cys Pro Pro Cys Pro Ala Pro Glu Ala Ala Gly
1 5 10 15

Gly Pro Ser Val Phe Leu Phe Pro Pro Lys Pro Lys Asp Thr Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Asn Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Pro Ala Pro Ile
100 105 110

Glu Lys Thr Ile Ser Lys Ala Lys Gly Gln Pro Arg Glu Pro Gln Val
115 120 125

Tyr Thr Leu Pro Pro Ser Arg Glu Glu Met Thr Lys Asn Gln Val Ser
130 135 140

Leu Thr Cys Leu Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Tyr Ser Lys Leu Thr Val
180 185 190

Asp Lys Ser Arg Trp Gln Gln Gly Asn Val Phe Ser Cys Ser Val Met
195 200 205

His Glu Ala Leu His Asn His Tyr Thr Gln Lys Ser Leu Ser Leu Ser
210 215 220

Pro Gly Lys
225

<210> 263
<211> 226
<212> PRT
<213> Artificial sequence

<220>
<223> IgG1(L234A,L235A, T250Q,N297A,P331S,M428L,K447-) mutant constant region amino acid sequence

<400> 263

Asp Lys Thr His Thr Cys Pro Pro Cys Pro Ala Pro Glu Ala Ala Gly
1 5 10 15

Gly Pro Ser Val Phe Leu Phe Pro Pro Lys Pro Lys Asp Gln Leu Met
20 25 30

Ile Ser Arg Thr Pro Glu Val Thr Cys Val Val Val Asp Val Ser His
35 40 45

Glu Asp Pro Glu Val Lys Phe Asn Trp Tyr Val Asp Gly Val Glu Val
50 55 60

His Asn Ala Lys Thr Lys Pro Arg Glu Glu Gln Tyr Ala Ser Thr Tyr
65 70 75 80

Arg Val Val Ser Val Leu Thr Val Leu His Gln Asp Trp Leu Asn Gly
85 90 95

Lys Glu Tyr Lys Cys Lys Val Ser Asn Lys Ala Leu Pro Ala Ser Ile
100 105 110

Glu Lys Thr Ile Ser Lys Ala Lys Gly Gln Pro Arg Glu Pro Gln Val
115 120 125

Tyr Thr Leu Pro Pro Ser Arg Asp Glu Leu Thr Lys Asn Gln Val Ser
130 135 140

Leu Thr Cys Leu Val Lys Gly Phe Tyr Pro Ser Asp Ile Ala Val Glu
145 150 155 160

Trp Glu Ser Asn Gly Gln Pro Glu Asn Asn Tyr Lys Thr Thr Pro Pro
165 170 175

Val Leu Asp Ser Asp Gly Ser Phe Phe Leu Tyr Ser Lys Leu Thr Val
180 185 190

Asp Lys Ser Arg Trp Gln Gln Gly Asn Val Phe Ser Cys Ser Val Leu
195 200 205

His Glu Ala Leu His Asn His Tyr Thr Gln Lys Ser Leu Ser Leu Ser
210 215 220

Pro Gly
225

<210> 264
<211> 752
<212> PRT
<213> Artificial sequence

<220>
<223> bispecific antibody AB1K1 amino acid sequence

<400> 264

Gln Val Gln Leu Gln Gln Ser Gly Ala Glu Leu Val Arg Pro Gly Ser
1 5 10 15

Ser Val Lys Ile Ser Cys Lys Ala Ser Gly Tyr Ala Phe Ser Ser Tyr
20 25 30

Trp Met Asn Trp Val Lys Gln Arg Pro Gly Gln Gly Leu Glu Trp Ile
35 40 45

Gly Gln Ile Trp Pro Gly Asp Gly Asp Thr Asn Tyr Asn Gly Lys Phe
50 55 60

Lys Gly Lys Ala Thr Leu Thr Ala Asp Glu Ser Ser Ser Thr Ala Tyr
65 70 75 80

Met Gln Leu Ser Ser Leu Ala Ser Glu Asp Ser Ala Val Tyr Phe Cys
85 90 95

Ala Arg Arg Glu Thr Thr Thr Val Gly Arg Tyr Tyr Tyr Ala Met Asp
100 105 110

Tyr Trp Gly Gln Gly Thr Thr Val Thr Val Ser Ser Gly Gly Gly Gly
115 120 125

Ser Gly Gly Gly Gly Ser Gly Gly Gly Gly Ser Asp Ile Gln Leu Thr
130 135 140

Gln Ser Pro Ala Ser Leu Ala Val Ser Leu Gly Gln Arg Ala Thr Ile
145 150 155 160

Ser Cys Lys Ala Ser Gln Ser Val Asp Tyr Asp Gly Asp Ser Tyr Leu
165 170 175

Asn Trp Tyr Gln Gln Ile Pro Gly Gln Pro Pro Lys Leu Leu Ile Tyr
180 185 190

Asp Ala Ser Asn Leu Val Ser Gly Ile Pro Pro Arg Phe Ser Gly Ser
195 200 205

Gly Ser Gly Thr Asp Phe Thr Leu Asn Ile His Pro Val Glu Lys Val
210 215 220

Asp Ala Ala Thr Tyr His Cys Gln Gln Ser Thr Glu Asp Pro Trp Thr
225 230 235 240

Phe Gly Gly Gly Thr Lys Leu Glu Ile Lys Gly Gly Gly Gly Gly Gly
245 250 255

Ser Gly Gly Gly Gly Ser Gly Gly Gly Gly Ser Ser Ser Ser Ser Lys
260 265 270

Ala Pro Pro Pro Ser Glu Val Gln Leu Leu Glu Ser Gly Gly Gly Leu
275 280 285

Val Gln Pro Gly Gly Ser Leu Lys Leu Ser Cys Ala Ala Ser Gly Phe
290 295 300

Thr Phe Asn Thr Tyr Ala Met Asn Trp Val Arg Gln Ala Pro Gly Lys
305 310 315 320

Gly Leu Glu Trp Val Ala Arg Ile Arg Ser Lys Tyr Asn Asn Tyr Ala
325 330 335

Thr Tyr Tyr Ala Asp Ser Val Lys Asp Arg Phe Thr Ile Ser Arg Asp
340 345 350

```
Asp Ser Lys Asn Thr Ala Tyr Leu Gln Met Asn Asn Leu Lys Thr Glu
        355                 360                 365

Asp Thr Ala Val Tyr Tyr Cys Val Arg His Gly Asn Phe Gly Asn Ser
    370                 375                 380

Tyr Val Ser Trp Phe Ala Tyr Trp Gly Gln Gly Thr Leu Val Thr Val
385                 390                 395                 400

Ser Ser Gly Gly Gly Gly Ser Gly Gly Gly Gly Ser Gly Gly Gly Gly
                405                 410                 415

Ser Glu Leu Val Val Thr Gln Glu Pro Ser Leu Thr Val Ser Pro Gly
            420                 425                 430

Gly Thr Val Thr Leu Thr Cys Arg Ser Ser Thr Gly Ala Val Thr Thr
        435                 440                 445

Ser Asn Tyr Ala Asn Trp Val Gln Gln Lys Pro Gly Gln Ala Pro Arg
    450                 455                 460

Gly Leu Ile Gly Gly Thr Asn Lys Arg Ala Pro Gly Thr Pro Ala Arg
465                 470                 475                 480

Phe Ser Gly Ser Leu Leu Gly Gly Lys Ala Ala Leu Thr Leu Ser Gly
                485                 490                 495

Val Gln Pro Glu Asp Glu Ala Glu Tyr Tyr Cys Ala Leu Trp Tyr Ser
            500                 505                 510

Asn Leu Trp Val Phe Gly Gly Gly Thr Lys Leu Thr Val Leu Asp Lys
        515                 520                 525

Thr His Thr Cys Pro Pro Cys Pro Ala Pro Glu Ala Ala Gly Gly Pro
    530                 535                 540

Ser Val Phe Leu Phe Pro Pro Lys Pro Lys Asp Gln Leu Met Ile Ser
545                 550                 555                 560

Arg Thr Pro Glu Val Thr Cys Val Val Val Asp Val Ser His Glu Asp
                565                 570                 575

Pro Glu Val Lys Phe Asn Trp Tyr Val Asp Gly Val Glu Val His Asn
            580                 585                 590

Ala Lys Thr Lys Pro Arg Glu Glu Gln Tyr Ala Ser Thr Tyr Arg Val
        595                 600                 605
```

Val Ser Val Leu Thr Val Leu His Gln Asp Trp Leu Asn Gly Lys Glu
610 615 620

Tyr Lys Cys Lys Val Ser Asn Lys Ala Leu Pro Ala Ser Ile Glu Lys
625 630 635 640

Thr Ile Ser Lys Ala Lys Gly Gln Pro Arg Glu Pro Gln Val Tyr Thr
645 650 655

Leu Pro Pro Ser Arg Asp Glu Leu Thr Lys Asn Gln Val Ser Leu Thr
660 665 670

Cys Leu Val Lys Gly Phe Tyr Pro Ser Asp Ile Ala Val Glu Trp Glu
675 680 685

Ser Asn Gly Gln Pro Glu Asn Asn Tyr Lys Thr Thr Pro Pro Val Leu
690 695 700

Asp Ser Asp Gly Ser Phe Phe Leu Tyr Ser Lys Leu Thr Val Asp Lys
705 710 715 720

Ser Arg Trp Gln Gln Gly Asn Val Phe Ser Cys Ser Val Leu His Glu
725 730 735

Ala Leu His Asn His Tyr Thr Gln Lys Ser Leu Ser Leu Ser Pro Gly
740 745 750

<210> 265
<211> 2256
<212> DNA
<213> Artificial sequence

<220>
<223> bispecific antibody AB1K1 nucleotide sequence

<400> 265
caggtgcagc tgcagcagtc tggagctgag ctggtgcggc ctggctccag cgtgaagatc 60

tcttgtaagg cttccggcta cgccttctct tcctattgga tgaattgggt gaagcagaga 120

cctggacagg gactggagtg gatcggacag atctggccag gcgatggcga cacaaactac 180

aatggcaagt ttaagggcaa ggctacactg accgccgacg agagctcttc caccgcctac 240

atgcagctga gctctctggc ttccgaggat agcgccgtgt atttctgcgc taggagggag 300

accacaaccg tgggccgcta ctattacgct atggattact ggggccaggg cacaaccgtg 360

accgtgtcca gcggaggagg aggatctgga ggaggaggct ccggaggagg aggaagcgac 420

atccagctga cacagagccc cgcttctctg gccgtgagcc tgggacagag ggctaccatc 480

tcttgcaagg cttcccagag cgtggactac gatggcgact cttacctgaa ctggtatcag 540
cagatccctg gccagccccc taagctgctg atctatgatg cctccaatct ggtgagcggc 600
atcccaccca ggttttctgg ctccggcagc ggcacagact tcaccctgaa catccacccc 660
gtggagaagg tggatgccgc tacctaccat tgccagcaga gcacagagga cccttggacc 720
tttggcggcg gcacaaagct ggagatcaag ggtggcggcg gtggaggatc cggcggtgga 780
ggtagcggcg gaggcggtag ctccagctct agtaaagctc cccctccttc cgaggtgcag 840
ctgctggagt ccggaggagg actggtgcag ccaggaggct ccctgaagct gagctgtgct 900
gcctctggct ttaccttcaa cacatatgcc atgaattggg tgcggcaggc tccaggcaag 960
ggactggagt gggtggctag gatcaggtct aagtacaaca attatgccac ctactatgct 1020
gattccgtga aggacaggtt caccatctcc cgcgacgata gcaagaacac agcctacctg 1080
cagatgaaca atctgaagac cgaggatacc gccgtgtact actgcgtgag acatggcaac 1140
tttggcaata gctacgtgtc ctggttcgct tactggggac agggcaccct ggtcacagtg 1200
agctctggag gaggaggatc tggaggagga ggctccggag gaggaggaag cgagctggtg 1260
gtgacccagg agccatctct gacagtgtcc cccggcggca cagtgaccct gacatgtaga 1320
tccagcaccg gcgccgtgac cacatccaac tacgctaatt gggtgcagca gaagccagga 1380
caggctccaa ggggactgat cggaggaacc aacaagaggg ctcctggaac accagctcgg 1440
tttagcggat ctctgctggg aggcaaggct gccctgaccc tgtccggagt gcagccagag 1500
gatgaggccg agtattattg cgctctgtgg tatagcaatc tgtgggtgtt cggaggagga 1560
accaagctga cagtgctgga caagacccat acatgcccac catgccctgc ccctgaagcc 1620
gccggaggac cttccgtgtt cctgttccct cccaagccaa aagatcagct gatgatctct 1680
agaacccccg aagtcacctg cgtggtcgtc gacgtgtccc atgaggaccc tgaagtcaag 1740
ttcaactggt acgtggacgg tgtcgaagtc cacaacgcca agaccaagcc tagggaggag 1800
cagtatgcca gcacataccg ggtggtgtct gtgctgaccg tgctgcatca ggattggctg 1860
aatggcaagg aatataaatg taaggtgagc aataaggctc tgccggctag cattgaaaaa 1920
accatttcca aggctaaggg ccagcccagg gagcctcagg tctacaccct gcctccatct 1980
agagatgaac tgaccaaaaa ccaggtgagc ctgacttgcc tggtcaaagg cttctacccc 2040
agcgacattg ccgtggagtg ggagtctaat ggccagcctg aaaataacta caaaactacc 2100
cctcctgtgc tggactctga tggctccttc tttctgtact ctaaactgac cgtggacaag 2160
tctcgctggc agcagggtaa cgtgttttct tgctccgtgc tgcacgaggc tctgcataac 2220
cattacaccc agaagagcct gtctctgtcc ccagga 2256

<210> 266
<211> 744

<212> PRT
<213> Artificial sequence

<220>
<223> bispecific antibody AB2K amino acid sequence

<400> 266

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ala Gly Gly Gly Gly Ser Gly Gly
        115                 120                 125

Gly Gly Ser Gly Gly Gly Gly Ser Gln Ile Val Leu Ser Gln Ser Pro
    130                 135                 140

Ala Ile Leu Ser Ala Ser Pro Gly Glu Lys Val Thr Met Thr Cys Arg
145                 150                 155                 160

Ala Ser Ser Ser Val Ser Tyr Ile His Trp Phe Gln Gln Lys Pro Gly
                165                 170                 175

Ser Ser Pro Lys Pro Trp Ile Tyr Ala Thr Ser Asn Leu Ala Ser Gly
            180                 185                 190

Val Pro Val Arg Phe Ser Gly Ser Gly Ser Gly Thr Ser Tyr Ser Leu
        195                 200                 205

Thr Ile Ser Arg Val Glu Ala Glu Asp Ala Ala Thr Tyr Tyr Cys Gln
    210                 215                 220
```

```
Gln Trp Thr Ser Asn Pro Pro Thr Phe Gly Gly Gly Thr Lys Leu Glu
225                 230                 235                 240

Ile Lys Gly Gly Gly Gly Gly Gly Ser Gly Gly Gly Gly Ser Gly Gly
                245                 250                 255

Gly Gly Ser Ser Ser Ser Ser Lys Ala Pro Pro Pro Ser Glu Val Gln
            260                 265                 270

Leu Leu Glu Ser Gly Gly Gly Leu Val Gln Pro Gly Gly Ser Leu Lys
        275                 280                 285

Leu Ser Cys Ala Ala Ser Gly Phe Thr Phe Asn Thr Tyr Ala Met Asn
    290                 295                 300

Trp Val Arg Gln Ala Pro Gly Lys Gly Leu Glu Trp Val Ala Arg Ile
305                 310                 315                 320

Arg Ser Lys Tyr Asn Asn Tyr Ala Thr Tyr Tyr Ala Asp Ser Val Lys
                325                 330                 335

Asp Arg Phe Thr Ile Ser Arg Asp Asp Ser Lys Asn Thr Ala Tyr Leu
            340                 345                 350

Gln Met Asn Asn Leu Lys Thr Glu Asp Thr Ala Val Tyr Tyr Cys Val
        355                 360                 365

Arg His Gly Asn Phe Gly Asn Ser Tyr Val Ser Trp Phe Ala Tyr Trp
    370                 375                 380

Gly Gln Gly Thr Leu Val Thr Val Ser Ser Gly Gly Gly Gly Ser Gly
385                 390                 395                 400

Gly Gly Gly Ser Gly Gly Gly Gly Ser Glu Leu Val Val Thr Gln Glu
                405                 410                 415

Pro Ser Leu Thr Val Ser Pro Gly Gly Thr Val Thr Leu Thr Cys Arg
            420                 425                 430

Ser Ser Thr Gly Ala Val Thr Thr Ser Asn Tyr Ala Asn Trp Val Gln
        435                 440                 445

Gln Lys Pro Gly Gln Ala Pro Arg Gly Leu Ile Gly Gly Thr Asn Lys
    450                 455                 460

Arg Ala Pro Gly Thr Pro Ala Arg Phe Ser Gly Ser Leu Leu Gly Gly
465                 470                 475                 480
```

```
Lys Ala Ala Leu Thr Leu Ser Gly Val Gln Pro Glu Asp Glu Ala Glu
                485                 490                 495

Tyr Tyr Cys Ala Leu Trp Tyr Ser Asn Leu Trp Val Phe Gly Gly Gly
            500                 505                 510

Thr Lys Leu Thr Val Leu Asp Lys Thr His Thr Cys Pro Pro Cys Pro
        515                 520                 525

Ala Pro Glu Ala Ala Gly Gly Pro Ser Val Phe Leu Phe Pro Pro Lys
    530                 535                 540

Pro Lys Asp Gln Leu Met Ile Ser Arg Thr Pro Glu Val Thr Cys Val
545                 550                 555                 560

Val Val Asp Val Ser His Glu Asp Pro Glu Val Lys Phe Asn Trp Tyr
                565                 570                 575

Val Asp Gly Val Glu Val His Asn Ala Lys Thr Lys Pro Arg Glu Glu
            580                 585                 590

Gln Tyr Ala Ser Thr Tyr Arg Val Val Ser Val Leu Thr Val Leu His
        595                 600                 605

Gln Asp Trp Leu Asn Gly Lys Glu Tyr Lys Cys Lys Val Ser Asn Lys
    610                 615                 620

Ala Leu Pro Ala Ser Ile Glu Lys Thr Ile Ser Lys Ala Lys Gly Gln
625                 630                 635                 640

Pro Arg Glu Pro Gln Val Tyr Thr Leu Pro Pro Ser Arg Asp Glu Leu
                645                 650                 655

Thr Lys Asn Gln Val Ser Leu Thr Cys Leu Val Lys Gly Phe Tyr Pro
            660                 665                 670

Ser Asp Ile Ala Val Glu Trp Glu Ser Asn Gly Gln Pro Glu Asn Asn
        675                 680                 685

Tyr Lys Thr Thr Pro Pro Val Leu Asp Ser Asp Gly Ser Phe Phe Leu
    690                 695                 700

Tyr Ser Lys Leu Thr Val Asp Lys Ser Arg Trp Gln Gln Gly Asn Val
705                 710                 715                 720

Phe Ser Cys Ser Val Leu His Glu Ala Leu His Asn His Tyr Thr Gln
```

725 730 735

Lys Ser Leu Ser Leu Ser Pro Gly
740

<210> 267
<211> 2232
<212> DNA
<213> Artificial sequence

<220>
<223> bispecific antibody AB2K nucleotide sequence

<400> 267
caagtgcagc tgcagcagcc tggagctgag ctggtgaaac ccggcgcctc cgtcaagatg 60

tcctgtaagg cctccggata caccttcacc agctacaaca tgcactgggt gaaacagacc 120

cctggaaggg gcctggagtg gatcggcgcc atttaccctg gcaacggcga tacctcctat 180

aatcagaagt ttaagggcaa ggccaccctg accgctgata agagcagcag cacagcctac 240

atgcagctgt ccagcctgac ctccgaggac agcgccgtgt actactgtgc tcggagcacc 300

tactacggcg gcgactggta ctttaacgtg tggggagctg gcaccacagt gaccgtgagc 360

gccggaggag gaggatctgg aggaggaggc tccggaggag gaggaagcca gatcgtgctg 420

agccagagcc ccgccatcct gagcgccagc cccggcgaga aggtgaccat gacctgccgc 480

gccagcagca gcgtgagcta catccactgg ttccagcaga agcccggcag cagccccaag 540

ccctggatct acgccaccag caacctggcc agcggcgtgc ccgtgcgctt cagcggcagc 600

ggcagcggca ccagctacag cctgaccatc agccgcgtgg aggccgagga cgccgccacc 660

tactactgcc agcagtggac cagcaacccc cccaccttcg gcggcggcac caagctggag 720

atcaagggtg gcggcggtgg aggatccggc ggtggaggta gcggcggagg cggtagctcc 780

agctctagta aagctccccc tccttccgag gtgcagctgc tggagtccgg aggaggactg 840

gtgcagccag gaggctccct gaagctgagc tgtgctgcct ctggctttac cttcaacaca 900

tatgccatga attgggtgcg gcaggctcca ggcaagggac tggagtgggt ggctaggatc 960

aggtctaagt acaacaatta tgccacctac tatgctgatt ccgtgaagga caggttcacc 1020

atctcccgcg acgatagcaa gaacacagcc tacctgcaga tgaacaatct gaagaccgag 1080

gataccgccg tgtactactg cgtgagacat ggcaactttg gcaatagcta cgtgtcctgg 1140

ttcgcttact ggggacaggg caccctggtc acagtgagct ctggaggagg aggatctgga 1200

ggaggaggct ccggaggagg aggaagcgag ctggtggtga cccaggagcc atctctgaca 1260

gtgtcccccg gcggcacagt gaccctgaca tgtagatcca gcaccggcgc cgtgaccaca 1320

tccaactacg ctaattgggt gcagcagaag ccaggacagg ctccaagggg actgatcgga 1380

ggaaccaaca agagggctcc tggaacacca gctcggttta gcggatctct gctgggaggc 1440

aaggctgccc tgaccctgtc cggagtgcag ccagaggatg aggccgagta ttattgcgct 1500

ctgtggtata gcaatctgtg ggtgttcgga ggaggaacca agctgacagt gctggacaag 1560

acccatacat gcccaccatg ccctgcccct gaagccgccg gaggaccttc cgtgttcctg 1620

ttccctccca agccaaaaga tcagctgatg atctctagaa cccccgaagt cacctgcgtg 1680

gtcgtcgacg tgtcccatga ggaccctgaa gtcaagttca actggtacgt ggacggtgtc 1740

gaagtccaca acgccaagac caagcctagg gaggagcagt atgccagcac ataccgggtg 1800

gtgtctgtgc tgaccgtgct gcatcaggat tggctgaatg gcaaggaata taaatgtaag 1860

gtgagcaata aggctctgcc ggctagcatt gaaaaaacca tttccaaggc taagggccag 1920

cccagggagc ctcaggtcta caccctgcct ccatctagag atgaactgac caaaaaccag 1980

gtgagcctga cttgcctggt caaaggcttc taccccagcg acattgccgt ggagtgggag 2040

tctaatggcc agcctgaaaa taactacaaa actacccctc ctgtgctgga ctctgatggc 2100

tccttctttc tgtactctaa actgaccgtg gacaagtctc gctggcagca gggtaacgtg 2160

ttttcttgct ccgtgctgca cgaggctctg cataaccatt acacccagaa gagcctgtct 2220

ctgtccccag ga 2232

<210> 268
<211> 749
<212> PRT
<213> Artificial sequence

<220>
<223> bispecific antibody AB3K amino acid sequence

<400> 268

Glu Val Gln Leu Val Glu Ser Gly Gly Gly Leu Val Gln Pro Gly Gly
1 5 10 15

Ser Leu Arg Leu Ser Cys Ala Ala Ser Gly Tyr Glu Phe Ser Arg Ser
20 25 30

Trp Met Asn Trp Val Arg Gln Ala Pro Gly Lys Gly Leu Glu Trp Val
35 40 45

Gly Arg Ile Tyr Pro Gly Asp Gly Asp Thr Asn Tyr Ser Gly Lys Phe
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ala Arg Asp Gly Ser Ser Trp Asp Trp Tyr Phe Asp Val Trp Gly Gln
100 105 110

Gly Thr Leu Val Thr Val Ser Ser Gly Gly Gly Gly Ser Gly Gly Gly
115 120 125

Gly Ser Gly Gly Gly Gly Ser Asp Ile Gln Met Thr Gln Ser Pro Ser
130 135 140

Ser Leu Ser Ala Ser Val Gly Asp Arg Val Thr Ile Thr Cys Arg Ser
145 150 155 160

Ser Gln Ser Ile Val His Ser Val Gly Asn Thr Phe Leu Glu Trp Tyr
165 170 175

Gln Gln Lys Pro Gly Lys Ala Pro Lys Leu Leu Ile Tyr Lys Val Ser
180 185 190

Asn Arg Phe Ser Gly Val Pro Ser Arg Phe Ser Gly Ser Gly Ser Gly
195 200 205

Thr Asp Phe Thr Leu Thr Ile Ser Ser Leu Gln Pro Glu Asp Phe Ala
210 215 220

Thr Tyr Tyr Cys Phe Gln Gly Ser Gln Phe Pro Tyr Thr Phe Gly Gln
225 230 235 240

Gly Thr Lys Val Glu Ile Lys Gly Gly Gly Gly Gly Gly Ser Gly Gly
245 250 255

Gly Gly Ser Gly Gly Gly Gly Ser Ser Ser Ser Ser Lys Ala Pro Pro
260 265 270

Pro Ser Glu Val Gln Leu Leu Glu Ser Gly Gly Gly Leu Val Gln Pro
275 280 285

Gly Gly Ser Leu Lys Leu Ser Cys Ala Ala Ser Gly Phe Thr Phe Asn
290 295 300

Thr Tyr Ala Met Asn Trp Val Arg Gln Ala Pro Gly Lys Gly Leu Glu
305 310 315 320

Trp Val Ala Arg Ile Arg Ser Lys Tyr Asn Asn Tyr Ala Thr Tyr Tyr
325 330 335

Ala Asp Ser Val Lys Asp Arg Phe Thr Ile Ser Arg Asp Asp Ser Lys
340 345 350

Asn Thr Ala Tyr Leu Gln Met Asn Asn Leu Lys Thr Glu Asp Thr Ala
        355                 360                 365

Val Tyr Tyr Cys Val Arg His Gly Asn Phe Gly Asn Ser Tyr Val Ser
    370                 375                 380

Trp Phe Ala Tyr Trp Gly Gln Gly Thr Leu Val Thr Val Ser Ser Gly
385                 390                 395                 400

Gly Gly Gly Ser Gly Gly Gly Gly Ser Gly Gly Gly Gly Ser Glu Leu
                405                 410                 415

Val Val Thr Gln Glu Pro Ser Leu Thr Val Ser Pro Gly Gly Thr Val
            420                 425                 430

Thr Leu Thr Cys Arg Ser Ser Thr Gly Ala Val Thr Thr Ser Asn Tyr
        435                 440                 445

Ala Asn Trp Val Gln Gln Lys Pro Gly Gln Ala Pro Arg Gly Leu Ile
    450                 455                 460

Gly Gly Thr Asn Lys Arg Ala Pro Gly Thr Pro Ala Arg Phe Ser Gly
465                 470                 475                 480

Ser Leu Leu Gly Gly Lys Ala Ala Leu Thr Leu Ser Gly Val Gln Pro
                485                 490                 495

Glu Asp Glu Ala Glu Tyr Tyr Cys Ala Leu Trp Tyr Ser Asn Leu Trp
            500                 505                 510

Val Phe Gly Gly Gly Thr Lys Leu Thr Val Leu Asp Lys Thr His Thr
        515                 520                 525

Cys Pro Pro Cys Pro Ala Pro Glu Ala Ala Gly Gly Pro Ser Val Phe
    530                 535                 540

Leu Phe Pro Pro Lys Pro Lys Asp Gln Leu Met Ile Ser Arg Thr Pro
545                 550                 555                 560

Glu Val Thr Cys Val Val Val Asp Val Ser His Glu Asp Pro Glu Val
                565                 570                 575

Lys Phe Asn Trp Tyr Val Asp Gly Val Glu Val His Asn Ala Lys Thr
            580                 585                 590

Lys Pro Arg Glu Glu Gln Tyr Ala Ser Thr Tyr Arg Val Val Ser Val

595 600 605

Leu Thr Val Leu His Gln Asp Trp Leu Asn Gly Lys Glu Tyr Lys Cys
610 615 620

Lys Val Ser Asn Lys Ala Leu Pro Ala Ser Ile Glu Lys Thr Ile Ser
625 630 635 640

Lys Ala Lys Gly Gln Pro Arg Glu Pro Gln Val Tyr Thr Leu Pro Pro
645 650 655

Ser Arg Asp Glu Leu Thr Lys Asn Gln Val Ser Leu Thr Cys Leu Val
660 665 670

Lys Gly Phe Tyr Pro Ser Asp Ile Ala Val Glu Trp Glu Ser Asn Gly
675 680 685

Gln Pro Glu Asn Asn Tyr Lys Thr Thr Pro Pro Val Leu Asp Ser Asp
690 695 700

Gly Ser Phe Phe Leu Tyr Ser Lys Leu Thr Val Asp Lys Ser Arg Trp
705 710 715 720

Gln Gln Gly Asn Val Phe Ser Cys Ser Val Leu His Glu Ala Leu His
725 730 735

Asn His Tyr Thr Gln Lys Ser Leu Ser Leu Ser Pro Gly
740 745

<210> 269
<211> 2247
<212> DNA
<213> Artificial sequence

<220>
<223> bispecific antibody AB3K nucleotide sequence

<400> 269
gaggtgcagc tggtggagtc cggaggagga ctggtgcagc caggaggaag cctgaggctg 60

tcttgtgctg cctccggcta tgagttttct cgctcctgga tgaactgggt gaggcaggct 120

ccaggcaagg gactggagtg ggtgggaagg atctaccctg gcgatggcga cacaaattat 180

tccggcaagt ttaagggcag attcaccatc agcgccgaca catctaagaa caccgcttac 240

ctgcagatga atagcctgcg ggctgaggac accgccgtgt attactgcgc tagagatggc 300

tccagctggg actggtattt cgacgtgtgg ggccagggca cactggtgac cgtgtcttcc 360

ggaggaggag gatctggagg aggaggctcc ggaggaggag gaagcgacat ccagatgacc 420

cagagcccaa gctctctgag cgcctctgtg ggcgatcgcg tgacaatcac ctgtaggtcc 480

agccagtcca tcgtgcacag cgtgggcaac acctttctgg agtggtacca gcagaagccc 540

ggcaaggctc caaaactgct gatctataag gtgtctaatc ggttttccgg agtgccaagc 600

agattctccg gaagcggctc tggcacagat ttcacactga ccatctcttc cctgcagcct 660

gaggactttg ccacctatta ctgcttccag ggctctcagt ttccatacac cttcggccag 720

ggcacaaagg tggagatcaa gggtggcggc ggtggaggat ccggcggtgg aggtagcggc 780

ggaggcggta gctccagctc tagtaaagct ccccctcctt ccgaggtgca gctgctggag 840

tccggaggag gactggtgca gccaggaggc tccctgaagc tgagctgtgc tgcctctggc 900

tttaccttca acacatatgc catgaattgg gtgcggcagg ctccaggcaa gggactggag 960

tgggtggcta ggatcaggtc taagtacaac aattatgcca cctactatgc tgattccgtg 1020

aaggacaggt tcaccatctc ccgcgacgat agcaagaaca cagcctacct gcagatgaac 1080

aatctgaaga ccgaggatac cgccgtgtac tactgcgtga gacatggcaa ctttggcaat 1140

agctacgtgt cctggttcgc ttactgggga cagggcaccc tggtcacagt gagctctgga 1200

ggaggaggat ctggaggagg aggctccgga ggaggaggaa gcgagctggt ggtgacccag 1260

gagccatctc tgacagtgtc ccccggcggc acagtgaccc tgacatgtag atccagcacc 1320

ggcgccgtga ccacatccaa ctacgctaat tgggtgcagc agaagccagg acaggctcca 1380

aggggactga tcggaggaac caacaagagg gctcctggaa caccagctcg gtttagcgga 1440

tctctgctgg gaggcaaggc tgccctgacc ctgtccggag tgcagccaga ggatgaggcc 1500

gagtattatt gcgctctgtg gtatagcaat ctgtgggtgt tcggaggagg aaccaagctg 1560

acagtgctgg acaagaccca tacatgccca ccatgccctg cccctgaagc cgccggagga 1620

ccttccgtgt tcctgttccc tcccaagcca aaagatcagc tgatgatctc tagaaccccc 1680

gaagtcacct gcgtggtcgt cgacgtgtcc catgaggacc ctgaagtcaa gttcaactgg 1740

tacgtggacg gtgtcgaagt ccacaacgcc aagaccaagc ctagggagga gcagtatgcc 1800

agcacatacc gggtggtgtc tgtgctgacc gtgctgcatc aggattggct gaatggcaag 1860

gaatataaat gtaaggtgag caataaggct ctgccggcta gcattgaaaa aaccatttcc 1920

aaggctaagg gccagcccag ggagcctcag gtctacaccc tgcctccatc tagagatgaa 1980

ctgaccaaaa accaggtgag cctgacttgc ctggtcaaag gcttctaccc cagcgacatt 2040

gccgtggagt gggagtctaa tggccagcct gaaaataact acaaaactac ccctcctgtg 2100

ctggactctg atggctcctt ctttctgtac tctaaactga ccgtggacaa gtctcgctgg 2160

cagcagggta acgtgttttc ttgctccgtg ctgcacgagg ctctgcataa ccattacacc 2220

cagaagagcc tgtctctgtc cccagga 2247

<210> 270

<211> 745
<212> PRT
<213> Artificial sequence

<220>
<223> bispecific antibody AB4K amino acid sequence

<400> 270

Gln Ile Gln Leu Gln Gln Ser Gly Pro Glu Val Val Lys Pro Gly Ala
1               5                   10                  15

Ser Val Lys Ile Ser Cys Lys Ala Ser Gly Tyr Thr Phe Thr Asp Tyr
            20                  25                  30

Tyr Ile Thr Trp Val Lys Gln Lys Pro Gly Gln Gly Leu Glu Trp Ile
        35                  40                  45

Gly Trp Ile Tyr Pro Gly Ser Gly Asn Thr Lys Tyr Asn Glu Lys Phe
    50                  55                  60

Lys Gly Lys Ala Thr Leu Thr Val Asp Thr Ser Ser Ser Thr Ala Phe
65                  70                  75                  80

Met Gln Leu Ser Ser Leu Thr Ser Glu Asp Thr Ala Val Tyr Phe Cys
                85                  90                  95

Ala Asn Tyr Gly Asn Tyr Trp Phe Ala Tyr Trp Gly Gln Gly Thr Gln
            100                 105                 110

Val Thr Val Ser Ala Gly Gly Gly Gly Ser Gly Gly Gly Gly Ser Gly
        115                 120                 125

Gly Gly Gly Ser Asp Ile Val Leu Thr Gln Ser Pro Ala Ser Leu Ala
    130                 135                 140

Val Ser Leu Gly Gln Arg Ala Thr Ile Ser Cys Lys Ala Ser Gln Ser
145                 150                 155                 160

Val Asp Phe Asp Gly Asp Ser Tyr Met Asn Trp Tyr Gln Gln Lys Pro
                165                 170                 175

Gly Gln Pro Pro Lys Val Leu Ile Tyr Ala Ala Ser Asn Leu Glu Ser
            180                 185                 190

Gly Ile Pro Ala Arg Phe Ser Gly Ser Gly Ser Gly Thr Asp Phe Thr
        195                 200                 205

Leu Asn Ile His Pro Val Glu Glu Glu Asp Ala Ala Thr Tyr Tyr Cys
    210                 215                 220

Gln Gln Ser Asn Glu Asp Pro Trp Thr Phe Gly Gly Gly Thr Lys Leu
225 230 235 240

Glu Ile Lys Gly Gly Gly Gly Gly Gly Ser Gly Gly Gly Gly Ser Gly
245 250 255

Gly Gly Gly Ser Ser Ser Ser Ser Lys Ala Pro Pro Pro Ser Glu Val
260 265 270

Gln Leu Leu Glu Ser Gly Gly Gly Leu Val Gln Pro Gly Gly Ser Leu
275 280 285

Lys Leu Ser Cys Ala Ala Ser Gly Phe Thr Phe Asn Thr Tyr Ala Met
290 295 300

Asn Trp Val Arg Gln Ala Pro Gly Lys Gly Leu Glu Trp Val Ala Arg
305 310 315 320

Ile Arg Ser Lys Tyr Asn Asn Tyr Ala Thr Tyr Tyr Ala Asp Ser Val
325 330 335

Lys Asp Arg Phe Thr Ile Ser Arg Asp Asp Ser Lys Asn Thr Ala Tyr
340 345 350

Leu Gln Met Asn Asn Leu Lys Thr Glu Asp Thr Ala Val Tyr Tyr Cys
355 360 365

Val Arg His Gly Asn Phe Gly Asn Ser Tyr Val Ser Trp Phe Ala Tyr
370 375 380

Trp Gly Gln Gly Thr Leu Val Thr Val Ser Ser Gly Gly Gly Gly Ser
385 390 395 400

Gly Gly Gly Gly Ser Gly Gly Gly Gly Ser Glu Leu Val Val Thr Gln
405 410 415

Glu Pro Ser Leu Thr Val Ser Pro Gly Gly Thr Val Thr Leu Thr Cys
420 425 430

Arg Ser Ser Thr Gly Ala Val Thr Thr Ser Asn Tyr Ala Asn Trp Val
435 440 445

Gln Gln Lys Pro Gly Gln Ala Pro Arg Gly Leu Ile Gly Gly Thr Asn
450 455 460

Lys Arg Ala Pro Gly Thr Pro Ala Arg Phe Ser Gly Ser Leu Leu Gly

465 470 475 480

Gly Lys Ala Ala Leu Thr Leu Ser Gly Val Gln Pro Glu Asp Glu Ala
485 490 495

Glu Tyr Tyr Cys Ala Leu Trp Tyr Ser Asn Leu Trp Val Phe Gly Gly
500 505 510

Gly Thr Lys Leu Thr Val Leu Asp Lys Thr His Thr Cys Pro Pro Cys
515 520 525

Pro Ala Pro Glu Ala Ala Gly Gly Pro Ser Val Phe Leu Phe Pro Pro
530 535 540

Lys Pro Lys Asp Gln Leu Met Ile Ser Arg Thr Pro Glu Val Thr Cys
545 550 555 560

Val Val Val Asp Val Ser His Glu Asp Pro Glu Val Lys Phe Asn Trp
565 570 575

Tyr Val Asp Gly Val Glu Val His Asn Ala Lys Thr Lys Pro Arg Glu
580 585 590

Glu Gln Tyr Ala Ser Thr Tyr Arg Val Val Ser Val Leu Thr Val Leu
595 600 605

His Gln Asp Trp Leu Asn Gly Lys Glu Tyr Lys Cys Lys Val Ser Asn
610 615 620

Lys Ala Leu Pro Ala Ser Ile Glu Lys Thr Ile Ser Lys Ala Lys Gly
625 630 635 640

Gln Pro Arg Glu Pro Gln Val Tyr Thr Leu Pro Pro Ser Arg Asp Glu
645 650 655

Leu Thr Lys Asn Gln Val Ser Leu Thr Cys Leu Val Lys Gly Phe Tyr
660 665 670

Pro Ser Asp Ile Ala Val Glu Trp Glu Ser Asn Gly Gln Pro Glu Asn
675 680 685

Asn Tyr Lys Thr Thr Pro Pro Val Leu Asp Ser Asp Gly Ser Phe Phe
690 695 700

Leu Tyr Ser Lys Leu Thr Val Asp Lys Ser Arg Trp Gln Gln Gly Asn
705 710 715 720

Val Phe Ser Cys Ser Val Leu His Glu Ala Leu His Asn His Tyr Thr
725 730 735

Gln Lys Ser Leu Ser Leu Ser Pro Gly
740 745

<210> 271
<211> 2235
<212> DNA
<213> Artificial sequence

<220>
<223> bispecific antibody AB4K nucleotide sequence

<400> 271
cagatccagc tgcagcagtc cggaccagag gtggtgaagc caggagcctc tgtgaagatc 60

tcctgtaagg ctagcggcta cacattcacc gactattaca tcacatgggt gaagcagaag 120

ccaggccagg gcctggagtg gatcggatgg atctaccccg gcagcggcaa caccaagtat 180

aatgagaagt ttaagggcaa ggccacactg accgtggaca caagctcttc caccgctttc 240

atgcagctgt cttccctgac atctgaggat accgccgtgt acttttgcgc taactacggc 300

aattattggt tcgcctattg ggacagggga acacaggtga ccgtgtccgc tggaggagga 360

ggatctggag gaggaggctc cggaggagga ggaagcgata tcgtgctgac acagtctcca 420

gcttccctgg ccgtgtccct gggccagaga gctaccatca gctgtaaagc ctcccagagc 480

gtggattttg acggcgattc ttacatgaat tggtaccagc agaaacctgg ccagcctcca 540

aaggtgctga tctacgctgc ctctaatctg gagtccggca tcccagccag attttctggt 600

tctggcagcg gcacagattt caccctgaac attcatccag tggaggagga ggacgctgct 660

acctattact gccagcagtc caatgaggac ccttggacct tcggcggtgg taccaaactg 720

gagatcaagg gtggcggcgg tggaggatcc ggcggtggag gtagcggcgg aggcggtagc 780

tccagctcta gtaaagctcc ccctccttcc gaggtgcagc tgctggagtc cggaggagga 840

ctggtgcagc caggaggctc cctgaagctg agctgtgctg cctctggctt taccttcaac 900

acatatgcca tgaattgggt gcggcaggct ccaggcaagg gactggagtg ggtggctagg 960

atcaggtcta agtacaacaa ttatgccacc tactatgctg attccgtgaa ggacaggttc 1020

accatctccc gcgacgatag caagaacaca gcctacctgc agatgaacaa tctgaagacc 1080

gaggataccg ccgtgtacta ctgcgtgaga catggcaact ttggcaatag ctacgtgtcc 1140

tggttcgctt actggggaca gggcaccctg gtcacagtga gctctggagg aggaggatct 1200

ggaggaggag gctccggagg aggaggaagc gagctggtgg tgacccagga gccatctctg 1260

acagtgtccc ccggcggcac agtgaccctg acatgtagat ccagcaccgg cgccgtgacc 1320

acatccaact acgctaattg ggtgcagcag aagccaggac aggctccaag gggactgatc 1380

```
ggaggaacca acaagagggc tcctggaaca ccagctcggt ttagcggatc tctgctggga     1440
ggcaaggctg ccctgaccct gtccggagtg cagccagagg atgaggccga gtattattgc     1500
gctctgtggt atagcaatct gtgggtgttc ggaggaggaa ccaagctgac agtgctggac     1560
aagacccata catgcccacc atgccctgcc cctgaagccg ccggaggacc ttccgtgttc     1620
ctgttccctc ccaagccaaa agatcagctg atgatctcta gaaccccga agtcacctgc      1680
gtggtcgtcg acgtgtccca tgaggaccct gaagtcaagt tcaactggta cgtggacggt     1740
gtcgaagtcc acaacgccaa gaccaagcct agggaggagc agtatgccag cacataccgg     1800
gtggtgtctg tgctgaccgt gctgcatcag gattggctga atggcaagga atataaatgt     1860
aaggtgagca ataaggctct gccggctagc attgaaaaa ccatttccaa ggctaagggc      1920
cagcccaggg agcctcaggt ctacaccctg cctccatcta gagatgaact gaccaaaaac     1980
caggtgagcc tgacttgcct ggtcaaaggc ttctacccca gcgacattgc cgtggagtgg     2040
gagtctaatg gccagcctga aaataactac aaaactaccc ctcctgtgct ggactctgat     2100
ggctccttct ttctgtactc taaactgacc gtggacaagt ctcgctggca gcagggtaac     2160
gtgttttctt gctccgtgct gcacgaggct ctgcataacc attacaccca gaagagcctg     2220
tctctgtccc cagga                                                      2235
```

<210> 272
<211> 751
<212> PRT
<213> Artificial sequence

<220>
<223> bispecific antibody AB5K amino acid sequence

<400> 272

```
Glu Val Gln Leu Leu Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

Ala Lys Asp Met Gly Trp Gly Ser Gly Trp Arg Pro Tyr Tyr Tyr Tyr
100 105 110

Gly Met Asp Val Trp Gly Gln Gly Thr Thr Val Thr Val Ser Ser Gly
115 120 125

Gly Gly Gly Ser Gly Gly Gly Gly Ser Gly Gly Gly Gly Ser Glu Leu
130 135 140

Gln Met Thr Gln Ser Pro Ser Ser Leu Ser Ala Ser Val Gly Asp Arg
145 150 155 160

Val Thr Ile Thr Cys Arg Thr Ser Gln Ser Ile Ser Ser Tyr Leu Asn
165 170 175

Trp Tyr Gln Gln Lys Pro Gly Gln Pro Pro Lys Leu Leu Ile Tyr Trp
180 185 190

Ala Ser Thr Arg Glu Ser Gly Val Pro Asp Arg Phe Ser Gly Ser Gly
195 200 205

Ser Gly Thr Asp Phe Thr Leu Thr Ile Ser Ser Leu Gln Pro Glu Asp
210 215 220

Ser Ala Thr Tyr Tyr Cys Gln Gln Ser Tyr Asp Ile Pro Tyr Thr Phe
225 230 235 240

Gly Gln Gly Thr Lys Leu Glu Ile Lys Gly Gly Gly Gly Gly Gly Ser
245 250 255

Gly Gly Gly Gly Ser Gly Gly Gly Gly Ser Ser Ser Ser Ser Lys Ala
260 265 270

Pro Pro Pro Ser Glu Val Gln Leu Leu Glu Ser Gly Gly Gly Leu Val
275 280 285

Gln Pro Gly Gly Ser Leu Lys Leu Ser Cys Ala Ala Ser Gly Phe Thr
290 295 300

Phe Asn Thr Tyr Ala Met Asn Trp Val Arg Gln Ala Pro Gly Lys Gly
305 310 315 320

Leu Glu Trp Val Ala Arg Ile Arg Ser Lys Tyr Asn Asn Tyr Ala Thr
325 330 335

Tyr Tyr Ala Asp Ser Val Lys Asp Arg Phe Thr Ile Ser Arg Asp Asp

340 345 350

Ser Lys Asn Thr Ala Tyr Leu Gln Met Asn Asn Leu Lys Thr Glu Asp
355 360 365

Thr Ala Val Tyr Tyr Cys Val Arg His Gly Asn Phe Gly Asn Ser Tyr
370 375 380

Val Ser Trp Phe Ala Tyr Trp Gly Gln Gly Thr Leu Val Thr Val Ser
385 390 395 400

Ser Gly Gly Gly Gly Ser Gly Gly Gly Gly Ser Gly Gly Gly Gly Ser
405 410 415

Glu Leu Val Val Thr Gln Glu Pro Ser Leu Thr Val Ser Pro Gly Gly
420 425 430

Thr Val Thr Leu Thr Cys Arg Ser Ser Thr Gly Ala Val Thr Thr Ser
435 440 445

Asn Tyr Ala Asn Trp Val Gln Gln Lys Pro Gly Gln Ala Pro Arg Gly
450 455 460

Leu Ile Gly Gly Thr Asn Lys Arg Ala Pro Gly Thr Pro Ala Arg Phe
465 470 475 480

Ser Gly Ser Leu Leu Gly Gly Lys Ala Ala Leu Thr Leu Ser Gly Val
485 490 495

Gln Pro Glu Asp Glu Ala Glu Tyr Tyr Cys Ala Leu Trp Tyr Ser Asn
500 505 510

Leu Trp Val Phe Gly Gly Gly Thr Lys Leu Thr Val Leu Asp Lys Thr
515 520 525

His Thr Cys Pro Pro Cys Pro Ala Pro Glu Ala Ala Gly Gly Pro Ser
530 535 540

Val Phe Leu Phe Pro Pro Lys Pro Lys Asp Gln Leu Met Ile Ser Arg
545 550 555 560

Thr Pro Glu Val Thr Cys Val Val Val Asp Val Ser His Glu Asp Pro
565 570 575

Glu Val Lys Phe Asn Trp Tyr Val Asp Gly Val Glu Val His Asn Ala
580 585 590

Lys Thr Lys Pro Arg Glu Glu Gln Tyr Ala Ser Thr Tyr Arg Val Val
595 600 605

Ser Val Leu Thr Val Leu His Gln Asp Trp Leu Asn Gly Lys Glu Tyr
610 615 620

Lys Cys Lys Val Ser Asn Lys Ala Leu Pro Ala Ser Ile Glu Lys Thr
625 630 635 640

Ile Ser Lys Ala Lys Gly Gln Pro Arg Glu Pro Gln Val Tyr Thr Leu
645 650 655

Pro Pro Ser Arg Asp Glu Leu Thr Lys Asn Gln Val Ser Leu Thr Cys
660 665 670

Leu Val Lys Gly Phe Tyr Pro Ser Asp Ile Ala Val Glu Trp Glu Ser
675 680 685

Asn Gly Gln Pro Glu Asn Asn Tyr Lys Thr Thr Pro Pro Val Leu Asp
690 695 700

Ser Asp Gly Ser Phe Phe Leu Tyr Ser Lys Leu Thr Val Asp Lys Ser
705 710 715 720

Arg Trp Gln Gln Gly Asn Val Phe Ser Cys Ser Val Leu His Glu Ala
725 730 735

Leu His Asn His Tyr Thr Gln Lys Ser Leu Ser Leu Ser Pro Gly
740 745 750

<210> 273
<211> 2253
<212> DNA
<213> Artificial sequence

<220>
<223> bispecific antibody AB5K nucleotide sequence

<400> 273
gaggtgcagc tgctggagtc tggaggagga gtggtgcagc caggcaggtc cctgaggctg 60

agctgtgctg cttctggctt cacctttagc tcttatggaa tgcactgggt gcggcaggct 120

ccaggcaagg gactggagtg ggtggccgtg atctcctacg acggcagcaa caagtactat 180

gctgattccg tgaagggcag attcacaatc tctcgcgaca actccaagaa taccctgtac 240

ctgcagatga actctctgag agctgaggac accgccgtgt actattgcgc caaggatatg 300

ggatggggct ccggatggag gccttactat tactatggca tggacgtgtg gggccagggc 360

accacagtga cagtgtcctc cggaggagga ggatctggag gaggaggctc cggaggagga 420

```
ggaagcgagc tgcagatgac acagagccca tcttccctga gcgcctctgt gggcgataga      480
gtgaccatca catgtcgcac ctcccagagc atcagctctt atctgaattg gtatcagcag      540
aagccaggcc agccacccaa gctgctgatc tactgggcta gcacaaggga gtctggagtg      600
ccagaccggt tctctggctc cggaagcgga accgacttca ccctgacaat ctccagcctg      660
cagccagagg actccgccac atactattgc cagcagagct atgacatccc ctacacattc      720
ggccagggta ctaagctgga gatcaagggt ggcggcggtg gaggatccgg cggtggaggt      780
agcggcggag gcggtagctc cagctctagt aaagctcccc ctccttccga ggtgcagctg      840
ctggagtccg gaggaggact ggtgcagcca ggaggctccc tgaagctgag ctgtgctgcc      900
tctggcttta ccttcaacac atatgccatg aattgggtgc ggcaggctcc aggcaaggga      960
ctggagtggg tggctaggat caggtctaag tacaacaatt atgccaccta ctatgctgat     1020
tccgtgaagg acaggttcac catctcccgc gacgatagca agaacacagc ctacctgcag     1080
atgaacaatc tgaagaccga ggataccgcc gtgtactact gcgtgagaca tggcaacttt     1140
ggcaatagct acgtgtcctg gttcgcttac tggggacagg gcaccctggt cacagtgagc     1200
tctggaggag gaggatctgg aggaggaggc tccggaggag gaggaagcga gctggtggtg     1260
acccaggagc catctctgac agtgtccccc ggcggcacag tgaccctgac atgtagatcc     1320
agcaccggcg ccgtgaccac atccaactac gctaattggg tgcagcagaa gccaggacag     1380
gctccaaggg gactgatcgg aggaaccaac aagagggctc ctggaacacc agctcggttt     1440
agcggatctc tgctgggagg caaggctgcc ctgaccctgt ccggagtgca gccagaggat     1500
gaggccgagt attattgcgc tctgtggtat agcaatctgt gggtgttcgg aggaggaacc     1560
aagctgacag tgctggacaa gacccataca tgcccaccat gccctgcccc tgaagccgcc     1620
ggaggacctt ccgtgttcct gttccctccc aagccaaaag atcagctgat gatctctaga     1680
acccccgaag tcacctgcgt ggtcgtcgac gtgtcccatg aggaccctga agtcaagttc     1740
aactggtacg tggacggtgt cgaagtccac aacgccaaga ccaagcctag ggaggagcag     1800
tatgccagca cataccgggt ggtgtctgtg ctgaccgtgc tgcatcagga ttggctgaat     1860
ggcaaggaat ataaatgtaa ggtgagcaat aaggctctgc cggctagcat tgaaaaaacc     1920
atttccaagg ctaagggcca gcccagggag cctcaggtct acaccctgcc tccatctaga     1980
gatgaactga ccaaaaacca ggtgagcctg acttgcctgg tcaaaggctt ctaccccagc     2040
gacattgccg tggagtggga gtctaatggc cagcctgaaa ataactacaa aactacccct     2100
cctgtgctgg actctgatgg ctccttcttt ctgtactcta aactgaccgt ggacaagtct     2160
cgctggcagc agggtaacgt gttttcttgc tccgtgctgc acgaggctct gcataaccat     2220
tacacccaga agagcctgtc tctgtcccca gga                                  2253
```

<210> 274
<211> 749
<212> PRT
<213> Artificial sequence

<220>
<223> bispecific antibody AB6K amino acid sequence

<400> 274

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Met His Trp Val Arg Gln Ala Pro Gly Lys Gly Leu Glu Trp Val
        35                  40                  45

Ala Arg Ile Asp Pro Ala Asn Gly Asn Ser Lys Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Asp Ile Gln Leu Thr Gln Ser Pro
    130                 135                 140

Ser Ser Leu Ser Ala Ser Val Gly Asp Arg Val Thr Ile Thr Cys Arg
145                 150                 155                 160

Ala Gly Glu Ser Val Asp Ile Phe Gly Val Gly Phe Leu His Trp Tyr
                165                 170                 175

Gln Gln Lys Pro Gly Lys Ala Pro Lys Leu Leu Ile Tyr Arg Ala Ser
            180                 185                 190

Asn Leu Glu Ser Gly Val Pro Ser Arg Phe Ser Gly Ser Gly Ser Arg
        195                 200                 205

Thr Asp Phe Thr Leu Thr Ile Ser Ser Leu Gln Pro Glu Asp Phe Ala

210 215 220

Thr Tyr Tyr Cys Gln Gln Thr Asn Glu Asp Pro Tyr Thr Phe Gly Gln
225 230 235 240

Gly Thr Lys Val Glu Ile Lys Gly Gly Gly Gly Gly Gly Ser Gly Gly
245 250 255

Gly Gly Ser Gly Gly Gly Gly Ser Ser Ser Ser Ser Lys Ala Pro Pro
260 265 270

Pro Ser Glu Val Gln Leu Leu Glu Ser Gly Gly Gly Leu Val Gln Pro
275 280 285

Gly Gly Ser Leu Lys Leu Ser Cys Ala Ala Ser Gly Phe Thr Phe Asn
290 295 300

Thr Tyr Ala Met Asn Trp Val Arg Gln Ala Pro Gly Lys Gly Leu Glu
305 310 315 320

Trp Val Ala Arg Ile Arg Ser Lys Tyr Asn Asn Tyr Ala Thr Tyr Tyr
325 330 335

Ala Asp Ser Val Lys Asp Arg Phe Thr Ile Ser Arg Asp Asp Ser Lys
340 345 350

Asn Thr Ala Tyr Leu Gln Met Asn Asn Leu Lys Thr Glu Asp Thr Ala
355 360 365

Val Tyr Tyr Cys Val Arg His Gly Asn Phe Gly Asn Ser Tyr Val Ser
370 375 380

Trp Phe Ala Tyr Trp Gly Gln Gly Thr Leu Val Thr Val Ser Ser Gly
385 390 395 400

Gly Gly Gly Ser Gly Gly Gly Gly Ser Gly Gly Gly Gly Ser Glu Leu
405 410 415

Val Val Thr Gln Glu Pro Ser Leu Thr Val Ser Pro Gly Gly Thr Val
420 425 430

Thr Leu Thr Cys Arg Ser Ser Thr Gly Ala Val Thr Thr Ser Asn Tyr
435 440 445

Ala Asn Trp Val Gln Gln Lys Pro Gly Gln Ala Pro Arg Gly Leu Ile
450 455 460

Gly Gly Thr Asn Lys Arg Ala Pro Gly Thr Pro Ala Arg Phe Ser Gly
465 470 475 480

Ser Leu Leu Gly Gly Lys Ala Ala Leu Thr Leu Ser Gly Val Gln Pro
485 490 495

Glu Asp Glu Ala Glu Tyr Tyr Cys Ala Leu Trp Tyr Ser Asn Leu Trp
500 505 510

Val Phe Gly Gly Gly Thr Lys Leu Thr Val Leu Asp Lys Thr His Thr
515 520 525

Cys Pro Pro Cys Pro Ala Pro Glu Ala Ala Gly Gly Pro Ser Val Phe
530 535 540

Leu Phe Pro Pro Lys Pro Lys Asp Gln Leu Met Ile Ser Arg Thr Pro
545 550 555 560

Glu Val Thr Cys Val Val Val Asp Val Ser His Glu Asp Pro Glu Val
565 570 575

Lys Phe Asn Trp Tyr Val Asp Gly Val Glu Val His Asn Ala Lys Thr
580 585 590

Lys Pro Arg Glu Glu Gln Tyr Ala Ser Thr Tyr Arg Val Val Ser Val
595 600 605

Leu Thr Val Leu His Gln Asp Trp Leu Asn Gly Lys Glu Tyr Lys Cys
610 615 620

Lys Val Ser Asn Lys Ala Leu Pro Ala Ser Ile Glu Lys Thr Ile Ser
625 630 635 640

Lys Ala Lys Gly Gln Pro Arg Glu Pro Gln Val Tyr Thr Leu Pro Pro
645 650 655

Ser Arg Asp Glu Leu Thr Lys Asn Gln Val Ser Leu Thr Cys Leu Val
660 665 670

Lys Gly Phe Tyr Pro Ser Asp Ile Ala Val Glu Trp Glu Ser Asn Gly
675 680 685

Gln Pro Glu Asn Asn Tyr Lys Thr Thr Pro Pro Val Leu Asp Ser Asp
690 695 700

Gly Ser Phe Phe Leu Tyr Ser Lys Leu Thr Val Asp Lys Ser Arg Trp
705 710 715 720

Gln Gln Gly Asn Val Phe Ser Cys Ser Val Leu His Glu Ala Leu His
725 730 735

Asn His Tyr Thr Gln Lys Ser Leu Ser Leu Ser Pro Gly
740 745

<210> 275
<211> 2247
<212> DNA
<213> Artificial sequence

<220>
<223> bispecific antibody AB6K nucleotide sequence

<400> 275
gaggtgcagc tggtggagtc tggaggagga ctggtgcagc caggaggatc tctgaggctg 60

tcctgcgccg ctagcggctt caacatcaag gatacctaca tgcattgggt gagacaggct 120

ccaggcaagg gactggagtg ggtggctagg atcgacccag ctaacggcaa tagcaagtac 180

gctgattctg tgaagggcag gttcaccatc tctgccgaca cctccaagaa cacagcttat 240

ctgcagatga actccctgcg ggccgaggat acagccgtgt actattgcgc ccctttttggc 300

tactatgtga gcgactacgc catggcttat tggggccagg gcaccctggt gacagtgtcc 360

tctggaggag gaggatctgg aggaggaggc tccggaggag gaggaagcga catccagctg 420

acccagtctc catcttccct gtctgcctcc gtgggcgata gggtgaccat cacatgtcgg 480

gctggcgagt ccgtggacat cttcggcgtg ggctttctgc actggtacca gcagaagccc 540

ggcaaggccc ctaagctgct gatctatagg gctagcaacc tggagtctgg cgtgccttcc 600

agattcagcg gctctggctc ccgcacagac tttaccctga caatcagctc tctgcagcct 660

gaggatttcg ccacctacta ttgccagcag acaaatgagg acccatacac ctttggccag 720

ggcacaaagg tggagatcaa gggtggcggc ggtggaggat ccggcggtgg aggtagcggc 780

ggaggcggta gctccagctc tagtaaagct ccccctcctt ccgaggtgca gctgctggag 840

tccggaggag gactggtgca gccaggaggc tccctgaagc tgagctgtgc tgcctctggc 900

tttaccttca acacatatgc catgaattgg gtgcggcagg ctccaggcaa gggactggag 960

tgggtggcta ggatcaggtc taagtacaac aattatgcca cctactatgc tgattccgtg 1020

aaggacaggt tcaccatctc ccgcgacgat agcaagaaca cagcctacct gcagatgaac 1080

aatctgaaga ccgaggatac cgccgtgtac tactgcgtga gacatggcaa ctttggcaat 1140

agctacgtgt cctggttcgc ttactgggga cagggcaccc tggtcacagt gagctctgga 1200

ggaggaggat ctggaggagg aggctccgga ggaggaggaa gcgagctggt ggtgacccag 1260

gagccatctc tgacagtgtc ccccggcggc acagtgaccc tgacatgtag atccagcacc 1320

ggcgccgtga ccacatccaa ctacgctaat tgggtgcagc agaagccagg acaggctcca 1380

aggggactga tcggaggaac caacaagagg gctcctggaa caccagctcg gtttagcgga 1440

tctctgctgg gaggcaaggc tgccctgacc ctgtccggag tgcagccaga ggatgaggcc 1500

gagtattatt gcgctctgtg gtatagcaat ctgtgggtgt tcggaggagg aaccaagctg 1560

acagtgctgg acaagaccca tacatgccca ccatgccctg cccctgaagc cgccggagga 1620

ccttccgtgt tcctgttccc tcccaagcca aaagatcagc tgatgatctc tagaaccccc 1680

gaagtcacct gcgtggtcgt cgacgtgtcc catgaggacc ctgaagtcaa gttcaactgg 1740

tacgtggacg gtgtcgaagt ccacaacgcc aagaccaagc ctagggagga gcagtatgcc 1800

agcacatacc gggtggtgtc tgtgctgacc gtgctgcatc aggattggct gaatggcaag 1860

gaatataaat gtaaggtgag caataaggct ctgccggcta gcattgaaaa aaccatttcc 1920

aaggctaagg gccagcccag ggagcctcag gtctacaccc tgcctccatc tagagatgaa 1980

ctgaccaaaa accaggtgag cctgacttgc ctggtcaaag gcttctaccc cagcgacatt 2040

gccgtggagt gggagtctaa tggccagcct gaaaataact acaaaactac ccctcctgtg 2100

ctggactctg atggctcctt ctttctgtac tctaaactga ccgtggacaa gtctcgctgg 2160

cagcagggta acgtgttttc ttgctccgtg ctgcacgagg ctctgcataa ccattacacc 2220

cagaagagcc tgtctctgtc cccagga 2247

<210> 276
<211> 2232
<212> DNA
<213> Artificial sequence

<220>
<223> bispecific antibody AB7K7 nucleotide sequence

<400> 276
gaggtgcagc tcgtagaatc aggaggtggc ctggtccagc ccgggggatc actgaggctg 60

tcctgtgctg cctcaggctt caacattaag gatacctaca tccattgggt ccgccaggct 120

ccaggaaaag ggcttgagtg ggtggctagg atctatccaa caaatggata tacaaggtat 180

gccgactctg tcaaaggccg ctttactatt tcagccgata caagcaagaa cactgcttac 240

ttgcagatga actctctgcg tgctgaagac acagcagtat actattgctc aaggtggggt 300

ggtgacgggt tttacgcaat ggattactgg ggtcagggca ccttggtgac cgtatcttct 360

ggaggaggag gatctggagg aggaggctcc ggaggaggag gaagcgacat tcaaatgaca 420

caatccccct catccctcag cgcctccgtg ggggataggg tcacaataac ctgccgtgcc 480

tcacaggacg tgaacaccgc agtggcatgg tatcagcaaa agccagggaa ggctccaaag 540

ctcctcatct acagcgccag cttcctctat agtggagtcc cttcacgatt ctctggtagc 600

cgctctggga cagactttac cctgactatt agcagtctgc agcctgagga ttttgcaact 660

tattactgtc agcagcacta caccacacca ccaacctttg gacaaggcac caaagtggaa 720

atcaagggtg gcggcggtgg aggatccggc ggtggaggta gcggcggagg cggtagctcc 780

agctctagta aagctccccc tccttccgag gtgcagctgc tggagtccgg aggaggactg 840

gtgcagccag gaggctccct gaagctgagc tgtgctgcct ctggctttac cttcaacaca 900

tatgccatga attgggtgcg gcaggctcca ggcaagggac tggagtgggt ggctaggatc 960

aggtctaagt acaacaatta tgccacctac tatgctgatt ccgtgaagga caggttcacc 1020

atctcccgcg acgatagcaa gaacacagcc tacctgcaga tgaacaatct gaagaccgag 1080

gataccgccg tgtactactg cgtgagacat ggcaactttg gcaatagcta cgtgtcctgg 1140

ttcgcttact ggggacaggg caccctggtc acagtgagct ctggaggagg aggatctgga 1200

ggaggaggct ccggaggagg aggaagcgag ctggtggtga cccaggagcc atctctgaca 1260

gtgtcccccg gcggcacagt gaccctgaca tgtagatcca gcaccggcgc cgtgaccaca 1320

tccaactacg ctaattgggt gcagcagaag ccaggacagg ctccaagggg actgatcgga 1380

ggaaccaaca agagggctcc tggaacacca gctcggttta gcggatctct gctgggaggc 1440

aaggctgccc tgaccctgtc cggagtgcag ccagaggatg aggccgagta ttattgcgct 1500

ctgtggtata gcaatctgtg ggtgttcgga ggaggaacca agctgacagt gctggacaag 1560

acccatacat gcccaccatg ccctgcccct gaagccgccg gaggaccttc cgtgttcctg 1620

ttccctccca agccaaaaga tcagctgatg atctctagaa cccccgaagt cacctgcgtg 1680

gtcgtcgacg tgtcccatga ggaccctgaa gtcaagttca actggtacgt ggacggtgtc 1740

gaagtccaca acgccaagac caagcctagg gaggagcagt atgccagcac ataccgggtg 1800

gtgtctgtgc tgaccgtgct gcatcaggat tggctgaatg gcaaggaata taaatgtaag 1860

gtgagcaata aggctctgcc ggctagcatt gaaaaaacca tttccaaggc taagggccag 1920

cccagggagc ctcaggtcta caccctgcct ccatctagag atgaactgac caaaaaccag 1980

gtgagcctga cttgcctggt caaaggcttc taccccagcg acattgccgt ggagtgggag 2040

tctaatggcc agcctgaaaa taactacaaa actacccctc ctgtgctgga ctctgatggc 2100

tccttctttc tgtactctaa actgaccgtg gacaagtctc gctggcagca gggtaacgtg 2160

ttttcttgct ccgtgctgca cgaggctctg cataaccatt acacccagaa gagcctgtct 2220

ctgtccccag ga 2232

<210> 277
<211> 743
<212> PRT
<213> Artificial sequence

<220>
<223> bispecific antibody AB8K amino acid sequence

<400> 277

Gln Val Gln Leu Lys Gln Ser Gly Pro Gly Leu Val Gln Pro Ser Gln
1 5 10 15

Ser Leu Ser Ile Thr Cys Thr Val Ser Gly Phe Ser Leu Thr Asn Tyr
20 25 30

Gly Val His Trp Val Arg Gln Ser Pro Gly Lys Gly Leu Glu Trp Leu
35 40 45

Gly Val Ile Trp Ser Gly Gly Asn Thr Asp Tyr Asn Thr Pro Phe Thr
50 55 60

Ser Arg Leu Ser Ile Asn Lys Asp Asn Ser Lys Ser Gln Val Phe Phe
65 70 75 80

Lys Met Asn Ser Leu Gln Ser Asn Asp Thr Ala Ile Tyr Tyr Cys Ala
85 90 95

Arg Ala Leu Thr Tyr Tyr Asp Tyr Glu Phe Ala Tyr Trp Gly Gln Gly
100 105 110

Thr Leu Val Thr Val Ser Ala Gly Gly Gly Gly Ser Gly Gly Gly Gly
115 120 125

Ser Gly Gly Gly Gly Ser Asp Ile Leu Leu Thr Gln Ser Pro Val Ile
130 135 140

Leu Ser Val Ser Pro Gly Glu Arg Val Ser Phe Ser Cys Arg Ala Ser
145 150 155 160

Gln Ser Ile Gly Thr Asn Ile His Trp Tyr Gln Gln Arg Thr Asn Gly
165 170 175

Ser Pro Arg Leu Leu Ile Lys Tyr Ala Ser Glu Ser Ile Ser Gly Ile
180 185 190

Pro Ser Arg Phe Ser Gly Ser Gly Ser Gly Thr Asp Phe Thr Leu Ser
195 200 205

Ile Asn Ser Val Glu Ser Glu Asp Ile Ala Asp Tyr Tyr Cys Gln Gln
210 215 220

Asn Asn Asn Trp Pro Thr Thr Phe Gly Ala Gly Thr Lys Leu Glu Leu
225 230 235 240

Lys Gly Gly Gly Gly Gly Gly Ser Gly Gly Gly Gly Ser Gly Gly Gly

245 250 255

Gly Ser Ser Ser Ser Ser Lys Ala Pro Pro Pro Ser Glu Val Gln Leu
260 265 270

Leu Glu Ser Gly Gly Gly Leu Val Gln Pro Gly Gly Ser Leu Lys Leu
275 280 285

Ser Cys Ala Ala Ser Gly Phe Thr Phe Asn Thr Tyr Ala Met Asn Trp
290 295 300

Val Arg Gln Ala Pro Gly Lys Gly Leu Glu Trp Val Ala Arg Ile Arg
305 310 315 320

Ser Lys Tyr Asn Asn Tyr Ala Thr Tyr Tyr Ala Asp Ser Val Lys Asp
325 330 335

Arg Phe Thr Ile Ser Arg Asp Asp Ser Lys Asn Thr Ala Tyr Leu Gln
340 345 350

Met Asn Asn Leu Lys Thr Glu Asp Thr Ala Val Tyr Tyr Cys Val Arg
355 360 365

His Gly Asn Phe Gly Asn Ser Tyr Val Ser Trp Phe Ala Tyr Trp Gly
370 375 380

Gln Gly Thr Leu Val Thr Val Ser Ser Gly Gly Gly Gly Ser Gly Gly
385 390 395 400

Gly Gly Ser Gly Gly Gly Gly Ser Glu Leu Val Val Thr Gln Glu Pro
405 410 415

Ser Leu Thr Val Ser Pro Gly Gly Thr Val Thr Leu Thr Cys Arg Ser
420 425 430

Ser Thr Gly Ala Val Thr Thr Ser Asn Tyr Ala Asn Trp Val Gln Gln
435 440 445

Lys Pro Gly Gln Ala Pro Arg Gly Leu Ile Gly Gly Thr Asn Lys Arg
450 455 460

Ala Pro Gly Thr Pro Ala Arg Phe Ser Gly Ser Leu Leu Gly Gly Lys
465 470 475 480

Ala Ala Leu Thr Leu Ser Gly Val Gln Pro Glu Asp Glu Ala Glu Tyr
485 490 495

Tyr Cys Ala Leu Trp Tyr Ser Asn Leu Trp Val Phe Gly Gly Gly Thr
500 505 510

Lys Leu Thr Val Leu Asp Lys Thr His Thr Cys Pro Pro Cys Pro Ala
515 520 525

Pro Glu Ala Ala Gly Gly Pro Ser Val Phe Leu Phe Pro Pro Lys Pro
530 535 540

Lys Asp Gln Leu Met Ile Ser Arg Thr Pro Glu Val Thr Cys Val Val
545 550 555 560

Val Asp Val Ser His Glu Asp Pro Glu Val Lys Phe Asn Trp Tyr Val
565 570 575

Asp Gly Val Glu Val His Asn Ala Lys Thr Lys Pro Arg Glu Glu Gln
580 585 590

Tyr Ala Ser Thr Tyr Arg Val Val Ser Val Leu Thr Val Leu His Gln
595 600 605

Asp Trp Leu Asn Gly Lys Glu Tyr Lys Cys Lys Val Ser Asn Lys Ala
610 615 620

Leu Pro Ala Ser Ile Glu Lys Thr Ile Ser Lys Ala Lys Gly Gln Pro
625 630 635 640

Arg Glu Pro Gln Val Tyr Thr Leu Pro Pro Ser Arg Asp Glu Leu Thr
645 650 655

Lys Asn Gln Val Ser Leu Thr Cys Leu Val Lys Gly Phe Tyr Pro Ser
660 665 670

Asp Ile Ala Val Glu Trp Glu Ser Asn Gly Gln Pro Glu Asn Asn Tyr
675 680 685

Lys Thr Thr Pro Pro Val Leu Asp Ser Asp Gly Ser Phe Phe Leu Tyr
690 695 700

Ser Lys Leu Thr Val Asp Lys Ser Arg Trp Gln Gln Gly Asn Val Phe
705 710 715 720

Ser Cys Ser Val Leu His Glu Ala Leu His Asn His Tyr Thr Gln Lys
725 730 735

Ser Leu Ser Leu Ser Pro Gly
740

<210> 278
<211> 2229
<212> DNA
<213> Artificial sequence

<220>
<223> bispecific antibody AB8K nucleotide sequence

<400> 278
caggtgcagc tgaagcagtc tggaccagga ctggtgcagc caagccagtc tctgtccatc 60

acctgcacag tgagcggctt ctctctgacc aactacggcg tgcactgggt gagacagtcc 120

ccaggcaagg gactggagtg gctgggcgtg atctggagcg gaggcaatac agattataac 180

accccctta catcccgcct gagcatcaat aaggacaaca gcaagtctca ggtgttcttt 240

aagatgaact ctctgcagtc caacgatacc gccatctact attgcgctag agccctgaca 300

tactatgact acgagttcgc ttattgggga cagggcaccc tggtgacagt gagcgccgga 360

ggaggaggat ctggaggagg aggctccgga ggaggaggaa gcgacatcct gctgacacag 420

tcccctgtga tcctgagcgt gtctccaggc gagagagtgt cctttagctg tcgcgcctct 480

cagtccatcg gcaccaatat ccattggtac cagcagagga caaacggctc tccccggctg 540

ctgatcaagt atgctagcga gtctatctcc ggcatccctt cccgcttcag cggatctggc 600

tccggaaccg acttcaccct gagcatcaat tctgtggagt ccgaggatat cgccgactac 660

tattgccagc agaacaataa ctggcctacc acattcggcg ctggcaccaa gctggagctg 720

aagggtggcg gcggtggagg atccggcggt ggaggtagcg gcggaggcgg tagctccagc 780

tctagtaaag ctccccctcc ttccgaggtg cagctgctgg agtccggagg aggactggtg 840

cagccaggag gctccctgaa gctgagctgt gctgcctctg gctttacctt caacacatat 900

gccatgaatt gggtgcggca ggctccaggc aagggactgg agtgggtggc taggatcagg 960

tctaagtaca acaattatgc cacctactat gctgattccg tgaaggacag gttcaccatc 1020

tcccgcgacg atagcaagaa cacagcctac ctgcagatga acaatctgaa gaccgaggat 1080

accgccgtgt actactgcgt gagacatggc aactttggca atagctacgt gtcctggttc 1140

gcttactggg gacagggcac cctggtcaca gtgagctctg gaggaggagg atctggagga 1200

ggaggctccg gaggaggagg aagcgagctg gtggtgaccc aggagccatc tctgacagtg 1260

tcccccggcg gcacagtgac cctgacatgt agatccagca ccggcgccgt gaccacatcc 1320

aactacgcta attgggtgca gcagaagcca ggacaggctc caaggggact gatcggagga 1380

accaacaaga gggctcctgg aacaccagct cggtttagcg gatctctgct gggaggcaag 1440

gctgccctga ccctgtccgg agtgcagcca gaggatgagg ccgagtatta ttgcgctctg 1500

tggtatagca atctgtgggt gttcggagga ggaaccaagc tgacagtgct ggacaagacc 1560

catacatgcc caccatgccc tgcccctgaa gccgccggag gaccttccgt gttcctgttc 1620

cctcccaagc caaaagatca gctgatgatc tctagaaccc ccgaagtcac ctgcgtggtc 1680

gtcgacgtgt cccatgagga ccctgaagtc aagttcaact ggtacgtgga cggtgtcgaa 1740

gtccacaacg ccaagaccaa gcctagggag gagcagtatg ccagcacata ccgggtggtg 1800

tctgtgctga ccgtgctgca tcaggattgg ctgaatggca aggaatataa atgtaaggtg 1860

agcaataagg ctctgccggc tagcattgaa aaaaccattt ccaaggctaa gggccagccc 1920

agggagcctc aggtctacac cctgcctcca tctagagatg aactgaccaa aaaccaggtg 1980

agcctgactt gcctggtcaa aggcttctac cccagcgaca ttgccgtgga gtgggagtct 2040

aatggccagc ctgaaaataa ctacaaaact acccctcctg tgctggactc tgatggctcc 2100

ttctttctgt actctaaact gaccgtggac aagtctcgct ggcagcaggg taacgtgttt 2160

tcttgctccg tgctgcacga ggctctgcat aaccattaca cccagaagag cctgtctctg 2220

tccccagga 2229

<210> 279
<211> 744
<212> PRT
<213> Artificial sequence

<220>
<223> bispecific antibody AB9K amino acid sequence

<400> 279

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Lys Gln Ala Pro Gly Gln Gly Leu Glu Trp Ile
35 40 45

Gly Ala Ile Asp Pro Gln Thr Gly Asn Thr Ala Phe Asn Gln Lys Phe
50 55 60

Lys Gly Arg Val Thr Leu Thr Arg Asp Lys Ser Ser Ser Thr Val Tyr
65 70 75 80

Met Glu Leu Ser Ser Leu Arg Ser Glu Asp Thr Ala Val Tyr Tyr Cys
85 90 95

Thr Arg Phe Tyr Ser Leu Thr Tyr Trp Gly Gln Gly Thr Leu Val Thr
100 105 110

Val Ser Ser Gly Gly Gly Gly Ser Gly Gly Gly Gly Ser Gly Gly Gly

115 120 125

Gly Ser Asp Val Leu Met Thr Gln Ser Pro Leu Ser Leu Pro Val Thr
130 135 140

Leu Gly Gln Pro Ala Ser Ile Ser Cys Arg Ser Ser Gln Ser Ile Val
145 150 155 160

His Ser Asn Gly Asn Thr Tyr Leu Gln Trp Tyr Leu Gln Arg Pro Gly
165 170 175

Gln Ser Pro Lys Leu Leu Ile Tyr Lys Val Ser Asn Arg Phe Ser Gly
180 185 190

Val Pro Asp Arg Phe Ser Gly Ser Gly Ser Gly Thr Tyr Phe Thr Leu
195 200 205

Lys Ile Ser Arg Val Glu Ala Glu Asp Val Gly Val Tyr Tyr Cys Phe
210 215 220

Gln Gly Ser His Phe Pro Tyr Ala Phe Gly Gly Gly Thr Lys Val Glu
225 230 235 240

Ile Lys Gly Gly Gly Gly Gly Gly Ser Gly Gly Gly Gly Ser Gly Gly
245 250 255

Gly Gly Ser Ser Ser Ser Ser Lys Ala Pro Pro Pro Ser Glu Val Gln
260 265 270

Leu Leu Glu Ser Gly Gly Gly Leu Val Gln Pro Gly Gly Ser Leu Lys
275 280 285

Leu Ser Cys Ala Ala Ser Gly Phe Thr Phe Asn Thr Tyr Ala Met Asn
290 295 300

Trp Val Arg Gln Ala Pro Gly Lys Gly Leu Glu Trp Val Ala Arg Ile
305 310 315 320

Arg Ser Lys Tyr Asn Asn Tyr Ala Thr Tyr Tyr Ala Asp Ser Val Lys
325 330 335

Asp Arg Phe Thr Ile Ser Arg Asp Asp Ser Lys Asn Thr Ala Tyr Leu
340 345 350

Gln Met Asn Asn Leu Lys Thr Glu Asp Thr Ala Val Tyr Tyr Cys Val
355 360 365

```
Arg His Gly Asn Phe Gly Asn Ser Tyr Val Ser Trp Phe Ala Tyr Trp
    370                 375                 380

Gly Gln Gly Thr Leu Val Thr Val Ser Ser Gly Gly Gly Gly Ser Gly
385                 390                 395                 400

Gly Gly Gly Ser Gly Gly Gly Gly Ser Glu Leu Val Val Thr Gln Glu
                405                 410                 415

Pro Ser Leu Thr Val Ser Pro Gly Gly Thr Val Thr Leu Thr Cys Arg
            420                 425                 430

Ser Ser Thr Gly Ala Val Thr Thr Ser Asn Tyr Ala Asn Trp Val Gln
        435                 440                 445

Gln Lys Pro Gly Gln Ala Pro Arg Gly Leu Ile Gly Gly Thr Asn Lys
    450                 455                 460

Arg Ala Pro Gly Thr Pro Ala Arg Phe Ser Gly Ser Leu Leu Gly Gly
465                 470                 475                 480

Lys Ala Ala Leu Thr Leu Ser Gly Val Gln Pro Glu Asp Glu Ala Glu
                485                 490                 495

Tyr Tyr Cys Ala Leu Trp Tyr Ser Asn Leu Trp Val Phe Gly Gly Gly
            500                 505                 510

Thr Lys Leu Thr Val Leu Asp Lys Thr His Thr Cys Pro Pro Cys Pro
        515                 520                 525

Ala Pro Glu Ala Ala Gly Gly Pro Ser Val Phe Leu Phe Pro Pro Lys
    530                 535                 540

Pro Lys Asp Gln Leu Met Ile Ser Arg Thr Pro Glu Val Thr Cys Val
545                 550                 555                 560

Val Val Asp Val Ser His Glu Asp Pro Glu Val Lys Phe Asn Trp Tyr
                565                 570                 575

Val Asp Gly Val Glu Val His Asn Ala Lys Thr Lys Pro Arg Glu Glu
            580                 585                 590

Gln Tyr Ala Ser Thr Tyr Arg Val Val Ser Val Leu Thr Val Leu His
        595                 600                 605

Gln Asp Trp Leu Asn Gly Lys Glu Tyr Lys Cys Lys Val Ser Asn Lys
    610                 615                 620
```

Ala Leu Pro Ala Ser Ile Glu Lys Thr Ile Ser Lys Ala Lys Gly Gln
625 630 635 640

Pro Arg Glu Pro Gln Val Tyr Thr Leu Pro Pro Ser Arg Asp Glu Leu
645 650 655

Thr Lys Asn Gln Val Ser Leu Thr Cys Leu Val Lys Gly Phe Tyr Pro
660 665 670

Ser Asp Ile Ala Val Glu Trp Glu Ser Asn Gly Gln Pro Glu Asn Asn
675 680 685

Tyr Lys Thr Thr Pro Pro Val Leu Asp Ser Asp Gly Ser Phe Phe Leu
690 695 700

Tyr Ser Lys Leu Thr Val Asp Lys Ser Arg Trp Gln Gln Gly Asn Val
705 710 715 720

Phe Ser Cys Ser Val Leu His Glu Ala Leu His Asn His Tyr Thr Gln
725 730 735

Lys Ser Leu Ser Leu Ser Pro Gly
740

<210> 280
<211> 2232
<212> DNA
<213> Artificial sequence

<220>
<223> bispecific antibody AB9K nucleotide sequence

<400> 280
caggtccagc tggtccagtc tggcgccgaa gtgaagaagc caggcgcctc cgtgaaggtg 60

agctgtaagg cctctggcta caccttcaca gactatgaga tgcattgggt gaagcaggct 120

ccaggacagg gactggagtg gatcggcgct atcgatcccc agaccggcaa cacagccttc 180

aatcagaagt ttaagggcag agtgaccctg acacgcgaca agagctcttc caccgtgtac 240

atggagctga gctctctgag gtccgaggat accgccgtgt actattgcac acggttttac 300

agcctgacct actggggtca gggcaccctg gtcacagtgt ccagcggagg aggaggatct 360

ggaggaggag gctccggagg aggaggaagc gatgtgctga tgacacagtc tccactgtcc 420

ctgccagtga ccctgggaca gccagcttcc atcagctgta gatcttccca gtccatcgtg 480

cacagcaacg gcaataccta cctgcagtgg tatctgcagc gccctggcca gagcccaaag 540

ctgctgatct acaaggtgtc taataggttc tccggcgtgc cagaccggtt ttctggctcc 600

ggcagcggca cctatttcac actgaagatc tctagagtgg aggccgagga tgtgggcgtg 660

tactattgtt ttcagggctc ccatttcccc tacgcttttg gcggtggtac taaagtggag 720

atcaagggtg gcggcggtgg aggatccggc ggtggaggta gcggcggagg cggtagctcc 780

agctctagta aagctccccc tccttccgag gtgcagctgc tggagtccgg aggaggactg 840

gtgcagccag gaggctccct gaagctgagc tgtgctgcct ctggctttac cttcaacaca 900

tatgccatga attgggtgcg gcaggctcca ggcaagggac tggagtgggt ggctaggatc 960

aggtctaagt acaacaatta tgccacctac tatgctgatt ccgtgaagga caggttcacc 1020

atctcccgcg acgatagcaa gaacacagcc tacctgcaga tgaacaatct gaagaccgag 1080

gataccgccg tgtactactg cgtgagacat ggcaactttg gcaatagcta cgtgtcctgg 1140

ttcgcttact ggggacaggg caccctggtc acagtgagct ctggaggagg aggatctgga 1200

ggaggaggct ccggaggagg aggaagcgag ctggtggtga cccaggagcc atctctgaca 1260

gtgtcccccg gcggcacagt gaccctgaca tgtagatcca gcaccggcgc cgtgaccaca 1320

tccaactacg ctaattgggt gcagcagaag ccaggacagg ctccaagggg actgatcgga 1380

ggaaccaaca agagggctcc tggaacacca gctcggttta gcggatctct gctgggaggc 1440

aaggctgccc tgaccctgtc cggagtgcag ccagaggatg aggccgagta ttattgcgct 1500

ctgtggtata gcaatctgtg ggtgttcgga ggaggaacca agctgacagt gctggacaag 1560

acccatacat gcccaccatg ccctgcccct gaagccgccg gaggaccttc cgtgttcctg 1620

ttccctccca agccaaaaga tcagctgatg atctctagaa cccccgaagt cacctgcgtg 1680

gtcgtcgacg tgtcccatga ggaccctgaa gtcaagttca actggtacgt ggacggtgtc 1740

gaagtccaca acgccaagac caagcctagg gaggagcagt atgccagcac ataccgggtg 1800

gtgtctgtgc tgaccgtgct gcatcaggat tggctgaatg gcaaggaata taaatgtaag 1860

gtgagcaata aggctctgcc ggctagcatt gaaaaaacca tttccaaggc taagggccag 1920

cccagggagc ctcaggtcta caccctgcct ccatctagag atgaactgac caaaaaccag 1980

gtgagcctga cttgcctggt caaaggcttc taccccagcg acattgccgt ggagtgggag 2040

tctaatggcc agcctgaaaa taactacaaa actacccctc ctgtgctgga ctctgatggc 2100

tccttctttc tgtactctaa actgaccgtg gacaagtctc gctggcagca gggtaacgtg 2160

ttttcttgct ccgtgctgca cgaggctctg cataaccatt acacccagaa gagcctgtct 2220

ctgtccccag ga 2232

<210> 281
<211> 746
<212> PRT
<213> Artificial sequence

<220>
<223> bispecific antibody AB10K amino acid sequence

<400> 281

Gln Val Tyr Leu Val Glu Ser Gly Gly Gly Val Val Gln Pro Gly Arg
1 5 10 15

Ser Leu Arg Leu Ser Cys Ala Ala Ser Gly Ile Thr Phe Ser Ile Tyr
20 25 30

Gly Met His Trp Val Arg Gln Ala Pro Gly Lys Gly Leu Glu Trp Val
35 40 45

Ala Val Ile Trp Tyr Asp Gly Ser His Glu Tyr Tyr Ala Asp Ser Val
50 55 60

Lys Gly Arg Phe Thr Ile Ser Arg Asp Asn Ser Lys Asn Thr Leu Tyr
65 70 75 80

Leu Leu Met Asn Ser Leu Arg Ala Glu Asp Thr Ala Val Tyr Tyr Cys
85 90 95

Ala Arg Asp Gly Asp Tyr Tyr Asp Ser Gly Ser Pro Leu Asp Tyr Trp
100 105 110

Gly Gln Gly Thr Leu Val Thr Val Ser Ser Gly Gly Gly Gly Ser Gly
115 120 125

Gly Gly Gly Ser Gly Gly Gly Gly Ser Glu Ile Val Leu Thr Gln Ser
130 135 140

Pro Ala Thr Leu Ser Leu Ser Pro Gly Glu Arg Ala Thr Leu Ser Cys
145 150 155 160

Arg Ala Ser Gln Ser Val Ser Ser Tyr Leu Ala Trp Tyr Gln Gln Lys
165 170 175

Pro Gly Gln Ala Pro Arg Leu Leu Ile Tyr Asp Ala Ser Asn Arg Ala
180 185 190

Thr Gly Ile Pro Ala Arg Phe Ser Gly Ser Gly Ser Gly Thr Asp Phe
195 200 205

Thr Leu Thr Ile Ser Ser Leu Glu Pro Glu Asp Phe Ala Val Tyr Tyr
210 215 220

Cys Gln Gln Arg Ser Asn Trp Pro Leu Thr Phe Gly Gly Gly Thr Lys
225 230 235 240

Val Glu Ile Lys Gly Gly Gly Gly Gly Gly Ser Gly Gly Gly Gly Ser
245 250 255

Gly Gly Gly Gly Ser Ser Ser Ser Ser Lys Ala Pro Pro Pro Ser Glu
260 265 270

Val Gln Leu Leu Glu Ser Gly Gly Gly Leu Val Gln Pro Gly Gly Ser
275 280 285

Leu Lys Leu Ser Cys Ala Ala Ser Gly Phe Thr Phe Asn Thr Tyr Ala
290 295 300

Met Asn Trp Val Arg Gln Ala Pro Gly Lys Gly Leu Glu Trp Val Ala
305 310 315 320

Arg Ile Arg Ser Lys Tyr Asn Asn Tyr Ala Thr Tyr Tyr Ala Asp Ser
325 330 335

Val Lys Asp Arg Phe Thr Ile Ser Arg Asp Asp Ser Lys Asn Thr Ala
340 345 350

Tyr Leu Gln Met Asn Asn Leu Lys Thr Glu Asp Thr Ala Val Tyr Tyr
355 360 365

Cys Val Arg His Gly Asn Phe Gly Asn Ser Tyr Val Ser Trp Phe Ala
370 375 380

Tyr Trp Gly Gln Gly Thr Leu Val Thr Val Ser Ser Gly Gly Gly Gly
385 390 395 400

Ser Gly Gly Gly Gly Ser Gly Gly Gly Gly Ser Glu Leu Val Val Thr
405 410 415

Gln Glu Pro Ser Leu Thr Val Ser Pro Gly Gly Thr Val Thr Leu Thr
420 425 430

Cys Arg Ser Ser Thr Gly Ala Val Thr Thr Ser Asn Tyr Ala Asn Trp
435 440 445

Val Gln Gln Lys Pro Gly Gln Ala Pro Arg Gly Leu Ile Gly Gly Thr
450 455 460

Asn Lys Arg Ala Pro Gly Thr Pro Ala Arg Phe Ser Gly Ser Leu Leu
465 470 475 480

Gly Gly Lys Ala Ala Leu Thr Leu Ser Gly Val Gln Pro Glu Asp Glu
485 490 495

Ala Glu Tyr Tyr Cys Ala Leu Trp Tyr Ser Asn Leu Trp Val Phe Gly
500 505 510

Gly Gly Thr Lys Leu Thr Val Leu Asp Lys Thr His Thr Cys Pro Pro
515 520 525

Cys Pro Ala Pro Glu Ala Ala Gly Gly Pro Ser Val Phe Leu Phe Pro
530 535 540

Pro Lys Pro Lys Asp Gln Leu Met Ile Ser Arg Thr Pro Glu Val Thr
545 550 555 560

Cys Val Val Val Asp Val Ser His Glu Asp Pro Glu Val Lys Phe Asn
565 570 575

Trp Tyr Val Asp Gly Val Glu Val His Asn Ala Lys Thr Lys Pro Arg
580 585 590

Glu Glu Gln Tyr Ala Ser Thr Tyr Arg Val Val Ser Val Leu Thr Val
595 600 605

Leu His Gln Asp Trp Leu Asn Gly Lys Glu Tyr Lys Cys Lys Val Ser
610 615 620

Asn Lys Ala Leu Pro Ala Ser Ile Glu Lys Thr Ile Ser Lys Ala Lys
625 630 635 640

Gly Gln Pro Arg Glu Pro Gln Val Tyr Thr Leu Pro Pro Ser Arg Asp
645 650 655

Glu Leu Thr Lys Asn Gln Val Ser Leu Thr Cys Leu Val Lys Gly Phe
660 665 670

Tyr Pro Ser Asp Ile Ala Val Glu Trp Glu Ser Asn Gly Gln Pro Glu
675 680 685

Asn Asn Tyr Lys Thr Thr Pro Pro Val Leu Asp Ser Asp Gly Ser Phe
690 695 700

Phe Leu Tyr Ser Lys Leu Thr Val Asp Lys Ser Arg Trp Gln Gln Gly
705 710 715 720

Asn Val Phe Ser Cys Ser Val Leu His Glu Ala Leu His Asn His Tyr
725 730 735

Thr Gln Lys Ser Leu Ser Leu Ser Pro Gly
740 745

<210> 282
<211> 2238
<212> DNA
<213> Artificial sequence

<220>
<223> bispecific antibody AB10K nucleotide sequence

<400> 282
caggtgtacc tggtggagag cggaggagga gtggtgcagc caggccgctc tctgaggctg 60

tcctgtgctg ccagcggcat caccttctcc atctatggaa tgcactgggt gaggcaggct 120

cctggcaagg gactggagtg ggtggctgtg atctggtacg atggctctca tgagtattat 180

gccgactccg tgaagggccg gtttacaatc tctagagata actccaagaa taccctgtat 240

ctgctgatga atagcctgcg cgccgaggac acagccgtgt actattgcgc tagggacggc 300

gattactatg attccggaag cccactggac tactggggac agggcacact ggtgaccgtg 360

agctctggag gaggaggatc tggaggagga ggctccggag gaggaggaag cgagatcgtg 420

ctgacacaga gcccagccac cctgtctctg tcccctggag agagggccac cctgtcttgt 480

agggctagcc agtccgtgtc cagctacctg gcttggtacc agcagaagcc aggacaggct 540

ccaaggctgc tgatctatga cgcttccaac agggctacag gaatcccagc taggttttcc 600

ggaagcggat ctggcaccga tttcacactg accatctctt ccctggagcc tgaggacttt 660

gccgtgtact actgtcagca gagaagcaat tggccactga cattcggcgg cggcaccaag 720

gtggagatca agggtggcgg cggtggagga tccggcggtg gaggtagcgg cggaggcggt 780

agctccagct ctagtaaagc tccccctcct tccgaggtgc agctgctgga gtccggagga 840

ggactggtgc agccaggagg ctccctgaag ctgagctgtg ctgcctctgg ctttaccttc 900

aacacatatg ccatgaattg ggtgcggcag gctccaggca agggactgga gtgggtggct 960

aggatcaggt ctaagtacaa caattatgcc acctactatg ctgattccgt gaaggacagg 1020

ttcaccatct cccgcgacga tagcaagaac acagcctacc tgcagatgaa caatctgaag 1080

accgaggata ccgccgtgta ctactgcgtg agacatggca actttggcaa tagctacgtg 1140

tcctggttcg cttactgggg acagggcacc ctggtcacag tgagctctgg aggaggagga 1200

tctggaggag gaggctccgg aggaggagga agcgagctgg tggtgaccca ggagccatct 1260

ctgacagtgt cccccggcgg cacagtgacc ctgacatgta gatccagcac cggcgccgtg 1320

accacatcca actacgctaa ttgggtgcag cagaagccag gacaggctcc aaggggactg 1380

atcggaggaa ccaacaagag ggctcctgga acaccagctc ggtttagcgg atctctgctg 1440

ggaggcaagg ctgccctgac cctgtccgga gtgcagccag aggatgaggc cgagtattat 1500

tgcgctctgt ggtatagcaa tctgtgggtg ttcggaggag gaaccaagct gacagtgctg 1560

gacaagaccc atacatgccc accatgccct gcccctgaag ccgccggagg accttccgtg 1620

ttcctgttcc ctcccaagcc aaaagatcag ctgatgatct ctagaacccc cgaagtcacc 1680

tgcgtggtcg tcgacgtgtc ccatgaggac cctgaagtca agttcaactg gtacgtggac 1740

ggtgtcgaag tccacaacgc caagaccaag cctagggagg agcagtatgc cagcacatac 1800

cgggtggtgt ctgtgctgac cgtgctgcat caggattggc tgaatggcaa ggaatataaa 1860

tgtaaggtga gcaataaggc tctgccggct agcattgaaa aaaccatttc caaggctaag 1920

ggccagccca gggagcctca ggtctacacc ctgcctccat ctagagatga actgaccaaa 1980

aaccaggtga gcctgacttg cctggtcaaa ggcttctacc ccagcgacat tgccgtggag 2040

tgggagtcta atggccagcc tgaaaataac tacaaaacta cccctcctgt gctggactct 2100

gatggctcct tctttctgta ctctaaactg accgtggaca agtctcgctg gcagcagggt 2160

aacgtgtttt cttgctccgt gctgcacgag gctctgcata accattacac ccagaagagc 2220

ctgtctctgt ccccagga 2238

<210> 283
<211> 743
<212> PRT
<213> Artificial sequence

<220>
<223> bispecific antibody AB1K2 amino acid sequence

<400> 283

Gln Val Gln Leu Gln Glu Ser Gly Pro Gly Leu Val Lys Pro Ser Gln
1 5 10 15

Thr Leu Ser Leu Thr Cys Thr Val Ser Gly Gly Ser Ile Ser Thr Ser
20 25 30

Gly Met Gly Val Gly Trp Ile Arg Gln His Pro Gly Lys Gly Leu Glu
35 40 45

Trp Ile Gly His Ile Trp Trp Asp Asp Asp Lys Arg Tyr Asn Pro Ala
50 55 60

Leu Lys Ser Arg Val Thr Ile Ser Val Asp Thr Ser Lys Asn Gln Phe
65 70 75 80

Ser Leu Lys Leu Ser Ser Val Thr Ala Ala Asp Thr Ala Val Tyr Tyr
85 90 95

Cys Ala Arg Met Glu Leu Trp Ser Tyr Tyr Phe Asp Tyr Trp Gly Gln
100 105 110

```
Gly Thr Leu Val Thr Val Ser Ser Gly Gly Gly Gly Ser Gly Gly Gly
        115                 120                 125

Gly Ser Gly Gly Gly Gly Ser Glu Ile Val Leu Thr Gln Ser Pro Ala
    130                 135                 140

Thr Leu Ser Leu Ser Pro Gly Glu Arg Ala Thr Leu Ser Cys Ser Ala
145                 150                 155                 160

Ser Ser Ser Val Ser Tyr Met His Trp Tyr Gln Gln Lys Pro Gly Gln
                165                 170                 175

Ala Pro Arg Leu Leu Ile Tyr Asp Thr Ser Lys Leu Ala Ser Gly Ile
            180                 185                 190

Pro Ala Arg Phe Ser Gly Ser Gly Ser Gly Thr Asp Phe Thr Leu Thr
        195                 200                 205

Ile Ser Ser Leu Glu Pro Glu Asp Val Ala Val Tyr Tyr Cys Phe Gln
    210                 215                 220

Gly Ser Val Tyr Pro Phe Thr Phe Gly Gln Gly Thr Lys Leu Glu Ile
225                 230                 235                 240

Lys Gly Gly Gly Gly Gly Gly Ser Gly Gly Gly Gly Ser Gly Gly Gly
                245                 250                 255

Gly Ser Ser Ser Ser Ser Lys Ala Pro Pro Pro Ser Glu Val Gln Leu
            260                 265                 270

Leu Glu Ser Gly Gly Gly Leu Val Gln Pro Gly Gly Ser Leu Lys Leu
        275                 280                 285

Ser Cys Ala Ala Ser Gly Phe Thr Phe Asn Thr Tyr Ala Met Asn Trp
    290                 295                 300

Val Arg Gln Ala Pro Gly Lys Gly Leu Glu Trp Val Ala Arg Ile Arg
305                 310                 315                 320

Ser Lys Tyr Asn Asn Tyr Ala Thr Tyr Tyr Ala Asp Ser Val Lys Asp
                325                 330                 335

Arg Phe Thr Ile Ser Arg Asp Asp Ser Lys Asn Thr Ala Tyr Leu Gln
            340                 345                 350

Met Asn Asn Leu Lys Thr Glu Asp Thr Ala Val Tyr Tyr Cys Val Arg
        355                 360                 365
```

```
His Gly Asn Phe Gly Asn Ser Tyr Val Ser Trp Phe Ala Tyr Trp Gly
    370                 375                 380

Gln Gly Thr Leu Val Thr Val Ser Ser Gly Gly Gly Gly Ser Gly Gly
385                 390                 395                 400

Gly Gly Ser Gly Gly Gly Gly Ser Glu Leu Val Val Thr Gln Glu Pro
                405                 410                 415

Ser Leu Thr Val Ser Pro Gly Gly Thr Val Thr Leu Thr Cys Arg Ser
            420                 425                 430

Ser Thr Gly Ala Val Thr Thr Ser Asn Tyr Ala Asn Trp Val Gln Gln
        435                 440                 445

Lys Pro Gly Gln Ala Pro Arg Gly Leu Ile Gly Gly Thr Asn Lys Arg
    450                 455                 460

Ala Pro Gly Thr Pro Ala Arg Phe Ser Gly Ser Leu Leu Gly Gly Lys
465                 470                 475                 480

Ala Ala Leu Thr Leu Ser Gly Val Gln Pro Glu Asp Glu Ala Glu Tyr
                485                 490                 495

Tyr Cys Ala Leu Trp Tyr Ser Asn Leu Trp Val Phe Gly Gly Gly Thr
            500                 505                 510

Lys Leu Thr Val Leu Asp Lys Thr His Thr Cys Pro Pro Cys Pro Ala
        515                 520                 525

Pro Glu Ala Ala Gly Gly Pro Ser Val Phe Leu Phe Pro Pro Lys Pro
    530                 535                 540

Lys Asp Gln Leu Met Ile Ser Arg Thr Pro Glu Val Thr Cys Val Val
545                 550                 555                 560

Val Asp Val Ser His Glu Asp Pro Glu Val Lys Phe Asn Trp Tyr Val
                565                 570                 575

Asp Gly Val Glu Val His Asn Ala Lys Thr Lys Pro Arg Glu Glu Gln
            580                 585                 590

Tyr Ala Ser Thr Tyr Arg Val Val Ser Val Leu Thr Val Leu His Gln
        595                 600                 605

Asp Trp Leu Asn Gly Lys Glu Tyr Lys Cys Lys Val Ser Asn Lys Ala
    610                 615                 620
```

Leu Pro Ala Ser Ile Glu Lys Thr Ile Ser Lys Ala Lys Gly Gln Pro
625 630 635 640

Arg Glu Pro Gln Val Tyr Thr Leu Pro Pro Ser Arg Asp Glu Leu Thr
645 650 655

Lys Asn Gln Val Ser Leu Thr Cys Leu Val Lys Gly Phe Tyr Pro Ser
660 665 670

Asp Ile Ala Val Glu Trp Glu Ser Asn Gly Gln Pro Glu Asn Asn Tyr
675 680 685

Lys Thr Thr Pro Pro Val Leu Asp Ser Asp Gly Ser Phe Phe Leu Tyr
690 695 700

Ser Lys Leu Thr Val Asp Lys Ser Arg Trp Gln Gln Gly Asn Val Phe
705 710 715 720

Ser Cys Ser Val Leu His Glu Ala Leu His Asn His Tyr Thr Gln Lys
725 730 735

Ser Leu Ser Leu Ser Pro Gly
740

<210> 284
<211> 2229
<212> DNA
<213> Artificial sequence

<220>
<223> bispecific antibody AB1K2 nucleotide sequence

<400> 284
caggtgcagc tgcaggagag cggaccagga ctggtgaagc catcccagac cctgagcctg 60

acctgtacag tgtctggcgg cagcatctct acatccggaa tgggagtggg atggatcagg 120

cagcacccag gcaagggcct ggaatggatc ggccatatct ggtgggacga tgacaagaga 180

tacaatcctg ccctgaagtc ccgcgtgaca atctctgtgg acacctccaa gaaccagttt 240

agcctgaagc tgtcttccgt gacagccgct gataccgccg tgtattactg cgctagaatg 300

gagctgtgga gctattactt cgactactgg ggccagggca cactggtgac cgtgagctcc 360

ggtggtggtg gttccggcgg cggcggttct ggcggcggcg gcagcgagat cgtgctgaca 420

cagtcccctg ccaccctgag cctgtctcca ggagagaggg ccaccctgtc ctgttccgcc 480

tccagctctg tgagctacat gcattggtat cagcagaagc caggccaggc tcccaggctg 540

ctgatctacg atacttctaa actggcctct ggcatccctg ctcggttttc tggctccggc 600

agcggcacag acttcaccct gacaatctcc agcctggagc cagaggatgt ggccgtgtat 660

tactgcttcc agggcagcgt gtatcccttc acatttggcc agggcactaa actggagatc 720

aagggtggcg gcggtggagg atccggcggt ggaggtagcg gcggaggcgg tagctccagc 780

tctagtaaag ctccccctcc ttccgaggtg cagctgctgg agtccggagg aggactggtg 840

cagccaggag gctccctgaa gctgagctgt gctgcctctg gctttacctt caacacatat 900

gccatgaatt gggtgcggca ggctccaggc aagggactgg agtgggtggc taggatcagg 960

tctaagtaca acaattatgc cacctactat gctgattccg tgaaggacag gttcaccatc 1020

tcccgcgacg atagcaagaa cacagcctac ctgcagatga acaatctgaa gaccgaggat 1080

accgccgtgt actactgcgt gagacatggc aactttggca atagctacgt gtcctggttc 1140

gcttactggg gacagggcac cctggtcaca gtgagctctg gaggaggagg atctggagga 1200

ggaggctccg gaggaggagg aagcgagctg gtggtgaccc aggagccatc tctgacagtg 1260

tcccccggcg gcacagtgac cctgacatgt agatccagca ccggcgccgt gaccacatcc 1320

aactacgcta attgggtgca gcagaagcca ggacaggctc caaggggact gatcggagga 1380

accaacaaga gggctcctgg aacaccagct cggtttagcg gatctctgct gggaggcaag 1440

gctgccctga ccctgtccgg agtgcagcca gaggatgagg ccgagtatta ttgcgctctg 1500

tggtatagca atctgtgggt gttcggagga ggaaccaagc tgacagtgct ggacaagacc 1560

catacatgcc caccatgccc tgcccctgaa gccgccggag gaccttccgt gttcctgttc 1620

cctcccaagc caaaagatca gctgatgatc tctagaaccc ccgaagtcac ctgcgtggtc 1680

gtcgacgtgt cccatgagga ccctgaagtc aagttcaact ggtacgtgga cggtgtcgaa 1740

gtccacaacg ccaagaccaa gcctagggag gagcagtatg ccagcacata ccgggtggtg 1800

tctgtgctga ccgtgctgca tcaggattgg ctgaatggca aggaatataa atgtaaggtg 1860

agcaataagg ctctgccggc tagcattgaa aaaaccattt ccaaggctaa gggccagccc 1920

agggagcctc aggtctacac cctgcctcca tctagagatg aactgaccaa aaaccaggtg 1980

agcctgactt gcctggtcaa aggcttctac cccagcgaca ttgccgtgga gtgggagtct 2040

aatggccagc ctgaaaataa ctacaaaact acccctcctg tgctggactc tgatggctcc 2100

ttctttctgt actctaaact gaccgtggac aagtctcgct ggcagcaggg taacgtgttt 2160

tcttgctccg tgctgcacga ggctctgcat aaccattaca cccagaagag cctgtctctg 2220

tccccagga 2229

<210> 285
<211> 746
<212> PRT
<213> Artificial sequence

<220>

<223> bispecific antibody AB11K amino acid sequence

<400> 285

Glu Val Gln Leu Val Glu Ser Gly Gly Gly Leu Val Gln Pro Gly Gly
1 5 10 15

Ser Met Arg Leu Ser Cys Val Ala Ser Gly Phe Pro Phe Ser Asn Tyr
20 25 30

Trp Met Asn Trp Val Arg Gln Ala Pro Gly Lys Gly Leu Glu Trp Val
35 40 45

Gly Glu Ile Arg Leu Lys Ser Asn Asn Tyr Thr Thr His Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Thr Arg His Tyr Tyr Phe Asp Tyr Trp Gly Gln Gly Thr Leu
100 105 110

Val Thr Val Ser Ser Gly Gly Gly Gly Ser Gly Gly Gly Gly Ser Gly
115 120 125

Gly Gly Gly Ser Asp Ile Val Met Thr Gln Ser Pro Leu Ser Asn Pro
130 135 140

Val Thr Pro Gly Glu Pro Ala Ser Ile Ser Cys Arg Ser Ser Lys Ser
145 150 155 160

Leu Leu His Ser Asn Gly Ile Thr Tyr Phe Phe Trp Tyr Leu Gln Lys
165 170 175

Pro Gly Gln Ser Pro Gln Leu Leu Ile Tyr Gln Met Ser Asn Leu Ala
180 185 190

Ser Gly Val Pro Asp Arg Phe Ser Gly Ser Gly Ser Gly Thr Asp Phe
195 200 205

Thr Leu Arg Ile Ser Arg Val Glu Ala Glu Asp Val Gly Val Tyr Tyr
210 215 220

Cys Ala Gln Asn Leu Glu Leu Pro Pro Thr Phe Gly Gln Gly Thr Lys
225 230 235 240

Val Glu Ile Lys Gly Gly Gly Gly Gly Gly Ser Gly Gly Gly Gly Ser
245 250 255

Gly Gly Gly Gly Ser Ser Ser Ser Ser Lys Ala Pro Pro Pro Ser Glu
260 265 270

Val Gln Leu Leu Glu Ser Gly Gly Gly Leu Val Gln Pro Gly Gly Ser
275 280 285

Leu Lys Leu Ser Cys Ala Ala Ser Gly Phe Thr Phe Asn Thr Tyr Ala
290 295 300

Met Asn Trp Val Arg Gln Ala Pro Gly Lys Gly Leu Glu Trp Val Ala
305 310 315 320

Arg Ile Arg Ser Lys Tyr Asn Asn Tyr Ala Thr Tyr Tyr Ala Asp Ser
325 330 335

Val Lys Asp Arg Phe Thr Ile Ser Arg Asp Asp Ser Lys Asn Thr Ala
340 345 350

Tyr Leu Gln Met Asn Asn Leu Lys Thr Glu Asp Thr Ala Val Tyr Tyr
355 360 365

Cys Val Arg His Gly Asn Phe Gly Asn Ser Tyr Val Ser Trp Phe Ala
370 375 380

Tyr Trp Gly Gln Gly Thr Leu Val Thr Val Ser Ser Gly Gly Gly Gly
385 390 395 400

Ser Gly Gly Gly Gly Ser Gly Gly Gly Gly Ser Glu Leu Val Val Thr
405 410 415

Gln Glu Pro Ser Leu Thr Val Ser Pro Gly Gly Thr Val Thr Leu Thr
420 425 430

Cys Arg Ser Ser Thr Gly Ala Val Thr Thr Ser Asn Tyr Ala Asn Trp
435 440 445

Val Gln Gln Lys Pro Gly Gln Ala Pro Arg Gly Leu Ile Gly Gly Thr
450 455 460

Asn Lys Arg Ala Pro Gly Thr Pro Ala Arg Phe Ser Gly Ser Leu Leu
465 470 475 480

Gly Gly Lys Ala Ala Leu Thr Leu Ser Gly Val Gln Pro Glu Asp Glu
485 490 495

```
Ala Glu Tyr Tyr Cys Ala Leu Trp Tyr Ser Asn Leu Trp Val Phe Gly
            500                 505                 510

Gly Gly Thr Lys Leu Thr Val Leu Asp Lys Thr His Thr Cys Pro Pro
        515                 520                 525

Cys Pro Ala Pro Glu Ala Ala Gly Gly Pro Ser Val Phe Leu Phe Pro
    530                 535                 540

Pro Lys Pro Lys Asp Gln Leu Met Ile Ser Arg Thr Pro Glu Val Thr
545                 550                 555                 560

Cys Val Val Val Asp Val Ser His Glu Asp Pro Glu Val Lys Phe Asn
                565                 570                 575

Trp Tyr Val Asp Gly Val Glu Val His Asn Ala Lys Thr Lys Pro Arg
            580                 585                 590

Glu Glu Gln Tyr Ala Ser Thr Tyr Arg Val Val Ser Val Leu Thr Val
        595                 600                 605

Leu His Gln Asp Trp Leu Asn Gly Lys Glu Tyr Lys Cys Lys Val Ser
    610                 615                 620

Asn Lys Ala Leu Pro Ala Ser Ile Glu Lys Thr Ile Ser Lys Ala Lys
625                 630                 635                 640

Gly Gln Pro Arg Glu Pro Gln Val Tyr Thr Leu Pro Pro Ser Arg Asp
                645                 650                 655

Glu Leu Thr Lys Asn Gln Val Ser Leu Thr Cys Leu Val Lys Gly Phe
            660                 665                 670

Tyr Pro Ser Asp Ile Ala Val Glu Trp Glu Ser Asn Gly Gln Pro Glu
        675                 680                 685

Asn Asn Tyr Lys Thr Thr Pro Pro Val Leu Asp Ser Asp Gly Ser Phe
    690                 695                 700

Phe Leu Tyr Ser Lys Leu Thr Val Asp Lys Ser Arg Trp Gln Gln Gly
705                 710                 715                 720

Asn Val Phe Ser Cys Ser Val Leu His Glu Ala Leu His Asn His Tyr
                725                 730                 735

Thr Gln Lys Ser Leu Ser Leu Ser Pro Gly
```

740 745

<210> 286
<211> 2238
<212> DNA
<213> Artificial sequence

<220>
<223> bispecific antibody AB11K nucleotide sequence

<400> 286
gaggtgcagc tggtggagtc cggaggaggc ctggtgcagc caggaggaag catgaggctg 60

tcttgcgtgg cttccggctt ccctttttagc aactactgga tgaattgggt gcggcaggct 120

ccaggcaagg gcctggagtg ggtgggcgag atcagactga agtctaacaa ttacacaacc 180

cattatgctg agtccgtgaa gggccgcttc acaatcagca gggacgatag caagaactct 240

ctgtatctgc agatgaattc cctgaagaca gaagatactg ccgtgtatta ttgcaccaga 300

cactattact ttgattactg gggtcaaggc acactggtta ccgtgagctc tggaggagga 360

ggatctggag gaggaggctc cggaggagga ggaagcgaca tcgtgatgac acagtcccct 420

ctgagcaacc cagtgacccc tggagagcca gcttccatca gctgtcggtc cagcaagagc 480

ctgctgcact ctaatggcat cacctacttc ttttggtatc tgcagaagcc tggccagagc 540

ccacagctgc tgatctacca gatgtccaac ctggccagcg gcgtgccaga tagattctct 600

ggctccggca gcggcacaga ctttaccctg cggatctcta gagtggaggc tgaggatgtg 660

ggcgtgtatt actgcgccca gaatctggag ctgcctccaa ccttcggcca gggcacaaag 720

gtggagatca agggtggcgg cggtggagga tccggcggtg gaggtagcgg cggaggcggt 780

agctccagct ctagtaaagc tcccctcct tccgaggtgc agctgctgga gtccggagga 840

ggactggtgc agccaggagg ctccctgaag ctgagctgtg ctgcctctgg ctttaccttc 900

aacacatatg ccatgaattg ggtgcggcag gctccaggca agggactgga gtgggtggct 960

aggatcaggt ctaagtacaa caattatgcc acctactatg ctgattccgt gaaggacagg 1020

ttcaccatct cccgcgacga tagcaagaac acagcctacc tgcagatgaa caatctgaag 1080

accgaggata ccgccgtgta ctactgcgtg agacatggca actttggcaa tagctacgtg 1140

tcctggttcg cttactgggg acagggcacc ctggtcacag tgagctctgg aggaggagga 1200

tctggaggag gaggctccgg aggaggagga agcgagctgg tggtgaccca ggagccatct 1260

ctgacagtgt cccccggcgg cacagtgacc ctgacatgta gatccagcac cggcgccgtg 1320

accacatcca actacgctaa ttgggtgcag cagaagccag gacaggctcc aaggggactg 1380

atcggaggaa ccaacaagag ggctcctgga acaccagctc ggtttagcgg atctctgctg 1440

ggaggcaagg ctgccctgac cctgtccgga gtgcagccag aggatgaggc cgagtattat 1500

tgcgctctgt ggtatagcaa tctgtgggtg ttcggaggag gaaccaagct gacagtgctg 1560

```
gacaagaccc atacatgccc accatgccct gcccctgaag ccgccggagg accttccgtg    1620
ttcctgttcc ctcccaagcc aaaagatcag ctgatgatct ctagaacccc cgaagtcacc    1680
tgcgtggtcg tcgacgtgtc ccatgaggac cctgaagtca agttcaactg gtacgtggac    1740
ggtgtcgaag tccacaacgc caagaccaag cctagggagg agcagtatgc cagcacatac    1800
cgggtggtgt ctgtgctgac cgtgctgcat caggattggc tgaatggcaa ggaatataaa    1860
tgtaaggtga gcaataaggc tctgccggct agcattgaaa aaaccatttc caaggctaag    1920
ggccagccca gggagcctca ggtctacacc ctgcctccat ctagagatga actgaccaaa    1980
aaccaggtga gcctgacttg cctggtcaaa ggcttctacc ccagcgacat tgccgtggag    2040
tgggagtcta atggccagcc tgaaaataac tacaaaacta cccctcctgt gctggactct    2100
gatggctcct tctttctgta ctctaaactg accgtggaca agtctcgctg gcagcagggt    2160
aacgtgtttt cttgctccgt gctgcacgag gctctgcata accattacac ccagaagagc    2220
ctgtctctgt ccccagga                                                  2238
```

## Description

CROSS-REFERENCE TO RELATED APPLICATION(S)

This application claims priority to Chinese patent application No. CN 201811294887.4 filed on Nov. 1, 2018, the disclosure of which is incorporated herein by reference in its entirety.

TECHNICAL FIELD

The present disclosure relates to the field of immunology and, more specifically, to an anti-CD3 bispecific antibody that mediates T-cell killing and the use of such an antibody, particularly in the use thereof for the treatment of cancer.

BACKGROUND

In 1985, the concept of killing tumor cells using T cells was proposed (Stearz UD et al., Nature, 314: 628-631, 1985). It is generally believed that the effective activation of T cells requires a dual signal, in which the first signal comes from the binding of the MHC-antigen complex to the T-cell receptor TCR-CD3 on the antigen-presenting cell and the second signal is a non-antigen-specific costimulatory signal resulting from the interaction of the T cell with a costimulatory molecule expressed by the antigen-presenting cell. Due to the down-regulated expression or even the deletion of MHC on the surface of most tumor cells, the tumor cells can escape the immune killing.

The bispecific antibodies can be classified according to the action mechanism into dual signal blocking type and cell-mediated functional type. Generally, the cell-mediated functional bispecific antibody refers to the anti-CD3 bispecific antibody that mediates the T-cell killing. The CD3 molecule is expressed on the surface of all mature T cells, and non-covalently binds to the TCR to form an intact TCR-CD3 complex, which jointly participates in the immune response to antigen stimulation and is the most used and the most successful trigger molecule on the surface of immune effector cells among bispecific antibodies. The bispecific antibody targeting CD3 can bind to the T cell surface CD3 and the tumor cell surface antigen, respectively, thus shortening the distance between cytotoxic T cells (Tc or CTL) and tumor cells and directly activating T cells to induce T cells to directly kill cancer cells instead of relying on the conventional dual activation signal of T cells. However, the agonistic antibody targeting the T cell antigen CD3, for example, the first generation of mouse monoclonal antibody OKT3 targeting human CD3 applied to the clinical practice (Kung P et al., Science, 206: 347-349, 1979), releases a large number of inflammatory factors such as interleukin-2 (IL-2), TNF-$\alpha$, IFN-$\gamma$, and interleukin-6 (IL-6) due to the hyperactivation of T cells, which clinically causes a severe "cytokine storm syndrome" (Hirsch R et al., J. Immunol., 142: 737-743, 1989) and thus resulting in "influenza-like" symptoms characterized by fever, chills, headache, nausea, vomiting, diarrhea, respiratory distress, aseptic meningitis, and hypotension. Thus, how to attenuate or avoid the excessive cytokine storm is a priority for the development of bifunctional antibodies targeting CD3.

In recent years, to solve the problem of correctly assembling two different half-antibodies, scientists have designed and developed bispecific antibodies of various structures. Overall, there are two categories. The one is that the bispecific antibody does not include an Fc region. The advantages of bispecific antibodies in such a structure are that they have a small molecular weight, can be expressed in prokaryotic cells, and do not need to be correctly assembled, while their disadvantages are that due to the lack of the antibody Fc fragment and the relatively low molecular weight, they have a short half-life period and that such bispecific antibodies are highly susceptible to polymerization, and thus have poor stability and low expression, and thus are limited in the clinical application. Such bispecific antibodies that have been reported so far include BiTE, DART, TrandAbs, bi-Nanobody, etc.

The other is that the bispecific antibody retains an Fc domain. Such bispecific antibodies form an IgG-like structure with a larger molecular structure and have a longer half-life period due to the FcRn-mediated endocytosis and recycling process; meanwhile, they also retain some or all of the Fc-mediated effector functions, such as antibody-dependent cell-mediated cytotoxicity (ADCC), complement-dependent cytotoxicity (CDC), and antibody-dependent cellular phagocytosis (ADCP). Such bispecific antibodies that have been reported so far include Triomabs, kih IgG, Crossmab, orthoFab IgG, DVD IgG, IgG scFv, $scFv_2$-Fc, etc. As for anti-CD3 bispecific antibodies, currently, except for configurations TandAb and scFv-Fv-scFv, other anti-CD3 bispecific antibodies are widely designed in the form of univalent anti-CD3, mainly because bivalent anti-CD3 bispecific antibodies can easily lead to hyperactivation and thus induce T cell apoptosis and massive transient release of cytokines (Kuhn C et al, Immunotherapy, 8: 889-906, 2016) and, more seriously, they may also trigger non-antigen-dependent activation of T cells and thus disrupt immune homeostasis. Therefore, most of the anti-CD3 bispecific antibodies in the existing art avoid the introduction of bivalent anti-CD3 antibodies. For example, bispecific antibodies in configurations of triFab-Fc, DART-Fc, and BiTE-Fc are designed asymmetrically (that is, heterodimer-type bispecific antibodies) (Z Wu et al, Pharmacology and Therapeutics, 182: 161-175, 2018), but such a design poses many challenges for downstream production of such heterodimeric bispecific antibodies, such as the generation of undesired homodimers or mismatched impurity molecules, which increase the difficulty of expression and purification of bispecific antibodies. Although the use of the "knobs-into-holes" technique to some extent solves the problem of inter-heavy chain mismatching of heteromeric bispecific antibody molecules, "light chain/heavy chain mismatching" brings about another challenge. One strategy for preventing heavy chain-light chain mismatching is to interchange the partial domains of the light chain and heavy chain of one of Fabs of a bispecific antibody to form a Crossmab (a hybrid antibody), which can allow selective pairing between the light chains/heavy chains. However, the disadvantages of this method are that the generation of mismatching products cannot be completely prevented, and residual fractions of any mismatching molecule are difficult to separate from the products, and in addition, this method requires a large number of genetic engineering modifications such as mutations for two antibody sequences. Thus, this method cannot become simple and universal.

Furthermore, for bispecific antibodies including CD3-specific IgG-like structures, such bispecific antibodies may cause unrestricted long-lasting T-cell activation due to their ability to bind to FcγR, and such activation is non-target cell-restricted, that is, activated T-cells can be found in tissues expressing FcγR (e.g., in hematopoietic, lymphoid, and reticuloendothelial systems), whether or not such bispecific antibodies bind to the target antigen. The systemic activation of such T cells will be accompanied by a substantial release of cytokines, which is a serious adverse effect during the therapeutic application of T cells-activated cytokines or antibodies. Therefore, such anti-CD3 bispecific antibodies that mediate the T cell killing need to avoid Fc-mediated systemic activation of T cells, thus allowing immune effector cells to be restrictedly activated within target cell tissues, that is, relying exclusively on the binding of the bispecific antibodies to the corresponding target antigens.

Therefore, there is an urgent need in the art to develop novel bispecific molecules with improved properties in terms of product half-life period, stability, safety, and productibility.

## SUMMARY

An object of the present disclosure is to provide a tetravalent homodimer-type bispecific antibody molecule targeting immune effector cell antigen CD3 and a tumor-associated antigen (TAA). Such a bispecific antibody can significantly inhibit or kill tumor cells *in vivo,* but has significantly reduced non-specific killing effect for normal cells with low TAA expression, and meanwhile has controlled toxic side effects that may be caused by excessive activation of effector cells, and significantly improved physicochemical and *in vivo* stabilities.

In a first aspect of the present disclosure, provided is a bispecific antibody which is a tetravalent homodimer formed by two identical polypeptide chains that bind to each other by a covalent bond, wherein each of the two identical polypeptide chains includes, in sequence from N-terminus to C-terminus, a first single-chain Fv that specifically binds to a tumor-associated antigen (anti-TAA scFv), a second single-chain Fv that specifically bind to effector cell antigen CD3 (anti-CD3 scFv), and an Fc fragment; wherein the first and the second single-chain Fvs are linked by a linker peptide, the second single-chain Fv and the Fc fragment are linked directly or by a linker peptide, and the Fc fragment does not have effector functions.

The first single-chain Fv has specificity to the tumor-associated antigen and includes a VH domain and a VL domain linked by a linker peptide (L1), wherein VH, L1, and VL are arranged in the order of VH-L1-VL or VL-L1-VH, and the amino acid sequence of the linker peptide L1 is $(GGGGX)_n$, wherein X includes Ser or Ala, preferably Ser, and n is a natural number of 1 to 5, preferably 3;

Illustratively, the tumor-associated antigen includes, but is not limited to, CD19, CD20, CD22, CD25, CD30, CD33, CD38, CD39, CD40, CD47, CD52, CD73, CD74, CD123, CD133, CD138, BCMA, CA125, CEA, CS1, DLL3, DLL4, EGFR, EpCAM, FLT3, gpA33, GPC-3, Her2, MEGE-A3, NYESO1, PSMA, TAG-72, CIX, folate-binding protein, GD2, GD3, GM2, VEGF, VEGFR2, VEGFR3, Cadherin, Integrin, Mesothelin, Claudin18, αVβ3, α5β1, ERBB3, c-MET, IGF1R, EPHA3, TRAILR1, TRAILR2, RANKL, B7 protein family, Mucin, FAP, and Tenascin; preferably, the tumor-associated antigen is CD19, CD20, CD22, CD30, CD38, BCMA, CS1, EpCAM, CEA, Her2, EGFR, CA125, Mucin1, GPC-3, and Mesothelin.

For example, some preferred amino acid sequences of the VH domain and its complementary determining regions (HCDR1, HCDR2, and HCDR3) and amino acid sequences of the VL domain and its complementary determining regions (LCDR1, LCDR2 and LCDR3) of a first single-chain Fv targeting the tumor-associated antigen are exemplified in Table 6-1 of the present disclosure.

Preferably, the first single-chain Fv specifically binds to CD19 and is selected from the group consisting of:

(i) a VH domain comprising HCDR1, HCDR2, and HCDR3 as shown in SEQ ID NOs: 9, 10, and 11, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 9, 10, and 11; and a VL domain comprising LCDR1, LCDR2, and LCDR3 as shown in SEQ ID NOs: 12, 13, and 14, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 12, 13, and 14;

(ii) a VH domain comprising HCDR1, HCDR2, and HCDR3 as shown in SEQ ID NOs: 17, 18, and 19, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 17, 18, and 19; and a VL domain comprising LCDR1, LCDR2, and LCDR3 as shown in SEQ ID NOs: 20, 21, and 22, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 20, 21, and 22;

(iii) a VH domain comprising HCDR1, HCDR2, and HCDR3 as shown in SEQ ID NOs: 25, 26, and 27, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 25, 26, and 27; and a VL domain comprising LCDR1, LCDR2, and LCDR3 as shown in SEQ ID NOs: 28, 29, and 30, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 28, 29, and 30; and

(iv) a VH domain comprising HCDR1, HCDR2, and HCDR3 as shown in SEQ ID NOs: 33, 34, and 35, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 33, 34, and 35; and a VL domain comprising LCDR1, LCDR2, and LCDR3 as shown in SEQ ID NOs: 36, 37, and 38, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 36, 37, and 38.

Preferably, the first single-chain Fv specifically binds to CD20 and is selected from the group consisting of:

(i) a VH domain comprising HCDR1, HCDR2, and HCDR3 as shown in SEQ ID NOs: 41, 42, and 43, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 41, 42, and 43; and a VL domain comprising LCDR1, LCDR2, and LCDR3 as shown in SEQ ID NOs: 44, 45, and 46, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 44, 45, and 46;

(ii) a VH domain comprising HCDR1, HCDR2, and HCDR3 as shown in SEQ ID NOs: 49, 50, and 51, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 49, 50, and 51; and a VL domain comprising LCDR1, LCDR2, and LCDR3 as shown in SEQ ID NOs: 52, 53, and 54, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 52, 53, and 54;

(iii) a VH domain comprising HCDR1, HCDR2, and HCDR3 as shown in SEQ ID NOs: 57, 58, and 59, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 57, 58, and 59; and a VL domain comprising LCDR1, LCDR2, and LCDR3 as shown in SEQ ID NOs: 60, 61, and 62, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 60, 61, and 62; and

(iv) a VH domain comprising HCDR1, HCDR2, and HCDR3 as shown in SEQ ID NOs: 65, 66, and 67, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 65, 66, and 67; and a VL domain comprising LCDR1, LCDR2, and LCDR3 as shown in SEQ ID NOs: 68, 69, and 70, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 68, 69, and 70.

Preferably, the first single-chain Fv specifically binds to CD22 and is selected from the group consisting of:

(i) a VH domain comprising HCDR1, HCDR2, and HCDR3 as shown in SEQ ID NOs: 73, 74, and 75, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 73, 74, and 75; and a VL domain comprising LCDR1, LCDR2, and LCDR3 as shown in SEQ ID NOs: 76, 77, and 78, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 76, 77, and 78; and

(ii) a VH domain comprising HCDR1, HCDR2, and HCDR3 as shown in SEQ ID NOs: 81, 82, and 83, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 81, 82, and 83; and a VL domain comprising LCDR1, LCDR2, and LCDR3 as shown in SEQ ID NOs: 84, 85, and 86, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 84, 85, and 86.

Preferably, the first single-chain Fv specifically binds to CD30 and is selected from the group consisting of:

(i) a VH domain comprising HCDR1, HCDR2, and HCDR3 as shown in SEQ ID NOs: 89, 90, and 91, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 89, 90, and 91; and a VL domain comprising LCDR1, LCDR2, and LCDR3 as shown in SEQ ID NOs: 92, 93, and 94, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 92, 93, and 94; and

(ii) a VH domain comprising HCDR1, HCDR2, and HCDR3 as shown in SEQ ID NOs: 97, 98, and 99, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 97, 98, and 99; and a VL domain comprising LCDR1, LCDR2, and LCDR3 as shown in SEQ ID NOs: 100, 101, and 102, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 100, 101, and 102.

Preferably, the first single-chain Fv specifically binds to EpCAM and is selected from the group consisting of:

(i) a VH domain comprising HCDR1, HCDR2, and HCDR3 as shown in SEQ ID NOs: 105, 106, and 107, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 105, 106, and 107; and a VL domain comprising LCDR1, LCDR2, and LCDR3 as shown in SEQ ID NOs: 108, 109, and 110, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 108, 109, and 110; and

(ii) a VH domain comprising HCDR1, HCDR2, and HCDR3 as shown in SEQ ID NOs: 113, 114, and 115, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 113, 114, and 115; and a VL domain comprising LCDR1, LCDR2, and LCDR3 as shown in SEQ ID NOs: 116, 117, and 118, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 116, 117, and 118.

Preferably, the first single-chain Fv specifically binds to CEA and is selected from the group consisting of:

(i) a VH domain comprising HCDR1, HCDR2, and HCDR3 as shown in SEQ ID NOs: 121, 122, and 123, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 121, 122, and 123; and a VL domain comprising LCDR1, LCDR2, and LCDR3 as shown in SEQ ID NOs: 124, 125, and 126, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 124, 125, and 126;

(ii) a VH domain comprising HCDR1, HCDR2, and HCDR3 as shown in SEQ ID NOs: 129, 130, and 131, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 129, 130, and 131; and a VL domain comprising LCDR1, LCDR2, and LCDR3 as shown in SEQ ID NOs: 132, 133, and 134, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 132, 133, and 134; and

(iii) a VH domain comprising HCDR1, HCDR2, and HCDR3 as shown in SEQ ID NOs: 137, 138, and 139, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 137, 138, and 139; and a VL domain comprising LCDR1, LCDR2, and LCDR3 as shown in SEQ ID NOs: 140, 141, and 142, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 140, 141, and 142.

Preferably, the first single-chain Fv specifically binds to Her2 and is selected from the group consisting of:

(i) a VH domain comprising HCDR1, HCDR2, and HCDR3 as shown in SEQ ID NOs: 145, 146, and 147, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 145, 146, and 147; and a VL domain comprising LCDR1, LCDR2, and LCDR3 as shown in SEQ ID NOs: 148, 149, and 150, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 148, 149, and 150;

(ii) a VH domain comprising HCDR1, HCDR2, and HCDR3 as shown in SEQ ID NOs: 153, 154, and 155, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 153, 154, and 155; and a VL domain comprising LCDR1, LCDR2, and LCDR3 as shown in SEQ ID NOs: 156, 157, and 158, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 156, 157, and 158; and

(iii) a VH domain comprising HCDR1, HCDR2, and HCDR3 as shown in SEQ ID NOs: 161, 162, and 163, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 161, 162, and 163; and a VL domain comprising LCDR1, LCDR2, and LCDR3 as shown in SEQ ID NOs: 164, 165, and 166, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 164, 165, and 166.

Preferably, the first single-chain Fv specifically binds to EGFR and is selected from the group consisting of:

(i) a VH domain comprising HCDR1, HCDR2, and HCDR3 as shown in SEQ ID NOs: 169, 170, and 171, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 169, 170, and 171; and a VL domain comprising LCDR1, LCDR2, and LCDR3 as shown in SEQ ID NOs: 172, 173, and 174, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 172, 173, and 174;

(ii) a VH domain comprising HCDR1, HCDR2, and HCDR3 as shown in SEQ ID NOs: 177, 178, and 179, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 177, 178, and 179; and a VL domain comprising LCDR1, LCDR2, and LCDR3 as shown in SEQ ID NOs: 180, 181, and 182, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 180, 181, and 182; and

(iii) a VH domain comprising HCDR1, HCDR2, and HCDR3 as shown in SEQ ID NOs: 185, 186, and 187, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 185, 186, and 187; and a VL domain comprising LCDR1, LCDR2, and LCDR3 as shown in SEQ ID NOs: 188, 189, and 190, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 188, 189, and 190.

Preferably, the first single-chain Fv specifically binds to GPC-3; the VH domain of the first single-chain Fv includes HCDR1, HCDR2, and HCDR3 as shown in SEQ ID NOs: 193, 194, and 195, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 193, 194, and 195; and the VL domain of the first single-chain Fv includes LCDR1, LCDR2, and LCDR3 as shown in SEQ ID NOs: 196, 197, and 198, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 196, 197, and 198.

Preferably, the first single-chain Fv specifically binds to Mesothelin; the VH domain of the first single-chain Fv includes HCDR1, HCDR2, and HCDR3 as shown in SEQ ID

NOs: 201, 202, and 203, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 201, 202, and 203; and the VL domain of the first single-chain Fv includes LCDR1, LCDR2, and LCDR3 as shown in SEQ ID NOs: 204, 205, and 206, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 204, 205, and 206.

Preferably, the first single-chain Fv specifically binds to Mucin1 and is selected from the group consisting of:

(i) a VH domain comprising HCDR1, HCDR2, and HCDR3 as shown in SEQ ID NOs: 209, 210, and 211, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 209, 210, and 211; and a VL domain comprising LCDR1, LCDR2, and LCDR3 as shown in SEQ ID NOs: 212, 213, and 214, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 212, 213, and 214; and

(ii) a VH domain comprising HCDR1, HCDR2, and HCDR3 as shown in SEQ ID NOs: 217, 218, and 219, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 217, 218, and 219; and a VL domain comprising LCDR1, LCDR2, and LCDR3 as shown in SEQ ID NOs: 220, 221, and 222, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 220, 221, and 222.

Preferably, the first single-chain Fv specifically binds to CA125; the VH domain of the first single-chain Fv includes HCDR1, HCDR2, and HCDR3 as shown in SEQ ID NOs: 225, 226, and 227, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 225, 226, and 227; and the VL domain of the first single-chain Fv includes LCDR1, LCDR2, and LCDR3 as shown in SEQ ID NOs: 228, 229, and 230, respectively or having sequences that are substantially identical the (for example, are at least 80%, 85%, 90%, 92%, 95%, 97%, 98%, 99% or more similar to or have one or more amino acid substitutions (for example, conservative substitutions) than) to any of SEQ ID NOs: 228, 229, and 230.

Preferably, the first single-chain Fv specifically binds to BCMA; the VH domain of the first single-chain Fv includes HCDR1, HCDR2, and HCDR3 as shown in SEQ ID NOs: 233, 234, and 235, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 233, 234, and 235; and the VL domain of the first single-chain Fv includes LCDR1, LCDR2, and LCDR3 as shown in SEQ ID NOs: 236, 237, and 238, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 236, 237, and 238.

More preferably, the first single-chain Fv specifically binds to CD19 and is selected from the group consisting of:

(i) a VH domain comprising an amino acid sequence as shown in SEQ ID NO: 15 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 15; and a VL domain comprising an amino acid sequence as shown in SEQ ID NO: 16 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 16;

(ii) a VH domain comprising an amino acid sequence as shown in SEQ ID NO: 23 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 23; and a VL domain comprising an amino acid sequence as shown in SEQ ID NO: 24 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 24;

(iii) a VH domain comprising an amino acid sequence as shown in SEQ ID NO: 31 or having a sequence that is substantially identical (for example, is at least 80%, 85%, 90%, 92%, 95%, 97%, 98%, 99% or more similar to or has one or more amino acid substitutions (for example, conservative substitutions) than)to SEQ ID NO: 31; and a VL domain comprising an amino acid sequence as shown in SEQ ID NO: 32 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 32; and

(iv) a VH domain comprising an amino acid sequence as shown in SEQ ID NO: 39 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 39; and a VL domain comprising an amino acid sequence as shown in SEQ ID NO: 40 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 40.

More preferably, the first single-chain Fv specifically binds to CD20 and is selected from the group consisting of:

(i) a VH domain comprising an amino acid sequence as shown in SEQ ID NO: 47 or having a sequence that is substantially identical (for example, is at least 80%, 85%, 90%, 92%, 95%, 97%, 98%, 99% or more similar to or hasone or more amino acid substitutions (for example, conservative substitutions) than) to SEQ ID NO: 47; and a VL domain comprising an amino acid sequence as shown in SEQ ID NO: 48 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 48;

(ii) a VH domain comprising an amino acid sequence as shown in SEQ ID NO: 55 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 55; and a VL domain comprising an amino acid sequence as shown in SEQ ID NO: 56 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 56;

(iii) a VH domain comprising an amino acid sequence as shown in SEQ ID NO: 63 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 63; and a VL domain comprising an amino acid sequence as shown in SEQ ID NO: 64 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 64; and

(iv) a VH domain comprising an amino acid sequence as shown in SEQ ID NO: 71 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 71; and a VL domain comprising an amino acid sequence as shown in SEQ ID NO: 72 or a sequence substantially identical to (for example, is at least 80%, 85%, 90%, 92%, 95%, 97%, 98%, 99% or more similar to or has one or more amino acid substitutions (for example, conservative substitutions) than) SEQ ID NO: 72.

More preferably, the first single-chain Fv specifically binds to CD22 and is selected from the group consisting of:

(i) a VH domain comprising an amino acid sequence as shown in SEQ ID NO: 79 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 79; and a VL domain comprising an amino acid sequence as shown in SEQ ID NO: 80 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 80; and

(ii) a VH domain comprising an amino acid sequence as shown in SEQ ID NO: 87 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 87; and a VL domain comprising an amino acid sequence as shown in SEQ ID NO: 88 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 88.

More preferably, the first single-chain Fv specifically binds to CD30 and is selected from the group consisting of:

(i) a VH domain comprising an amino acid sequence as shown in SEQ ID NO: 95 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 95; anda VL domain comprising an amino acid sequence as shown in SEQ ID NO: 96 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 96; and
(ii) a VH domain comprising an amino acid sequence as shown in SEQ ID NO: 103 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 103; and a VL domain comprising an amino acid sequence as shown in SEQ ID NO: 104 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 104.

More preferably, the first single-chain Fv specifically binds to EpCAM and is selected from the group consisting of:

(i) a VH domain comprising an amino acid sequence as shown in SEQ ID NO: 111 or having a sequence that is substantially identical (for example, is at least 80%, 85%, 90%, 92%, 95%, 97%, 98%, 99% or more similar to or hs one or more amino acid substitutions (for example, conservative substitutions) than) to SEQ ID NO: 111; and a VL domain comprising an amino acid sequence as shown in SEQ ID NO: 112 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 112; and

(ii) a VH domain comprising an amino acid sequence as shown in SEQ ID NO: 119 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 119; and a VL domain comprising an amino acid sequence as shown in SEQ ID NO: 120 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 120.

More preferably, the first single-chain Fv specifically binds to CEA and is selected from the group consisting of:

(i) a VH domain comprising an amino acid sequence as shown in SEQ ID NO: 127 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 127,; and a VL domain comprising an amino acid sequence as shown in SEQ ID NO: 128 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 128;

(ii) a VH domain comprising an amino acid sequence as shown in SEQ ID NO: 135 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 135; and a VL domain comprising an amino acid sequence as shown in SEQ ID NO: 136 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 136; and

(iii) a VH domain comprising an amino acid sequence as shown in SEQ ID NO: 143 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 143; and a VL domain comprising an amino acid sequence as shown in SEQ ID NO: 144 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 144.

More preferably, the first single-chain Fv specifically binds to Her2 and is selected from the group consisting of:

(i) a VH domain comprising an amino acid sequence as shown in SEQ ID NO: 151 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 151; and a VL domain comprising an amino acid sequence as shown in SEQ ID NO: 152 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 152;

(ii) a VH domain comprising an amino acid sequence as shown in SEQ ID NO: 159 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 159; and a VL domain comprising an amino acid sequence as shown in SEQ ID NO: 160 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 160; and

(iii) a VH domain comprising an amino acid sequence as shown in SEQ ID NO: 167 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 167; and a VL domain comprising an amino acid sequence as shown in SEQ ID NO: 168 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 168.

More preferably, the first single-chain Fv specifically binds to EGFR and is selected from the group consisting of:

(i) a VH domain comprising an amino acid sequence as shown in SEQ ID NO: 175 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 175; and a VL domain comprising an amino acid sequence as shown in SEQ ID NO: 176 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 176;

(ii) a VH domain comprising an amino acid sequence as shown in SEQ ID NO: 183 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 183; and a VL domain comprising an amino acid sequence as shown in SEQ ID NO: 184 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 184; and

(iii) a VH domain comprising an amino acid sequence as shown in SEQ ID NO: 191 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 191; and a VL domain comprising an amino acid sequence as shown in SEQ ID NO: 192 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 192.

More preferably, the first single-chain Fv specifically binds to GPC-3; the VH domain of the first single-chain Fv includes an amino acid sequence as shown in SEQ ID NO: 199 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 199; and the VL domain of the first single-chain Fv includes an amino acid sequence as shown in SEQ ID NO: 200 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 200.

More preferably, the first single-chain Fv specifically binds to Mesothelin; the VH domain of the first single-chain Fv includes an amino acid sequence as shown in SEQ ID NO: 207 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 207; and the VL domain of the first single-chain Fv includes an amino acid sequence as shown in SEQ ID NO: 208 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 208.

More preferably, the first single-chain Fv specifically binds to Mucin1 and is selected from the group consisting of:

(i) a VH domain comprising an amino acid sequence as shown in SEQ ID NO: 215 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 215; and a VL domain comprising an amino acid sequence as shown in SEQ ID NO: 216 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 216; and

(ii) a VH domain comprising an amino acid sequence as shown in SEQ ID NO: 223 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 223; and a VL domain comprising an amino acid sequence as shown in SEQ ID NO: 224 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 224.

More preferably, the first single-chain Fv specifically binds to CA125; the VH domain of the first single-chain Fv includes an amino acid sequence as shown in SEQ ID NO: 231 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 231; and the VL domain of the first single-chain Fv includes an amino acid sequence as shown in SEQ ID NO: 232 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 232.

More preferably, the first single-chain Fv specifically binds to BCMA; the VH domain of the first single-chain Fv includes an amino acid sequence as shown in SEQ ID NO: 239 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 239; and the VL domain of the first single-chain Fv includes an amino acid sequence as shown in SEQ ID NO: 240 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 240.

The linker peptide (L2) connecting the first single-chain Fv and the second single-chain Fv in the present disclosure consists of a flexible peptide and a rigid peptide.

Furthermore, the flexible peptide includes two or more amino acids that are preferably selected from the group consisting of the following amino acids: Gly(G), Ser(S), Ala(A), and Thr(T). More preferably, the flexible peptide includes G and S residues. Most preferably, the amino acid composition structure of the flexible peptide has a general formula $G_xS_y(GGGGS)_z$, where x, y, and z are integers greater than or equal to 0, and $x + y + z \geq 1$. For example, in a preferred embodiment, the amino acid sequence of the flexible peptide is $G_2(GGGGS)_3$.

Furthermore, the rigid peptide is derived from a full-length sequence (as shown in SEQ ID NO: 257) consisting of amino acids at positions 118 to 145 of the carboxy-terminus of the natural human chorionic gonadotropin beta-subunit, or a truncated fragment thereof (hereinafter collectively referred to as CTP). Preferably, the CTP rigid peptide includes 10 amino acids at the N-terminal of SEQ ID NO: 257, that is, SSSSKAPPPS ($CTP^1$); or the CTP rigid peptide includes 14 amino acids at the C-terminal of SEQ ID NO: 257, that is, SRLPGPSDTPILPQ ($CTP^2$); as another example, in another embodiment, the CTP rigid peptide includes 16 amino acids at the N-terminal of SEQ ID NO: 257, that is, SSSSKA-PPPSLPSPSR ($CTP^3$); for another example, in another embodiment, the CTP rigid peptide includes 28 amino acids that begin at the position 118 and end at position 145 of the human chorionic gonadotropin beta-subunit, that is, SSSSKA-PPPSLPSPSRLPGPSDTPILPQ ($CTP^4$).

For example, some preferred amino acid sequences of the linker peptide L2 that links the first single-chain Fv and second single-chain Fv are exemplified listed in Table 6-3 of the present disclosure.

In a preferred embodiment of the present disclosure, the linker peptide has an amino acid sequence as shown in SEQ ID NO: 258, wherein the amino acid composition of the flexible peptide is $G_2(GGGGS)_3$, and the amino acid composition of the rigid peptide is SSSSKAPPPS (that is, $CTP^1$).

The second single-chain Fv has specificity to immune effector cell antigen CD3 and includes a VH domain and a VL domain linked by a linker peptide (L3), wherein VH, L3, and VL are arranged in the order of VH-L3-VL or VL-L3-VH, and the amino acid sequence of the linker peptide L3 is $(GGGGX)_n$, wherein X includes Ser or Ala, preferably Ser; and n is a natural number of 1 to 5, preferably 3;

Preferably, the second single-chain Fv of the bispecific antibody binds to an effector cell at an $EC_{50}$ value greater than about 50 nM, or greater than 100 nM, or greater than 300 nM, or greater than 500 nM in an *in vitro* FACS binding affinity assay; more preferably, the second single-chain Fv of the bispecific antibody not only binds to human CD3, but also specifically binds to CD3 of a cynomolgus monkey or a rhesus monkey. In a preferred embodiment of the present disclosure, the bispecific antibody specifically binds to the effector cell at an $EC_{50}$ value of 132.3 nM.

For example, some preferred amino acid sequences of the VH domain and its complementary determining regions (HCDR1, HCDR2, and HCDR3) and amino acid sequences of the VL domain and its complementary determining regions (LCDR1, LCDR2 and LCDR3) of the anti-CD3 scFv are exemplified in Table 6-2 of the present disclosure.

Preferably, the second single-chain Fv specifically binds to CD3; the VH domain of the second single-chain Fv includes HCDR1, HCDR2, and HCDR3 as shown in SEQ ID NOs: 241, 242, and 243, respectively or having sequences that are substantially identical to (for example, are at least 80%, 85%, 90%, 92%, 95%, 97%, 98%, 99% or more similar to or have one or more amino acid substitutions (for example, conservative substitutions) than) SEQ ID NOs: 241, 242, and 243; and the VL domain of the second single-chain Fv includes LCDR1, LCDR2, and LCDR3 as shown in SEQ ID NOs: 244, 245, and 246, respectively or having sequences that are substantially identical to (for example, are at least 80%, 85%, 90%, 92%, 95%, 97%, 98%, 99% or more similar to or have one or more amino acid substitutions (for example, conservative substitutions) than) SEQ ID NOs: 244, 245, and 246.

Preferably, the second single-chain Fv specifically binds to CD3; the VH domain of the second single-chain Fv includes HCDR1, HCDR2, and HCDR3 as shown in SEQ ID NOs: 249, 250, and 251, respectively or having sequences are substantially identical to (for example, are at least 80%, 85%, 90%, 92%, 95%, 97%, 98%, 99% or more similar to or have one or more amino acid substitutions (for example, conservative substitutions) than) SEQ ID NOs: 249, 250, and 251; and the VL domain of the second single-chain Fv includes LCDR1, LCDR2, and LCDR3 as shown in SEQ ID NOs: 252, 253, and 254, respectively or having sequences that are substantially identical to (for example, are at least 80%, 85%, 90%, 92%, 95%, 97%, 98%, 99% or more similar to or have one or more amino acid substitutions (for example, conservative substitutions) than) SEQ ID NOs: 252, 253, and 254.

More preferably, the second single-chain Fv specifically binds to CD3; the VH domain of the second single-chain Fv includes an amino acid sequence as shown in SEQ ID NO: 247 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 247; and the VL domain of the second single-chain Fv includes an amino acid sequence as shown in SEQ ID NO: 248 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 248.

More preferably, the second single-chain Fv specifically binds to CD3; the VH domain of the second single-chain Fv includes an amino acid sequence as shown in SEQ ID NO: 255 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 255; and the VL domain of the second single-chain Fv includes an amino acid sequence as shown in SEQ ID NO: 256 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 256.

The Fc fragment of the present disclosure is linked to the second single-chain Fv directly or by the linker peptide L4, and the linker peptide L4 includes 1 to 20 amino acids, and preferably selected from the following amino acids: Gly(G), Ser(S), Ala(A), and Thr(T); more preferably, the linker peptide L4 is selected from Gly(G) and Ser(S); further preferably, the linker peptide L4 consists of $(GGGGS)_n$, wherein n = 1, 2, 3 or 4. In a preferred embodiment of the present disclosure, the Fc fragment is directly linked to the second single-chain Fv.

In another aspect, the Fc fragment of the present disclosure includes a hinge region, a CH2 domain, and a CH3 domain from a human immunoglobulin heavy chain constant region. For example, in some embodiments, the Fc fragment of the present disclosure is selected from heavy chain constant regions of human IgG1, IgG2, IgG3, IgG4, IgM, IgA1, IgA2, IgD, and IgE; particularly selected from heavy chain constant regions of human IgG1, IgG2, IgG3, and IgG4, and more particularly selected from a heavy chain constant region of human IgG1 or IgG4; and the Fc fragment has one or more amino acid substitutions, deletions or additions (for example, at most 20, at most 15, at most 10, or at most 5 substitutions, deletions or additions) than a natural sequence from which the Fc fragment is derived.

In some preferred embodiments, the Fc fragment is changed, for example, mutated to modify the properties of the bispecific antibody molecule of the present disclosure (for example, to change one or more of the following properties: Fc receptor binding, antibody glycosylation, an effector cell function or a complement function).

For example, the bispecific antibody provided by the present disclosure includes an Fc variant containing amino acid substitutions, deletions or additions that change (for example, reduce or eliminate) effector functions. Fc region of the antibody mediates several important effector functions, such as ADCC, ADCP, and CDC. Methods for changing the affinity of the antibody to an effector ligand (such as FcγR or a complement C1q) by substituting amino acid residues in the Fc region of the antibody to change the effector functions are known in the art (see, for example, EP 388151A1; U.S. 5648260; U.S. 5624821; Natsume A et al., Cancer Res., 68: 3863-3872, 2008; Idusogie EE et al., J. Immunol., 166: 2571-2575, 2001; Lazar GA et al., PNAS, 103: 4005-4010, 2006; Shields RL et al., JBC, 276: 6591-6604, 2001; Stavenhagen JB et al., Cancer Res., 67: 8882-8890, 2007; Stavenhagen JB et al., Advan. Enzyme. Regul., 48: 152-164, 2008; Alegre ML et al., J. Immunol., 148: 3461-3468, 1992; Kaneko E et al., Biodrugs, 25: 1-11, 2011). In some preferred embodiments of the present disclosure, amino acid L235 (EU numbering) in the constant region of the antibody is modified to change an interaction with an Fc receptor, such as L235E or L235A. In some other preferred embodiments, amino acids 234 and 235 in the constant region of the antibody are modified simultaneously, such as L234A and L235A (L234A/L235A) (EU numbering).

For example, the bispecific antibody provided by the present disclosure may include an Fc variant containing amino acid substitutions, deletions or additions that extend a circulating half-life. Studies show that M252Y/S254T/T256E, M428L/N434S or T250Q/M428L can extend the half-life of the antibody in primates. For more mutation sites included in the Fc variant with enhanced binding affinity to a neonatal receptor (FcRn), see Chinese invention patent CN 201280066663.2, U.S. 2005/0014934A1, WO 97/43316, U.S. 5,869,046, U.S. 5,747,03 and WO 96/32478. In some preferred embodiments of the present disclosure, amino acid M428 (EU numbering) in the constant region of the antibody is modified to enhance the binding affinity for the FcRn receptor, such as M428L. In some other preferred embodiments, amino acids 250 and 428 (EU numbering) in the constant region of the antibody are modified simultaneously, such as T250Q and M428L (T250Q/M428L).

For example, the bispecific antibody provided by the present disclosure may also include an Fc variant containing amino acid substitutions, deletions or additions that may reduce or eliminate Fc glycosylation. For example, the Fc variant contains reduced glycosylation of the N-linked glycan normally present at amino acid site 297 (EU numbering). The glycosylation at position N297 has a great effect on the activity of IgG. If the glycosylation at this position is eliminated, the conformation of the upper half of CH2 of an IgG molecule is affected, thus losing the ability of binding to FcγRs and affecting the biological activity related to the antibody. In some preferred embodiments of the present disclosure, amino acid N297 (EU numbering) in the constant region of human IgG is modified to avoid the glycosylation of the antibody, such as N297A.

For example, the bispecific antibody provided by the present disclosure may also include an Fc variant containing amino acid substitutions, deletions or additions that eliminate charge heterogeneity. Various post-translational modifications during expression in engineered cells will cause the charge heterogeneity of monoclonal antibodies. The heterogeneity of lysine at C-terminus of an IgG antibody is one of the main reasons for charge heterogeneity. Lysine K at C-terminus of a heavy chain may be deleted at a certain proportion during the production of the antibody, resulting in charge heterogeneity and affecting the stability, effectiveness, immunogenicity or pharmacokinetic of the antibody. In some preferred embodiments of the present disclosure, K447 (EU numbering) at the C-terminus of the IgG antibody is removed or deleted to eliminate the charge heterogeneity of the antibody and improve the homogeneity of the expressed product.

The amino acid sequences of some preferred Fc fragments are exemplarily listed in Table 6-4 of the present disclosure. Compared with a bispecific antibody containing the Fc region of wild-type human IgG, the bispecific antibody provided by the present disclosure contains an Fc fragment that exhibits reduced affinity for at least one of human FcyRs (FcγRI, FcγRIIa, or FcγRIIIa) and C1q, and has reduced effector cell functions or complement functions. For example, in a preferred embodiment of the present disclosure, the bispecific antibody includes an Fc fragment that is derived from human IgG1, has L234A and L235A substitutions (L234A/L235A), and exhibits reduced binding ability for FcγRI. In addition, the Fc fragment included in the bispecific antibody provided by the present disclosure may also contain amino acid substitutions that change one or more other properties (e.g., ability of binding to the FcRn receptor, the glycosylation of the antibody or the charge heterogeneity of the antibody). For example, in a preferred embodiment of the present disclosure, the Fc fragment has an amino acid sequence as shown in SEQ ID NO: 263, which has amino acid substitutions L234A/L235A/T250Q/N297A/P331S/M428L and a deleted or removed K447 compared with the native sequence from which it is derived.

The bispecific antibody molecule of the present disclosure is a tetravalent homodimer formed by two identical polypeptide chains that bind to each other by an interchain disulfide bond in the hing region of the Fc fragment, wherein each polypeptide chain consists of, in sequence from N-terminus to C-terminus, an anti-TAA scFv, a linker peptide, an anti-CD3 scFv, and an Fc fragment. For example, the amino acid sequences of some preferred bispecific antibodies are exemplified in Table 6-5 of the present disclosure.

In a preferred embodiment of the present disclosure, the bispecific antibody binds to human CD19 and CD3 and has an amino acid sequence as follows:

(i) a sequence as shown in SEQ ID NO: 264;

(ii) a sequence with one or more substitutions, deletions or additions (such as 1, 2, 3, 4 or 5 substitutions, deletions or additions) compared to the sequence as shown in SEQ ID NO: 264; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity relative to the sequence as shown in SEQ ID NO: 264;

In some preferred embodiments, the substitutions in (ii) are conservative substitutions.

In a preferred embodiment of the present disclosure, the bispecific antibody binds to human CD19 and CD3 and has an amino acid sequence as follows:

(i) a sequence as shown in SEQ ID NO: 283;

(ii) a sequence with one or more substitutions, deletions or additions (such as 1, 2, 3, 4 or 5 substitutions, deletions or additions) compared to the sequence as shown in SEQ ID NO: 283; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 283.

In some preferred embodiments, the substitutions in (ii) are conservative substitutions.

In a preferred embodiment of the present disclosure, the bispecific antibody binds to human CD20 and CD3 and has an amino acid sequence as follows:

(i) a sequence as shown in SEQ ID NO: 266;

(ii) a sequence with one or more substitutions, deletions or additions (such as 1, 2, 3, 4 or 5 substitutions, deletions or additions) compared to the sequence as shown in SEQ ID NO: 266; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 266.

In some preferred embodiments, the substitutions in (ii) are conservative substitutions.

In a preferred embodiment of the present disclosure, the bispecific antibody binds to human CD22 and CD3 and has an amino acid sequence as follows:

(i) a sequence as shown in SEQ ID NO: 268;

(ii) a sequence with one or more substitutions, deletions or additions (such as 1, 2, 3, 4 or 5 substitutions, deletions or additions) compared to the sequence as shown in SEQ ID NO: 268; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 268.

In some preferred embodiments, the substitutions in (ii) are conservative substitutions.

In a preferred embodiment of the present disclosure, the bispecific antibody binds to human CD30 and CD3 and has an amino acid sequence as follows:

(i) a sequence as shown in SEQ ID NO: 270;

(ii) a sequence with one or more substitutions, deletions or additions (such as 1, 2, 3, 4 or 5 substitutions, deletions or additions) compared to the sequence as shown in SEQ ID NO: 270; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 270.

In some preferred embodiments, the substitutions in (ii) are conservative substitutions.

In a preferred embodiment of the present disclosure, the bispecific antibody binds to human EpCAM and CD3 and has an amino acid sequence as follows:

(i) a sequence as shown in SEQ ID NO: 272;

(ii) a sequence with one or more substitutions, deletions or additions (such as 1, 2, 3, 4 or 5 substitutions, deletions or additions) compared to the sequence as shown in SEQ ID NO: 272; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 272.

In some preferred embodiments, the substitutions in (ii) are conservative substitutions.

In a preferred embodiment of the present disclosure, the bispecific antibody binds to human CEA and CD3 and has an amino acid sequence as follows:

(i) a sequence as shown in SEQ ID NO: 274;

(ii) a sequence with one or more substitutions, deletions or additions (such as 1, 2, 3, 4 or 5 substitutions, deletions or additions) compared to the sequence as shown in SEQ ID NO: 274; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 274.

In some preferred embodiments, the substitutions in (ii) are conservative substitutions.

In a preferred embodiment of the present disclosure, the bispecific antibody binds to human Her2 and CD3 and has an amino acid sequence as follows:

(i) a sequence as shown in SEQ ID NO: 8;

(ii) a sequence with one or more substitutions, deletions or additions (such as 1, 2, 3, 4 or 5 substitutions, deletions or additions) compared to the sequence as shown in SEQ ID NO: 8; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 8.

In some preferred embodiments, the substitutions in (ii) are conservative substitutions.

In a preferred embodiment of the present disclosure, the bispecific antibody binds to human EGFR and CD3 and has an amino acid sequence as follows:

(i) a sequence as shown in SEQ ID NO: 277;

(ii) a sequence with one or more substitutions, deletions or additions (such as 1, 2, 3, 4 or 5 substitutions, deletions or additions) compared to the sequence as shown in SEQ ID NO: 277; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 277.

In some preferred embodiments, the substitutions in (ii) are conservative substitutions.

In a preferred embodiment of the present disclosure, the bispecific antibody binds to human GPC-3 and CD3 and has an amino acid sequence as follows:

(i) a sequence as shown in SEQ ID NO: 279;

(ii) a sequence with one or more substitutions, deletions or additions (such as 1, 2, 3, 4 or 5 substitutions, deletions or additions) compared to the sequence as shown in SEQ ID NO: 279; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 279.

In some preferred embodiments, the substitutions in (ii) are conservative substitutions.

In a preferred embodiment of the present disclosure, the bispecific antibody binds to human Mesothelin and CD3 and has an amino acid sequence as follows:

(i) a sequence as shown in SEQ ID NO: 281;

(ii) a sequence with one or more substitutions, deletions or additions (such as 1, 2, 3, 4 or 5 substitutions, deletions or additions) compared to the sequence as shown in SEQ ID NO: 281; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 281.

In some preferred embodiments, the substitutions in (ii) are conservative substitutions.

In a preferred embodiment of the present disclosure, the bispecific antibody binds to human Mucin1 and CD3 and has an amino acid sequence as follows:

(i) a sequence as shown in SEQ ID NO: 285;

(ii) a sequence with one or more substitutions, deletions or additions (such as 1, 2, 3, 4 or 5 substitutions, deletions or additions) compared to the sequence as shown in SEQ ID NO: 285; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 285.

In some preferred embodiments, the substitutions in (ii) are conservative substitutions.

In a second aspect of the present disclosure, a DNA molecule encoding the bispecific antibody as described above is provided.

In a preferred embodiment of the present disclosure, the DNA molecule encoding the bispecific antibody as described above is a nucleotide sequence as shown in SEQ ID NO: 265.

In a preferred embodiment of the present disclosure, the DNA molecule encoding the bispecific antibody as described above is a nucleotide sequence as shown in SEQ ID NO: 267.

In a preferred embodiment of the present disclosure, the DNA molecule encoding the bispecific antibody as described above is a nucleotide sequence as shown in SEQ ID NO: 269.

In a preferred embodiment of the present disclosure, the DNA molecule encoding the bispecific antibody as described above is a nucleotide sequence as shown in SEQ ID NO: 271.

In a preferred embodiment of the present disclosure, the DNA molecule encoding the bispecific antibody as described above is a nucleotide sequence as shown in SEQ ID NO: 273.

In a preferred embodiment of the present disclosure, the DNA molecule encoding the bispecific antibody as described above is a nucleotide sequence as shown in SEQ ID NO: 275.

In a preferred embodiment of the present disclosure, the DNA molecule encoding the bispecific antibody as described above is a nucleotide sequence as shown in SEQ ID NO: 276.

In a preferred embodiment of the present disclosure, the DNA molecule encoding the bispecific antibody as described above is a nucleotide sequence as shown in SEQ ID NO: 278.

In a preferred embodiment of the present disclosure, the DNA molecule encoding the bispecific antibody as described above is a nucleotide sequence as shown in SEQ ID NO: 280.

In a preferred embodiment of the present disclosure, the DNA molecule encoding the bispecific antibody as described above is a nucleotide sequence as shown in SEQ ID NO: 282.

In a preferred embodiment of the present disclosure, the DNA molecule encoding the bispecific antibody as described above is a nucleotide sequence as shown in SEQ ID NO: 284.

In a preferred embodiment of the present disclosure, the DNA molecule encoding the bispecific antibody as described above is a nucleotide sequence as shown in SEQ ID NO: 286.

In a third aspect of the present disclosure, a vector comprising the DNA molecule as described above is provided.

In a fourth aspect of the present disclosure, a host cell comprising the vector as described above is provided; the host cell includes a prokaryotic cell, a yeast or a mammalian cell, such as a CHO cell, an NS0 cell or another mammalian cell, preferably a CHO cell.

In a fifth aspect of the present disclosure, provided is a pharmaceutical composition, comprising the bispecific antibody as described above and a pharmaceutically acceptable excipient, carrier or diluent.

In a sixth aspect of the present disclosure, further provided a method for preparing the bispecific antibody as described in the present disclosure, comprising: (a) obtaining a fusion gene of the bispecific antibody to construct an expression vector of the bispecific antibody; (b) transfecting the expression vector into a host cell by a genetic engineering method; (c) culturing the host cell under conditions that allow the bispecific antibody to be generated; and (d) separating and purifying the generated bispecific antibody;

The expression vector in step (a) is one or more selected from plasmids, bacteria, and viruses, and preferably the expression vector is a pCDNA3.1 vector;

The host cell into which the constructed vector is transfected by the genetic engineering method in step (b) includes a prokaryotic cell, a yeast or a mammalian cell, such as a CHO cell, an NS0 cell or another mammalian cell, preferably a CHO cell.

The bispecific antibody is separated and purified in step (d) by a conventional immunoglobulin purification method comprising protein A affinity chromatography and ion exchange, hydrophobic chromatography or molecular sieve.

In a seventh aspect of the present disclosure, use of the bispecific antibody in the preparation of a medicament for the treatment, prevention or alleviation of tumor is provided; examples of the cancer include, but are not limited to, mesothelioma, squamous cell carcinoma, myeloma, osteosarcoma, glioblastoma, neuroglioma, malignant epithelial tumours, adenocarcinoma, melanoma, sarcoma, acute and chronic leukemia, lymphoma and meningioma, Hodgkin's lymphoma, Sezary syndrome, multiple myeloma, lung cancer, non-small cell lung cancer, small cell lung cancer, laryngeal cancer, breast cancer, head and neck cancer, bladder cancer, uterine cancer, skin cancer, prostate cancer, cervical cancer, vaginal cancer, gastric cancer, renal cell carcinoma, renal carcinoma, pancreatic cancer, colorectal cancer, endometrial carcinoma, esophageal carcinoma, hepatobiliary cancer, bone cancer, skin cancer and blood cancer, and carcinoma of nasal cavity and sinus, nasopharyngeal carcinoma, oral cancer, oropharyngeal cancer, laryngeal cancer, sublaryngeal cancer, salivary cancer, mediastinal cancer, stomach cancer, small intestine cancer, colon cancer, cancer of rectum and anal regions, ureter cancer, urethral cancer, carcinoma of penis, testicular cancer, vulva cancer, cancer of endocrine system, cancer of central nervous system, and plasmocytoma.

In an eighth aspect of the present disclosure, provided is the bispecific antibody for use in a method for enhancing or stimulating an immune response or function, comprising administering to a patient/subject individual a therapeutically effective amount of the bispecific antibody.

In a ninth aspect of the present disclosure, provided is the bispecific antibody for use in a method for treating, delaying development of, or reducing/inhibiting recurrence of a tumor, comprising: giving or administering an effective amount of the bispecific antibody to an individual suffering from the foregoing disease or discorder; examples of the tumor include, but are not limited to, mesothelioma, squamous cell carcinoma, myeloma, osteosarcoma, glioblastoma, neuroglioma, malignant epithelial tumours, adenocarcinoma, melanoma, sarcoma, acute and chronic leukemia, lymphoma and meningioma, Hodgkin's lymphoma, Sezary syndrome, multiple myeloma, lung cancer, non-small cell lung cancer, small cell lung cancer, laryngeal cancer, breast cancer, head and neck cancer, bladder cancer, uterine cancer, skin cancer, prostate cancer, cervical cancer, vaginal cancer, gastric cancer, renal cell carcinoma, renal carcinoma, pancreatic cancer, colorectal cancer, endometrial carcinoma, esophageal carcinoma, hepatobiliary cancer, bone cancer, skin cancer and blood cancer, and carcinoma of nasal cavity and sinus, nasopharyngeal carcinoma, oral cancer, oropharyngeal cancer, laryngeal cancer, sublaryngeal cancer, salivary cancer, mediastinal cancer, cervical cancer, small intestine cancer, colon cancer, cancer of rectum and anal regions, ureter cancer, carcinoma of urethral cancer, carcinoma of penis, testicular cancer, vulva cancer, cancer of endocrine system, cancer of central nervous system, and plasmocytoma.

The technical solutions provided by the present disclosure have beneficial effects summarized as follows:

1. The bispecific antibody provided by the present disclosure includes anti-TAA scFv located at the N-terminus of the bispecific antibody and having changed spatial conformation, so that the bispecific antibody has reduced binding ability to TAA under some conditions, especially that the bispecific antibody is difficult to bind to normal cells with weak expression or low expression of TAAs, thereby exhibiting reduced non-specific killing effect. However, the binding specificity to cells with over-expression or high expression of TAA is not significantly reduced, and the bispecific antibody exhibits a good killing effect *in vivo.* It can be known that when a target antigen is merely expressed on tumor cells or the bispecific antibody of the present disclosure specifically binds to tumor cells over-expressing the target antigen, immune effector cells are activated restrictively and merely in target cell tissues, which can minimize the non-specific killing of the bispecific antibody on the normal cells and the accompanying release of cytokines and reduce the toxic side effects of the bispecific antibody in clinical treatment.

2. The anti-CD3 scFv selected by the bispecific antibody provided by the present disclosure specifically binds to effector cells with a weak binding affinity ($EC_{50}$ value greater than about 50 nM, or greater than 100 nM, or greater than 300 nM, or greater than 500 nM). In addition, the anti-CD3 scFv is embedded between the anti-TAA scFv and Fc, and the CTP rigid peptide contained in the linker peptide L3 at the N-terminus and the Fc fragment located at its C-terminus partially "cover" or "shield" the antigen-binding domain of the anti-CD3 scFv. Such steric hindrance effect makes the anti-CD3 scFv bind to CD3 with a weaker binding affinity (for example, greater than 1 $\mu$M), which reduces its ability to activate and stimulate T cells, limits the excessive release of cytokines, and provides higher safety. In addition, the anti-CD3 scFv used in the present disclosure can bind to CD3 native antigens from humans and cynomolgus monkeys and/or rhesus monkeys at the same time, so that no alternative molecule needs to be constructed for preclinical toxicology evaluation and the effective dose, toxic dose and toxic side effects obtained are more objective and accurate and can be directly converted into a clinical dose to reduce the risk of clinical studies. Further, the bispecific antibody provided by the present disclosure creatively adopts a divalent anti-CD3 scFv, which avoids an asymmetric structure of a heterodimer (including a monovalent anti-CD3 scFv) commonly used in the existing art in terms of the configuration design of the bispecific antibody and solves the problem of heavy chain mismatches, thereby simplifying downstream purification steps. Moreover, unexpectedly, the non-specific binding of the anti-CD3 scFv to T cells is not observed in an *in vitro* cell binding assay, and the degree of cell activation (the release of cytokines such as IL-2) is controlled within a safe and effective range. That is, the bivalent anti-CD3 scFv structure used in the present disclosure has not induced the over-activation of T cells in a non-antigen-dependent manner, whereas for other bispecific antibodies including bivalent anti-CD3 domains, the uncontrollable over-activation of T cells is common and thus anti-CD3 bispecific antibodies are generally designed to avoid the introduction of a bivalent anti-CD3 structure.

3. The modified Fc fragment included in the bispecific antibody provided by the present disclosure has no ability of binding to FcγR, avoiding the systemic activation of T cells mediated by FcγR and allowing immune effector cells to be activated restrictively and merely in target cell tissues.

4. The bispecific antibody provided by the present disclosure is homodimeric without mismatches of heavy chains and light chains. The bispecific antibody is produced by a stable downstream process and purified by simple and efficient steps, with a homogeneous expression product and significantly improved physicochemical and *in vivo* stability.

5. The bispecific antibody provided by the present disclosure has a relatively long *in vivo* circulating half-life due to the inclusion of the Fc fragment. Pharmacokinetic tests show that the *in vivo* circulating half-life in mice and cynomolgus monkeys are about 40 hours and 8 hours, respectively, which will greatly reduce a clinical administration frequency.

## Detailed description

Abbreviations and definitions

| | |
|---|---|
| Her2 | Human epidermal growth factor receptor 2 |
| BiAb | Bispecific antibody |
| CDR | Complementarity determining region in a variable region of an immunoglobulin, defined by a Kabat numbering system |
| $EC_{50}$ | A concentration at which 50% efficacy or binding is generated |
| ELISA | Enzyme-linked immunosorbent assay |
| FR | Framework region of an antibody: a variable region of an immunoglobulin excluding CDRs |
| HRP | Horseradish peroxidase |
| IL-2 | Interleukin 2 |
| IFN | Interferon |
| $IC_{50}$ | A concentration at which 50% inhibition is generated |
| IgG | Immunoglobulin G |
| Kabat | Immunoglobulin comparison and numbering system advocated by Elvin A Kabat |
| mAb | Monoclonal antibody |
| PCR | Polymerase chain reaction |
| V region | IgG chain fragment whose sequence is variable for different antibodies; the V region extends to Kabat residue 109 of a light chain and residue 113 of a heavy chain. |
| VH | Heavy chain variable region of an immunoglobulin |
| VK | κ light chain variable region of an immunoglobulin |
| $K_D$ | Equilibrium dissociation constant |
| $k_a$ | Association rate constant |
| $k_d$ | Dissociation rate constant |

In the present disclosure, unless otherwise specified, the scientific and technical terms used herein have meanings generally understood by those skilled in the art. The antibody or fragments thereof used in the present disclosure may be further modified using conventional techniques known in the art alone or in combination, such as amino acid deletion, insertion, substitution, addition, and/or recombination and/or other modification methods. A method for introducing such modifications into a DNA sequence of an antibody according to the amino acid sequence of the antibody is well known to those skilled in the art. See, for example, Sambrook, Molecular Cloning: A Laboratory Manual, Cold Spring Harbor Laboratory (1989), N.Y. Such modifications are preferably performed at a nucleic acid level. Meanwhile, for a better understanding of the present disclosure, the definitions and explanations of related terms are provided below.

"CD19", as known as Cluster of Differentiation 19 polypeptide, is a single channel Type I transmembrane glycoprotein with two C2-set Ig-like (immunoglobulin-like) domains and a relatively large cytoplasmic tail that is highly conserved among mammalian species. CD19 is expressed in almost all B lineage cells and follicular cells and plays an indispensable role in B lymphocyte differentiation. It works with CD21, CD81, and CD225 as the B cell key co-receptor. Therefore, CD19 acts as a biomarker for B lymphocyte development, B-cell lymphoma diagnosis, and B-lymphoblastic leukemia diagnosis. In addition, mutations in CD19 are associated with severe immunodeficiency syndromes. Indications for CD19 targets also include other related diseases or disorders found in the existing art or about to be discovered in the future. The term also includes any variants, isotypes, derivatives, and species homologues of CD19 that are naturally expressed by cells including tumor cells or expressed by cells transfected with CD19 genes or cDNA.

"CD20", as known as Cluster of Differentiation 20 polypeptide, belongs to a four transmembrane protein and is a B lymphocyte surface-specific differentiation antigen. It is expressed on more than 90% of B lymphoma cells and normal B lymphocytes but not expressed on hematopoietic stem cells, primary B lymphocytes, normal blood cells, and other tissues, and exhibits no significant internalization and shedding or antigen apoptosis and change after binding to antibodies, which thus may be used as an ideal target for treating B cell lymphoma. CD20 exerts an anti-tumor effect mainly through the action of ADCC, CDC, etc. In recent years, indications for CD20 targets have been developed, including, for example, autoimmune diseases (including multiple sclerosis (MS), Crohn's disease (CD)), inflammatory diseases (e.g., ulcerative colitis (UC)), and the like. Indications for CD20 targets also include other related diseases or disorders found in the existing art or about to be discovered in the future. The term also includes any variants, isotypes, derivatives, and species homologues of CD20 that are naturally expressed by cells including tumor cells or expressed by cells transfected with CD20 genes or cDNA.

"CD22", as known as Cluster of Differentiation 22 polypeptide, has an Ig domain and is a transmembrane receptor on the surface of mature B cells. In humans, CD22 primarily inhibits the over-activation of B cell surface receptors and reduces the risk of developing autoimmune diseases (e.g., systemic lupus erythematosus). Indications associated with CD22 include, for example, B-cell lymphoma, acute and chronic leukemia, and disorders associated with other B-cell dysplasia and B-cell dependent autoimmune diseases. Indications for CD22 targets also include other related diseases or disorders found in the existing art or about to be discovered in the future. The term also includes any variants, isotypes, derivatives, and species homologues of CD22 that are naturally expressed by cells including tumor cells or expressed by cells transfected with CD22 genes or cDNA.

"CD30", is a member of the tumor necrosis factor (TNF) receptor superfamily, belongs to the Type I transmembrane glycoprotein, and is physiologically expressed by activated T and B lymphocytes. CD30 is mainly expressed in tumors originate from lymph, such as all Hodgkin's lymphoma (HL), some certain B-cell lymphomas, some certain T-cell lymphomas and NK-cell lymphomas, is low-expressed on the surface of T-cells and B-cells activated in a non-pathological state, and is not expressed in normal cells, and therefore may be used as a corresponding tumor marker and an indicator of disease diagnosis. Indications for CD30 targets also include other related diseases or disorders found in the existing art or about to be discovered in the future. The term also includes any variants, isotypes, derivatives, and species homologues of CD30 that are naturally expressed by cells including tumor cells or expressed by cells transfected with CD30 genes or cDNA.

"EpCAM (epithelial cell adhesion molecule)" is a transmembrane glycoprotein and is one of the earliest TAAs found in colon cancer. EpCAM is overexpressed to varying degrees in most human tumors, including, for example, lung cancer, esophageal cancer, gastric cancer, breast cancer, colorectal cancer, liver cancer, prostate cancer, and ovarian cancer, and is closely related to tumor diagnosis and prognosis. In addition, the overexpression of EpCAM has been developed and applied in clinical trials of EpCAM antibodies and tumor-associated vaccines. Indications for EpCAM targets also include other related diseases or disorders found in the existing art or about to be discovered in the future. The term also includes any variants, isotypes, derivatives, and species homologues of EpCAM that are naturally expressed by cells including tumor cells or expressed by cells transfected with EpCAM genes or cDNA.

"CEA (carcinoembryonic antigen)" is an acid glycoprotein. It is an antigen on the surface of tumor cells, has the properties of human embryonic antigen and widely exists in the digestive system cancers originated from endoderm, including gastrointestinal cancer, liver cancer, pancreatic cancer, and may also exist in small cell lung cancer, breast cancer, medullary thyroid cancer and carcinoid tumor. Therefore, it can be used as a broad-spectrum tumor marker for the diagnosis and treatment of various tumors. Indications for CEA targets also include other related diseases or disorders found in the existing art or about to be discovered in the future. The term also includes any variants, isotypes, derivatives, and species homologues of CEA that are naturally expressed by cells including tumor cells or expressed by cells transfected with CEA genes or cDNA.

"Her2 (human epidermal growth factor receptor 2)" is a member of the human epidermal growth factor receptor family. The occurrence, development and severity of various tumors are closely related to the activity of Her2. In addition to gene mutations or amplification, the up-regulation of Her2 may also activate two downstream signaling pathways, trigger a series of cascade reactions, promote unlimited cell proliferation, and ultimately lead to cancer. In addition, Her2 may initiate multiple metastasis-related mechanisms to increase tumor cell metastasis ability. The amplification or over-

expression of Her2 genes occurs in various tumors such as breast cancer, ovarian cancer, gastric cancer, lung adenocarcinoma, prostate cancer, and invasive uterine cancer. Indications targeting Her2 include other related diseases or disorders found in the existing art or about to be discovered in the future. The term also includes any variants, isotypes, derivatives, and species homologues of Her2 that are naturally expressed by cells including tumor cells or expressed by cells transfected with Her2 genes or cDNA. Species homologues include rhesus monkey Her2.

"EGFR (epidermal growth factor receptor)" is a member of the epidermal growth factor receptor family. It is widely distributed on the surface of mammalian epithelial cells, fibroblasts, glial cells, keratinocytes and other cells, and is associated with the proliferation of tumor cells, angiogenesis, tumor invasion, tumor metastasis and the inhibition of apoptosis. Mutations or overexpression of EGFR generally lead to tumors, and high or abnormal expression of EGFR is found in various solid tumors including glial cells, renal carcinoma, lung cancer, prostate cancer, pancreatic cancer, breast cancer, and tumors in other tissues. Indications for EGFR targets also include other related diseases or disorders found in the existing art or about to be discovered in the future. The term also includes any variants, isotypes, derivatives, and species homologues of EGFR that are naturally expressed by cells including tumor cells or expressed by cells transfected with EGFR genes or cDNA.

"GPC-3 (glypican-3)" is a member of the glypican family, is highly expressed in most embryonic tissues, and is an inhibitor of cell proliferation. The lack of GPC-3 may lead to simpson-golabi-behmel syndrome (SGBS). It is overexpressed in early hepatocellular carcinoma (HCC) tissues and is associated with various cancers such as HCC, melanoma, ovarian cancer, and prostate cancer. In addition, GPC-3 is silenced in malignant tumors such as malignant mesothelioma, breast cancer, lung cancer, gastric cancer and ovarian cell carcinoma, but not expressed or low expressed in normal tissues, and thus can be used as a biomarker for the treatment and diagnosis of various tumors. Indications for GPC-3 targets also include other related diseases or disorders found in the existing art or about to be discovered in the future. The term also includes any variants, isotypes, derivatives, and species homologues of GPC-3 that are naturally expressed by cells including tumor cells or expressed by cells transfected with GPC-3 genes or cDNA.

"Mesothelin" belongs to the mesothelin family and is a pre-pro-megakaryocyte potentiating factor. It can be proteolytically cleaved, with a furin invertase protein, into two chains: megakaryocyte-potentiating factor (MPF) and mesothelin. Mesothelin is a tumor differentiation antigen and usually found on mesothelial cells lining the pleura, peritoneum and pericardium. Mesothelin is overexpressed and immunogenic in a variety of tumors such as mesothelioma, ovarian cancer, lung cancer, and pancreatic cancer, and therefore can be used as a tumor marker or antigenic target for therapeutic cancer vaccines. Indications targeting Mesothelin include other related diseases or disorders found in the existing art or about to be discovered in the future. The term also includes any variants, isotypes, derivatives, and species homologues of Mesothelin that are naturally expressed by cells including tumor cells or expressed by cells transfected with Mesothelin genes or cDNA.

"Mucin1 (cell surface associated mucin protein 1)" is a member of the mucin protein family, and is expressed on the apical surface of epithelial cells in tissue organs including lungs, breast, stomach and pancreas. The overexpression, aberrant intracellular localization, and changes in glycosylation of Mucin1 are associated with cancer including, but not limited to, colon cancer, breast cancer, ovarian cancer, lung cancer, and pancreatic cancer. Using immunohistochemistry, Mucin1 can be identified in a wide range of secretory epithelial cells, mesenchymal tumors (e.g., synovial sarcoma and granulosa cell tumor of ovary), and their neoplastic equivalents. Moreover, Mucin1 can be used to distinguish mesothelioma (in which it is restricted to the cell membranes and associated microvilli), from adenocarcinoma, in which it is diffusely spread through the cytoplasm. Therefore, Mucin1 can be used to diagnose and treat the above related diseases or disorders and other related diseases or disorders found in the existing art or about to be discovered in the future. The term also includes any variants, isotypes, and species homologues of Mucin1 that are naturally expressed by cells including tumor cells or expressed by cells transfected with Mucin1 genes or cDNA.

"CA125 (carbohydrate antigen 125)" is an ovarian cancer-associated antigen that originates from fetal coelomic epithelial tissue and is widely distributed on the surface of mesothelial cells such as pleura, pericardium, peritoneum, endometrium, genital tract and amniotic membrane. CA-125 levels in serum are significantly elevated when malignant lesions occur in these sites or these sites are stimulated by inflammation. As the most studied ovarian cancer marker, CA125 has been reported in the study of early screening, diagnosis, treatment and prognosis of ovarian cancer. CA125 levels in the puncture fluid of benign cystic tumor and malignant cystic epithelioma of ovarian cancer are significantly elevated. CA125 serum levels are also elevated in gastrointestinal malignant tumors (e.g., pancreatic cancer, liver cancer, gastric cancer, and bowel cancer), chronic pancreatitis, chronic hepatitis, liver cirrhosis, lung adenocarcinoma, pelvic inflammatory disease, and endometriosis. Given the research of CA125 in a variety of cancers and inflammation, CA125 can be widely used in screening, diagnosis and treatment of the above related diseases. Indications for CA125 targets also include other related diseases or disorders found in the existing art or about to be discovered in the future. The term also includes any variants, isotypes, and species homologues of CA125 that are naturally expressed by cells including tumor cells or expressed by cells transfected with CA125 genes or cDNA.

"BCMA (B-cell maturation antigen)" is a member of the tumor necrosis factor receptor superfamily. BCMA is preferentially expressed in mature B lymphocytes and is also expressed on the surface of plasmablasts (i.e., plasma cell precursors) and plasma cells. RNAs of BCMA are detected in the spleen, lymph node, thymus, adrenal gland and liver, and the level of BCMA mRNA in multiple B-cell lines also increases after maturation. BCMA is associated with a variety of diseases such as leukemia, lymphoma (e.g., Hodgkin's lymphoma), multiple myeloma, and autoimmune diseases (e.g., systemic lupus erythematosus), and therefore can be used as a potential target for related B-cell diseases. Indications for BCMA targets also include other related diseases or disorders found in the existing art or about to be discovered in the future. The term also includes any variants, isotypes, and species homologues of BCMA that are naturally expressed by cells including tumor cells or expressed by cells transfected with BCMA genes or cDNA.

CD3 molecule is an important differentiation antigen on the T cell membrane and a characteristic marker of mature T cells. It is composed of six peptide chains, and these chains are associated with the T cell antigen receptor (TCR) with a non-covalent bond to constitute a TCR-CD3 complex. CD3 molecule not only participates in the intracytoplasmic assembly of the TCR-CD3 complex but also transmits antigen stimulation signals through the immunoreceptor tyrosine-based activation motif (ITAM) of the cytoplasmic regions of polypeptide chains. The main functions of CD3 molecule are to stabilize TCR structure and transmit T cell activation signal. When TCR specifically recognizes and binds to the antigen, CD3 is involved in signal transduction into T cell cytoplasm as the first signal to induce T cell activation and plays a very important role in T cell antigen recognition and immune response generation.

"CD3" refers to a part of a T-cell receptor complex and consists of three different chains CD3ε, CD3δ and CD3γ. CD3 is clustered on T cells by, for example, being immobilized by an anti-CD3 antibody, leading to the activation of T cells, which is similar to T cell receptor-mediated activation but independent of the specificity of TCR clones. Most anti-CD3 antibodies recognize the chain CD3ε. The second functional domain that specifically recognizes the T cell surface receptor CD3 in the present disclosure is not specifically limited as long as it can specifically recognize CD3, for example, but not limited to, CD3 antigens mentioned in the following patents: U.S. 7,994,289; U.S. 6,750,325; U.S. 6,706,265; U.S. 5,968,509; U.S. 8,076,459; U.S. 7,728,114; and U.S. 20100183615. Preferably, the antibody against human CD3 used in the present disclosure is cross-reactive with cynomolgus monkeys and/or rhesus monkeys, for example, but not limited to, CD3 antigens mentioned in the following patents: WO 2016130726, U.S. 20050176028, WO 2007042261, or WO 2008119565. The term also includes any variants, isotypes, derivatives, and species homologues of CD3 that are naturally expressed by cells or expressed by cells transfected with a gene or cDNA encoding the preceding chains.

The term "hypervariable region", "CDR" or "complementarity determining region" refers to amino acid residues of an antibody, which are responsible for antigen binding, and is a discontinuous amino acid sequence. CDR sequences are amino acid residues in the variable region that may be defined by the IMGT, Kabat, Chothia or AbM method or identified by any CDR sequence determination method well known in the art. For example, the hypervariable region includes the following amino acid residues: amino acid residues from a "complementarity determining region" or "CDR" defined by sequence comparison, for example, residues at positions 24-34 (L1), 50-56 (L2) and 89-97 (L3) in a light chain variable domain and residues at positions 31-35 (H1), 50-65 (H2) and 95-102 (H3) in a heavy chain variable domain (see Kabat et al., 1991, Sequences of Proteins of Immunological Interest (5th edition), Public Health Service, National Institutes of Health, Bethesda, Md.), and/or amino acid residues from a "hypervariable loop" (HVL) defined according to the structure, for example, residues at positions 26-32 (L1), 50-52 (L2) and 91-96 (L3) in the light chain variable domain and residues at positions 26-32 (H1), 53-55 (H2) and 96-101 (H3) in the heavy chain variable domain (see Chothia and Leskl, J. Mol Biol, 196: 901-917, 1987). "Framework" residues or "FR" residues refer to variable domain residues other than the hypervariable region residues as defined in the present disclosure. In some embodiments, the antibody or the antigen-binding fragment thereof in the present disclosure is preferably determined through the Kabat, Chothia or IMGT numbering system. Those skilled in the art may explicitly assign each system to any variable domain sequence without relying on any experimental data beyond the sequence itself. For example, the Kabat residue numbering method of a given antibody may be determined by comparing the sequence of the given antibody to each "standard" numbered sequence. Based on the numbers of the sequences provided herein, the numbering scheme of determining any variable region sequence in the sequence table is entirely within the conventional technical scope of those skilled in the art.

The term "single-chain Fv antibody" (or "scFv antibody") refers to an antibody fragment comprising VH and VL domains of an antibody. It is a fusion protein of the variable regions of the heavy (VH) and light chains (VL) connected with a linker. The linker enables these two domains to be cross-linked to form an antigen-binding site, and the sequence of the linker generally consists of a flexible peptide, for example, but not limited to, $G_2(GGGGS)_3$. The size of scFv is generally 1/6 of an intact antibody. The single-chain antibody is preferably an amino acid chain sequence encoded by a nucleotide chain. For the review of scFv, reference may be made to Pluckthun (1994), The Pharmacology of Monoclonal Antibodies, Vol. 113, edited by Rosenburg and Moore, Springer-Verlag, New York, pages 269-315. Reference may also be made to International Patent Application Publication No. WO 88/01649 and U.S. Patent Nos. 4,946,778 and 5,260,203.

The term "Fab fragment" consists of a light chain and a CH1 and a variable domain of each of a heavy chain. The heavy chain of the Fab molecule cannot form a disulfide bond with another heavy chain molecule. The size of "Fab antibody" is 1/3 of an intact antibody, and "Fab antibody" includes only one antigen-binding site.

The term "Fab' fragment" contains a light chain, and a VH domain and a CH1 domain of a heavy chain, and a constant region between CH1 and CH2 domains.

The term "F(ab')$_2$ fragment" contains two light chains, VH domains and CH1 domains of two heavy chains, and constant regions between CH1 and CH2 domains, so that an inter-chain disulfide bond is formed between the two heavy chains. Therefore, the F(ab')$_2$ fragment is composed of two Fab' fragments held together by the disulfide bond between the two heavy chains.

The term "Fc" region refers to the antibody heavy chain constant region fragment, including at least a hinge region and CH2 and CH3 domains.

The term "Fv region" includes variable regions from the heavy chain and the light chain but lacks the constant regions, and is the minimum fragment containing a complete antigen recognition and binding site.

The term "antibody fragment" and "antigen-binding fragment" refers to an antigen-binding fragment of the antibody that retains a specific binding ability to an antigen (e.g., Her2), as well as antibody analogs. It generally includes at least part of an antigen-binding region or a variable region of a parental antibody. The antibody fragment retains at least part of the binding specificity of the parental antibody. Generally, when the activity is represented in moles, the antibody fragment retains at least 10% of parental binding activity. Preferably, the antibody fragment retains at least 20%, 50%, 70%, 80%, 90%, 95% or 100% of the binding affinity of the parental antibody to a target. The antibody fragments include, but are not limited to, Fab fragments, Fab' fragments, F(ab')2 fragments, Fv fragments, Fd fragments, complementarity determining region (CDR) fragments, disulfide-stabilized variable fragments (dsFv); linear antibodies, single-chain antibodies (e.g., scFv monoclonal antibodies) (technology from Genmab), bivalent single-chain antibodies, single-chain phage antibodies, single domain antibodies (e.g., VH domain antibodies), domain antibodies (technology from Ablynx); multispecific antibodies formed from antibody fragments (e.g., triabodies and tetrabodies); and engineered antibodies such as chimeric antibodies (e.g., humanized mouse antibodies) and heteroconjugate antibodies. These antibody fragments can be obtained using any conventional technologies known to those skilled in the art, and the utility of these fragments can be screened in the same way as the intact antibody.

The term "linker peptide" refers to a peptide linking two polypeptides, wherein the linker peptide may be two immunoglobulin variable regions or one variable region. The length of the linker peptide may be 0 to 30 amino acids or 0 to 40 amino acids. In some embodiments, the linker peptide may be in the length of 0 to 25, 0 to 20, or 0 to 18 amino acids. In some embodiments, the linker peptide may be a peptide having no more than 14, 13, 12, 11, 10, 9, 8, 7, 6, or 5 amino acids. In other embodiments, the linker peptide may include 0 to 25, 5 to 15, 10 to 20, 15 to 20, 20 to 30, or 30 to 40 amino acids. In other embodiments, the linker peptide may have about 0, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids. The linker peptide is known to those skilled in the art. The linker peptide may be prepared by any method in the art. For example, the linker peptide may be originated from synthesis.

The term "heavy chain constant region" includes an amino acid sequence from the immunoglobulin heavy chain. The polypeptide comprising the heavy chain constant region includes at least one of: a CH1 domain, a hinge domain (e.g., an upper hinge region, an intermediate hinge region, and/or a lower hinge region), a CH2 domain, a CH3 domain, or a variant or fragment thereof. For example, the antigen-binding polypeptide used herein may include a polypeptide chain having a CH1 domain; a polypeptide having a CH1 domain, at least part of a hinge domain, and a CH2 domain; a polypeptide chain having a CH1 domain and a CH3 domain; a polypeptide chain having a CH1 domain, at least part of a hinge domain, and a CH3 domain; or a polypeptide chain having a CH1 domain, at least part of a hinge domain, a CH2 domain, and a CH3 domain. In another embodiment, the polypeptide of the present application includes a polypeptide chain having a CH3 domain. In addition, the antibody used in the present application may lack at least part of a CH2 domain (e.g., all or part of a CH2 domain). As described above, it is appreciated by those of ordinary skill in the art that heavy chain constant regions may be modified such that they differ in amino acid sequence from naturally immunoglobulin molecules.

The term "light chain constant region" includes an amino acid sequence from the antibody light chain. Preferably, the light chain constant region includes at least one of a constant kappa domain and a constant lambda domain.

The term "VH domain" includes an amino-terminal variable domain of the immunoglobulin heavy chain, while the term "CH1 domain" includes a first (mostly amino-terminal) constant region of the immunoglobulin heavy chain. The CH1 domain is adjacent to the VH domain and is the amino-terminal of the hinge region of the immunoglobulin heavy chain molecule.

"Binding" defines the affinity interaction between a specific epitope on an antigen and its corresponding antibody and is generally understood as "specific recognition." "Specific recognition" means that the bispecific antibody of the present disclosure does not or substantially does not have cross-reaction with any polypeptide other than the target antigen. The degree of specificity may be determined by immunological techniques, including, but not limited to, immunoblotting, immunoaffinity chromatography, and flow cytometry. In the present disclosure, the specific recognition is preferably determined by flow cytometry, and the criteria for the specific recognition in particular cases can be judged by those of ordinary skill in the art according to the general knowledge of the art which he/she knows.

The term "*in vivo* half-life" refers to the biological half-life of the polypeptide of interest in the circulation of a given animal and is expressed as the time it takes to clear half of the amount present in the circulation of the animal from the circulation and/or other tissues in the animal.

The term "identity" refers to the matching of sequences between two polypeptides or between two nucleic acids. When a certain position in each of two sequences for comparison is occupied by the same base group or amino acid monomer subunit (e.g., a certain position in each of the two DNA molecules is occupied by adenine, or a certain position in each of the two polypeptides is occupied by lysine), then the molecules are identical at that position. The "percentage identity" between two sequences is a function of the number of matching positions of the two sequences divided by the number of positions to be compared and then multiplied by 100. For example, if 6 of the 10 positions in two sequences are matched, the identity between the two sequences is 60%. For example, DNA sequences CTGACT and CAGGTT have a total identity of 50% (three of a total of six positions are matched). Generally, the comparison is made when two sequences are aligned to produce maximum identity. Such alignment may be implemented, for example, through a computer program, such as an Align program (DNAstar, Inc.) to conveniently perform the method described by Needleman et al. Mol. Biol., 48: 443-453. The percentage identity between the two amino acid sequences may also be determined by using the algorithm proposed by E. Meyers and W. Miller (Comput. Appl Biosci., 4: 11-17) which has been incorporated into ALIGN program (version 2), using the PAM 120 weight residue table with a gap length penalty score of 12 and a gap penalty score of 4. In addition, the percentage identity between the two amino acid sequences may also be determined by using the algorithm proposed by Needleman and Wunsch (J. Mol. Biol., 48: 444-453) which has been incorporated into the GAP program in the GCG software package (available on www.gcg.com), using Blossum 62 matrix or PAM 250 matrix with a gap weight of 16, 14, 12, 10, 8, 6 or 4 and a length weight of 1, 2, 3, 4, 5 or 6.

The term "Fc region" or "Fc fragment" refers to the C-terminal regions of the immunoglobulin heavy chain, which includes at least part of a hinge region, a CH2 domain and a CH3 domain. It mediates the binding of immunoglobulins to host tissues or factors, including the binding of immunoglobulins to Fc receptors located on various cells (e.g., effector cells) of the immune system or the binding of immunoglobulins to the first component (C1q) of the classical complement system. It includes the native sequence Fc region and the variant Fc region.

Generally, the human IgG heavy chain Fc region is a segment from the amino acid residue at the position Cys226 or Pro230 of the human IgG heavy chain Fc region to the carboxy terminus, but its boundaries may vary. The C-terminal lysine of the Fc region (residue 447, according to the EU numbering system) may or may not be present. Fc may also refer to this region in isolation, or in the case of a protein polypeptide comprising Fc, for example, "binding protein comprising an Fc region", and is also referred to as "Fc fusion protein" (e.g., antibody or immunoadhesin). The native Fc region of the antibody of the present disclosure includes mammalian (e.g., human) IgG1, IgG2 (IgG2A, IgG2B), IgG3, and IgG4. Among human IgG1 Fc regions, at least two allotypes are known. In some embodiments, there is a single amino acid substitution, insertion, and/or deletion of about 10 amino acids per 100 amino acids in the amino acid sequences of two Fc polypeptide chains relative to the sequence of the amino acid sequence of the mammalian Fc polypeptide. In some embodiments, the difference may be changes in Fc that extend the half-life, changes that increase FcRn binding, changes that inhibit Fcγ receptor (FcyR) binding, and/or changes that decrease or remove ADCC and CDC.

The term "Fc receptor" or "FcR" refers to a receptor that binds to the Fc region of an immunoglobulin. FcR may be a native sequence human FcR, or may be an FcR that binds to the IgG antibody (a γ receptor), as well as allelic variants and alternatively spliced forms of these receptors. The FcyR family is composed of three activating receptors (FcγRI, FcγRIII and FcγRIV in mice; FcγRIA, FcγRIIA and FcγRIIIA in humans) and one inhibitory receptor (FcγRIIb or equivalent FcγRIIB). The FcγRII receptor includes FcγRIIA ("activating receptor") and FcγRIIB ("inhibitory receptor") which have similar amino acid sequences. The cytoplasmic domain of FcγRIIA includes an immunoreceptor tyrosine-based activation motif (ITAM). The cytoplasmic domain of FcγRIIB contains an immunoreceptor tyrosine-based inhibitory motif (ITIM) (see M. Annu. Rev. Immunol., 15: 203-234 (1997)). Most native effector cell types co-express one or more activating FcyRs and inhibitory FcγRIIbs, while NK cells selectively express one activating Fc receptor (FcγRIII in mice and FcγRIIIA in humans) but do not express inhibitory FcγRIIb in mice and humans. Human IgG1 binds to most human Fc receptors and is considered equivalent to murine IgG2a in terms of the type of activating Fc receptor to which Human IgG1 binds. The term "FcR" herein covers other FcRs, including those which will be identified in the future. Methods of measuring the binding to FcRn are known (see, e.g., Ghetie V et al., Immunol Today, 18: 592-8, 1997); Ghetie V et al., Nature Biotechnology, 15: 637-40, 1997)). The *in vivo* binding and serum half-life of the human FcRn high-affinity binding polypeptide to FcRn can be determined, for example, in transgenic mice expressing human FcRn or transfected human cell lines. The term "Fc receptor" or "FcR" also includes the neonatal receptor FcRn which is responsible for transferring maternal IgG to the fetus (Guyer RL et al., J. Immunol., 117: 587, 1976) and Kim YJ et al., J. Immunol., 24: 249, 1994)).

The term "humanized antibody" refers to a genetically engineered non-human antibody whose amino acid sequence has been modified to increase homology to the sequence of the human antibody. Most or all of the amino acids outside the CDR domain of a non-human antibody, for example, a mouse antibody, are substituted by corresponding amino acids from human immunoglobulins, while most or all of the amino acids within one or more CDR regions are not altered. The addition, deletion, insertion, substitution or modification of small molecule amino acids is permissible as long as they do not eliminate the ability of the antibody to bind a particular antigen. The "humanized" antibody retains antigen specificity similar to that of the original antibody. The origin of the CDRs is not particularly limited and may be derived from any animal. For example, antibodies derived from mouse antibodies, rat antibodies, rabbit antibodies, or non-human primate (e.g., cynomolgus monkeys) antibodies may be used. Examples of human frameworks useful in the present disclosure are KOL, NEWM, REI, EU, TUR, TEI, LAY, and POM (Kabat et al., ibid). For example, KOL and NEWM may be used for heavy chains, REI may be used for light chains, and EU, LAY, and POM may be used for both heavy and light chains. Alternatively, human germline sequences may be used, and these sequences are available at http://www2.mrc-lmb.cam.ac.uk/vbase/list2.php. In the humanized antibody molecules of the present disclosure, the receptor heavy and light chains do not need to be derived from the same antibody, and may, if needed, include complex chains having framework regions derived from different chains.

The term "cytokine" generally refers to a protein that is released by one cell population and that acts as an intercellular medium on another cell or has an autocrine effect on the cells from which the protein is produced. Examples of such cytokines include lymphokines, monokines, interleukins ("IL") such as IL-2, IL-6, and IL-17A-F, tumor necrosis factors such as TNF-$\alpha$ and TNF-$\beta$, and other polypeptide factors such as leukemia inhibitory factor ("LIF").

The term "immunobinding" and "immunobinding property" refers to a non-covalent interaction between an immunoglobulin molecule and an antigen (to the antigen, the immunoglobulin is specific). The strength or affinity of the immunobinding interaction may be represented by the equilibrium dissociation constant ($K_D$) of the interaction, where the smaller the $K_D$ value, the higher the affinity. The immunobinding property of the selected polypeptide may be quantified using a method known in the art. One method relates to the measurement of rates at which an antigen-binding site/antigen complex is formed and dissociated. Both the "binding rate constant" ($K_a$ or $K_{on}$) and the "dissociation rate constant" ($K_d$ or $K_{off}$) may be calculated according to the concentration and actual rates of association and dissociation (see Malmqvist M et al., Nature, 361: 186-187, 1993). The ratio of $k_d/k_a$ is the dissociation constant $K_D$ (generally see Davies et al., Annual Rev Biochem., 1990, 59: 439-473). Any effective method may be used for measuring values of $K_D$, $k_a$ and $k_d$.

The term "cross-reaction" refers to the ability of the antibody described herein to bind to tumor-associated antigens from different species. For example, the antibody described herein that binds to human TAA may also bind to TAAs from other species (e.g., cynomolgus monkey TAA). Cross-reactivity may be measured by detecting specific reactivity with purified antigens in binding assays (e.g., SPR, ELISA), or detecting the binding to cells physiologically expressing TAA or the interaction with the function of cells physiologically expressing TAA. Examples of assays known in the art for determining the binding affinity include surface plasmon resonance (e.g., Biacore) or similar techniques (e.g., Kinexa or Octet).

The term "$EC_{50}$" refers to the maximum response of the concentration of the antibody or antigen-binding fragment thereof that induces a 50% response in an *in vitro* or *in vivo* assay using the antibody or antigen-binding fragment thereof, that is, half between the maximum response and the baseline.

"Effector cell" refers to a cell of the immune system, which expresses one or more FcRs and mediates one or more effector functions. Preferably, the cell expresses at least one type of activating Fc receptors such as human Fc$\gamma$RIII and performs ADCC effector function. Examples of human leukocytes which mediate ADCC include peripheral blood mononuclear cells (PBMCs), natural killer (NK) cells, monocytes, macrophages, neutrophils, and eosinophils. Effector cells also include, for example, T cells. They may be derived from any organism including, but not limited to, humans, mice, rats, rabbits or monkeys.

The term "effector function" refers to biological activities that can be attributed to the biological activities of the antibody Fc region (a native sequence Fc region or amino acid sequence variant Fc region) and that vary with antibody isotypes. Examples of antibody effector functions include, but are not limited to, Fc receptor binding affinity, ADCC, ADCP, CDC, downregulation of cell surface receptors (e.g., B cell receptors), B cell activation, cytokine secretion, and half-life/clearance rate of antibodies and antigen-antibody complexes. Methods of altering the effector function of antibodies are known in the art, for example, the effector function of antibodies may be altered by introducing mutations in the Fc region.

The term "antibody-dependent cell-mediated cytotoxicity (ADCC)" refers to a cytotoxic form in which Ig binds to FcRs on cytotoxic cells (e.g., NK cells, neutrophils or macrophages) to enable these cytotoxic effector cells to specifically bind to antigen-attached target cells and then secret cytotoxins to kill the target cells. Methods for detecting the ADCC activity of an antibody are known in the art, for example, the ADCC activity may be detected by measuring the binding activity between a to-be-tested antibody and FcR (e.g., CD16a).

The term "antibody-dependent cell-mediated phagocytosis (ADCP)" refers to a cell-mediated reaction in which a non-specific cytotoxic active cell expressing FcyR recognizes a bound antibody on a target cell and subsequently causes phagocytosis of the target cell.

The term "complement-dependent cytotoxicity (CDC)" refers to a cytotoxic form that activates the complement cascade by binding the complement component C1q to the antibody Fc. Methods for detecting the CDC activity of an antibody are known in the art, for example, the CDC activity may be detected by measuring the binding activity between a to-be-tested antibody and an Fc receptor (e.g., C1q).

The term "pharmaceutically acceptable carrier and/or excipient and/or diluent" refers to a carrier and/or excipient and/or stabilizer which is pharmacologically and/or physiologically compatible with the subject and the active ingredient and which is nontoxic to the cell or mammal exposed to such a carrier and/or excipient and/or stabilizer at the dosage and concentration employed. Examples include, but are not limited to, pH regulators, surfactants, adjuvants, ionic strength enhancers, diluents, reagents to maintain osmotic pressure, reagents to delay absorption, and preservatives. For example, pH adjusting agents include, but are not limited to, phosphate buffers. Surfactants include, but are not limited to, cationic surfactant, anionic surfactant or nonionic surfactants, for example, Tween-80. Ionic strength enhancers include, but are not limited to, sodium chloride. Preservatives include, but are not limited to, various antibacterial reagent and antifungal reagent, such as parabens, chlorobutanol, phenol, and sorbic acid. Reagents to maintain osmotic pressure include, but are not limited to, sugars, NaCl, and analogs thereof. Reagents to delay absorption include, but are not limited to, monostearate and gelatin. Diluents include, but are not limited to, water, aqueous buffers (e.g., buffered saline), alcohols, and polyols (e.g., glycerol). Preservatives include, but are not limited to, various antibacterial reagent and antifungal reagent, such as thiomersal, 2-phenoxyethanol, parabens, chlorobutanol, phenol, and sorbic acid. Stabilizers have the meaning as commonly understood by those of ordinary skill in the art. Stabilizers are those capable of stabilizing the desired activity of the active ingredient in a drug, including, but not limited to, sodium glutamate, gelatin, SPGA, sugars (e.g., sorbitol, mannitol, starch, sucrose, lactose, dextran or glucose), amino acids (e.g., glutamic acid or glycine), proteins (e.g., dry whey, albumin or casein), or degradation products thereof (e.g., lactalbumin hydrolysate).

The term "effective amount" refers to an amount sufficient to obtain or at least partially obtain the desired effect. For example, a prophylactically (e.g., tumor or infection) effective amount refers to an amount sufficient to prevent, arrest, or delay the onset of a disease (e.g., tumor or infection) when used alone or used together with one or more therapeutic agents; a therapeutically effective amount refers to an amount sufficient to cure or at least partially arrest the disease and complications thereof in a patient already suffering from the disease when used alone or used together with one or more therapeutic agents. It is well within the ability of those skilled in the art to determine such effective amounts. For example, the amount effective for therapeutic use depends on the severity of the to-be-treated disease, the overall state of the patient's own immune system, the general condition of the patient such as age, weight and sex, the mode of administration of the drug, and other treatments administered concurrently. The terms "efficacy" and "effectiveness" with respect to treatment include both pharmacological effectiveness and physiological safety. The pharmacological effectiveness refers to the ability of a drug to promote regression of a condition or symptom in a patient. The physiological safety refers to the level of toxicity or other adverse physiological effects (adverse effects) at the cellular, organ and/or organism level due to drug administration.

"Treatment" or "therapy" on a subject refers to any type of intervention or treatment of, or administration of an active agent to, a subject for the purpose of reversing, alleviating, ameliorating, inhibiting, slowing or preventing the occurrence, progression, progression, severity, or recurrence of symptoms, complications, disorders or biochemical indicators associated with a disease.

The term "T-cell receptor (TCR)" is a specific receptor present on the surface of T cells, i.e., T lymphocytes. *In vivo,* the T-cell receptor is present as a complex of several proteins. The T-cell receptor generally has two separate peptide chains, typically T-cell receptors $\alpha$ and $\beta$ (TCR$\alpha$ and TCR$\beta$) chains, and in some T cells, they are T-cell receptor $\gamma$ and $\delta$ (TCR$\gamma$ and TCR$\delta$). The other proteins in the complex are CD3 proteins: CD3$\varepsilon\gamma$ and CD3$\varepsilon\delta$ heterodimers, most importantly, CD3$\zeta$ homodimers having six ITAMs. The ITAMs on CD3$\zeta$ can be phosphorylated by Lck, which in turn recruit ZAP-70. Lck and/or ZAP-70 may also phosphorylate tyrosine on many other molecules, particularly CD28, LAT, and SLP-76, which allows aggregation of signal transduction complexes around these proteins.

The term "bispecific antibody" refers to the bispecific antibody of the present disclosure, for example, an anti-Her2 antibody or an antigen-binding fragment thereof, which may be derivatized or linked to another functional molecule, for example, another peptide or protein (e.g., TAA, a cytokine and a cell surface receptor), to generate a bispecific antibody that binds to at least two different binding sites or target molecules. To produce the bispecific molecule of the present disclosure, the antibody of the present disclosure may be functionally linked (e.g., by chemical coupling, gene fusion, non-covalent binding or other means) to one or more other binding molecules, for example, another antibody, antibody fragment, peptide or binding mimetic, to produce the bispecific molecule. For example, the "bispecific antibody" refers to one including two variable domains or ScFv units such that the antibody obtained recognizes two different antigens. Various different forms and uses of the bispecific antibody are known in the art (Chames P et al., Curr. Opin. Drug Disc. Dev., 12:.276, 2009; Spiess C et al., Mol. Immunol., 67: 95-106, 2015).

The term "hCG-$\beta$ carboxy terminal peptide (CTP)" is a short peptide from the carboxy terminus of a human chorionic gonadotropin (hCG) $\beta$-subunit. Four reproduction-related polypeptide hormones, follicle-stimulating hormone (FSH), luteinizing hormone (LH), thyroid-stimulating hormone (TSH), and human chorionic gonadotropin (hCG), each contain the same $\alpha$-subunit and their respective specific $\beta$-subunits. The *in vivo* half-life of hCG is significantly longer than those of the other three hormones, mainly due to the specific carboxy terminal peptide (CTP) on the $\beta$-subunit of hCG. The CTP includes 37 amino acid residues and four O-glycosylation sites, in which sugar side chain terminals are sialic acid residues. The electronegative highly-sialyl CTP can resist renal clearance and thus extend the half-life *in vivo* (Fares F A et al., Proc Natl Acad. Sci. USA, 1992, 89: 4304-4308, 1992).

The term "glycosylation" means that an oligosaccharide (a carbohydrate containing two or more monosaccharides that are linked together, e.g., a carbohydrate containing 2 to about 12 monosaccharides that are linked together) is attached to form a glycoprotein. The oligosaccharide side chains are generally linked to the backbone of the glycoprotein via *N*- or *O*-linkages. The oligosaccharides of the antibodies disclosed herein are generally CH2 domains linked to the Fc region as *N*-linked oligosaccharides. "*N*-linked glycosylation" refers to carbohydrate moiety attachment to an asparagine residue of a glycoprotein chain. For example, the skilled artisan can recognize a single site useful for *N*-linked glycosylation at residue 297 of each of CH2 domains of murine IgG1, IgG2a, IgG2b and IgG3 and human IgG1, IgG2, IgG3, IgG4, IgA and IgD.

**Homologous antibody**

In another aspect, amino acid sequences included in the heavy and light chain variable regions of the antibody of the present disclosure are homologous with amino acid sequences of the preferred antibody described herein, and the antibody retains desired functional properties of the bispecific antibody of the present disclosure, for example, Her2×CD3 bispecific antibody.

**Antibody with conservative modifications**

The term "conservative modification" is intended to mean that an amino acid modification does not significantly affect or change the binding characteristics of the antibody containing an amino acid sequence. Such conservative modifications include amino acid substitutions, additions and deletions. A modification may be introduced into the antibody of the present disclosure by using a standard technology known in the art, such as a site-directed mutagenesis and a PCR-mediated mutagenesis. A conservative amino acid substitution refers to the substitution of an amino acid residue with an amino acid residue with a similar side chain. Families of amino acid residues with similar side chains have been described in detail in the art. These families include amino acids with basic side chains (such as lysine, arginine and histidine), amino acids with acidic side chains (such as aspartic acid and glutamic acid), amino acids with uncharged polar side chains (such as glycine, asparagine, glutamine, serine, threonine, tyrosine, cysteine and tryptophan), amino acids with non-polar side chains (such as alanine, valine, leucine, isoleucine, proline, phenylalanine and methionine), amino acids with β-branched side chains (such as threonine, valine and isoleucine) and amino acids with aromatic side chains (such as tyrosine, phenylalanine, tryptophan and histidine). Therefore, one or more amino acid residues in the CDR of the antibody of the present disclosure may be substituted with other amino acid residues from the same side chain family.

**Fc variant with altered binding affinity for the neonatal receptor (FcRn)**

"FcRn" used herein refers to a protein that binds to at least part of the Fc region of the IgG antibody and that is encoded by the FcRn gene. FcRn may be derived from any organism including, but not limited to, humans, mice, rats, rabbits or monkeys. The functional FcRn protein includes two polypeptides that often referred to as heavy and light chains, in which the light chain is β-2-microglobulin and the heavy chain is encoded by the FcRn gene.

The present disclosure relates to an antibody whose binding to FcRn is regulated (the regulation includes to increase or decrease the binding). For example, in some cases, increased binding may result in cell recirculating antibodies, and thus extends, for example, the half-life of the therapeutic antibody. Sometimes, it is desirable to decrease the FcRn binding, for example, when the antibody is used as a diagnostic or therapeutic antibody including a radiolabel. In addition, antibodies exhibiting increased binding to FcRn and altered binding to other Fc receptors such as Fcγ Rs may be used in the present disclosure.

The present application relates to an antibody including an amino acid modification that regulates the binding to FcRn. Of particular interest is that at lower pH, the binding affinity for FcRn exhibits an increase, while at higher pH, the binding basically does not exhibit an altered antibody that minimally includes the Fc region or functional variants thereof.

**Fc variant with enhanced binding affinity for the neonatal receptor (FcRn)**

The plasma half-life of IgG depends on its binding to FcRn, where IgG generally binds to FcRn at a pH of 6.0 and dissociates from FcRn at a pH of 7.4 (the pH of plasma). Through studies on the binding site, a binding site of IgG to FcRn is modified to increase the binding capacity at the pH of 6.0. It has been proved that mutations of some residues of a human Fcγ domain, which are essential to the binding to FcRn, can increase the serum half-life. It has been reported that mutations at T250, M252, S254, T256, V308, E380, M428, and N434 (EU numbering) can increase or decrease the binding affinity for FcRn (Roopenian et al., Nat. Rev. Immunol., 7: 715-725, 2007). Korean Patent No. KR 10-1027427 discloses trastuzumab (Herceptin, Genentech) variants with enhanced binding affinity for FcRn, where these variants include one or more amino acid modifications selected from 257C, 257M, 257L, 257N, 257Y, 279Q, 279Y, 308F, and 308Y. Korean Patent Publication No. KR 2010-0099179 provides bevacizumab (Avastin, Genentech) variants, where these variants exhibit an increased *in vivo* half-life through amino acid modifications included in N434S, M252Y/M428L, M252Y/N434S, and M428L/N434S. In addition, Hinton et al. have found that T250Q and M428L mutants increase the binding to FcRn threefold and sevenfold, respectively. At the same time, the mutation of two sites increases the binding 28-fold. In rhesus monkeys, the M428L or T250QM/428 L mutant exhibits the plasma half-life increased twofold (Hinton P.R. et al., J. Immunol., 176: 346-356, 2006). For more mutational sites included in the Fc variant with the enhanced binding affinity for the neonatal receptor (FcRn), see Chinese invention patent CN 201280066663.2. In addition, studies have shown that through the T250Q/M428L mutation on Fc fragments of five humanized antibodies, the interaction between Fc and FcRn is improved, and in subsequent *in vivo* pharmacokinetic assays, the pharmacokinetic parameters of the Fc-mutation antibody are improved compared with the wild-type antibody through subcutaneous administration, for example, the *in vivo* exposure is increased, the clearance rate is reduced, and the subcutaneous bioavailability is increased (Datta-Mannan A et al., MAbs. Taylor&Francis, 4: 267-273, 2012).

Other mutational sites capable of enhancing the affinity of the antibody of the present disclosure for FcRn include, but are not limited to, the following amino acid modifications: 226, 227, 230, 233, 239, 241, 243, 246, 259, 264, 265, 267, 269, 270, 276, 284, 285, 288, 289, 290, 291, 292, 294, 298, 299, 301, 302, 303, 305, 307, 309, 311, 315, 317, 320, 322, 325, 327, 330, 332, 334, 335, 338, 340, 342, 343, 345, 347, 350, 352, 354, 355, 356, 359, 360, 361, 362, 369, 370, 371, 375, 378, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 433, 438, 439, 440, 443, 444, 445, and 446, where the numbers of the amino acids in the Fc region is numbers of the EU indexes in Kabat.

Fc variants with enhanced binding affinity for FcRn also include all other known amino acid modification sites as well as amino acid modification sites that have not yet been found.

In an optional embodiment, the IgG variant can be optimized to gain increased or decreased affinity for FcRn and increased or decreased affinity for human FcyR including, but not limited to, FcγRI, FcγRIIa, FcγRIIb, FcγRIIc, FcγRIIIa and FcγRIIIb, including allelic variants thereof.

Preferentially, the Fc ligand specificity of an IgG variant determines its therapeutic application. The given IgG variant for therapeutic purposes depends on the epitope or form of the target antigen as well as the to-be-treated disease or indication. Enhanced FcRn binding may be more preferred for most targets and indications because enhanced FcRn binding may result in extended serum half-life. A relatively long serum half-life allows administration at relatively low frequencies and doses during treatment. This property may be particularly preferred when the therapeutic agent is administered in order to respond to indications requiring repeated administration. For some targets and indications, the reduced affinity for FcRn may be particularly preferred when the variant Fc is required to have an increased clearance or reduced serum half-life, for example, when the Fc polypeptide is used as an imaging agent or a radiotherapy agent.

The affinity of the polypeptide for FcRn can be evaluated by methods well known in the art. For example, those skilled in the art can perform appropriate ELISA assays. As illustrated in Example 5.6, appropriate ELISA assays enabled the comparison of the binding strengths of the variants and parents to FcRn. At the pH of 7.0, the specific signals detected for the variant and the parent polypeptide are compared, if the specific signal of the variant is at least 1.9 times weaker than the specific signal of the parent polypeptide, this variant is a preferred variant of the present disclosure and is more suitable for clinical application.

FcRn may be derived from any organism including, but not limited to, humans, mice, rats, rabbits or monkeys.

**Alterations to inhibit FcγR binding**

As used herein, "alterations to inhibit FcyR binding" refers to one or more insertions, deletions or substitutions in the Fc polypeptide chain that inhibit binding of FcγRIIA, FcγRIIB and/or FcγRIIIA, in which the binding is determined, for example, by a competitive binding assay (PerkinElmer, Waltham, MA). These alterations may be included in the Fc polypeptide chain as part of the bispecific antibody. More specifically, alterations that inhibit binding of the Fcγ receptor (FcyR) include L234A, L235A, or any alteration that inhibits glycosylation at the position N297, including any substitution at N297. In addition, along with the alterations that inhibit glycosylation of the position N297, additional alterations to stabilize the dimer Fc region by establishing additional disulfide bridges are also expected. Further examples of alterations that inhibit FcyR binding include D265A alterations in one Fc polypeptide chain and A327Q alterations in another Fc polypeptide chain. Some of the above mutations are described, for example, in Xu D et al., Cellular Immunol., 200: 16-26, 2000, of which the section about the above mutations and the activity evaluation thereof is incorporated herein by reference. The above numbers are based on EU numbering.

For example, the Fc fragment included by the bispecific antibody provided by the present disclosure in the alterations that inhibit FcyR binding exhibits reduced affinity for at least one of human FcyR (FcγRI, FcγRIIa or FcγRIIIa) or C1q, and has reduced effector cell functions or complement functions.

Other alterations that inhibit FcγR binding include sites and modifications thereof that are well known in the art or may be discovered in the future.
FcγR may be derived from any organism including, but not limited to, humans, mice, rats, rabbits or monkeys.

### Fc alterations to extend the half-life

As used herein, "Fc alterations to extend the half-life" refers to an alteration to extend the *in vivo* half-life of a protein that includes an altered Fc polypeptide in the Fc polypeptide chain as compared with the half-life of a protein that includes the same Fc polypeptide but does not include any altered but similar Fc. These alterations may be included in the Fc polypeptide chain as part of the bispecific antibody. Alterations T250Q, M252Y, S254T and T256E (alteration of threonine at position 250 to glutamine; alteration of methionine at position 252 to tyrosine; alteration of serine at position 254 to threonine; and alteration of threonine at position 256 to glutamic acid; where numbers are based on EU numbering) is an Fc alteration to extend half-life and may be used jointly, alone or in any combination. These and other alterations are described in detail in U.S. Pat. No. 7,083,784. The section about this alteration described in U.S. Pat. No. 7,083,784 is incorporated herein by reference.
Similarly, M428L and N434S are Fc alterations that extend the half-life and can be used jointly, alone or in any combination. These alterations and other alterations are described in detail in U.S. Patent Application Publication No. 2010/0234575 and U.S. Pat. No. 7,670,600. Sections of such alterations described in U.S. Patent Application Publication No. 2010/0234575 and U.S. Pat. No. 7,670,600 are incorporated herein by reference.
In addition, according to the meaning herein, any substitution at one of the following positions can be considered to be an Fc alternation that extends the half-life: 250, 251, 252, 259, 307, 308, 332, 378, 380, 428, 430, 434, and 436. Each of these alterations or a combination of these alterations may be used to extend the half-life of the bispecific antibody described herein. Other alternations that can be used to extend the half-life are described in detail in International Application No. PCT/US2012/070146 (Publication No. WO 2013/096221) filed December 17, 2012. The section about the above alterations of this application is incorporated herein by reference.
Fc alternations that extend the half-life also include sites and modifications thereof that are well known in the art or may be discovered in the future.
Fc may be derived from any organism including, but not limited to, humans, mice, rats, rabbits or monkeys.

### Method for preparing a bispecific antibody

The bispecific antibody of the present disclosure may be prepared by any method known in the art. Early methods for constructing the bispecific antibody include chemical cross-linking or hybridoma heterozygosis or quadroma method (e.g., Staerz UD et al., Nature, 314: 628-31, 1985; Milstein C et al., Nature, 305: 537-540, 1983; Karpovsky B et al., J. Exp. Med., 160: 1686-1701, 1984). The chemical coupling method is to connect two different monoclonal antibodies by chemical coupling to prepare a bispecific monoclonal antibody. For example, two different monoclonal antibodies chemically bind to each other, or two antibody fragments, for example, two Fab fragments chemically bind to each other. The heterozygosis-hybridoma method is to prepare a bispecific monoclonal antibody by a cell hybridization method or a ternary hybridoma method, where the cell hybridoma or the ternary hybridoma is obtained by the fusion of constructed hybridomas or the fusion of a constructed hybridoma and lymphocytes derived from mice. Although these techniques are used to manufacture BiAb, various generation problems make such complexes difficult to use, such as the generation of mixed populations containing different combinations of antigen-binding sites, difficulties in protein expression, the need for purifying the target BiAb, low yields, and high production costs.
Recent methods utilize genetically engineered constructs that can produce a single homogeneous product of BiAb so that there is no need for thorough purification to remove unwanted by-products. Such constructs include tandem scFv, diabodies, tandem diabodies, double variable domain antibodies, and heterdimeric antibodies using the Ch1/Ck domain or DNLTM motifs (Chames & Baty, Curr. Opin. Drug. Discov. Devel., 12: 276-83, 2009; Chames & Baty, mAbs, 1: 539-47). Related purification techniques are well known.

### Tumor surface antigen

The term "tumor surface antigen" refers to antigens that are or can be present on the tumor cells or on the inner surface of the tumor cells. Some cancer cell antigens are also expressed on the surface of some normal cells, which thus may be referred to as tumor-associated antigens. These tumor-associated antigens may be overexpressed on tumor cells compared with their expression on normal cells, or are susceptible to binding to antibodies in tumor cells due to the less compact structure of the tumor tissue compared with normal tissues. These antigens may be presented solely by tumor cells and not by normal cells. Tumor antigens may also be expressed only on tumor cells or may represent tumor-specific mutations compared with normal cells. The corresponding antigens may be called tumor-specific antigens.

The "tumor-associated antigen" can trigger an immune response in host and can be used for identifying tumor cells and as a possible candidate in cancer therapy. Such an antigen may include normal proteins that evade the immune system well, proteins that are usually produced in very small amounts, proteins that are usually produced only in certain developmental stages, or proteins whose structure is modified by mutations.

A large number of tumor antigens are known in the art, and new tumor antigens can be readily determined through screening and identification. Non-limiting examples of tumor antigens include: α-fetoprotein (AFP), α-actinin-4, A3, antigens specific to A33 antibodies, ART-4, B7, Ba733, BAGE, BrE3-antigen, CA125, CAMEL, CAP-1, carbonic anhydrase IX, CASP-8/m, CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD47, CD52, CD54, CD55, CD59, CD64, CD66a-e, CD67, CD70, CD70L, CD74, CD79a, CD80, CD83, CD95, CD123, CD126, CD132, CD133, CD138, CD147, CD154, CDC27, BCMA, CS1, DLL3, DLL4, EpCAM, FLT3, gpA33, GPC-3, Her2, MEGE-A3, NYESO1, CIX, GD2, GD3, GM2, CDK-4/m, CDKN2A, CTLA-4, CXCR4, CXCR7, CXCL12, HIF-1α, Colon-specific antigen p (CSAp), CEA(CEACAM5), CEACAM6, c-Met, DAM, EGFR, EGFRvIII, EGP-1(TROP-2), EGP-2, ELF2-M, Ep-CAM, fibroblast growth factor (FGF), Flt-1, Flt-3, folate-binding protein, G250 antigen, GAGE, gp100, GRO-β, HLA-DR, HM1.24, human chorionic gonadotropin (HCG) and subunits thereof, HER2/neu, HMGB-1, hypoxia-inducible factor (HIF-1), HSP70-2M, HST-2, Ia, IGF-1R, IFN-γ, IFN-α, IFN-β, IFN-λ, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, PAM4 antigen, pancreatic cancer mucoprotein, PD-1 receptor, placental growth factor, p53, PLAGL2, prostatic acid phosphatase, PSA, PRAME, PSMA, PIGF, ILGF, ILGF-1R, IL-6, IL-25, RS5, RANTES, T101, SAGE, S100, survivin, survivin-2B, TAC, TAG-72, cytotactin, TRAIL receptor, TNF-α, Tn antigen, Thomson-Friedenreich antigen, tumor necrosis antigen, VEGF, VEGFR2, VEGFR3, Cadherin, Integrin, Mesothelin, Claudin18, αVβ3, α5β1, ERBB3, IGF1R, EPHA3, TRAILR1, TRAILR2, RANKL, Mucin family, FAP, Tenascin, ED-B fibronectin, WT-1, 17-1A-antigen, complementation factor C3, C3a, C3b, C5a, C5, angiogenesis marker, bcl-2, bcl-6, Kras, oncogene marker and oncogene products (see, for example, Sensi M et al., Clin. Cancer Res., 12: 5023-32, 2006; Parmiani J et al., J. Immunol., 178: 1975-79, 2007; Novellino Let al., Cancer Immunol. Immunother., 54: 187-207, 2005). Preferably, TAA in the present disclosure is CD19, CD20, CD22, CD30, CD38, BCMA, CS1, EpCAM, CEA, Her2, EGFR, Mucin1, CA125, GPC-3, or Mesothelin.

The term also includes any variants, isotypes, derivatives, and species homologues of TAA that are naturally expressed by cells including tumor cells or expressed by cells transfected with TAA genes or cDNA.

TAA may be derived from any organism including, but not limited to, humans, mice, rats, rabbits or monkeys.

**Target cell and target cell protein expressed on the target cell**

As described above, the bispecific antibody can bind to effector cell proteins and target cell proteins. For example, the target cell protein may be expressed on the surface of cancer cells, cells infected by pathogen, or cells mediating diseases (e.g., inflammatory and autoimmune diseases). In some embodiments, the target cell protein can be highly expressed on the surface of target cells, although such high-level expression is not required. In some embodiments, the target cell protein is not expressed or low-expressed on the surface of target cells.

When the target cell is a cancer cell, the homodimeric bispecific antibody as described herein can bind to the cancer cell antigen as described above. The cancer cell antigen may be a human protein or a protein derived from other species.

In some embodiments, the target cell protein may be a protein that is selectively expressed or overexpressed or not expressed on the surface of tumor cells.

In some embodiments, the target cell protein may be a protein on the surface on cells that mediate lymphatic system-related diseases.

In other aspects, the target cell may be a cell that mediates autoimmune diseases or inflammatory diseases. For example, human eosinophils in asthma may be the target cell, and in this case, for example, the EGF-like module-containing mucin-like hormone receptor (EMR1) may be the target cell protein. Optionally, excess human B cells in patients suffering from systemic lupus erythematosus may be the target cell, and in this case, for example, CD19 or CD20 may be the target cell protein. In other autoimmune diseases, excess human Th2T cells may be the target cell, and in this case, for example, CCR4 may be the target cell protein. Similarly, the target cell may be a fibrotic cell that mediates, for example, atherosclerosis, chronic obstructive pulmonary disease (COPD), liver cirrhosis, scleroderma, renal transplantation fibrosis, renal allograft nephropathy or pulmonary fibrosis (including idiopathic pulmonary fibrosis and/or idiopathic pulmonary hypertension). For the fibrosis, for example, fibroblast activation protein α (FAPα) may be the target cell protein.

In some embodiments, the target cell protein may be a protein that is selectively expressed on the surface of infected cells. For example, in the case of hepatitis B virus (HBV) or hepatitis C virus (HCV) infection, the target cell protein may be an envelope protein of HBV or HCV expressed on the surface of the infected cells. In other embodiments, the target cell protein may be gp120 encoded by human immunodeficiency virus (HIV) on HIV-infected cells.

In some embodiments, the target cell may be a cell that mediates infections and infectious-related diseases.

In some embodiments, the target cell may be a cell that mediates immunodeficiency-related diseases.

In some embodiments, the target cell may be a cell that mediates other related diseases, including diseases well known in the art or about to be discovered in the future.

The bispecific antibody may bind to target cell proteins from species of mice, rats, rabbits, New World monkeys, and/or Old World monkeys. The species include, but are not limited to, the following species: Mus musculus, Rat tusrattus, Rattus norvegicus, Cynomolgus monkeys, Macaca fascicularis, Hamadryas baboon, Papio hamadryas, Guinea baboon, Papio papio, Olive baboon, Papio anubis, Yellow baboon, Papio cynocephalus, Chacma baboon, Papio ursinus, Callithrix jacchus, Saguinus Oedipus and Saimiri sciureus.

**Cancer**

The term "cancer" refers to a broad class of diseases characterized by uncontrolled growth of abnormal cells *in vivo.* "Cancer" includes benign and malignant cancers as well as dormant tumors or micrometastases. Cancer includes primary malignant cells or tumors (e.g., tumors in which cells have not migrated to a site other than the site of the original malignant disease or tumor in the subject) and secondary malignant cells or tumors (e.g., tumors resulting from metastasis in which cells are metastasized to malignant cells or tumor cells and then migrate to a secondary site different from the original tumor site). Cancers also include hematologic malignancies. "Hematologic malignancies" include lymphomas, leukemias, myelomas or lymphoid malignancies, as well as spleen cancer and lymph node tumors.

In a preferred embodiment, the bispecific antibody of the present disclosure or nucleic acids or polynucleotides encoding the antibody of the present disclosure or immunoconjugates or pharmaceutical compositions or combination therapies are useful for the treatment, prevention or alleviation of cancer. Examples of cancers include, but are not limited to, carcinomas, lymphomas, glioblastomas, melanomas, sarcomas, leukemia, myelomas or lymphoid malignancies. More specific examples of such cancers are described below and include: squamous cell carcinoma (e.g., epithelial squamous cell carcinoma), Ewing's sarcoma, Wilms' tumor, astrocytoma, lung cancer (including small-cell lung cancer, non-small-cell lung cancer, lung adenocarcinoma, and lung squamous-cell carcinoma), peritoneal carcinoma, hepatocellular carcinoma, stomach or gastric cancer (including gastrointestinal cancer), pancreatic cancer, glioblastoma multiforme, cervical cancer, ovarian cancer, liver cancer, bladder cancer, hepatoma, hepatocellular carcinoma, neuroendocrine tumor, medullary thyroid cancer, differentiated thyroid cancer, breast cancer, ovarian cancer, colon cancer, rectal cancer, endometrial or uterine carcinoma, salivary gland tumors, kidney or renal carcinoma, prostate cancer, vaginal cancer, anal cancer, penile cancer, and head and neck cancer.

Other examples of cancers or malignancies include, but are not limited to, childhood acute lymphoblastic leukemia, acute lymphoblastic leukemia, acute lymphocytic leukemia, acute myelogenous leukemia, adrenocortical carcinoma, adult (primary) hepatocellular carcinoma, adult (primary) liver cancer, adult acute lymphocytic leukemia, adult acute myelogenous leukemia, adult Hodgkin's lymphoma, adult lymphocytic lymphoma, adult non-Hodgkin's lymphoma, adult primary liver cancer, adult soft tissue sarcoma, AIDS-related lymphoma, AIDS-related malignancies, anal cancer, astrocytoma, cholangiocarcinoma, bladder cancer, bone cancer, brain stem glioma, brain tumor, breast cancer, renal pelvis and ureter cancer, central nervous system (primary) lymphoma, central nervous system lymphoma, cerebellar astrocytoma, cerebral astrocytoma, cervical cancer, childhood (primary) hepatocellular carcinoma, childhood (primary) liver cancer, childhood acute lymphoblastic leukemia, childhood acute myelogenous leukemia, childhood brain stem glioma, childhood cerebellar astrocytoma, childhood cerebral astrocytoma, childhood extracranial blastoma, childhood Hodgkin's disease, childhood Hodgkin's lymphoma, childhood hypothalamic and visual pathway glioma, childhood lymphoblastic leukemia, childhood medulloblastoma, childhood non-Hodgkin's lymphoma, childhood pineal and supratentorial primitive neuroectodermal tumors, childhood primary liver cancer, childhood rhabdomyosarcoma, childhood soft-tissue sarcoma, childhood visual pathway and hypothalamic glioma, chronic lymphocytic leukemia, chronic myelogenous leukemia, colon cancer, cutaneous T-cell lymphoma, endocrine pancreas islet cell carcinoma, endometrial cancer, ependymoma, epithelial cancer, esophageal cancer, Ewing's sarcoma and related tumors, exocrine pancreatic cancer, extracranial blastoma, extragonadal blastoma, cholangiocarcinoma, retinoblastoma, female breast cancer, Gaucher's disease, gallbladder carcinoma, gastric cancer, gastrointestinal benign tumor, gastrointestinal tumors, blastoma, gestational trophoblastic tumor, hairy cell leukemia, head and neck cancer, hepatocellular carcinoma, Hodgkin's lymphoma, hypergammaglobulinemia, hypopharyngeal cancer, intestinal cancers, intraocular melanoma, islet cell carcinoma, islet cell pancreatic cancer, Kaposi's sarcoma, kidney cancer, laryngeal cancer, lip and oral cavity cancer, liver cancer, lung cancer, lymphoproliferative disorders, macroglobulinemia, male breast cancer, malignant mesothelioma, malignant thymoma, medulloblastoma, melanoma, mesothelioma, metastatic primary latent squamous neck cancer, metastatic primary squamous neck cancer, metastatic squamous neck cancer, multiple myeloma, multiple myeloma/plasma cell neoplasm, myelodysplastic syndrome, myelogenous leukemia, myeloid leukemia, myeloproliferative disorders, nasal cavity and paranasal sinus cancer, nasopharyngeal cancer, neuroblastoma, non-Hodgkin's lymphoma, non-melanoma skin cancer, non-small-cell lung cancer, metastatic primary latent metastatic squamous neck cancer, oropharyngeal cancer, osteosarcoma/malignant fibrous sarcoma, osteosarcoma/malignant fibrous histiocytoma, osteosarcoma/malignant fibrous histiocytoma of bone, ovarian epicytoma, ovarian blastoma, ovarian low malignant potential tumor, pancreatic cancer, paraproteinemias, polycythemia vera, parathyroid cancer, penile cancer, pheochromocytoma, pituitary tumor, primary central nervous system lymphoma, primary liver cancer, prostate cancer, rectal cancer, renal cell cancer, renal pelvis and ureter cancer, retinoblastoma, rhabdomyosarcoma, salivary gland cancer, sarcoidosis sarcomas, Sezary syndrome, skin cancer, small-cell lung cancer, small intestine cancer, soft-tissue sarcoma, squamous neck cancer, stomach cancer, supratentorial primitive neuroectodermal and pineal tumors, T-cell lymphoma, testicular cancer, thymoma, thyroid cancer, transitional cell cancer of the renal pelvis and ureter, transitional renal pelvis and ureter cancer, trophoblastic tumors, ureter and renal pelvis cell cancer, urethral cancer, uterine cancer, uterine sarcoma, vaginal cancer, visual pathway and hypothalamic glioma, vulvar cancer, Waldenstrom's macroglobulinemia, Wilm's tumor, and any other hyperproliferative disease, besides neoplasia, located in an organ system listed above.

**Combination therapy**

The present disclosure relates to uses of a combination of the bispecific antibody or nucleic acids or polynucleotides encoding the antibody of the present disclosure or immunoconjugates or pharmaceutical compositions and one or more active therapeutic agents (e.g., chemotherapeutic agents) or other prophylactic or therapeutic modes (e.g., radiation). In such combination therapies, the various active agents often have different complementary mechanisms of action, and the combination therapy may lead to synergistic effects. The combination therapy includes therapeutic agents that affect immune responses (e.g., an enhanced or activated response) and therapeutic agents that affect (e.g., inhibit or kill) tumor/cancer cells. The combination therapy may reduce the likelihood of drug-resistant cancer cells. The combination therapy may allow the dose reduction of one or more reagents to reduce or eliminate adverse effects associated with the one or more reagents. Such combination therapies may have synergistic therapeutic or prophylactic effects on underlying diseases, disorders or symptoms.

The "combination" includes therapies that can be administered separately, for example, separate formulations for individual administration (e.g., which may be provided in a kit), and therapies that can be administered together in a single formulation (i.e., "co-formulation"). In some embodiments, the bispecific antibody of the present disclosure or nucleic acids or polynucleotides encoding the antibody of the present disclosure or immunoconjugates or pharmaceutical compositions may be administered sequentially. In some embodiments, the bispecific antibody of the present disclosure or nucleic acids or polynucleotides encoding the antibody of the present disclosure or immunoconjugates or pharmaceutical compositions may be administered simultaneously. The bispecific antibody or nucleic acids or polynucleotides encoding the antibody of the present disclosure or immunoconjugates or pharmaceutical compositions may be used in any manner in combination with at least one other (active) agent.

Treatment with the bispecific antibody of the present disclosure may be combined with other treatments that are effective against the to-be-treated disease. Non-limiting examples of antibody combination therapies of the present disclosure include surgery, chemotherapy, radiation therapy, immunotherapy, gene therapy, DNA therapy, RNA therapy, nanotherapy, viral therapy, and adjuvant therapy.

Combination therapies also include all other combination therapies known in the art or developed in the future.

BRIEF DESCRIPTION OF DRAWINGS

FIG. 1-1 illustrates configurations of bispecific antibodies AB7K, AB7K4, AB7K5, AB7K6, AB7K7, and AB7K8 as shown in a, b, c, d, e and f, respectively.

FIG. 1-2 illustrates an expression plasmid map of bispecific antibody AB7K7. The expression plasmid has a full length of 9293 bp and contains nine major gene fragments which are (1) an hCMV promoter, (2) target genes, (3) EMCV IRES, (4) mDHFR screening gene, (5) a Syn discontinuation sequence, (6) an SV40 promoter, (7) Kalamycin resistance gene; (8) an SV40 termination sequence, and (9) a PUC replicon.

FIG. 1-3 illustrates SEC-HPLC detection results of a purified sample of bispecific antibody AB7K7.

FIG. 1-4 illustrates SDS-PAGE electrophoresis results of a purified sample of bispecific antibody AB7K7.

FIG. 1-5 illustrates SDS-PAGE results of bispecific antibody AB7K7 in an acceleration test at 25°C.

FIG. 1-6 illustrates SDS-PAGE results of bispecific antibody AB7K7 in a freeze-thaw test.

FIG. 2-1 illustrates abilities, detected by FACS, of bispecific antibodies AB7K and AB7K4 to bind to tumor cells BT474.

FIG. 2-2 illustrates abilities, detected by FACS, of bispecific antibodies AB7K and AB7K5 to bind to tumor cells BT474.

FIG. 2-3 illustrates abilities, detected by FACS, of bispecific antibodies AB7K and AB7K6 to bind to tumor cells BT474.

FIG. 2-4 illustrates abilities, detected by FACS, of bispecific antibodies AB7K and AB7K7 to bind to tumor cells BT474.

FIG. 2-5 illustrates an ability, detected by FACS, of bispecific antibody AB7K8 to bind to tumor cells BT474.

FIG. 2-6 illustrates abilities, detected by FACS, of bispecific antibodies AB7K and AB7K4 to bind to effector cells CIK.

FIG. 2-7 illustrates abilities, detected by FACS, of bispecific antibodies AB7K and AB7K5 to bind to effector cells CIK.

FIG. 2-8 illustrates an ability, detected by FACS, of bispecific antibody AB7K6 to bind to effector cells CIK.

FIG. 2-9 illustrates abilities, detected by FACS, of bispecific antibodies AB7K and AB7K7 to bind to effector cells CIK.

FIG. 2-10 illustrates an ability, detected by FACS, of bispecific antibody AB7K8 to bind to effector cells CIK.

FIG. 2-11 illustrates an ability, detected by FACS, of bispecific antibody AB7K to bind to cynomolgus monkey T cells.

FIG. 2-12 illustrates abilities, detected by ELISA, of five Anti-Her2xCD3 bispecific antibodies to bind to CD3 molecules and Her2 molecules.

FIG. 2-13 illustrates abilities, detected by a microplate reader, of five Anti-Her2xCD3 bispecific antibodies to activate Jurkat T cells of a reporter gene cell strain.

FIG. 2-14 illustrates structural modeling of a CTP linker and anti-CD3 scFv VH.

FIG. 2-15 illustrates structural modeling of a GS linker and anti-CD3 scFv VH.

FIG. 2-16 illustrates a molecular docking model of anti-CD3 scFv and a CD3 epsilon chain.

FIG. 3-1 illustrates *in vivo* anti-tumor effects of bispecific antibodies AB7K4 and AB7K7 in an NCG mouse model of transplanted tumor constructed by subcutaneously co-inoculating human CIK cells and HCC1954 cells.

FIG. 3-2 illustrates an *in vivo* anti-tumor effect of bispecific antibody AB7K7 in an NPG mouse model of transplanted tumor constructed by subcutaneously co-inoculating human CIK cells and human breast cancer cells HCC1954.

FIG. 3-3 illustrates *in vivo* anti-tumor effects of bispecific antibodies AB7K7 and AB7K8 at different administration frequencies in an NPG mouse model of transplanted tumor constructed by subcutaneously co-inoculating human CIK cells and human breast cancer cells HCC1954.

FIG. 3-4 illustrates an *in vivo* anti-tumor effect of bispecific antibody AB7K7 in an NPG mouse of transplanted tumor constructed by subcutaneously co-inoculating human CIK cells and SK-OV-3 cells.

FIG. 3-5 illustrates an *in vivo* anti-tumor effect of bispecific antibody AB7K7 in an NPG mouse of transplanted tumor constructed by subcutaneously co-inoculating human CIK cells and HT-29 cells.

FIG. 3-6 illustrates an *in vivo* anti-tumor effect of bispecific antibody AB7K7 in a CD34 immune-reconstituted NPG mouse model of transplanted tumor constructed by subcutaneously inoculating human breast cancer cells HCC1954.

FIG. 3-7 illustrates an *in vivo* anti-tumor effect of bispecific antibody AB7K7 in a PBMC immune-reconstituted NPG mouse model of transplanted tumor constructed by subcutaneously inoculating human breast cancer cells HCC1954.

FIG. 4-1 illustrates *in vivo* anti-tumor effects of bispecific antibodies AB7K4 and AB7K7 in an NCG mouse model of transplanted tumor constructed by subcutaneously co-inoculating human CIK cells and human Burkkit's lymphoma Raji cells.

FIG. 4-2 illustrates an anti-tumor effect of bispecific antibody AB7K7 in an NPG mouse model of transplanted tumor constructed by subcutaneously inoculating human breast cancer cells HCC1954.

FIG. 4-3 illustrates changes of weights in normal cynomolgus monkeys administered with bispecific antibodies AB7K7 and AB7K8 multiple times.

FIG. 5-1 illustrates concentration-time curves of bispecific antibody AB7K7 in SD rats by two ELISA methods.

FIG. 5-2 illustrates concentration-time curves of bispecific antibody AB7K8 in SD rats by two ELISA methods.

FIG. 5-3 illustrates concentration-time curves of bispecific antibodies AB7K7 and AB7K8 in cynomolgus monkeys.

FIG. 5-4 illustrates abilities, detected at a pH of 6.0, of bispecific antibodies AB7K, AB7K5, and AB7K7 to bind to FcRn.

FIG. 5-5 illustrates abilities, detected at a pH of 7.0, of bispecific antibodies AB7K, AB7K5, and AB7K7 to bind to FcRn.

FIG. 6-1 illustrates an *in vivo* anti-tumor effect of bispecific antibody AB9K in a NOD-SCID mouse of transplanted tumor model constructed by subcutaneously co-inoculating human PBMC cells and Huh-7 cells.

FIG. 6-2 illustrates an *in vivo* anti-tumor effect of bispecific antibody AB9K in a CD34 immune-reconstituted NPG mouse model of transplanted tumor constructed by subcutaneously inoculating human liver cancer cells Huh-7.

FIG. 6-3 illustrates an *in vivo* anti-tumor effect of bispecific antibody AB9K in a CD34 immune-reconstituted NPG mouse model of transplanted tumor constructed by subcutaneously inoculating human liver cancer cells Huh-7.

FIG. 7-1 illustrates an ability, detected by flow cytometry, of bispecific antibody AB2K to bind to CD20-positive tumor cells.

FIG. 7-2 illustrates abilities of bispecific antibodies AB2K and AB7K7 to mediate effector cells to kill Raji-luc cells.

FIG. 7-3 illustrates abilities, detected by reporter gene assay, of bispecific antibodies AB2K and AB7K7 to activate Jurkat NFATRE Luc cells.

FIG. 7-4 illustrates an *in vivo* anti-tumor effect of bispecific antibody AB2K in an NPG mouse model of transplanted tumor constructed by subcutaneously co-inoculating human CIK cells and human Burkkit's lymphoma Raji cells.

FIG. 7-5 illustrates an *in vivo* anti-tumor effect of bispecific antibody AB2K in an NPG mouse model of transplanted tumor constructed by subcutaneously co-inoculating human CIK cells and human Burkkit's lymphoma Daudi cells.

FIG. 8 illustrates changes of leukocytes and lymphocytes in normal cynomolgus monkeys administered with bispecific antibody AB2K multiple times.

FIG. 9-1 illustrates an ability, detected by FACS, of an Anti-CD19xCD3 bispecific antibody to bind to tumor cells Raji.

FIG. 9-2 illustrates an ability, detected by FACS, of an Anti-CD19xCD3 bispecific antibody to bind to effector cells CIK.

FIG. 9-3 illustrates abilities, detected by FACS, of bispecific antibodies AB1K2 and AB23P10 to bind to cynomolgus monkey T cells.

FIG. 9-4 illustrates abilities, detected through an ELISA, of four Anti-CD19×CD3 bispecific antibodies to bind to CD3 molecules and CD19 molecules.

FIG. 9-5 illustrates abilities, detected by a microplate reader, of bispecific antibodies AB1K2 and AB23P8 to activate Jurkat T cells of a reporter gene cell strain.

FIG. 9-6 illustrates abilities, detected through a microplate reader, of four Anti-CD19×CD3 bispecific antibodies to activate Jurkat T cells of a reporter gene cell strain.

FIG. 10-1 illustrates binding of AB11K to tumor cells that overexpress antigen Mucin1 and to primary T cells of human or cynomolgus monkeys.

FIG. 10-2 illustrates an ability of AB11K to mediate expanded T cells to kill tumor cells.

FIG. 10-3 illustrates an ability of AB11K to mediate PBMC to kill tumor cells.

FIG. 10-4 illustrates an ability of AB11K to specifically activate T cells.

FIG. 11 illustrates an *in vivo* anti-tumor effect of bispecific antibody AB8K in an NPG mouse model of transplanted tumor constructed by subcutaneously co-inoculating human CIK cells and human skin cancer cells A431.

## DETAILED DESCRIPTION

The present disclosure is further described through examples that should not be construed as further limitations. All drawings, all reference documents, and the contents of patents and published patent applications cited in the entire application are expressly incorporated herein by reference.

Example 1 Design and preparation of Anti-Her2xCD3 bispecific antibodies having different structures

1.1 Design of bispecific antibodies having different structures

In order to screen bispecific antibodies having suitable configuration, bispecific antibodies having six different configurations were designed for Her2 and CD3, among which AB7K5, AB7K6, and AB7K8 are single-chain bivalent bispecific antibodies while AB7K, AB7K4, and AB7K7 are double-chain tetravalent bispecific antibodies (see FIG. 1-1), where only AB7K8 is free of Fc fragments. Specifically, the configuration of the bispecific antibodies with the above four configurations and their composition from the N-terminus to the C-terminus as well as their amino acid sequence numbers are shown in Table 1-1. The specific structural composition properties of the six bispecific antibodies are described below: Bispecific antibody AB7K consists of an anti-Her2 full-length antibody whose two heavy chains are each linked at the C-terminus to an anti-CD3 scFv domain by a linker peptide (L1). For the amino acid sequence of the intact antibody against Her2 contained in AB7K, reference is made to the sequence of monoclonal antibody Herceptin® (IMGT database INN 7637), wherein AB7K contains an Fc fragment from human IgG1 and has D356E/L358M mutations (EU numbering). The linker peptide L1 consists of a flexible peptide and a rigid peptide, wherein the composition of the flexible peptide is GS$(GGGGS)_3$ and the rigid peptide is SSSSKAPPPSLPSPSRLPGPSDTPILPQ, wherein the composition of the linker peptide L2 between VH and VL of the anti-CD3 scFv is $(GGGGS)_3$.

Bispecific antibody AB7K4 consists of an anti-Her2 full-length antibody whose two light chains are each linked at the C-terminus to an anti-CD3 scFv domain by a linker peptide (L1). For the amino acid sequence of the heavy chain variable region of the intact antibody against Her2 contained in AB7K4, reference is made to the available region sequence of the monoclonal antibody Herceptin®, and for the light chain amino acid sequence thereof, reference is made to the light chain amino acid sequence of the monoclonal antibody Herceptin® (IMGT database INN 7637). The AB7K4 heavy chain contains an Fc fragment from human IgG1, has multiple amino acid substitutions/replacements, which are L234A, L235A, T250Q, N297A, P331S, and M428L (EU numbering), respectively, and also has a deleted/missed K447 (EU numbering) at the C-terminus of the Fc fragment. The linker peptide L1 consists of a flexible peptide and a rigid peptide, wherein the composition of the flexible peptide is $G_2(GGGGS)_3$ and the rigid peptide is SSSSKAPPPS, wherein the composition of the linker peptide L2 between VH and VL of the anti-CD3 scFv is $(GGGGS)_3$.

Bispecific antibody AB7K5 consists of an anti-Her2 scFv, an Fc fragment, a linker peptide L2 and an anti-CD3 scFv, which are sequentially connected in series, wherein VH and VL in the anti-Her2 scFv are connected by a linker peptide L1, and VH and VL in the anti-CD3 scFv are connected by a linker peptide L3. For the amino acid sequence of the scFv against Her2 contained in AB7K5, reference is made to the available region sequence of the monoclonal antibody Herceptin®. The AB7K5 contains an Fc fragment from human IgG1 and has multiple amino acid substitutions/replacements, which are C226S, C229S, L234A, L235A, T250Q, N297A, P331S, T366R, L368H, K409T, and M428L (EU numbering), respectively. Mutations at the five sites C226S, C229S, T366R, L368H, and K409T can prevent polymerization between Fc fragments, thereby promoting the formation of a single-chain bivalent bispecific antibody.

ADCC and CDC activities are removed from Fc fragments carrying the mutations L234A/L235A/P331S. The mutations T250Q/M428L can enhance the binding affinity of Fc fragments for the receptor FcRn, thereby extending the half-life. The mutation N297A avoids antibody glycosylation and loses the ability to bind FcγRs. In addition, K447 (EU numbering) at the C-terminus of the Fc fragment is deleted/missed, thereby eliminating the charge heterogeneity of the antibody. The linker peptide (L2) consists of a flexible peptide and a rigid peptide, wherein the flexible peptide is $G_2(GGGGS)_3$ and the rigid peptide is SSSSKAPPPS. The composition of the linker peptides L1 and L3 inside each scFv is $(GGGGS)_3$.

Bispecific antibody AB7K6 consists of an anti-Her2 scFv, a linker peptide L2, an anti-CD3 scFv, and an Fc fragment, which are sequentially connected in series, wherein VH and VL in the anti-Her2 scFv are connected by a linker peptide L1, and VH and VL in the anti-CD3 scFv are connected by a linker peptide L3. The AB7K6 contains an Fc fragment from human IgG1 and has multiple amino acid substitutions/replacements, which are C226S, C229S, L234A, L235A, T250Q, N297A, P331S, T366R, L368H, K409T, and M428L (EU numbering), respectively. Mutations at the five sites C226S, C229S, T366R, L368H, and K409T can prevent polymerization between Fc fragments, thereby promoting the formation of a single-chain bivalent bispecific antibody. ADCC and CDC activities are removed from Fc fragments carrying the mutations L234A/L235A/P331S. The mutations T250Q/M428L can enhance the binding affinity of Fc fragments for the receptor FcRn, thereby extending the half-life. The mutation N297A avoids antibody glycosylation and loses the ability to bind FcγRs. In addition, K447 (EU numbering) at the C-terminus of the Fc fragment is deleted/missed, thereby eliminating the charge heterogeneity of the antibody. The linker peptide (L2) consists of a flexible peptide and a rigid peptide, wherein the flexible peptide is $G_2(GGGGS)_3$ and the rigid peptide is SSSSKAPPPS. The composition of the linker peptides L1 and L3 inside each scFv is $(GGGGS)_3$.

Bispecific antibody AB7K7 consists of an anti-Her2 scFv, a linker peptide L2, an anti-CD3 scFv, and an Fc fragment, which are sequentially connected in series, wherein VH and VL in the anti-Her2 scFv are connected by a linker peptide L1, and VH and VL in the anti-CD3 scFv are connected by a linker peptide L3. For the amino acid sequence of the scFv against Her2 contained in AB7K7, reference is made to the available region sequence of the monoclonal antibody Herceptin®. The AB7K7 contains an Fc fragment from human IgG1, and has multiple amino acid substitutions/replacements, which were L234A, L235A, T250Q, N297A, P331S, and M428L (EU numbering), respectively, and also has a deleted/missed K447 (EU numbering) at the C-terminus of the Fc fragment. The linker peptide (L2) consists of a flexible peptide and a rigid peptide, wherein the flexible peptide is $G_2(GGGGS)_3$ and the rigid peptide is SSSSKAPPPS. The composition of the linker peptides L1 and L3 inside each scFv is $(GGGGS)_3$.

Bispecific antibody AB7K8 consists of an anti-Her2 scFv, a linker peptide L2, an anti-CD3 scFv, and a His-tag, which are sequentially connected in series, wherein VH and VL in the anti-Her2 scFv are connected by a linker peptide L1, and VH and VL in the anti-CD3 scFv are connected by a linker peptide L3. For the amino acid sequence of the scFv against Her2 contained in AB7K8, reference is made to the available region sequence of the monoclonal antibody Herceptin®. AB7K8 is added with a His-tag at the C-terminus of the anti-CD3 scFv to facilitate antibody purification, wherein the composition of the His tag is HHHHHHHH. The linker peptide (L2) consists of a flexible peptide and a rigid peptide, wherein the flexible peptide is $G_2(GGGGS)_3$ and the rigid peptide is SSSSKAPPPS. The composition of the linker peptides L1 and L3 inside each scFv is $(GGGGS)_3$.

VH and VL amino acid sequences of the anti-CD3 scFv contained in the above six bispecific antibodies are as shown in SEQ ID NO: 247 and SEQ ID NO: 248, respectively, wherein VH and VL are connected to each other by $(GGGGS)_3$. The monoclonal antibody (designated as CD3-3) specifically binds to human and cynomolgus monkey CD3 antigens and has a weak binding affinity for CD3.

Table 1-1 Bispecific antibodies with four different structures against Her2 and CD3

| | Code | Configuration | Composition from N-terminus to C-terminus | Amino acid sequence No. |
|---|---|---|---|---|
| Single-chain bivalent bispecific antibody | AB7K5 | scFv-mFc-scFv | $[VH-L1-VL]_{Her2}$-mFc-L2-$[VH-L3-VL]_{CD3}$ | SEQ ID NO: 1 |
| | AB7K6 | scFv-scFv-mFc | $[VH-L1-VL]_{Her2}$-L2-$[VH-L3-VL]_{CD3}$-MFC | SEQ ID NO: 2 |
| | AB7K8 | scFv-scFv-His tag | $[VH-L1-VL]_{Her2}$-L2-$[VH-L3-VL]_{CD3}$-$H_8$ | SEQ ID NO: 3 |
| Double-chain tetravalent | AB7K | IgG(H)-scFv | $[VH-CH]_{He2}$-L1-$[VH-L2-VL]_{CD3}$ | SEQ ID NO: 4 |

(continued)

| | Code | Configuration | Composition from N-terminus to C-terminus | Amino acid sequence No. |
|---|---|---|---|---|
| bispecific antibody | | | $[VL\text{-}CL]_{Her2}$ | SEQ ID NO: 5 |
| | AB7K4 | IgG(L)-scFv | $[VH\text{-}CH]_{Her2}$ | SEQ ID NO: 6 |
| | | | $[VL\text{-}CL]_{Her2}$-L1-$[VH\text{-}L2\text{-}VL]_{CD3}$ | SEQ ID NO: 7 |
| | AB7K7 | scFv-scFv-mFc | $[VH\text{-}L1\text{-}VL]_{Her2}$-L2-$[VH\text{-}L3\text{-}VL]_{CD3}$-mFc | SEQ ID NO: 8 |
| Note: Ln in the table represents the linker peptides between different structural units, wherein n is numbered sequentially in the order of the linker peptides contained between different structural units from the N-terminus to the C-terminus of the bispecific antibody. | | | | |

1.2 Construction of an expression vector of a bispecific antibody molecule

Genes encoding the preceding five bispecific antibodies were synthesized by conventional molecular biology method, and cDNAs encoding the obtained fusion genes were inserted into corresponding restriction endonuclease sites of eukaryotic expression plasmids pCMAB2M modified with PCDNA3.1. The heavy chains and light chains of AB7K and AB7K4 may be constructed into one vector or separately into two different vectors. For example, the expression plasmid map of AB7K7 is as shown in FIG. 1-2, wherein the plasmid contains cytomegalovirus early promoter. The promoter is an enhancer required for the high-level expression of foreign genes in mammalian cells. The plasmid pCMAB2M also contains a selective marker so that kanamycin resistance may be present in bacteria and G418 resistance may be present in mammalian cells. In addition, when host cells are deficient in the expression of DHFR genes, the pCMAB2M expression vector contains mouse dihydrofolate reductase (DHFR) genes so that target genes and the DHFR genes can be co-amplified in the presence of methotrexate (MTX) (see U.S. patent No. 4,399,216).

1.3 Expression of bispecific antibody molecules

The preceding constructed expression plasmids were transfected into a mammalian host cell line to express bispecific antibodies. To maintain stable and high-level expression, the preferred host cell line is a DHFR deficient CHO-cell (see U.S. Patent No. 4,818,679), and in this Example, the host cell was selected as the CHO-derived cell strain DXB11. A preferred transfection method is electroporation. Other methods, including calcium phosphate co-precipitation and lipofection may also be used. During electroporation, 50 μg of expression vector plasmids DNA were added to $5 \times 10^7$ cells in a cuvette with a Gene Pulser Electroporator (Bio-Rad Laboratories, Hercules, CA) with an electric field of 300 V and capacitance of 1500 μFd. After two-day transfection, the medium was changed to a growth medium containing 0.6 mg/mL G418. Transfectants were subcloned by the limiting dilution method, and the secretion rate of each cell line was determined by ELISA. Cell strains expressing bispecific antibodies at high levels were screened.

To achieve the high-level expression of fusion proteins, DHFR genes inhibited by MTX should be used for co-amplification. The transfected fusion protein genes were co-amplified with the DHFR genes in growth media containing MTX with increasing concentrations. Subclones that were positive for DHFR expression were subjected to limiting dilution with gradually increased pressure to screen transfectants capable of growing in media with MTX of up to 6 μM. The secretion rates of the transfectants were determined and cell lines with high foreign protein expression were screened. Cell lines with a secretion rate of greater than about 5 μg/$10^6$ (millions) cells/24 hours (preferably about 15 μg/$10^6$ cells/24 hours) were adaptively suspended using a serum-free medium. Cell supernatants were collected and bispecific antibodies were separated and purified.

Hereinafter, the purification process, stability, *in vitro* and *in vivo* biological functions, safety, and pharmacokinetics of the bispecific antibodies of several configurations were evaluated to screen the bispecific antibody of an appropriate configuration.

1.4 Purification process and stability detection of bispecific antibodies

Antibodies are generally purified by a three-step purification strategy: crude purification (sample capture), intermediate purification, and fine purification. In the crude purification stage, the target antibodies are generally captured by affinity chromatography which can effectively remove a large number of impurities such as heterologous proteins, nucleic acids, endotoxins, and viruses from the sample. The intermediate purification is often carried out using hydrophobic chromatography or CHT hydroxyapatite chromatography to remove most of the remaining impurity proteins and polymers. Fine purification is mostly carried out using anion exchange chromatography or gel filtration chromatography (molecular sieve) to remove the small or trace amount of remaining impurity proteins whose nature is similar to the nature of the target antibodies and further to remove contaminants such as HCP and DNA.

In the present disclosure, the culture supernatant of bispecific antibody AB7K8 fused with His-tag can be crudely purified using a metal chelation affinity chromatography column (e.g., HisTrap FF from GE). The bispecific antibodies AB7K4, AB7K5, AB7K6, AB7K, and AB7K7 containing Fc can be crudely purified using a Protein A/G affinity chromatography column (e.g., Mabselect SURE from GE). The products obtained after the above crude purification are then subjected to the intermediate purification and the fine purification to finally obtain purified target antibodies of high purity and high quality. The preservation buffers for the above bispecific antibodies are then replaced with PBS or other suitable buffers using desalination columns (e.g., HiTrap desalting from GE).

a) Purification of double-chain tetravalent bispecific antibody AB7K7

Specific purification steps and solutions for such bispecific antibodies of a tetravalent homodimer configuration are illustrated below by using an example of AB7K7. The bispecific antibody AB7K7 was purified by three-step chromatography. The three-step chromatography included affinity chromatography, hydrophobic chromatography, and anion exchange chromatography. (The protein purifier used in this example was AKTA pure 25 M from GE, U.S. Reagents used in this example were purchased from Sinopharm Chemical Reagent Co., Ltd and had purity at an analytical grade).

In a first step, affinity chromatography was performed. Sample capture and concentration and the removal of partial pollutants were performed using an affinity chromatography medium MabSelect Sure from GE or other commercially available affinity media (e.g., Diamond Protein A from Bestchrom). First, chromatography columns were equilibrated with 3-5 column volumes (CVs) of an equilibration buffer (20 mM PB, 140 mM NaCl, pH 7.4) at a linear flow rate of 100-200 cm/h. The clarified fermentation broth was loaded at a linear flow rate of 100-200 cm/h with a load not higher than 20 mg/mL. After loading, the chromatography columns were equilibrated with 3-5 column volumes (CVs) of an equilibration buffer (20 mM PB, 140 mM NaCl, pH 7.4) at a linear flow rate of 100-200 cm/h to remove unbound components. The chromatography columns were rinsed with 3-5 column volumes of decontamination buffer 1 (50 mM NaAc-HAc, 1 M NaCl, pH 5.0) at a linear flow rate of 100-200 cm/h to remove partial pollutants. The chromatography columns were equilibrated with 3-5 column volumes (CVs) of decontamination buffer 2 (50 mM NaAc-HAc, pH 5.0) at a linear flow rate of 100-200 cm/h. The target product was eluted using an elution buffer (40 mM NaAc-HAc, pH 3.5) at a linear flow rate not higher than 100 cm/h and target peaks were collected.

In a second step, hydrophobic chromatography was performed. Intermediate purification was performed using Butyl HP from Bestchrom or other commercially available hydrophobic chromatography media to reduce the content of polymers. After the target proteins were polymerized, since the polymers and monomers differed in property such as charge characteristics and hydrophobicity, the polymers and the monomers could be separated on the basis of the above differences between them. First, chromatography columns were equilibrated with 3-5 column volumes (CVs) of an equilibration buffer (20 mM PB, 0.3 M $(NH_4)_2SO_4$, pH 7.0) at a linear flow rate of 100-200 cm/h. The target proteins separated through the affinity chromatography in the first step were subjected to conductivity adjustment to 40-50 ms/cm with the solution of 2 M $(NH_4)_2SO_4$ and then loaded with a load controlled to be less than 20 mg/mL. After loading, the chromatography columns were rinsed with 3-5 column volumes (CVs) of an equilibration buffer (20 mM PB, 0.3 M $(NH_4)_2SO_4$, pH 7.0) at a linear flow rate of 100-200 cm/h. Finally, the target proteins were eluted using 3-5 column volumes (CVs) of an elution buffer (20 mM PB, pH 7.0) with gradients of 40%, 80% and 100% at a linear flow rate not higher than 100 cm/h. Eluted fractions were collected and sent for SEC-HPLC, respectively. Target components with the percentage of monomers being greater than 90% were combined for chromatography in the next step.

In a third step, anion exchange chromatography was performed. Fine purification was performed by using Q-HP from Bestchrom or other commercially available anion exchange chromatography media (e.g., Q HP from GE, Toyopearl GigaCap Q-650 from TOSOH, DEAE Beads 6FF from Smart-Lifesciences, Generik MC-Q from Sepax Technologies, Inc, Fractogel EMD TMAE from Merck, and Q Ceramic HyperD F from Pall) to separate structural variants and further remove pollutants such as HCP and DNA. First, chromatography columns were rinsed with 3-5 column volumes (CVs) of an equilibration buffer (20 mM PB, pH 7.0) at a linear flow rate of 100-200 cm/h. The target proteins separated through the hydroxyapatite chromatography in the second step were loaded and through-flow peaks were collected. After loading, the chromatography columns were rinsed with 3-5 column volumes (CVs) of an equilibration buffer (20 mM PB, pH 7.0) at a linear flow rate of 100-200 cm/h. The through-flow components were collected and sent for the detection of protein content, SEC-HPLC and electrophoresis.

The SEC-HPLC purity results and SDS-PAGE electrophoresis results of the samples are shown in FIG. 1-3 and FIG. 1-4. The SEC-HPLC results show that the purity of the main peak of the bispecific antibody was more than 95% after three-step chromatography. The band pattern in the SDS-PAGE electrophoresis was as expected, wherein a band was shown at 180 KDa in the non-reducing electrophoresis and a clear single-chain band (90 KDa) was obtained after reduction.

b) Purification of single-chain bivalent bispecific antibodies AB7K5 and AB7K6

The bispecific antibody AB7K5 was purified by Protein A affinity chromatography and hydroxyapatite (CHT) chromatography. After the SEC-HPLC test, it was found that the purity of bispecific antibody AB7K5 was low, its yield was not high, and there was a problem of extremely low expression yield.

For another single-chain bivalent bispecific antibody AB7K6, there also was a problem of high process development difficulty. The bispecific antibody AB7K6 was subjected to two-step purification, that is, Protein A affinity chromatography and molecular sieve chromatography Superdex 200. After the SEC-HPLC test, it was found that it was difficult to quantify the purity of bispecific antibody AB7K6, and there was a significant "shoulder peak" in the main peak; in addition, the expression yield of AB7K6 was very low and very unstable. After 24 hours of standing in a refrigerator at 4 °C, it was found that the peak shape in the SEC-HPLC result was changed from two peaks to one main peak, which attributed, presumably, to the conversion from the single-chain structure to the double-chain structure in AB7K6. From the above, it can be seen that the current process development difficulty of AB7K6 is too high to achieve process scale-up and industrialization.

In summary, AB7K7 had significant advantages over AB7K5 and AB7K6 in terms of process development and had advantages such as high yield, simple and efficient purification methods, and stable downstream processes. The physicochemical stability of AB7K7 in different buffer systems and at different storage conditions was further studied.

c) Assay on stability of bispecific antibody AB7K7

The stability of AB7K7 proteins in a citrate buffer system (20 mM citrate, pH 5.5) and a histidine buffer system (20 mM histidine, pH 5.5) was studied, respectively. AB7K7 proteins were stored for four weeks under accelerated conditions at 25°C for the evaluation of protein stability.

AB7K7 proteins were transferred to the preceding citrate buffer system (F2) and the histidine buffer system (F3), respectively, with the concentration adjusted to 0.5 mg/mL, wherein 8% sucrose (w/v) and 0.02% PS80 (w/v) were added to both buffer systems as excipients. The above buffer systems were filtered using a 0.22 μm PES membrane needle filter, and then vialed into 2 mL penicillin bottles, respectively, 0.8 mL in each bottle. After the vialing, a stopper was immediately pressed and capped. Samples were placed in different stability chambers according to the schemes in Table 1-2. Samples were taken at each sampling point for detection and analysis, wherein the detection terms included appearance, concentration, purity (detected by SEC-HPLC), HMW%, LMW%, and turbidity (A340) of the sample.

Table 1-2 Stability detection scheme

| **Condition** | **$T_0$** | Sampling point and detection term | | |
|---|---|---|---|---|
| 40 °C | X, Y | 1 Week (W) | 2W | 4W |
| | | X, Y | X | X, Y |
| 25 °C | | 1W | 2W | 4W |
| | | X, Y | X | X, Y |
| Freeze-thaw (-70 °C/room temperature) | | 3 cycles | | |
| | | X, Y | | |
| Note: X = appearance, concentration, SEC-HPLC, SDS-PAGE (reducing & non-reducing);<br>Y = turbidity (A340) | | | | |

The appearance, concentration, turbidity and SEC-HPLC detection results of two preparations stored for 0-4 weeks at 25°C are shown in Table 1-3 and Table 1-4, and SDS-PAGE (reducing/non-reducing) results thereof are shown in FIG. 1-5. There was no significant change in the appearance and concentration of the two preparations. In the SEC-HPLC results, the SEC results of the F2 and F3 preparations did not show any significant change. The purity after 4 weeks was 97.9% and 98.2%, respectively. SDS-PAGE (reducing/non-reducing) results were generally consistent with the trend of LMW% results, and F2 and F3 slightly changed.

To know the unfolding temperature of AB7K7 proteins in the two buffer systems, the Tm (unfolding temperature) and Tmonset (the temperature at which the protein begins to unfold) in the two preparations were measured by DSF and the results are shown in Table 1-5. Both preparations had low Tmonset values, and F2 and F3 had Tmonset values less than 45 °C.

Table 1-3 Appearance, concentration, and turbidity results in the acceleration test at 25 °C

| | Appearance | | | | Concentration | | | | Turbidity A340 | | |
|---|---|---|---|---|---|---|---|---|---|---|---|
| | T0 | 1W | 2W | 4W | T0 | 1W | 2W | 4W | T0* | 1W | 4W |
| F2 | Colorless clear liquid without visible foreign matter | | | | 0.46 | 0.46 | 0.45 | 0.46 | 0.003 | 0.004 | 0.002 |
| F3 | | | | | 0.47 | 0.46 | 0.45 | 0.47 | 0.004 | 0.003 | 0.005 |
| *T0 turbidity: the sample to be detected was a sample subjected to 1 cycle of freeze-thaw. | | | | | | | | | | | |

Table 1-4 SEC-HPLC results in the acceleration test at 25 °C

| | SEC-Purity% | | | | SEC-HMW% | | | | SEC-LMW% | | | |
|---|---|---|---|---|---|---|---|---|---|---|---|---|
| | T0 | 1W | 2W | 4W | T0 | 1W | 2W | 4W | T0 | 1W | 2W | 4W |
| F2 | 97.5 | 98.3 | 98.5 | 97.9 | 2.5 | 1.5 | 1.5 | 1.5 | 0 | 0.3 | 0.0 | 0.6 |
| F3 | 97.7 | 98.8 | 98.7 | 98.2 | 2.3 | 1.2 | 1.3 | 1.2 | 0 | 0.0 | 0.0 | 0.6 |

Table 1-5 DSF results

| | Tmonset (°C) | Tm1 (°C) | Tm2 (°C) |
|---|---|---|---|
| F2 | 42.0 | 46.0 | 60.5 |
| F3 | 41.0 | 45.0 | 58.0 |

The stability of AB7K7 proteins in the above two buffer systems during freeze-thaw (-70 °C/room temperature, 3 cycles of freeze-thaw) was studied by performing 3 cycles of freeze-thaw. The preparation and detection solution of the sample were the same as those described above.

The appearance, concentration, turbidity and SEC-HPLC detection results of samples are shown in Table 1-6, and SDS-PAGE (reducing/non-reducing) results thereof are shown in FIG. 1-6. In the SDS-PAGE (non-reducing) results, there were no significant changes in the results of each detection item of both F2 and F3 preparations subjected to three cycles of freeze-thaw.

Table 1-6 Appearance, concentration, turbidity, and SEC-HPLC results in the freeze-thaw test

| | Appearance | | Concentration | | Turbidity A340 | | SEC-Purity% | | SEC-HMW% | | SEC-LMW% | FT-3C |
|---|---|---|---|---|---|---|---|---|---|---|---|---|
| F2 | Colorless -clear liquid without visible foreign matter | Colorless clear liquid without visible foreign matter | 0.46 | 0.46 | 0.003 | 0.005 | 97.5 | 98.4 | 2.5 | 1.6 | 0 | 0.0 |
| F3 | | | 0.47 | 0.48 | 0.004 | 0.005 | 97.7 | 98.5 | 2.3 | 1.4 | 0 | 0.2 |

*T0 turbidity: the sample to be detected was a sample subjected to 1 cycle of freeze-thaw.

Example 2 Evaluation of *in vitro* biological functions of Anti-Her2×CD3 bispecific antibodies

2.1 Detection of binding activities of bispecific antibodies to effector cells and target cells (FACS)

a) Detection of binding activities of bispecific antibodies to Her2-positive tumor cells BT-474 by flow cytometry

Tumor cells BT-474 that were positive for Her2 expression (from the cell bank of Chinese Academy of Sciences, Shanghai) were cultured, then digested with 0.25% trypsin, and centrifuged to collect cells. The collected cells were resuspended with 1% PBSB, placed in 96-well plates after the cell density was adjusted to (2 × $10^6$) cells/ml, 100 μl (2 × $10^5$ cells) per well, and blocked for 0.5 hours at 4 °C. The blocked cells were centrifuged to discard the supernatant, and a series of diluted bispecific antibodies were added to the cells. The cells were incubated for 1 hour at 4 °C, then centrifuged to discard the supernatant, and washed three times using a PBS solution with 1% BSA (PBSB). Diluted AF488-labeled goat anti-human IgG antibodies or murine anti-6×His IgG antibodies were added to the cells, and the cells were incubated for 1 hour at 4 °C in the dark. The obtained cells were centrifuged to discard the supernatant, and washed twice with 1% PBSB, and cells in each well were resuspended with 100 μl of 1% paraformaldehyde (PF). The signal intensity was detected by flow cytometry. The analysis was performed with the average fluorescence intensity as the Y-axis and the antibody concentration as the X-axis through software GraphPad to calculate the $EC_{50}$ value for the binding of bispecific antibodies to tumor cells BT-474.

The results show that bispecific antibodies with different structures had good binding activity to tumor cells overexpressing Her2. FIG. 2-1 to FIG. 2-5 show binding curves of bispecific antibodies with different structures to tumor cells BT-474. As shown in Table 2-1, the $EC_{50}$ for the binding of AB7K to tumor cells and the $EC_{50}$ for the binding of AB7K4 to tumor cells both were around 5 nM, the $EC_{50}$ for the binding of AB7K7 to tumor cells was close to 50 nM, the $EC_{50}$ for the binding of AB7K5 to tumor cells and the $EC_{50}$ for the binding of AB7K8 to tumor cells both were greater than 100 nM, and the $EC_{50}$ for the binding of AB7K6 to tumor cells was greater than 200 nM.

Table 2-1 Detection of abilities of Anti-Her2×CD3 bispecific antibodies to bind to tumor cells BT474

| | AB7K | AB7K4 | AB7K5 | AB7K6 | AB7K7 | AB7K8 |
|---|---|---|---|---|---|---|
| $EC_{50}$ (nM) | 5.009 | 4.388 | 125.0 | 239.9 | 51.98 | 125.3 |

b) Detection of binding activities of bispecific antibodies to human T cells by FACS

PBMCs were prepared from fresh human blood by density gradient centrifugation. The prepared PBMCs were resuspended in a 1640 medium containing 10% heat-inactivated FBS, added with 2 μg/ml OKT3 for activation for 24 h, then added with 250 IU/ml IL-2 for amplification for 7 days, to prepare cytokine-induced killer (CIK) cells which were detected by flow cytometry to be positive for CD3 expression on the surface. The to-be-detected samples were prepared and detected in the same manner as in a) of Example 2.1. Cells resuspended with 1% PF were detected on a machine and, with the average fluorescence intensity, analyzed by software OriginPro 8 to calculate the $EC_{50}$ value for the binding of each bispecific antibody to human CIK cells.

The results show that there were great differences among the binding of each bispecific antibody to CIK cells (FIG. 2-6 to FIG. 2-10). As shown in Table 2-2, the $EC_{50}$ of AB7K was about 20 nM, which was roughly equal to the $EC_{50}$ of AB7K4, the $EC_{50}$ of AB7K7 was more than 6 times higher than the $EC_{50}$ of AB7K, and the $EC_{50}$ of AB7K5, AB7K6 and AB7K8 was more than 10 times higher than the $EC_{50}$ of AB7K.

Table 2-2 Detection of abilities of Anti-Her2×CD3 bispecific antibodies to bind to effector cells CIK

| | AB7K | AB7K4 | AB7K5 | AB7K6 | AB7K7 | AB7K8 |
|---|---|---|---|---|---|---|
| $EC_{50}$ (nM) | 20.51 | 19.44 | 375.2 | 241.7 | 132.3 | 504.1 |

c) Detection of cross-reactivity of bispecific antibodies with cynomolgus monkey CIK cell membrane CD3 by FACS

PBMCs were prepared from fresh cynomolgus monkey blood by density gradient centrifugation. The prepared PBMCs were resuspended in a 1640 medium containing 10% heat-inactivated FBS, added with 2 μg/ml OKT3 for activation for 24 h, then added with 250 IU/ml IL-2 for amplification for 7 days, to prepare cynomolgus monkey CIK cells for use. Human CIK cells and cynomolgus monkey CIK cells were collected by centrifugation, followed by the same test procedure as in the above examples. Cells resuspended with 1% paraformaldehyde solution were detected on a machine and, with the average fluorescence intensity, analyzed by software OriginPro 8 to calculate the $EC_{50}$ values for the

binding of bispecific antibodies to human CIK cells and the $EC_{50}$ values for the binding of bispecific antibodies to cynomolgus monkey CIK cells.

As shown in FIG. 2-11, the bispecific antibody AB7K bound well to cynomolgus monkey T cells, the ability of AB7K to bind to cynomolgus monkey T cells was roughly equal to the ability of AB7K to bind to human T cells, and the $EC_{50}$ for the binding of AB7K to cynomolgus monkey T cells was approximately 26 nM as detected by flow cytometry. Bispecific antibodies AB7K4, AB7K5, AB7K6, AB7K7, and AB7K8 bound specifically to cynomolgus monkey T cells, as did AB7K.

2.2 Detection of abilities of bispecific antibodies to bind to antigens The binding of bispecific antibodies to soluble CD3 and Her2 was detected by double antigen sandwich ELISA.

Her2 proteins (SinoBiological, Beijing, Cat. No. 10004-H08H4) were diluted with PBS to a concentration of 0.1 μg/ml and added to 96-well plates, 100 μl per well. The plates were coated at 4 °C overnight. The plates were then blocked with 1% skimmed milk powder for 1 hour at room temperature. Each bispecific antibody was diluted simultaneously with a 4-fold gradient for a total of 11 concentration gradients. The 96-well plates were then washed with PBST, and then the diluted bispecific antibodies were added. Control wells without antibodies were set. Incubated for 1 hour at room temperature. Unbound bispecific antibodies were washed away with PBST. Biotinylated CD3E and CD3D (ACRO Biosystem, Cat. No. CDD-H82W1) were mixed at 50 ng/ml with streptavdin HRP (BD, Cat. No. 554066), added in the 96-well plates, 100 μl per well, and incubated for 1 hour at room temperature. 96-well plates were washed with PBST, and TMB was added to the plates, 100 μl per wellss. Color development was performed at room temperature for 15 minutes, and then 0.2 M $H_2SO_4$ was added to stop the color development reaction. The light absorbance values at A450-620 nm were measured by a microplate reader. Analysis was performed by software OriginPro 8, and the $EC_{50}$ values for the binding of bispecific antibodies to two antigens were calculated.

The results show that each bispecific antibody bound specifically to both CD3 and Her2 molecules and exhibited good dose-dependence as the concentration of the antibodies changed (FIG. 2-12). The abilities of several bispecific antibodies to bind to soluble CD3 and Her2 are shown in Table 2-3, with $EC_{50}$ values ranging from 0.03 nM to 3.8 nM which differ by two orders of magnitude. AB7K had the best binding activity, binding activities of AB7K4 and AB7K7 differed by one order of magnitude, and AB7K5 and AB7K8 had the weakest binding activity.

Table 2-3 Detection of abilities of Anti-Her2×CD3 bispecific antibodies to bind to CD3 and Her2 molecules

| | AB7K | AB7K4 | AB7K5 | AB7K7 | AB7K8 |
|---|---|---|---|---|---|
| $EC_{50}$ (nM) | 0.03128 | 0.1518 | 1.004 | 0.1398 | 3.815 |

2.3 Evalution of abilities of bispecific antibodies to activate T cells through reporter gene cell strains

Jurkat T cells containing NFAT RE reporter genes (BPS Bioscience, Cat. No. 60621) can overexpress luciferase in the presence of bispecific antibodies and target cells, and the degree of activation of the Jurkat T cells can be quantified by detecting the activity of the luciferase. A four-parameter curve was fitted using the concentration of bispecific antibodies as the X-axis and the fluorescein signal as the Y-axis.

The test results from FIG. 2-13 show that the monoclonal antibody Herceptin targeting Her2 cannot activate Jurkat T cells. T cells can be activated only in the presence of both antibodies. The ability of each antibody to activate Jurkat T cells is shown in Table 2-4. AB7K4 had the strongest ability to activate T cells, AB7K8 had the weakest ability to activate T cells, and their $EC_{50}$ values differed by one order of magnitude.

Table 2-4 Detection of abilities of Anti-Her2×CD3 bispecific antibodies to a reporter gene cell strain that are Jurkat T cells

| | AB7K | AB7K4 | AB7K5 | AB7K7 | AB7K8 | Herceptin |
|---|---|---|---|---|---|---|
| $EC_{50}$ (nM) | 0.02263 | 0.01338 | 0.05357 | 0.08952 | 0.1575 | 0.009907 |

2.4 Abilities of bispecific antibodies to mediate T cells to kill tumor cells

Normally cultured tumor cell lines, including SK-BR-3, MCF-7, HCC1937, NCI-N87, HCC1954 cells (all purchased from the cell bank of Chinese Academy of Sciences, Shanghai), as target cells, were digested with 0.25% trypsin to prepare single-cell suspensions, added to 96-well cell culture plates after the cell density was adjusted to $2 \times 10^5$ cells/ml, 100 μl per well, and cultured overnight. The antibodies were diluted according to the test design, and added to

the cells, 50 μl per well, while wells without the addition of antibodies were supplemented with the same volume of the medium. Effector cells (human PBMCs or expanded CIK cells) whose number was five times larger than the number of target cells, were then added, 100 μl per well. Control wells were set, and wells without the addition of effector cells were supplemented with the same volume of the medium. After incubation for 48 hours, the supernatant was discarded from the 96-well plates. The cells were then washed three times with PBS, and a complete medium containing 10% CCK-8 was added, 100 μl per well, and the cells were incubated for 4 hours at 37 °C. The light absorbance values at A450-620 nm were measured by a microplate reader. Analysis was performed by software OriginPro 8, and the ability of each bispecific antibody to mediate the killing of tumor cells and the ability of the same target monoclonal antibody Herceptin to mediate the killing of tumor cells were calculated and compared.

The $EC_{50}$ values of each bispecific antibody to mediate effector cells to kill tumor cells are shown in Table 2-5. The results show that each bispecific antibody exhibited a very significant killing effect on tumor cells (e.g., SK-BR-3, NCI-N87, and HCC1954) with high expression of Her2 in a dose-dependent manner. Each bispecific antibody, in particular AB7K7, also exhibited a good killing effect on breast cancer cells MCF-7 with low expression of Her2. Each bispecific antibody also had a good killing effect on the Herceptin-resistant cell strain HCC1954 while each bispecific antibody exhibited the killing effect on the cell strain HCC1937 that was negative for Her2 expression (with little expression) only at two highest concentrations.

Table 2-5 $EC_{50}$ values of bispecific antibodies to mediate PBMCs to kill different tumor cells

| $EC_{50}$ (nM) | AB7K7 | AB7K8 | AB7K5 | Herceptin |
|---|---|---|---|---|
| SK-BR-3 | ~ 0.001 | ~ 0.002 | ~ 0.001 | - |
| | ~ 0.001 | 0.011 | - | 0.067 |
| MCF-7 | ~ 0.005 | 0.079 | 0.055 | - |
| HCC1937 | 0.659 | ~ 2.269 | 1.223 | - |
| | 0.579 | 4.011 | - | >6.667 |
| NCI-N87 | 0.015 | 0.034 | - | 0.129 |
| HCC1954 | 0.002 | 0.018 | - | 0.050 |
| Note: ~ means approximately equal to, and - means that no detection is performed. | | | | |

2.5 Evaluation of the effect of GS-CTP linker peptide on the ability of anti-CD3 scFv to bind to CD3 molecules by computer techniques

The anti-CD3 scFv VH containing the GS-CTP linker peptide was structurally modeled using computer software and the spatial conformation of molecular docking of anti-CD3 scFv and its antigen CD3 epsilon chain was simulated and predicted.

The sequence of the GS-CTP linker peptide between anti-Her2 scFv and anti-CD3 scFv in the bispecific antibody AB7K7 is (GGGGGGSGGGGSGGGGSSSSSKAPPPS), wherein the first half of the sequence is a GS-flexible peptide (GGGGGGSGGGGSGGGGS), and the second half is CTP-rigid peptide (SSSSKAPPPS). The rigid CTP portion (SSSSKAPPPS) is connected to the N-terminus of the anti-CD3 scFv VH. Through three-dimensional structural modeling using software phyre2, it is found that the CTP peptide fragment structurally overlays on the CDR1 region of VH of the anti-CD3 scFv (FIG. 2-14), which may hinder or disrupt the binding of the CD3 antibody to its antigen. The VH of the anti-CD3 scFv connected to the GS linker peptide (containing only the GS flexible peptide with the removal of CTP) was subjected to three-dimensional structural modeling using software phyre2, and then it is found that the GS linker peptide is far from the CDR region (FIG. 2-15) and does not affect antigen-antibody binding. Even if the GS linker peptide is close to the CDR region, the GS linker peptide can freely move away from the antigen-antibody binding region due to its own flexibility and thus does not affect antigen-antibody binding.

Further, the molecular docking between the anti-CD3 scFv and its antigen CD3 epsilon chain was simulated by software Discovery Studio. Since the structure of the double-chain anti-CD3 FV is highly similar to the structure of the anti-CD3 scFv, the structure simulation was performed using the double-chain anti-CD3 FV instead of the anti-CD3 scFv. The simulation results show that the antigen CD3 epsilon chain binds to CDR2 and CDR3 of VH of the anti-CD3 Fv while does not bind to the CDR1 region (FIG. 2-16), which indicates that the CTP overlaying the VH CDR1 region of the anti-CD3 Fv does not interfere with the binding of the anti-CD3 scFv to the antigen. However, given that the CD3 molecule is a complex including one CD3 gamma chain, one CD3 delta chain, and two CD3 epsilon chains, the CD3 molecule, together with the TCR and Zeta chains, constitutes a T-cell receptor complex. Although the CTP peptide fragment covering the VH CDR1 of the anti-CD3 scFv does not directly interfere with the binding of the anti-CD3 scFv to its antigen CD3 epsilon chain, the CTP peptide fragment may indirectly affect the binding of the anti-CD3 scFv to its antigen CD3 epsilon chain by making spatial structural contact with a certain constituent protein of the T-cell receptor complex.

Due to the presence of CTP covering the VH CDR1 region of the anti-CD3 scFv, the binding affinity of the anti-CD3 scFv for its antigen is greatly diminished so that there is no substantial release of cytokines caused by the overactivation of T cells, thereby avoiding some unnecessary T cell-mediated non-specific killing.

Example 3 Pharmacodynamics study of Anti-Her2×CD3 bispecific antibodies in a mouse transplanted tumor model

3.1 NCG mouse model of transplanted tumor constructed by subcutaneously co-inoculating human CIK cells and human breast cancer cells HCC1954

Her2-positive human breast cancer cells HCC1954 were selected to study the effect of bispecific antibodies in inhibiting tumor growth *in vivo* in an NCG mouse model of transplanted tumor constructed by subcutaneously co-inoculating human CIK cells and HCC1954 cells.

The peripheral blood of a normal human was subjected to density gradient centrifugation (Lymphoprep™, Lymphocytes Separation Medium, STEMCELL) to separate human PBMCs. Then the human PBMCs were resuspended in RPMI-1640 culture medium added with 10% inactivated FBS, and added with OKT3 at a final concentration of 1 μg/mL and human IL-2 at 250 IU/mL. After three days of culture, the human PBMCs were centrifuged at 300 g for 5 minutes, and the medium was changed. The cells were cultured in RPMI-1640 added with 10% inactivated FBS and added with human IL-2 at 250 IU/mL. After that, a fresh medium was then added every 2 days and CIK cells were collected on the tenth day of culture. Female NCG mice at the age of seven to eight weeks (purchased from Jiangsu GemPharmatech Co. Ltd Company) were selected and HCC1954 cells in the logarithmic growth stage were collected. $5 \times 10^6$ HCC1954 cells and $5 \times 10^5$ CIK cells were mixed and inoculated subcutaneously on the right back of each NCG mouse. One hour later, the mice were randomly divided into seven groups with five mice in each group according to their weights and intraperitoneally administered with corresponding drugs. All positive control groups and PBS control group were administered twice a week for a total of 3 doses, wherein the positive control groups were administered with Herceptin (from Roche) at doses of 1 mg/kg and 3 mg/kg, respectively, and the PBS control group was administered with a PBS solution of the same volume as Herceptin. The treated groups were administered with bispecific antibodies AB7K4 and AB7K7 every day at doses of 0.1 mg/kg and 1 mg/kg, respectively, for a total of 10 doses. The day of administration was recorded as Day 0. The maximum diameter (D) and the minimum diameter (d) of the tumor were measured weekly with an electronic vernier caliper. The volume of the tumor was calculated using the following formula: volume ($mm^3$) = $[D \times d^2]/2$. The tumor growth inhibition rate (TGI) was calculated for each treated group using the following formula: TGI (%) = (1 - volume of the treated group/volume of the control group) × 100%.

As shown in FIG. 3-1, on Day 33 of administration, the average tumor volume of the PBS control group was 1494.61 ± 500.28 $mm^3$; the average tumor volume of the treated group administrated with Herceptin at a dose of 1 mg/kg was 1327.29 ± 376.65 $mm^3$; the average tumor volume of the treated group administrated with Herceptin at a dose of 3 mg/kg was 510.49 ± 106.07 $mm^3$, and the TGI was 65.84%, which was not significantly different from that of the control group. The average tumor volumes of treated groups administrated with AB7K4 at doses of 0.1 mg/kg and 1 mg/kg were 304.10 ± 108.50 $mm^3$ and 79.70 ± 58.14 $mm^3$, respectively, and TGIs thereof were 79.65% and 94.67%, respectively, which were significantly different from that of the PBS control group ($P < 0.05$). The average tumor volumes of treated groups administrated with AB7K7 at doses of 0.1 mg/kg and 1 mg/kg were 385.82 ± 95.41 $mm^3$ and 209.98 ± 51.74 $mm^3$, respectively, and TGIs thereof were 74.19% and 85.95%, respectively, which were significantly different from that of the PBS control group ($P < 0.05$). In summary, the results show that the bispecific antibodies AB7K4 and AB7K7 at different doses could inhibit the growth of tumor cells by activating human immune cells in animals and exhibited great anti-tumor effects; and at the same dose of 1 mg/kg, the anti-tumor effect of the bispecific antibody was better than that of the monoclonal antibody Herceptin.

3.2 NPG mouse model of transplanted tumor constructed by subcutaneously co-inoculating human CIK cells and human breast cancer cells HCC1954

Her2-positive human breast cancer cells HCC1954 were selected to study the inhibiting effect of bispecific antibodies on tumor growth *in vivo* in an NPG mouse model of transplanted tumor constructed by subcutaneously co-inoculating human CIK cells and human breast cancer cells HCC1954.

CIK cells were prepared in the method as described in Example 3.1. Female NPG mice at the age of seven to eight weeks (purchased from Beijing Vitalstar Biotechnology Co., Ltd.) were selected and HCC1954 cells in the logarithmic growth stage were collected. $5 \times 10^6$ HCC1954 cells and $5 \times 10^5$ CIK cells were mixed and inoculated subcutaneously on the right back of each NPG mouse. After 6 days of tumor growth, the mice were randomly divided into three groups with six mice in each group according to the tumor volumes and weights and intraperitoneally administered with corresponding drugs. Specifically, AB7K7 treated groups were administered twice a week at doses of 0.1 mg/kg and 1 mg/kg, respectively, and the control group was administered with a PBS solution of the same volume as AB7K7. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 3-2, on Day 21 of administration, the average tumor volume of the PBS control group was 821.73 $\pm$ 201.82 $mm^3$; the average tumor volume of the treated group administrated with AB7K7 at a dose of 0.1 mg/kg was 435.60 $\pm$ 51.04 $mm^3$, and the TGI was 50.83%, which was not significantly different from that of the control group; the average tumor volume of the treated group administrated with AB7K7 at a dose of 1 mg/kg was 40.98 $\pm$ 12.64 $mm^3$, and the TGI was 95.37%, which was significantly different from that of the control group ($P < 0.01$). The above results show that the administration of the bispecific antibody AB7K7 had a good therapeutic effect even when tumors had grown to a certain volume, wherein 50% tumor inhibition effect was achieved at the low dose of 0.1 mg/kg, and there was complete tumor regression in 4 of 6 mice in the treated group at the dose of 1 mg/kg and the tumor volumes in the other 2 mice were both less than 100 $mm^3$, which was smaller than the tumor volume at the time of grouping (the average tumor volume of this group at the time of grouping was 161.37 $\pm$ 18.98 $mm^3$). Therefore, the bispecific antibody AB7K7 had a great therapeutic effect on tumors.

In addition, the inhibiting effect of bispecific antibodies AB7K7 and AB7K8 on tumor growth in the above-described transplanted tumor model at two administration frequencies were also studied. CIK cells were prepared in the method as described above. Female NPG mice at the age of seven to eight weeks were selected, and 5 $\times$ $10^6$ HCC1954 cells and 5 $\times$ $10^5$ CIK cells were mixed and inoculated subcutaneously on the right back of each NPG mouse. One hour later, the mice were randomly divided into six groups with six mice in each group according to their weights and intraperitoneally administered with corresponding drugs. Specifically, the control group and the Herceptin treated group were administered twice a week, wherein Herceptin was administrated at a dose of 3 mg/kg and the control group was administered with a PBS solution of the same volume as Herceptin. The bispecific antibody AB7K7 was administered at a dose of 1 mg/kg and AB7K8 was administered at a dose of 0.7 mg/kg. Two administration frequencies were set for each of the two bispecific antibodies, wherein the QD group was administered once a day for 10 consecutive days and the BIW group was administered twice a week. The day of administration was recorded as Day 0. The maximum diameter (D) and the minimum diameter (d) of the tumor were measured weekly. The tumor volume ($mm^3$) of each group and the tumor growth inhibition rate (TGI) (%) of each treated group were calculated using the formulas as shown above.

As shown in FIG. 3-3, on Day 25 of administration, the average tumor volume of the PBS control group was 1588.12 $\pm$ 120.46 $mm^3$; the average tumor volume of the treated group administrated with Herceptin at a dose of 3 mg/kg was 361.72 $\pm$ 134.70 $mm^3$; the average tumor volumes of the QD group and the BIW group administrated with AB7K7 were 260.18 $\pm$ 45.96 $mm^3$ and 239.39 $\pm$ 40.62 $mm^3$, respectively, and TGIs were 83.62% and 84.93%, respectively, which were significantly different from that of the PBS control group ($P < 0.01$); the average tumor volumes of the QD group and the BIW group administrated with AB7K8 were 284.98$\pm$ 26.62 $mm^3$ and 647.14 $\pm$ 118.49 $mm^3$, respectively, and TGIs were 82.06% and 59.25 %, respectively, which were significantly different from that of the PBS control group ($P < 0.01$). As can be seen from the above results, the anti-tumor effect of the bispecific antibody AB7K7 was superior to that of the Herceptin in both the QD group and the BIW group; at equimolar doses, AB7K8 and AB7K7 in the QD group exhibited basically equal tumor inhibiting effects, while the anti-tumor effect of AB7K7 in the BIW group was significantly superior than that of AB7K8, presumably due to the fact that AB7K8 is a bispecific antibody of BiTE configuration with no Fc domain, and therefore AB7K7 has a longer half-life than AB7K8, from which it is anticipated that the clinical administration frequency of AB7K7 is reduced and AB7K7 has a better therapeutic effect.

3.3 NPG mouse model of transplanted tumor constructed by subcutaneously co-inoculating human CIK cells and human ovarian cancer cells SK-OV-3

Her2-positive human ovarian cancer cells SK-OV-3 were selected to study the inhibiting effect of bispecific antibodies on tumor growth *in vivo* in an NPG mouse model of transplanted tumor constructed by subcutaneously co-inoculating human CIK cells and SK-OV-3 cells.

The peripheral blood of a normal human was subjected to density gradient centrifugation to separate human PBMCs. Then the human PBMCs were resuspended in McCoy's 5A culture medium added with 10% inactivated FBS, and added with OKT3 at a final concentration of 1 $\mu$g/mL and human IL-2 at 250 IU/mL. After three days of culture, the human PBMCs were centrifuged at 300 g for 5 minutes, and the supernatant was discarded. The cells were resuspended in RPMI-1640 added with 10% inactivated FBS and added with 250 IU/mL of human IL-2. After that, a fresh medium was then added every 2 days and CIK cells were collected on the tenth day of culture. Female NPG mice at the age of seven to eight weeks were selected and SK-OV-3 cells (purchased from the cell bank of Chinese Academy of Sciences, Shanghai) in the logarithmic growth stage were collected. $3 \times 10^6$ SK-OV-3 cells and $3 \times 10^5$ CIK cells were mixed and inoculated subcutaneously on the right back of each NPG mouse. One hour after inoculation, the mice were randomly divided into seven groups with six mice in each group according to their weights and intraperitoneally administered with corresponding drugs. Herceptin and AB7K7 treated groups were administered twice a week at doses of 1 mg/kg, 0.2 mg/kg, and 0.04 mg/kg, respectively, and the control group was administered with a PBS solution of the same volume. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 3-4, on Day 21 of administration, the average tumor volume of the PBS control group was $834.09 \pm 45.64$ $mm^3$; the average tumor volumes of the treated groups administrated with Herceptin at doses of 1 mg/kg, 0.2 mg/kg, and 0.04 mg/kg were $644.84 \pm 58.22$ $mm^3$, $884.95 \pm 38.63$ $mm^3$, and $815.79 \pm 78.39$ $mm^3$, respectively; and the tumors in all AB7K7 treated groups were completely regressed. The above results show that in the ovarian cancer SK-OV-3 model, AB7K7 enabled the tumor to completely regress even at a very low dose of 0.04 mg/kg, exhibiting an excellent anti-tumor effect.

3.4 NPG mouse model of transplanted tumor constructed by subcutaneously co-inoculating human CIK cells and human colon cancer cells HT-29

Her2-positive human colon cancer cells HT-29 were selected to study the inhibiting effect of bispecific antibodies on tumor growth *in vivo* in an NPG mouse model of transplanted tumor constructed by subcutaneously co-inoculating human CIK cells and HT-29 cells.

CIK cells were prepared in the method as described in Example 3.1. Female NPG mice at the age of seven to eight weeks were selected and HT-29 cells (purchased from the cell bank of Chinese Academy of Sciences, Shanghai) in the logarithmic growth stage were collected. $3 \times 10^6$ HT-29 cells and $3 \times 10^6$ CIK cells were mixed and inoculated subcutaneously on the right back of each NPG mouse. One hour after inoculation, the mice were randomly divided into five groups with six mice in each group according to their weights and intraperitoneally administered with corresponding drugs. Specifically, Herceptin was administered at a dose of 3 mg/kg, and AB7K7 was administered at doses of 3 mg/kg, 1 mg/kg, and 0.3 mg/kg, respectively. All treated groups were administered twice a week. The control group was administered with a PBS solution of the same volume. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 3-5, on Day 21 of administration, the average tumor volume of the PBS control group was $1880.52 \pm 338.26$ $mm^3$; the average tumor volume of the treated group administrated with Herceptin at a dose of 3 mg/kg was $1461.36 \pm 177.94$ $mm^3$; the average tumor volumes of the treated groups administrated with AB7K7 at doses of 3 mg/kg, 1 mg/kg, and 0.3 mg/kg were $13.94 \pm 7.06$ $mm^3$, $26.31 \pm 10.75$ $mm^3$, and $10.47 \pm 6.71$ $mm^3$, wherein tumors in four mice in the treated group at a dose of 0.3 mg/kg were completely regressed, tumors in three mice in the treated group at a dose of 1 mg/kg were completely regressed, and tumors in four mice in the treated group at a dose of 3 mg/kg were completely regressed. The above results show that in the colon cancer HT-29 model, Herceptin had few pharmacological effect on this tumor model, whereas AB7K7 exhibited complete tumor regression in mice at all three doses and exhibited excellent anti-tumor effect even at very low doses.

3.5 CD34 immune-reconstituted NPG mouse model of transplanted tumor constructed by inoculating human breast cancer cells HCC1954

Her2-positive human breast cancer cells HCC1954 were selected to study the inhibiting effect of bispecific antibodies on tumor growth *in vivo* in a CD34 immune-reconstituted NPG mouse model of transplanted tumor constructed by subcutaneously inoculating human breast cancer cells HCC1954.

CD34-positive hematopoietic stem cells were enriched from fresh umbilical cord blood using CD34-positive selective magnetic beads (purchased from Miltenyi Biotec, Germany). Female NPG mice at the age of seven to eight weeks (purchased from Beijing Vitalstar Biotechnology Co., Ltd.) were selected and injected with CD34-positive hematopoietic stem cells via the tail vein to reconstitute a human immune system in each mouse. Sixteen weeks later, blood was collected from the orbital venous plexus of mice for flow cytometry, and when the proportion of human CD45 in mice was greater than 15%, it was considered that the immune reconstitution succeeded. HCC1954 cells in the logarithmic growth stage were collected and $5 \times 10^6$ HCC1954 cells were inoculated subcutaneously on the right back of the mice with successful immune reconstitution. One hour after inoculation, the mice were randomly divided into three groups with six mice in each group according to their weights. The treated groups were intraperitoneally administered with AB7K7 and Herceptin at a dose of 1 mg/kg, and the control group was administered with a PBS solution of the same volume, twice a week for a total of 6 doses. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 3-6, on Day 21 of administration, the average tumor volume of the PBS control group was 475.23 ± 58.82 $mm^3$; the average tumor volume of the treated group administrated with Herceptin was 293.27 ± 66.35 $mm^3$, and the TGI was 38.29%, which was not significantly different from that of the control group; the average tumor volume of the treated group administrated with AB7K7 was 0.67 ± 0.67 $mm^3$, and the TGI was 99.86%, meaning that basically all tumors were regressed, which was significantly different from that of the control group (*P < 0.01*). In summary, the above results show that the bispecific antibody AB7K7 had an excellent anti-tumor effect in the CD34 immune-reconstituted model.

3.6 PBMC immune-reconstituted NPG mouse model of transplanted tumor constructed by inoculating human breast cancer cells HCC1954

Her2-positive HCC1954 cells were selected to study the inhibiting effect of bispecific antibodies on tumor growth *in vivo* in a PBMC immune-reconstituted NPG mouse model of transplanted tumor constructed by inoculating human breast cancer cells HCC1954.

The peripheral blood of a normal human was subjected to density gradient centrifugation to separate human PBMCs. Female NPG mice at the age of five to six weeks were selected and intraperitoneally injected with human PBMC cells to reconstitute a human immune system in each mouse. After seven days of PBMC injection, HCC1954 cells in the logarithmic growth stage were collected and 5 × $10^6$ HCC1954 cells were inoculated subcutaneously on the right back of each mouse. After 13 days of PBMC injection, blood was collected from the orbital venous plexus for flow cytometry, and when the proportion of human CD45 in mice was greater than 15%, it was considered that the immune reconstitution succeeded. After 14 days of PBMC injection, the mice with successful immune reconstitution were randomly divided into two groups with six mice in each group according to the tumor volumes and weights. The treated group was intraperitoneally administered with AB7K7 at a dose of 1 mg/kg, and the control group was administered with PBS, three times a week. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 3-7, on Day 23 of administration, the average tumor volume of the PBS control group was 1224.05 ± 224.39 $mm^3$; the average tumor volume of the treated group administrated with AB7K7 was 32.00 ± 0.00 $mm^3$, and the TGI was 97.41%, meaning that basically all tumors were regressed, which was significantly different from that of the control group (*P* < *0.001*). In summary, the above results show that the bispecific antibody AB7K7 had an excellent anti-tumor effect in the PBMC immune-reconstituted model.

Example 4 Evaluation of the safety of Anti-Her2xCD3 bispecific antibodies

4.1 Bispecific antibodies being incapable of mediating non-specific killing on Her2-negative tumor cells

Her2-negative human Burkkit's lymphoma Raji cells were selected to study whether bispecific antibodies can inhibit tumor growth in an NCG mouse model of transplanted tumor constructed by subcutaneously co-inoculating human CIK cells and human Burkkit's lymphoma Raji cells.

CIK cells were prepared in the method as described in Example 3.1. Female NCG mice at the age of seven to eight weeks were selected and Raji cells (purchased from the cell bank of Chinese Academy of Sciences, Shanghai) in the logarithmic growth stage were collected. 5 × $10^6$ Raji cells and 2 × $10^6$ CIK cells were mixed and inoculated subcutaneously on the right back of each NCG mouse. One hour after inoculation, the mice were randomly divided into three groups with five mice in each group according to their weights. The treated groups were intraperitoneally administered with AB7K4 and AB7K7 at a dose of 1 mg/kg, and the control group was administered with a PBS solution of the same volume, once a day continuously for 10 days. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 4-1, on Day 25 of administration, the average tumor volume of the PBS control group was 2439.88 ± 193.66 $mm^3$; the average tumor volume of the treated group administrated with AB7K4 was 2408.81 ± 212.44 $mm^3$; the average tumor volume of the treated group administrated with AB7K7 was 2598.11 ± 289.35 $mm^3$; and there was no difference between the average tumor volume of each of the two treated groups and the average tumor volume of the control group. In summary, the results show that bispecific antibodies AB7K4 and AB7K7 exhibited no non-specific killing on Her2-negative cell strains, which indicates that AB7K4 and AB7K7 do not mediate T cells to kill non-target tissues *in vivo* (i.e., specifically dependent on binding of bispecific antibodies to corresponding target antigens), there is no off-target toxicity, and the safety is high.

4.2 Bispecific antibodies killing tumor cells depending on the activation of T cells

Her2-positive human breast cancer cells HCC1954 were selected to study whether bispecific antibodies inhibit tumor growth in an NPG mouse model of transplanted tumor constructed by subcutaneously inoculating human breast cancer cells HCC1954.

Female NPG mice at the age of seven to eight weeks were selected and HCC1954 cells in the logarithmic growth stage were collected. $5 \times 10^6$ HCC1954 cells and Matrigel (Corning, Cat. No. 354234) were mixed in a volume ratio of 1 : 1 and then inoculated subcutaneously on the right back of each NPG mouse. After 6 days of tumor growth, the mice were randomly divided into three groups with six mice in each group according to the tumor volumes and weights. The treated groups were intraperitoneally administered with Herceptin at a dose of 3 mg/kg and AB7K7 at a dose of 1 mg/kg, respectively, and the control group was administered with a PBS solution of the same volume, twice a week. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 4-2, on Day 21 of administration, the average tumor volume of the PBS control group was $1311.35 \pm 215.70$ $mm^3$; the average tumor volume of the treated group administrated with Herceptin was $273.98 \pm 60.10$ $mm^3$; the average tumor volume of the treated group administrated with AB7K7 was $1243.20 \pm 340.31$ $mm^3$, which was not different from the average tumor volume of the control group. In summary, the results show that AB7K7 did not inhibit the growth of HCC1954 subcutaneous tumors in the absence of human immune cells, indicating that bispecific antibody AB7K7 needs to be mediated by immune effector cells so as to kill tumor cells, unlike Herceptin, which primarily depends on FcγR-mediated ADCC or CDC effects to kill tumor cells. Thus, it is proved that Fc variants contained in AB7K7 cannot bind to FcγR, which avoids mediating systemic activation of T cells caused by extensive expression of its receptor FcγR, resulting in higher drug safety.

4.3 Evaluation of toxicity of bispecific antibodies to normal cynomolgus monkeys

Adult cynomolgus monkeys (purchased from Guangzhou Xiangguan Biotechnology Co., Ltd.) at the age of 3-4 years and with the weight of 3-4 kg were divided into three groups with one monkey in each group, wherein the three groups were a vehicle control group, an AB7K7 treated group and an AB7K8 treated group. The groups were administrated via intravenous drip by a peristaltic pump for 1 hour on Day 0 (D0), Day 7 (D7), Day 21 (D21), and Day 28 (D28), respectively, for a total of four doses, and the drug dose was gradually increased each time. The monkeys were weighed weekly. The dose amount and volume administered are shown in Table 4-1.

On D0, after administration, cynomolgus monkeys in the AB7K8 treated group exhibited somnolence and pupil contraction and recovered to normal the next day while there was no abnormality in the other groups. On D7, after administration, cynomolgus monkeys in the AB7K7 treated group exhibited vomiting symptoms 2-3 hours after administration and recovered to normal the next day of administration while there was no abnormality in the other groups. On D21, after administration, cynomolgus monkeys in the AB7K7 treated group exhibited symptoms of vomiting food 3 hours after administration and excreted jelly-like feces, cynomolgus monkeys in the AB7K8 treated group exhibited symptoms of vomiting food 1 hour after administration, and cynomolgus monkeys in both groups recovered to normal on the second day after administration; On D28, after administration, cynomolgus monkeys in both AB7K7 treated group and AB7K8 treated group exhibited vomiting symptoms 40 to 50 minutes later and excreted feces 3 hours later, in which jelly-like mucus was found; cynomolgus monkeys in the AB7K7 treated group excreted watery feces with fishy smelling; and 24 hours later, all the animals recovered to normal and ingested normally. The body weight change of cynomolgus monkeys is shown in FIG. 4-3, wherein the arrow represents the administration time. It can be seen that the body weight of each group does not change too much and fluctuates within the normal physiological range.

Table 4-1 Dosing schedule for cynomolgus monkey acute toxicity evaluation

| Group | To-be-tested drugs name | Dose volume | Dose amount |
|---|---|---|---|
| G1 | Vehicle control group | D0: 5 mL/kg<br>D7: 5 mL/kg | N/A |
| | | D21: 10 mL/kg<br>D28: 10 mL/kg | |

(continued)

| Group | To-be-tested drugs name | Dose volume | Dose amount |
|---|---|---|---|
| G2 | AB7K7 | D0: 5 mL/kg<br>D7: 5 mL/kg<br>D21: 10 mL/kg<br>D28: 10 mL/kg | D0: 0.06 mg/kg<br>D7: 0.3 mg/kg<br>D21: 1.5 mg/kg<br>D28: 3 mg/kg |
| G3 | AB7K8 | D0: 5 mL/kg<br>D7: 5 mL/kg<br>D21: 10 mL/kg<br>D28: 10 mL/kg | D0: 0.04 mg/kg<br>D7: 0.2 mg/kg<br>D21: 1 mg/kg<br>D28: 2 mg/kg |

The different degree of diarrhea observed in this example may be related to the expression of related receptors in the gut, which is supposed to be caused by the imbalance of chloride ion in the gut caused by the inhibition of heterodimer of Her1/Her2 or Her2/Her3 by bispecific antibodies, which belongs to the extension of pharmacological action and can recover to normal after 24 hours of administration. Cynomolgus monkeys were still well tolerated when administrated with AB7K7 at a high dose of 3 mg/kg. The results of pharmacodynamics test in mice show that AB7K7 at a low dose shows a good anti-tumor effect, which indicates that AB7K7 has a wide treatment window and high safety.

Example 5 Pharmacokinetics study of Anti-Her2×CD3 antibodies

5.1 *In vivo* pharmacokinetics test of bispecific antibody AB7K7 in SD mice

AB7K7 was administered to four healthy Sprague-Dawley (SD) rats (purchased from Shanghai Salccas Laboratory Animals Co., Ltd.,) via the tail vein at a dose of 1 mg/kg. The blood sampling time points were Hour 1, Hour 3, Hour 6, Hour 24, Hour 72, Hour 96, Hour 120, Hour 168, Hour 216 and Hour 264, respectively. A certain amount of whole blood was taken at each time point, the serum was separated, and then the drug concentration in the serum was detected by two ELISA methods.

Method I. Plates were coated with the anti-AB7K7 antibody A (Ampsource Biopharma Shanghai Inc., mouse-anti-herceptin) at a concentration of 0.5 μg/mL. AB7K7 was formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled anti-AB7K7 antibody B (Ampsource Biopharma Shanghai Inc., anti-herceptin-HRP) was used at a concentration of 1 : 5000, and developed with TMB. The pharmacokinetics parameters were calculated using software PKSolver. Specific parameters are shown in Table 5-1.

Method II. The drug concentration in the serum of the SD rats was detected. Plates were coated with the anti-AB7K7 antibody A (Ampsource Biopharma Shanghai Inc., mouse-anti-herceptin) at a concentration of 0.5 μg/mL. AB7K7 was formulated at concentrations of 5 ng/mL, 2.5 ng/mL, 1.25 ng/mL, 0.625 ng/mL, 0.3125 ng/mL, 0.156 ng/mL and 0.078 ng/mL, separately. Standard curves were established. Mouse anti-human IgG Fc-HRP (Ampsource Biopharma Shanghai Inc.) was added at a concentration of 1 : 5000, and developed with TMB. The pharmacokinetics parameters were calculated using software PKSolver. Specific parameters are shown in Table 5-2.

FIG. 5-1 shows the blood drug concentration of AB7K7 in the body of rats detected using two different detection methods. It can be seen that the blood drug concentrations obtained by detecting the concentration of AB7K7in blood using two different detection methods were basically the same, and the calculated pharmacokinetics parameters were roughly equivalent, which indicates that AB7K7 can be metabolized in the form of intact molecules *in vivo,* thereby ensuring the biological function of AB7K7.

Table 5-1 Pharmacokinetics parameters of bispecific antibody AB7K7 in SD rats

| (Method 1) | | | | |
|---|---|---|---|---|
| AB7K7 | $t_{1/2}$ (h) | AUC 0-inf_ob (ng/mL*h) | Vz_obs (μg)/(ng/mL) | Cl_obs (μg)/(ng/mL)/h |
| Pharmacoki netics parameter | 42.10 | 550236.77 | 0.02351 | 3.811E-4 |

Table 5-2 Pharmacokinetics parameters of bispecific antibody AB7K7 in SD rats

| (Method 2) | | | | |
|---|---|---|---|---|
| AB7K7 | $t_{1/2}$ (h) | AUC 0-inf_ob (ng/mL*h) | Vz_obs (μg)/(ng/mL) | Cl_obs (μg)/(ng/mL)/h |
| Pharmaco kinetics parameter | 41.02 | 706126.89 | 0.01720 | 2.899E-4 |

5.2 *In vivo* pharmacokinetics test on bispecific antibody AB7K7 in NPG model mice

NPG mice (purchased from Beijing Vitalstar Biotechnology Co., Ltd.) were inoculated with HCC1954 cells (purchased from the Institute of Cells, Chinese Academy of Sciences) one week before administration, with an inoculum density of 3.5 × $10^6$/mouse. CIK cells were resuscitated two days before administration, cultured for 24 hours and then collected and injected intravenously into mice. The mice were randomly divided into three groups with four mice in each group. The three treated groups were administrated at doses of 0.3 mg/kg, 1 mg/kg and 3 mg/kg, respectively. The blood sampling time points were Hour 1, Hour 3, Hour 6, Hour 24, Hour 48, Hour 72, Hour 96, Hour 120, Hour 168, Hour 216 and Hour 264, respectively. A certain amount of whole blood was taken at each time point, the serum was separated, and then the drug concentration in the serum was detected by ELISA.

Plates were coated with the anti-AB7K7 antibody A (Ampsource Biopharma Shanghai Inc., mouse-anti-herceptin) at a concentration of 0.5 μg/mL. AB7K7 was formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled anti-AB7K7 antibody B (Ampsource Biopharma Shanghai Inc., mouse-anti-herceptin) was used at a concentration of 1 : 5000, and developed with TMB. The pharmacokinetics parameters were calculated using software PKSolver. Specific parameters are shown in Table 5-3. It can be seen from Table 5-3 that the pharmacokinetics parameters of AB7K7 in NPG model mice were not significantly different from those in SD rats.

Table 5-3 Pharmacokinetics parameters of bispecific antibody AB7K7 in NPG model mice

| Parameter / Group | $t_{1/2}$ (h) | AUC0-inf_obs (ng/mL*h) | Vz_ob (μg)/(ng/mL) | Cl_obs (μg)/(ng/mL)/h |
|---|---|---|---|---|
| 0.3 mg/kg IV | 39.54 | 79932.39 | 0.004872 | 8.968E-05 |
| 1 mg/kg IV | 42.70 | 597036.63 | 0.002461 | 3.996E-05 |
| 3 mg/kg IV | 46.03 | 2171649.41 | 0.002292 | 3.469E-05 |

5.3 *In vivo* pharmacokinetics test on bispecific antibody AB7K8 in SD rats

AB7K8 was administered to three healthy SD rats via the tail vein at doses of 1 mg/kg and 3 mg/kg, respectively. The blood sampling time points were Hour 0.25, Hour 0.5, Hour 1, Hour 2, Hour 3, Hour 4, Hour 5, and Hour 7, respectively. A certain amount of whole blood was taken at each time point, the serum was separated, and then the drug concentration in the serum was detected by ELISA.

Plates were coated with the anti-AB7K8 antibody C (Ampsource Biopharma Shanghai Inc., mouse-anti-herceptin) at a concentration of 2.5 μg/mL. AB7K8 was formulated at concentrations of 25 ng/mL, 12.5 ng/mL, 6.25 ng/mL, 3.125 ng/mL, 1.56 ng/mL, and 0.78 ng/mL, separately. Standard curves were established. HRP-labeled anti-his antibody (Ampsource Biopharma Shanghai Inc.) was used at a concentration of 1 : 5000, and developed with TMB. The pharmacokinetics parameters were calculated using software PKSolver. Specific parameters are shown in Table 5-4.

The pharmacokinetics parameter $T_{1/2}$ of AB7K8 were almost the same at two doses, which indicates that AB7K8 showed linear metabolic kinetics in SD rats. Since AB7K8 does not contain Fc, $T_{1/2}$ of AB7K8 is very short, about twenty times shorter than $T_{1/2}$ of AB7K7.

Table 5-4 Pharmacokinetics parameters of bispecific antibody AB7K8 in SD rats

| AB7K8 | $t_{1/2}$ (h) | AUC 0-inf_ob (ng/mL*h) | Vz_obs (μg)/(ng/mL) | Cl_obs (μg)/(ng/mL)/h |
|---|---|---|---|---|
| 1 mg/kg IV | 2.27 | 4623.14 | 0.17082 | 0.05191 |
| 3 mg/kg IV | 1.98 | 20608.77 | 0.10220 | 0.03579 |

5.4 *In vivo* pharmacokinetics test on bispecific antibody AB7K in SD rats

AB7K was administered to four healthy SD rats via the tail vein at a dose of 0.8 mg/kg. The blood sampling time points were Hour 2, Hour 24, Hour 48, Hour 72, Hour 96, Hour 120, Hour 144, Hour 168, Hour 216 and Hour 264, respectively. A certain amount of whole blood was taken at each time point, the serum was separated, and then the drug concentration in the serum was detected by two ELISA methods.

Method I. Plates were coated with the anti-AB7K antibody A (Ampsource Biopharma Shanghai Inc., mouse-anti-herceptin) at a concentration of 1 μg/mL. AB7K was formulated at concentrations of 20 ng/mL, 10 ng/mL, 5 ng/mL, 2.5 ng/mL, 1.25 ng/mL, 0.625 ng/mL and 0.3125 ng/mL, separately. Standard curves were established. 25 ng/mL of biotin-labeled human CD3E&CD3D (Acro, Cat. No. CDD-H82W0) was added, incubated for 1 hour, and after that, HRP-labeled streptavidin (BD Pharmingen, Cat. No. 554066) diluted at a factor of 1 : 500 was added, and developed with TMB. The pharmacokinetics parameters were calculated using software PKSolver. Specific parameters are shown in Table 5-5.

Table 5-5 Pharmacokinetics parameters of bispecific antibody AB7K

| AB7K | $t_{1/2}$ (h) | AUC 0-inf_ob (ng/mL*h) | Vz_obs (μg)/(ng/mL) | Cl_obs (μg)/(ng/mL)/h |
|---|---|---|---|---|
| Pharmacokinetics parameter | 60.47 | 1022788.69 | 0.01726 | 1.985E-4 |

Method II. Plates were coated with the anti-AB7K antibody A (Ampsource Biopharma Shanghai Inc., mouse-anti-herceptin) at a concentration of 1 μg/mL. AB7K was formulated at concentrations of 20 ng/mL, 10 ng/mL, 5 ng/mL, 2.5 ng/mL, 1.25 ng/mL, 0.625 ng/mL and 0.3125 ng/mL, separately. Standard curves were established. Mouse anti-human IgG Fc-HRP (diluted at 1 : 10000) (Ampsource Biopharma Shanghai Inc.) was added, incubated in an incubator for 1 hour, and developed with TMB.

FIG. 5-2 shows the blood drug concentration of AB7K in rats by using two different detection methods. From the results, it is showed that the difference between the two detection methods is large. The concentrations of the first two points (2h, 1D) on the curve were close, but after the next day, the concentrations detected by the two methods differed greatly, which is supposed to be caused by the fact that the linkage peptide between the heavy chains of anti-CD3 scFv and anti-Her2 antibodies was broken. AB7K is structurally unstable in vivo and thus can't play its biological function, while the improved AB7K7 can metabolize in complete form in vivo and thus can play its biological function normally.

5.5 *In vivo* pharmacokinetics test on bispecific antibodies AB7K7 and AB7K8 in cynomolgus monkeys

Female cynomolgus monkeys (purchased from Guangzhou Xiangguan Biotechnology Co., Ltd.) with the weight of 3-4 kg were divided into three groups with one monkey in each group. The first group (G1-1) was a blank control group; the second group (G2-1) was an AB7K7 treated group administrated at a dose of 0.3 mg/kg; and the third group (G3-1) was an AB7K8 treated group administrated at a dose of 0.2 mg/kg. The blood sampling time points were Minute 15, Hour 1, Hour 3, Hour 6, Hour 24, Hour 48, Hour 72, Hour 96, Hour 144, Hour 192, Hour 240 and Hour 288, respectively, a total of 13 time points. Serum was collected from blood and frozen at -80°C. The concentration of the drug in serum was determined by ELISA.

Plates were coated with the anti-AB7K7 antibody A (Ampsource Biopharma Shanghai Inc., mouse-anti-herceptin) at a concentration of 0.5 μg/mL. AB7K7 was formulated at concentrations of 100 ng/mL, 50 ng/mL, 25 ng/mL, 12.5 ng/mL, 6.25 ng/mL, 3.125 ng/mL and 1.56 ng/mL, separately. Standard curves were established. HRP-labeled anti-AB7K7 antibody B (Ampsource Biopharma Shanghai Inc., mouse-anti-herceptin) was used at a concentration of 1 : 5000, and developed with TMB. The pharmacokinetics parameters were calculated using software PKSolver. Specific parameters are shown in Table 5-6.

FIG. 5-3 shows the blood drug concentration of AB7K7 in rats. $T_{1/2}$ of AB7K7 in the normal cynomolgus monkey was only about eight hours. Pharmacokinetics parameters of AB7K8 could not be calculated due to too few points on the concentration-time curve of AB7K8. However, it can be seen from the concentration-time curve that the half-life of AB7K7 in the normal cynomolgus monkey was much longer than the half-life of AB7K8.

Table 5-6 Pharmacokinetics parameters of bispecific antibody AB7K7 in cynomolgus monkeys

| AB7K7 | $t_{1/2}$ (h) | AUC 0-inf_obs (ng/mL*h) | Vz_obs (μg)/ (ng/mL) | Cl_obs (μg)/(ng/mL)/h |
|---|---|---|---|---|
| Pharmacok inetics parameter | 7.95 | 87995.48 | 0.1563 | 0.01364 |

5.6 Evaluation of abilities of bispecific antibodies to bind to FcRn by ELISA

Each bispecific antibody was diluted with the PBS solution to a concentration of 10 μg/ml and added to 96-well plates, 100 μl per well. The plates were coated at 4 °C overnight. The plates were then blocked with 1% skimmed milk powder for 1 hour at room temperature. Biotin-labeled FcRn proteins (ACRO Biosystem, Cat. No. FCM-H8286) were diluted using diluents at pH of 6.0 and 7.0, respectively, with a 4-fold gradient for a total of 11 concentration gradients. The 96-well plates were then washed with PBST of the same pH, and then each of the bispecific antibodies diluted with the diluent at the same pH was added. Control wells without antibodies were set. Incubated for 1 hour at room temperature. Plates were washed with the PBST solution of the same pH, streptavidin-HRP (BD, Cat. No. 554066) was added to 96-well plates, 100 μl per well, and the plates were incubated for 0.5 hours at room temperature. 96-well plates were washed with PBST, and TMB was added to the plates, 100 μl per wells. Color development was performed at room temperature for 15 minutes, and then 0.2 M $H_2SO_4$ was added to stop the color development reaction. The light absorbance values at A450-620 nm were measured by a microplate reader. Analysis was performed by software OriginPro 8, and the $EC_{50}$ values for the binding of bispecific antibodies to FcRn were calculated.

The results show that the ability of each antibody to bind to FcRn was different under different pH conditions, and it is analyzed in conjunction with data of *in vivo* PK that the half-life of bispecific antibody AB7K7 was longer than the half-life of AB7K but shorter than the half-life of Herceptin, which may be more favorable for clinical application (FIGS. 5-4 and 5-5). Table 5-7 and Table 5-8 show the detection results of the ability of each antibody to bind to FcRn at pHs 6.0 and 7.0, respectively.

Table 5-7 Detection of abilities of bispecific antibodies AB7K, AB7K5 and AB7K7 to bind to FcRn at pH 6.0

| | Herceptin | AB7K | AB7K5 | AB7K7 |
|---|---|---|---|---|
| $EC_{50}$ (μg/ml) | 2.591 | 0.8027 | 1.706 | 0.4630 |

Table 5-8 Detection of abilities of bispecific antibodies AB7K, AB7K5 and AB7K7 to bind to FcRn at pH 7.0

| | Herceptin | AB7K | AB7K5 | AB7K7 |
|---|---|---|---|---|
| $EC_{50}$ (μg/ml) | ~287.1 | 1.651 | 13.43 | 4.838 |

Example 6 Preparation of bispecific antibody with scFv1-scFv2-Fc configuration

According to the above research results of six kinds of anti-Her2×CD3 bispecific antibodies, it can be determined that the bispecific antibody with scFv1-scFv2-Fc configuration such as AB7K7 is easy to be prepared, can be purified in a simple and efficient method, and has great stability in preparation and storage process. More advantageously, such a bispecific antibody has a weak non-specific killing effect on normal cells, has significant advantages of controlled toxic and side effects possibly caused by overactivation of effector cells, and has good druggability.

With reference to the design and preparation method of the bispecific antibody AB7K7 in Example 1, a series of bispecific antibody molecules that target immune effector cell antigen CD3 and a tumor-associated antigen were constructed. Such bispecific antibody molecules are tetravalent homodimers formed by two identical polypeptide chains that bind to each other by an interchain disulfide bond in the hing region of the Fc fragment, wherein each polypeptide chain consists of, in sequence from N-terminus to C-terminus, an anti-TAA scFv, a linker peptide, an anti-CD3 scFv, and an Fc fragment. The molecular composition of each structural unit of each bispecific antibody is described below in detail.

The tumor-associated antigen includes, but is not limited to, CD19, CD20, CD22, CD25, CD30, CD33, CD38, CD39, CD40, CD47, CD52, CD73, CD74, CD123, CD133, CD138, BCMA, CA125, CEA, CS1, DLL3, DLL4, EGFR, EpCAM, FLT3, gpA33, GPC-3, Her2, MEGE-A3, NYESO1, PSMA, TAG-72, CIX, folate-binding protein, GD2, GD3, GM2, VEGF, VEGFR2, VEGFR3, Cadherin, Integrin, Mesothelin, Claudin18, αVβ3, α5β1, ERBB3, c-MET, IGF1R, EPHA3, TRAILR1, TRAILR2, RANKL, B7 protein family, Mucin family, FAP, and Tenascin; preferably, the tumor-associated antigen is CD19, CD20, CD22, CD30, CD38, BCMA, CS1, EpCAM, CEA, Her2, EGFR, CA125, Mucin1, GPC-3, and Mesothelin.

Some preferred amino acid sequences of the VH domain and its complementary determining regions (HCDR1, HCDR2, and HCDR3) and amino acid sequences of the VL domain and its complementary determining regions (LCDR1, LCDR2 and LCDR3) of a first single-chain Fv targeting the tumor-associated antigen are exemplified in Table 6-1, wherein the amino acid composition of the linker peptide between VH and VL of the anti-TAA scFv is (GGGGS)n, wherein n = 1, 2, 3, 4 or 5.

Table 6-1 Amino acid sequences of the anti-TAA scFv included in the bispecific antibody and amino acid sequences of its CDR regions

| CD19 | | |
|---|---|---|
| SEQ ID NO: 9 | HCDR1 | SYWMN |
| SEQ ID NO: 10 | HCDR2 | QIWPGDGDTNYNGKFKG |
| SEQ ID NO: 11 | HCDR3 | RETTTVGRYYYAMDY |
| SEQ ID NO: 12 | LCDR1 | KASQSVDYDGDSYLN |
| SEQ ID NO: 13 | LCDR2 | DASNLVS |
| SEQ ID NO: 14 | LCDR3 | QQSTEDPWT |
| SEQ ID NO: 15 | VH | QVQLQQSGAELVRPGSSVKISCKASGYAFSSYWMN WVKQRPGQGLEWIGQIWPGDGDTNYNGKFKGKATL TADESSSTAYMQLSSLASEDSAVYFCARRETTTVG RYYYAMDYWGQGTTVTVSS |
| SEQ ID NO: 16 | VL | DIQLTQSPASLAVSLGQRATISCKASQSVDYDGDS YLNWYQQIPGQPPKLLIYDASNLVSGIPPRFSGSG SGTDFTLNIHPVEKVDAATYHCQQSTEDPWTFGGG TKLEIK |
| CD19 | | |
| SEQ ID NO: 17 | HCDR1 | SNWMH |
| SEQ ID NO: 18 | HCDR2 | EIDPSDSYTNYNQNFQG |
| SEQ ID NO: 19 | HCDR3 | GSNPYYYAMDY |
| SEQ ID NO: 20 | LCDR1 | SASSGVNYMH |
| SEQ ID NO: 21 | LCDR2 | DTSKLAS |
| SEQ ID NO: 22 | LCDR3 | HQRGSYT |

(continued)

| CD19 | | |
|---|---|---|
| SEQ ID NO: 23 | VH | QVQLVQPGAEVVKPGASVKLSCKTSGYTFTSNWMHWVKQAPGQGLEWIGEIDPSDSYTNYNQNFQGKAKLTVDKSTSTAYMEVSSLRSDDTAVYYCARGSNPYYYAMDYWGQGTSVTVSS |
| SEQ ID NO: 24 | VL | EIVLTQSPAIMSASPGERVTMTCSASSGVNYMHWYQQKPGTSPRRWIYDTSKLASGVPARFSGSGSGTDYSLTISSMEPEDAATYYCHQRGSYTFGGGTKLEIK |
| CD19 | | |
| SEQ ID NO: 25 | HCDR1 | TSGMGVG |
| SEQ ID NO: 26 | HCDR2 | HIWWDDDKRYNPALKS |
| SEQ ID NO: 27 | HCDR3 | MELWSYYFDY |
| SEQ ID NO: 28 | LCDR1 | SASSSVSYMH |
| SEQ ID NO: 29 | LCDR2 | DTSKLAS |
| SEQ ID NO: 30 | LCDR3 | FQGSVYPFT |
| SEQ ID NO: 31 | VH | QVQLQESGPGLVKPSQTLSLTCTVSGGSISTSGMGVGWIRQHPGKGLEWIGHIWWDDDKRYNPALKSRVTISVDTSKNQFSLKLSSVTAADTAVYYCARMELWSYYFDYWGQGTLVTVSS |
| SEQ ID NO: 32 | VL | EIVLTQSPATLSLSPGERATLSCSASSSVSYMHWYQQKPGQAPRLLIYDTSKLASGIPARFSGSGSGTDFTLTISSLEPEDVAVYYCFQGSVYPFTFGQGTKLEIK |
| CD19 | | |
| SEQ ID NO: 33 | HCDR1 | SSWMN |
| SEQ ID NO: 34 | HCDR2 | RIYPGDGDTNYNVKFKG |
| SEQ ID NO: 35 | HCDR3 | SGFITTVRDFDY |
| SEQ ID NO: 36 | LCDR1 | RASESVDTFGISFMN |
| SEQ ID NO: 37 | LCDR2 | EASNQGS |
| SEQ ID NO: 38 | LCDR3 | QQSKEVPFT |

(continued)

| CD19 | | |
|---|---|---|
| SEQ ID NO: 39 | VH | EVQLVESGGGLVQPGGSLRLSCAASGFTFSSSWMN WVRQAPGKGLEWVGRIYPGDGDTNYNVKFKGRFTI SRDDSKNSLYLQMNSLKTEDTAVYYCARSGFITTV RDFDYWGQGTLVTVSS |
| SEQ ID NO: 40 | VL | EIVLTQSPDFQSVTPKEKVTITCRASESVDTFGIS FMNWFQQKPDQSPKLLIHEASNQGSGVPSRFSGSG SGTDFTLTINSLEAEDAATYYCQQSKEVPFTFGGG TKVEIK |
| CD20 | | |
| SEQ ID NO: 41 | HCDR1 | SYNMH |
| SEQ ID NO: 42 | HCDR2 | AIYPGNGDTSYNQKFKG |
| SEQ ID NO: 43 | HCDR3 | STYYGGDWYFNV |
| SEQ ID NO: 44 | LCDR1 | RASSSVSYIH |
| SEQ ID NO: 45 | LCDR2 | ATSNLAS |
| SEQ ID NO: 46 | LCDR3 | QQWTSNPPT |
| SEQ ID NO: 47 | VH | QVQLQQPGAELVKPGASVKMSCKASGYTFTSYNMH WVKQTPGRGLEWIGAIYPGNGDTSYNQKFKGKATL TADKSSSTAYMQLSSLTSEDSAVYYCARSTYYGGD WYFNVWGAGTTVTVSA |
| SEQ ID NO: 48 | VL | QIVLSQSPAILSASPGEKVTMTCRASSSVSYIHWF QQKPGSSPKPWIYATSNLASGVPVRFSGSGSGTSY SLTISRVEAEDAATYYCQQWTSNPPTFGGGTKLEI K |
| CD20 | | |
| SEQ ID NO: 49 | HCDR1 | NYYIH |
| SEQ ID NO: 50 | HCDR2 | WIYPGDGNTKYNEKFKG |
| SEQ ID NO: 51 | HCDR3 | DSYSNYYFDY |
| SEQ ID NO: 52 | LCDR1 | RASSSVSYMH |
| SEQ ID NO: 53 | LCDR2 | APSNLAS |

(continued)

| CD20 | | |
|---|---|---|
| SEQ ID NO: 54 | LCDR3 | QQWSFNPPT |
| SEQ ID NO: 55 | VH | EVQLVQSGAEVKKPGASVKVSCKASGYTFTNYYIH WVRQAPGQGLEWIGWIYPGDGNTKYNEKFKGRATL TADTSTSTAYLELSSLRSEDTAVYYCARDSYSNYY FDYWGQGTLVTVSS |
| SEQ ID NO: 56 | VL | DIQMTQSPSSLSASVGDRVTITCRASSSVSYMHWY QQKPGKAPKPLIYAPSNLASGVPSRFSGSGSGTDF TLTISSLQPEDFATYYCQQWSFNPPTFGQGTKVEI |
| | | K |
| CD20 | | |
| SEQ ID NO: 57 | HCDR1 | YSWIN |
| SEQ ID NO: 58 | HCDR2 | RIFPGDGDTDYNGKFKG |
| SEQ ID NO: 59 | HCDR3 | NVFDGYWLVY |
| SEQ ID NO: 60 | LCDR1 | RSSKSLLHSNGITYLY |
| SEQ ID NO: 61 | LCDR2 | QMSNLVS |
| SEQ ID NO: 62 | LCDR3 | AQNLELPYT |
| SEQ ID NO: 63 | VH | QVQLVQSGAEVKKPGSSVKVSCKASGYAFSYSWIN WVRQAPGQGLEWMGRIFPGDGDTDYNGKFKGRVTI TADKSTSTAYMELSSLRSEDTAVYYCARNVFDGYW LVYWGQGTLVTVSS |
| SEQ ID NO: 64 | VL | DIVMTQTPLSLPVTPGEPASISCRSSKSLLHSNGI TYLYWYLQKPGQSPQLLIYQMSNLVSGVPDRFSGS GSGTDFTLKISRVEAEDVGVYYCAQNLELPYTFGG GTKVEIK |
| CD20 | | |
| SEQ ID NO: 65 | HCDR1 | DYAMH |
| SEQ ID NO: 66 | HCDR2 | TISWNSGSIGYADSVKG |
| SEQ ID NO: 67 | HCDR3 | DIQYGNYYYGMDV |
| SEQ ID NO: 68 | LCDR1 | RASQSVSSYLA |

(continued)

| CD20 | | |
|---|---|---|
| SEQ ID NO: 69 | LCDR2 | DASNRAT |
| SEQ ID NO: 70 | LCDR3 | QQRSNWPIT |
| SEQ ID NO: 71 | VH | EVQLVESGGGLVQPGRSLRLSCAASGFTFNDYAMHWVRQAPGKGLEWVSTISWNSGSIGYADSVKGRFTISRDNAKKSLYLQMNSLRAEDTALYYCAKDIQYGNYYYGMDVWGQGTTVTVSS |
| SEQ ID NO: 72 | VL | EIVLTQSPATLSLSPGERATLSCRASQSVSSYLAWYQQKPGQAPRLLIYDASNRATGIPARFSGSGSGTDFTLTISSLEPEDFAVYYCQQRSNWPITFGQGTRLEIK |
| CD22 | | |
| SEQ ID NO: 73 | HCDR1 | RSWMN |
| SEQ ID NO: 74 | HCDR2 | RIYPGDGDTNYSGKFKG |
| SEQ ID NO: 75 | HCDR3 | DGSSWDWYFDV |
| SEQ ID NO: 76 | LCDR1 | RSSQSIVHSVGNTFLE |
| SEQ ID NO: 77 | LCDR2 | KVSNRFS |
| SEQ ID NO: 78 | LCDR3 | FQGSQFPYT |
| SEQ ID NO: 79 | VH | EVQLVESGGGLVQPGGSLRLSCAASGYEFSRSWMNWVRQAPGKGLEWVGRIYPGDGDTNYSGKFKGRFTISADTSKNTAYLQMNSLRAEDTAVYYCARDGSSWDWYFDVWGQGTLVTVSS |
| SEQ ID NO: 80 | VL | DIQMTQSPSSLSASVGDRVTITCRSSQSIVHSVGNTFLEWYQQKPGKAPKLLIYKVSNRFSGVPSRFSGSGSGTDFTLTISSLQPEDFATYYCFQGSQFPYTFGQGTKVEIK |
| CD22 | | |
| SEQ ID NO: 81 | HCDR1 | IYDMS |
| SEQ ID NO: 82 | HCDR2 | YISSGGGTTYYPDTVKG |
| SEQ ID NO: 83 | HCDR3 | HSGYGTHWGVLFAY |

(continued)

| CD22 | | |
|---|---|---|
| SEQ ID NO: 84 | LCDR1 | RASQDISNYLN |
| SEQ ID NO: 85 | LCDR2 | YTSILHS |
| SEQ ID NO: 86 | LCDR3 | QQGNTLPWT |
| SEQ ID NO: 87 | VH | EVQLVESASTGGGLVKPGGSLKLSCAASGFAFSIY DMSWVRQTPEKCLEWVAYISSGGGTTYYPDTVKGR FTISRDNAKNTLYLQMSSLKSEDTAMYYCARHSGY GTHWGVLFAYWGQGTLVT |
| SEQ ID NO: 88 | VL | DIQMTQTTSSLSASLGDRVTISCRASQDISNYLNW YQQKPDGTVKLLIYYTSILHSGVPSRFSGSGSGTD YSLTISNLEQEDFATYFCQQGNTLPWTFGCGTKLE IK |
| CD30 | | |
| SEQ ID NO: 89 | HCDR1 | DYYIT |
| SEQ ID NO: 90 | HCDR2 | WIYPGSGNTKYNEKFKG |
| SEQ ID NO: 91 | HCDR3 | YGNYWFAY |
| SEQ ID NO: 92 | LCDR1 | KASQSVDFDGDSYMN |
| SEQ ID NO: 93 | LCDR2 | AASNLES |
| SEQ ID NO: 94 | LCDR3 | QQSNEDPWT |
| SEQ ID NO: 95 | VH | QIQLQQSGPEVVKPGASVKISCKASGYTFTDYYIT WVKQKPGQGLEWIGWIYPGSGNTKYNEKFKGKATL TVDTSSSTAFMQLSSLTSEDTAVYFCANYGNYWFA YWGQGTQVTVSA |
| SEQ ID NO: 96 | VL | DIVLTQSPASLAVSLGQRATISCKASQSVDFDGDS YMNWYQQKPGQPPKVLIYAASNLESGIPARFSGSG SGTDFTLNIHPVEEEDAATYYCQQSNEDPWTFGGG TKLEIK |
| SEQ ID NO: 97 | HCDR1 | AYYWS |
| SEQ ID NO: 98 | HCDR2 | DINHGGGTNYNPSLKS |
| SEQ ID NO: 99 | HCDR3 | LTAY |

(continued)

| CD30 | | |
|---|---|---|
| SEQ ID NO: 100 | LCDR1 | RASQGISSWLT |
| SEQ ID NO: 101 | LCDR2 | AASSLQS |
| SEQ ID NO: 102 | LCDR3 | QQYDSYPIT |
| SEQ ID NO: 103 | VH | QVQLQQWGAGLLKPSETLSLTCAVYGGSFSAYYWS WIRQPPGKGLEWIGDINHGGGTNYNPSLKSRVTIS VDTSKNQFSLKLNSVTAADTAVYYCASLTAYWGQG SLVTVSS |
| SEQ ID NO: 104 | VL | DIQMTQSPTSLSASVGDRVTITCRASQGISSWLTW YQQKPEKAPKSLIYAASSLQSGVPSRFSGSGSGTD FTLTISSLQPEDFATYYCQQYDSYPITFGQGTRLE IK |
| EpCAM | | |
| SEQ ID NO: 105 | HCDR1 | SYGMH |
| SEQ ID NO: 106 | HCDR2 | VISYDGSNKYYADSVKG |
| SEQ ID NO: 107 | HCDR3 | DMGWGSGWRPYYYYGMDV |
| SEQ ID NO: 108 | LCDR1 | RTSQSISSYLN |
| SEQ ID NO: 109 | LCDR2 | WASTRES |
| SEQ ID NO: 110 | LCDR3 | QQSYDIPYT |
| SEQ ID NO: 111 | VH | EVQLLESGGGVVQPGRSLRLSCAASGFTFSSYGMH WVRQAPGKGLEWVAVISYDGSNKYYADSVKGRFTI |
| | | SRDNSKNTLYLQMNSLRAEDTAVYYCAKDMGWGSG WRPYYYYGMDVWGQGTTVTVSS |
| SEQ ID NO: 112 | VL | ELQMTQSPSSLSASVGDRVTITCRTSQSISSYLNW YQQKPGQPPKLLIYWASTRESGVPDRFSGSGSGTD FTLTISSLQPEDSATYYCQQSYDIPYTFGQGTKLE IK |
| EpCAM | | |
| SEQ ID NO: 113 | HCDR1 | NYGMN |
| SEQ ID NO: 114 | HCDR2 | WINTYTGESTYADSFKG |

(continued)

| EpCAM | | |
|---|---|---|
| SEQ ID NO: 115 | HCDR3 | FAIKGDY |
| SEQ ID NO: 116 | LCDR1 | RSTKSLLHSNGITYLY |
| SEQ ID NO: 117 | LCDR2 | QMSNLAS |
| SEQ ID NO: 118 | LCDR3 | AQNLEIPRT |
| SEQ ID NO: 119 | VH | EVQLVQSGPGLVQPGGSVRISCAASGYTFTNYGMN WVKQAPGKGLEWMGWINTYTGESTYADSFKGRFTF SLDTSASAAYLQINSLRAEDTAVYYCARFAIKGDY WGQGTLLTVSS |
| SEQ ID NO: 120 | VL | DIQMTQSPSSLSASVGDRVTITCRSTKSLLHSNGI TYLYWYQQKPGKAPKLLIYQMSNLASGVPSRFSSS GSGTDFTLTISSLQPEDFATYYCAQNLEIPRTFGQ GTKVELK |
| CEA | | |
| SEQ ID NO: 121 | HCDR1 | DTYMH |
| SEQ ID NO: 122 | HCDR2 | RIDPANGNSKYADSVKG |
| SEQ ID NO: 123 | HCDR3 | FGYYVSDYAMAY |
| SEQ ID NO: 124 | LCDR1 | RAGESVDIFGVGFLH |
| SEQ ID NO: 125 | LCDR2 | RASNLES |
| SEQ ID NO: 126 | LCDR3 | QQTNEDPYT |
| SEQ ID NO: 127 | VH | EVQLVESGGGLVQPGGSLRLSCAASGFNIKDTYMH WVRQAPGKGLEWVARIDPANGNSKYADSVKGRFTI SADTSKNTAYLQMNSLRAEDTAVYYCAPFGYYVSD YAMAYWGQGTLVTVSS |
| SEQ ID NO: 128 | VL | DIQLTQSPSSLSASVGDRVTITCRAGESVDIFGVG FLHWYQQKPGKAPKLLIYRASNLESGVPSRFSGSG SRTDFTLTISSLQPEDFATYYCQQTNEDPYTFGQG TKVEIK |
| CEA | | |
| SEQ ID NO: 129 | HCDR1 | TYWMS |

(continued)

| CEA | | |
|---|---|---|
| SEQ ID NO: 130 | HCDR2 | EIHPDSSTINYAPSLKD |
| SEQ ID NO: 131 | HCDR3 | LYFGFPWFAY |
| SEQ ID NO: 132 | LCDR1 | KASQDVGTSVA |
| SEQ ID NO: 133 | LCDR2 | WTSTRHT |
| SEQ ID NO: 134 | LCDR3 | QQYSLYRS |
| SEQ ID NO: 135 | VH | EVQLVESGGGVVQPGRSLRLSCSASGFDFTTYWMS WVRQAPGKGLEWIGEIHPDSSTINYAPSLKDRFTI SRDNAKNTLFLQMDSLRPEDTGVYFCASLYFGFPW FAYWGQGTPVTVSS |
| SEQ ID NO: 136 | VL | DIQLTQSPSSLSASVGDRVTITCKASQDVGTSVAW YQQKPGKAPKLLIYWTSTRHTGVPSRFSGSGSGTD FTFTISSLQPEDIATYYCQQYSLYRSFGQGTKVEI K |
| CEA | | |
| SEQ ID NO: 137 | HCDR1 | EFGMN |
| SEQ ID NO: 138 | HCDR2 | WINTKTGEATYVEEFKG |
| SEQ ID NO: 139 | HCDR3 | WDFAYYVEAMDY |
| SEQ ID NO: 140 | LCDR1 | KASAAVGTYVA |
| SEQ ID NO: 141 | LCDR2 | SASYRKR |
| SEQ ID NO: 142 | LCDR3 | HQYYTYPLFT |
| SEQ ID NO: 143 | VH | QVQLVQSGAEVKKPGASVKVSCKASGYTFTEFGMN WVRQAPGQGLEWMGWINTKTGEATYVEEFKGRVTF TTDTSTSTAYMELRSLRSDDTAVYYCARWDFAYYV EAMDYWGQGTTVTVSS |
| SEQ ID NO: 144 | VL | DIQMTQSPSSLSASVGDRVTITCKASAAVGTYVAW YQQKPGKAPKLLIYSASYRKRGVPSRFSGSGSGTD FTLTISSLQPEDFATYYCHQYYTYPLFTFGQGTKL EIK |

(continued)

| Her2 | | |
|---|---|---|
| SEQ ID NO: 145 | HCDR1 | DTYIH |
| SEQ ID NO: 146 | HCDR2 | RIYPTNGYTRYADSVKG |
| SEQ ID NO: 147 | HCDR3 | WGGDGFYAMDY |
| SEQ ID NO: 148 | LCDR1 | RASQDVNTAVA |
| SEQ ID NO: 149 | LCDR2 | SASFLYS |
| SEQ ID NO: 150 | LCDR3 | QQHYTTPPT |
| SEQ ID NO: 151 | VH | EVQLVESGGGLVQPGGSLRLSCAASGFNIKDTYIH WVRQAPGKGLEWVARIYPTNGYTRYADSVKGRFTI SADTSKNTAYLQMNSLRAEDTAVYYCSRWGGDGFY AMDYWGQGTLVTVSS |
| SEQ ID NO: 152 | VL | DIQMTQSPSSLSASVGDRVTITCRASQDVNTAVAW YQQKPGKAPKLLIYSASFLYSGVPSRFSGSRSGTD FTLTISSLQPEDFATYYCQQHYTTPPTFGQGTKVE IK |
| Her2 | | |
| SEQ ID NO: 153 | HCDR1 | DYTMD |
| SEQ ID NO: 154 | HCDR2 | DVNPNSGGSIYNQRFKG |
| SEQ ID NO: 155 | HCDR3 | NLGPSFYFDY |
| SEQ ID NO: 156 | LCDR1 | KASQDVSIGVA |
| SEQ ID NO: 157 | LCDR2 | SASYRYT |
| SEQ ID NO: 158 | LCDR3 | QQYYIYPYT |
| SEQ ID NO: 159 | VH | EVQLVESGGGLVQPGGSLRLSCAASGFTFTDYTMD WVRQAPGKGLEWVADVNPNSGGSIYNQRFKGRFTL SVDRSKNTLYLQMNSLRAEDTAVYYCARNLGPSFY FDYWGQGTLVTVSS |
| SEQ ID NO: 160 | VL | DIQMTQSPSSLSASVGDRVTITCKASQDVSIGVAW YQQKPGKAPKLLIYSASYRYTGVPSRFSGSGSGTD FTLTISSLQPEDFATYYCQQYYIYPYTFGQGTKVE IK |

(continued)

| Her2 | | |
|---|---|---|
| SEQ ID NO: 161 | HCDR1 | DTYIH |
| SEQ ID NO: 162 | HCDR2 | RIYPTNGYTRYDPKFQD |
| SEQ ID NO: 163 | HCDR3 | WGGDGFYAMDY |
| SEQ ID NO: 164 | LCDR1 | KASQDVNTAVA |
| SEQ ID NO: | LCDR2 | SASFRYT |
| 165 | | |
| SEQ ID NO: 166 | LCDR3 | QQHYTTPPT |
| SEQ ID NO: 167 | VH | QVQLQQSGPELVKPGASLKLSCTASGFNIKDTYIH WVKQRPEQGLEWIGRIYPTNGYTRYDPKFQDKATI TADTSSNTAYLQVSRLTSEDTAVYYCSRWGGDGFY AMDYWGQGASVTVSS |
| SEQ ID NO: 168 | VL | DIVMTQSHKFMSTSVGDRVSITCKASQDVNTAVAW YQQKPGHSPKLLIYSASFRYTGVPDRFTGSRSGTD FTFTISSVQAEDLAVYYCQQHYTTPPTFGGGTKVE IK |
| EGFR | | |
| SEQ ID NO: 169 | HCDR1 | NYGVH |
| SEQ ID NO: 170 | HCDR2 | VIWSGGNTDYNTPFTS |
| SEQ ID NO: 171 | HCDR3 | ALTYYDYEFAY |
| SEQ ID NO: 172 | LCDR1 | RASQSIGTNIH |
| SEQ ID NO: 173 | LCDR2 | YASESIS |
| SEQ ID NO: 174 | LCDR3 | QQNNNWPTT |
| SEQ ID NO: 175 | VH | QVQLKQSGPGLVQPSQSLSITCTVSGFSLTNYGVH WVRQSPGKGLEWLGVIWSGGNTDYNTPFTSRLSIN KDNSKSQVFFKMNSLQSNDTAIYYCARALTYYDYE FAYWGQGTLVTVSA |
| SEQ ID NO: 176 | VL | DILLTQSPVILSVSPGERVSFSCRASQSIGTNIHW YQQRTNGSPRLLIKYASESISGIPSRFSGSGSGTD FTLSINSVESEDIADYYCQQNNNWPTTFGAGTKLE LK |

(continued)

| EGFR | | |
|---|---|---|
| SEQ ID NO: 177 | HCDR1 | SGDYYWS |
| SEQ ID NO: | HCDR2 | YIYYSGSTDYNPSLKS |
| 178 | | |
| SEQ ID NO: 179 | HCDR3 | VSIFGVGTFDY |
| SEQ ID NO: 180 | LCDR1 | RASQSVSSYLA |
| SEQ ID NO: 181 | LCDR2 | DASNRAT |
| SEQ ID NO: 182 | LCDR3 | HQYGSTPLT |
| SEQ ID NO: 183 | VH | QVQLQESGPGLVKPSQTLSLTCTVSGGSISSGDYY WSWIRQPPGKGLEWIGYIYYSGSTDYNPSLKSRVT MSVDTSKNQFSLKVNSVTAADTAVYYCARVSIFGV GTFDYWGQGTLVTVSS |
| SEQ ID NO: 184 | VL | EIVMTQSPATLSLSPGERATLSCRASQSVSSYLAW YQQKPGQAPRLLIYDASNRATGIPARFSGSGSGTD FTLTISSLEPEDFAVYYCHQYGSTPLTFGGGTKAE IK |
| EGFR | | |
| SEQ ID NO: 185 | HCDR1 | NYYIY |
| SEQ ID NO: 186 | HCDR2 | GINPTSGGSNFNEKFKT |
| SEQ ID NO: 187 | HCDR3 | QGLWFDSDGRGFDF |
| SEQ ID NO: 188 | LCDR1 | RSSQNIVHSNGNTYLD |
| SEQ ID NO: 189 | LCDR2 | KVSNRFS |
| SEQ ID NO: 190 | LCDR3 | FQYSHVPWT |
| SEQ ID NO: 191 | VH | QVQLQQSGAEVKKPGSSVKVSCKASGYTFTNYYIY WVRQAPGQGLEWIGGINPTSGGSNFNEKFKTRVTI TVDESTNTAYMELSSLRSEDTAFYFCARQGLWFDS DGRGFDFWGQGSTVTVSS |
| SEQ ID NO: 192 | VL | DIQMTQSPSSLSASVGDRVTITCRSSQNIVHSNGN TYLDWYQQTPGKAPKLLIYKVSNRFSGVPSRFSGS |
| | | GSGTDFTFTISSLQPEDIATYYCFQYSHVPWTFGQ GTKLQIT |

(continued)

| GPC-3 | | |
|---|---|---|
| SEQ ID NO: 193 | HCDR1 | DYEMH |
| SEQ ID NO: 194 | HCDR2 | AIDPQTGNTAFNQKFKG |
| SEQ ID NO: 195 | HCDR3 | FYSLTY |
| SEQ ID NO: 196 | LCDR1 | RSSQSIVHSNGNTYLQ |
| SEQ ID NO: 197 | LCDR2 | KVSNRFS |
| SEQ ID NO: 198 | LCDR3 | FQGSHFPYA |
| SEQ ID NO: 199 | VH | QVQLVQSGAEVKKPGASVKVSCKASGYTFTDYEMH WVKQAPGQGLEWIGAIDPQTGNTAFNQKFKGRVTL TRDKSSSTVYMELSSLRSEDTAVYYCTRFYSLTYW GQGTLVTVSS |
| SEQ ID NO: 200 | VL | DVLMTQSPLSLPVTLGQPASISCRSSQSIVHSNGN TYLQWYLQRPGQSPKLLIYKVSNRFSGVPDRFSGS GSGTYFTLKISRVEAEDVGVYYCFQGSHFPYAFGG GTKVEIK |
| Mesothelin | | |
| SEQ ID NO: 201 | HCDR1 | IYGMH |
| SEQ ID NO: 202 | HCDR2 | VIWYDGSHEYYADSVKG |
| SEQ ID NO: 203 | HCDR3 | DGDYYDSGSPLDY |
| SEQ ID NO: 204 | LCDR1 | RASQSVSSYLA |
| SEQ ID NO: 205 | LCDR2 | DASNRAT |
| SEQ ID NO: | LCDR3 | QQRSNWPLT |
| 206 | | |
| SEQ ID NO: 207 | VH | QVYLVESGGGVVQPGRSLRLSCAASGITFSIYGMH WVRQAPGKGLEWVAVIWYDGSHEYYADSVKGRFTI SRDNSKNTLYLLMNSLRAEDTAVYYCARDGDYYDS GSPLDYWGQGTLVTVSS |
| SEQ ID NO: 208 | VL | EIVLTQSPATLSLSPGERATLSCRASQSVSSYLAW YQQKPGQAPRLLIYDASNRATGIPARFSGSGSGTD FTLTISSLEPEDFAVYYCQQRSNWPLTFGGGTKVE IK |

(continued)

| Mucin1 | | |
|---|---|---|
| SEQ ID NO: 209 | HCDR1 | SYVLH |
| SEQ ID NO: 210 | HCDR2 | YINPYNDGTQYNEKFKG |
| SEQ ID NO: 211 | HCDR3 | GFGGSYGFAY |
| SEQ ID NO: 212 | LCDR1 | SASSSVSSSYLY |
| SEQ ID NO: 213 | LCDR2 | STSNLAS |
| SEQ ID NO: 214 | LCDR3 | HQWNRYPYT |
| SEQ ID NO: 215 | VH | QVQLQQSGAEVKKPGASVKVSCEASGYTFPSYVLH WVKQAPGQGLEWIGYINPYNDGTQYNEKFKGKATL TRDTSINTAYMELSRLRSDDTAVYYCARGFGGSYG FAYWGQGTLVTVSS |
| SEQ ID NO: 216 | VL | DIQLTQSPSSLSASVGDRVTMTCSASSSVSSSYLY WYQQKPGKAPKLWIYSTSNLASGVPARFSGSGSGT DFTLTISSLQPEDSASYFCHQWNRYPYTFGGGTRL EIK |
| Mucin1 | | |
| SEQ ID NO: 217 | HCDR1 | NYWMN |
| SEQ ID NO: 218 | HCDR2 | EIRLKSNNYTTHYAESVKG |
| SEQ ID NO: | HCDR3 | HYYFDY |
| 219 | | |
| SEQ ID NO: 220 | LCDR1 | RSSKSLLHSNGITYFF |
| SEQ ID NO: 221 | LCDR2 | QMSNLAS |
| SEQ ID NO: 222 | LCDR3 | AQNLELPPT |
| SEQ ID NO: 223 | VH | EVQLVESGGGLVQPGGSMRLSCVASGFPFSNYWMN WVRQAPGKGLEWVGEIRLKSNNYTTHYAESVKGRF TISRDDSKNSLYLQMNSLKTEDTAVYYCTRHYYFD YWGQGTLVTVSS |
| SEQ ID NO: 224 | VL | DIVMTQSPLSNPVTPGEPASISCRSSKSLLHSNGI TYFFWYLQKPGQSPQLLIYQMSNLASGVPDRFSGS GSGTDFTLRISRVEAEDVGVYYCAQNLELPPTFGQ GTKVEIK |

(continued)

| CA125 | | |
|---|---|---|
| SEQ ID NO: 225 | HCDR1 | SYAMS |
| SEQ ID NO: 226 | HCDR2 | TISSAGGYIFYSDSVQG |
| SEQ ID NO: 227 | HCDR3 | QGFGNYGDYYAMDY |
| SEQ ID NO: 228 | LCDR1 | KSSQSLLNSRTRKNQLA |
| SEQ ID NO: 229 | LCDR2 | WASTRQS |
| SEQ ID NO: 230 | LCDR3 | QQSYNLLT |
| SEQ ID NO: 231 | VH | VKLQESGGGFVKPGGSLKVSCAASGFTFSSYAMSW VRLSPEMRLEWVATISSAGGYIFYSDSVQGRFTIS RDNAKNTLHLQMGSLRSGDTAMYYCARQGFGNYGD YYAMDYWGQGTTVTVSS |
| SEQ ID NO: 232 | VL | DIELTQSPSSLAVSAGEKVTMSCKSSQSLLNSRTR KNQLAWYQQKPGQSPELLIYWASTRQSGVPDRFTG SGSGTDFTLTISSVQAEDLAVYYCQQSYNLLTFGP GTKLEVK |
| BCMA | | |
| SEQ ID NO: 233 | HCDR1 | NYWMH |
| SEQ ID NO: 234 | HCDR2 | ATYRGHSDTYYNQKFKG |
| SEQ ID NO: 235 | HCDR3 | GAIYDGYDVLDN |
| SEQ ID NO: 236 | LCDR1 | SASQDISNYLN |
| SEQ ID NO: 237 | LCDR2 | YTSNLHS |
| SEQ ID NO: 238 | LCDR3 | QQYRKLPWT |
| SEQ ID NO: 239 | VH | QVQLVQSGAEVKKPGSSVKVSCKASGGTFSNYWMH WVRQAPGQGLEWMGATYRGHSDTYYNQKFKGRVTI TADKSTSTAYMELSSLRSEDTAVYYCARGAIYDGY DVLDNWGQGTLVTVSS |
| SEQ ID NO: 240 | VL | DIQMTQSPSSLSASVGDRVTITCSASQDISNYLNW YQQKPGKAPKLLIYYTSNLHSGVPSRFSGSGSGTD FTLTISSLQPEDFATYYCQQYRKLPWTFGQGTKLE IK |

The anti-CD3 scFv binds to an effector cell at an $EC_{50}$ value greater than about 50 nM, or greater than 100 nM, or greater than 300 nM, or greater than 500 nM in an *in vitro* FACS binding affinity assay; more preferably, the second single-chain Fv of the bispecific antibody not only binds to human CD3 but also specifically binds to CD3 of cynomolgus monkeys or rhesus monkeys.

Some preferred amino acid sequences of the VH domain and its complementary determining regions (HCDR1, HCDR2, and HCDR3) and amino acid sequences of the VL domain and its complementary determining regions (LCDR1, LCDR2 and LCDR3) of the anti-CD3 scFv are exemplified in Table 6-2, wherein the amino acid residues contained in the CDRs are defined according to the Kabat rule, wherein the amino acid composition of the linker peptide between VH and VL of the anti-CD3 scFv is (GGGGS)n, wherein n = 1, 2, 3, 4 or 5.

Table 6-2 Amino acid sequences of the anti-CD3 scFv included in the bispecific antibody and amino acid sequences of its CDR regions

| CD3-3 | | |
|---|---|---|
| SEQ ID NO: 241 | HCDR1 | TYAMN |
| SEQ ID NO: 242 | HCDR2 | RIRSKYNNYATYYADSVKD |
| SEQ ID NO: 243 | HCDR3 | HGNFGNSYVSWFAY |
| SEQ ID NO: 244 | LCDR1 | RSSTGAVTTSNYAN |
| SEQ ID NO: 245 | LCDR2 | GTNKRAP |
| SEQ ID NO: 246 | LCDR3 | ALWYSNLWV |
| SEQ ID NO: 247 | VH | EVQLLESGGGLVQPGGSLKLSCAASGFTFNTYAMN WVRQAPGKGLEWVARIRSKYNNYATYYADSVKDRF TISRDDSKNTAYLQMNNLKTEDTAVYYCVRHGNFG NSYVSWFAYWGQGTLVTVSS |
| SEQ ID NO: 248 | VL | ELVVTQEPSLTVSPGGTVTLTCRSSTGAVTTSNYA NWVQQKPGQAPRGLIGGTNKRAPGTPARFSGSLLG GKAALTLSGVQPEDEAEYYCALWYSNLWVFGGGTK LTVL |
| CD3-4 | | |
| SEQ ID NO: 249 | HCDR1 | KYAMN |
| SEQ ID NO: 250 | HCDR2 | RIRSKYNNYATYYADSVKD |
| SEQ ID NO: 251 | HCDR3 | HGNFGNSYISYWAY |
| SEQ ID NO: 252 | LCDR1 | GSSTGAVTSGYYPN |
| SEQ ID NO: 253 | LCDR2 | GTKFLAP |
| SEQ ID NO: 254 | LCDR3 | ALWYSNRWV |

(continued)

| CD3-4 | | |
|---|---|---|
| SEQ ID NO: 255 | VH | EVQLLESGGGLVQPGGSLKLSCAASGFTFNKYAMN WVRQAPGKGLEWVARIRSKYNNYATYYADSVKDRF |
| | | TISRDDSKNTAYLQMNNLKTEDTAVYYCVRHGNFG NSYISYWAYWGQGTLVTVSS |
| SEQ ID NO: 256 | VL | ELVVTQEPSLTVSPGGTVTLTCGSSTGAVTSGYYP NWVQQKPGQAPRGLIGGTKFLAPGTPARFSGSLLG GKAALTLSGVQPEDEAEYYCALWYSNRWVFGGGTK LTVL |

The linker peptide that connects the anti-TAA scFv to the anti-CD3 scFv consists of a flexible peptide and a rigid peptide; preferably, the amino acid composition of the flexible peptide has a general formula $G_xS_y(GGGGS)_z$, wherein x, y, and z are integers greater than or equal to 0, and x + y + z ≥ 1. The rigid peptide is derived from a full-length sequence (as shown in SEQ ID NO: 257) consisting of amino acids at positions 118 to 145 of the carboxy terminus of the natural human chorionic gonadotropin beta subunit, or a truncated fragment thereof; preferably, the composition of the CTP rigid peptide is SSSSKAPPPS ($CTP^1$). Some preferred amino acid sequences of the linker peptide that connects the anti-TAA scFv and the anti-CD3 scFv are exemplified in Table 6-3.

Table 6-3 Amino acid sequences of the linker peptide that connects the anti-TAA scFv and the anti-CD3 scFv

| | | |
|---|---|---|
| SEQ ID NO: 258 | $G_2$ $(GGGGS)_3CTP^1$ | GGGGGGSGGGGSGGGGSSSSSKAPPPS |
| SEQ ID NO: 259 | $(GGGGS)_3CTP^1$ | GGGGSGGGGSGGGGSSSSSKAPPPS |
| SEQ ID NO: 260 | $GS(GGGGS)_2CTP^1$ | GSGGGGSGGGGSSSSSKAPPPS |
| SEQ ID NO: 261 | $(GGGGS)_1CTP^4$ | GGGGSSSSSKAPPPSLPSPSRLPGPSDTPILPQ |

The Fc fragment is directly connected or connected by a linker peptide to the anti-CD3 scFv, wherein the linker peptide includes 1 to 20 amino acids that are preferably selected from the following amino acids: Gly(G), Ser(S), Ala(A), and Thr(T), more preferably Gly(G) and Ser(S); and most preferably, the linker peptide consists of (GGGGS)n, wherein n = 1, 2, 3 or 4.

The Fc fragment is preferably selected from heavy chain constant regions of human IgG1, IgG2, IgG3 and IgG4 and more particularly selected from heavy chain constant regions of human IgG1 or IgG4; and Fc is mutated to modify the properties of the bispecific antibody molecule, e.g., reduced affinity to at least one of human FcγRs (FcγRI, FcγRIIa or FcγRIIIa) and C1q, a reduced effector cell function, or a reduced complement function. In addition, the Fc fragment may also contain amino acid substitutions that change one or more other characteristics (e.g., an ability of binding to an FcRn receptor, the glycosylation of the antibody or the charge heterogeneity of the antibody).

Some amino acid sequences of the Fc fragment with one or more amino acid mutations are exemplified in Table 6-4.

Table 6-4 Amino acid sequences of Fc from human IgG

| **Amino acid sequence of a constant region of an IgG1 Fc (L234A/L235A) mutant (EU numbering)** | |
|---|---|
| SEQ ID NO: 262 | DKTHTCPPCP APEAAGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPGK |

(continued)

| Amino acid sequence of a constant region of an IgG1 (L234A/L235A/T250Q/N297A/P331S/M428L/K447-) mutant (EU numbering) | |
|---|---|
| SEQ ID NO: 263 | DKTHTCPPCP APEAAGGPSV FLFPPKPKDQ LMISRTPEVT CVVVDVSHED PEVKFNWYVD GVEVHNAKTK PREEQYASTY RVVSVLTVLH QDWLNGKEYK CKVSNKALPA SIEKTISKAK GQPREPQVYT LPPSRDELTK NQVSLTCLVK GFYPSDIAVE WESNGQPENN YKTTPPVLDS DGSFFLYSKL TVDKSRWQQG NVFSCSVLHE ALHNHYTQKS LSLSPG~~K~~ |

Amino acid sequences of some preferred bispecific antibodies and corresponding nucleotide sequences thereof are exemplified in Table 6-5.

Table 6-5 several bispecific antibodies of scFv1-scFv2-Fc configuration

| Antibody code | Target site | Amino acid sequence No. | Nucleotide sequence No. |
|---|---|---|---|
| AB1K1 | Anti-CD19×CD3 | SEQ ID NO: 264 | SEQ ID NO: 265 |
| AB1K2 | Anti-CD19×CD3 | SEQ ID NO: 283 | SEQ ID NO: 284 |
| AB2K | Anti-CD20×CD3 | SEQ ID NO: 266 | SEQ ID NO: 267 |
| AB3K | Anti-CD22×CD3 | SEQ ID NO: 268 | SEQ ID NO: 269 |
| AB4K | Anti-CD30×CD3 | SEQ ID NO: 270 | SEQ ID NO: 271 |
| AB5K | Anti-EpCAM×CD3 | SEQ ID NO: 272 | SEQ ID NO: 273 |
| AB6K | Anti-CEA×CD3 | SEQ ID NO: 274 | SEQ ID NO: 275 |
| AB7K7 | Anti-Her2×CD3 | SEQ ID NO: 8 | SEQ ID NO: 276 |
| AB8K | Anti-EGFR×CD3 | SEQ ID NO: 277 | SEQ ID NO: 278 |
| AB9K | Anti-GPC-3×CD3 | SEQ ID NO: 279 | SEQ ID NO: 280 |
| AB10K | Anti-Mesothelin×CD3 | SEQ ID NO: 281 | SEQ ID NO: 282 |
| AB11k | Anti-Mucin1×CD3 | SEQ ID NO: 285 | SEQ ID NO: 286 |

Example 7 Pharmacodynamics study of Anti-GPC-3xCD3 bispecific antibodies in a mouse transplanted tumor model

7.1 NOD-SCID mouse model of transplanted tumor constructed by subcutaneously co-inoculating human PBMCs and human liver cancer cells Huh-7

GPC-3-positive human liver cancer cells Huh-7 were selected to study the inhibiting effect of bispecific antibodies on tumor growth *in vivo* in a NOD-SCID mouse model of transplanted tumor constructed by subcutaneously co-inoculating human PBMC cells and Huh-7 cells.

The peripheral blood of a normal human was subjected to density gradient centrifugation to separate human PBMCs. Female NOD-SCID mice (purchased from Shanghai Lingchang Biotechnology Co., Ltd.) at the age of seven to eight weeks were selected and Huh-7 cells in the logarithmic growth stage were collected. $3 \times 10^6$ Huh-7 cells and $3 \times 10^6$ PBMCs were mixed and inoculated subcutaneously on the right back of each NOD-SCID mouse. One hour after inoculation, the mice were randomly divided into two groups with six mice in each group according to their weights. The treated group was intraperitoneally administered with AB9K at a dose of 1 mg/kg, and the control group was administered with a PBS solution of the same volume, once a day continuously for 6 days. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 6-1, on Day 21 of administration, the average tumor volume of the PBS control group was 1311.03 ± 144.89 $mm^3$; the average tumor volume of the treated group administrated with AB9K was 60.83 ± 12.63 $mm^3$, and the TGI was 95.36%, wherein the tumor in one mouse was completely regressed, which was significantly different from that of the control group (P < *0.001*). The above results show that most of PBMCs are inactivated primary T cells, the bispecific antibody AB9K can activate the primary T cells in the animals and draw the distance between the T cells and the target cell Huh-7 so that the T cells can directly kill the tumor cells and inhibit the growth of the tumor, and thus AB9K has a very good anti-tumor effect at a dose of 1 mg/kg.

7.2 NPG mouse model of transplanted tumor constructed by subcutaneously co-inoculating human CIK cells and human Burkkit's lymphoma Raji cells

GPC-3-negative human Burkkit's lymphoma Raji cells were selected to study the inhibiting effect of bispecific antibodies on tumor growth *in vivo* in an NPG mouse model of transplanted tumor constructed by subcutaneously co-inoculating human CIK cells and human Burkkit's lymphoma Raji cells.

CIK cells were prepared in the method as described in Example 3.1. Female NPG mice at the age of seven to eight weeks were selected, and Raji cells in the logarithmic growth stage were collected. 5 × $10^6$ Raji cells and 2 × $10^6$ CIK cells were mixed and inoculated subcutaneously on the right back of each NPG mouse. One hour after inoculation, the mice were randomly divided into three groups with five mice in each group according to their weights. The treated group was intraperitoneally administered with AB9K at a dose of 1 mg/kg, and the control group was administered with a PBS solution of the same volume, once a day continuously for 10 days. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 6-2, on Day 26 of administration, the average tumor volume of the PBS control group was 2636.66 ± 196.62 $mm^3$; the average tumor volume of the treated group administrated with AB9K was 2739.57 ± 220.13 $mm^3$, which was not significantly different from that of the control group. In summary, the results show that bispecific antibody AB9K exhibited no non-specific killing on GPC-3-negative cell strains, which indicates that the bispecific antibody does not mediate T cells to kill non-target tissues *in vivo,* there is no drug toxicity, and the safety is high.

7.3 CD34 immune-reconstituted NPG mouse model of transplanted tumor constructed by inoculating human liver cancer Huh-7 cells

GPC-3-positive human liver cancer Huh-7 cells were selected to study the inhibiting effect of bispecific antibodies on tumor growth *in vivo* in a CD34 immune-reconstituted NPG mouse model of transplanted tumor constructed by subcutaneously inoculating human liver cancer Huh-7 cells.

CD34 immune-reconstituted NPG mice were prepared in the method as described in Example 3.5. Huh-7 cells in the logarithmic growth stage were collected and 2.5 × $10^6$ Huh-7 cells were inoculated subcutaneously on the right back of the immune-reconstituted mice. Four days after inoculation, the mice were randomly divided into two groups with seven mice in each group according to the tumor volumes and weights. The treated group was intraperitoneally administered with AB9K at a dose of 1 mg/kg, and the control group was administered with a PBS solution of the same volume, once a day until the test was completed. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 6-3, on Day 21 of administration, the average tumor volume of the PBS control group was 2102.84 ± 275.71 $mm^3$; the average tumor volume of the treated group administrated with AB9K at the dose of 1 mg/kg was 325.01 ± 282.21 $mm^3$, and the TGI was 86.53%, wherein the tumor in four mice was completely regressed, which was significantly different from that of the control group (*P* < *0.001*). The above results show that the bispecific antibody AB9K had an excellent anti-tumor effect in the CD34 immune-reconstituted model.

Example 8 *In vitro* biological function evaluation of Anti-CD20xCD3 bispecific antibodies and pharmacodynamics study of Anti-CD20xCD3 bispecific antibodies in a mouse transplanted tumor model

8.1 Detection of the binding activity of AB2K to CD20-positive tumor cells by flow cytometry

Raji cells (purchased from the cell bank of Chinese Academy of Sciences) were cultured and collected by centrifugation. The collected cells were resuspended with 1% PBSB and placed in 96-well plates, 100 μl (i.e., 2 × $10^5$ cells) per well, after the cell density was adjusted to (2 × $10^6$) cells/ml. Diluted bispecific antibodies with a series of concentrations were added and incubated for 1 hour at 4 °C. The cells were centrifuged to discard the supernatant and then washed three times using a PBS solution with 1% BSA (PBSB). Diluted AF488-labeled goat anti-human IgG antibodies (Jackson Immuno Research Inc., Cat. No. 109-545-088) or mouse anti-6xHis IgG antibodies (R&D Systems, Cat. No. IC050P) were added to the cells, and the cells were incubated for 1 hour at 4 °C in the dark. The obtained cells were centrifuged to discard the supernatant and then washed twice with 1% PBSB, and cells in each well were resuspended with 100 μl of 1% paraformaldehyde. The signal intensity was detected by flow cytometry. The analysis was performed with the average fluorescence intensity as the Y-axis and the antibody concentration as the X-axis through software GraphPad to calculate the $EC_{50}$ value for the binding of AB2K to Raji cells.

As shown in FIG. 7-1, AB2K bound well to CD20-positive cells, the signal intensity was proportional to the antibody concentration, and the $EC_{50}$ value for AB2K binding to Raji cells was calculated, which was about 69.97 nM.

8.2 AB2K mediating effector cells to target and kill CD20-positive tumor cells

Normally cultured Raji-luc cells (purchased from Beijing Biocytogen Biotechnology Co., Ltd.) were added to 96-well white plates after the cell density was adjusted to $1 \times 10^5$ cells/ml, 40 μl per well. AB2K antibodies were diluted into a series of gradients and added to the 96-well white plates. After the CIK cell density was adjusted to $5 \times 10^5$ cells/ml, the CIK cells were added to the 96-well white plates, 40 μl per well, to make the effector: target ratio (E : T) equal to 5 : 1, and cultured for 24 hours at 37 °C. After 24 hours, the white plates were taken out, 100 μl of One-Glo (Promega, Cat. No. E6120) solution was added to each well, and then the white plates were placed for at least three minutes at room temperature. The luminescence value was measured by a microplate reader. The analysis was performed with the fluorescence intensity as the Y-axis and the antibody concentration as the X-axis through software GraphPad to calculate the $EC_{50}$ value of AB2K killing Raji-luc cells.

As shown in FIG. 7-2, the $EC_{50}$ for AB2K mediating effector cells to kill Raji-luc cells was only 42.8 ng/ml and AB2K had target specificity, while the $EC_{50}$ of AB7K7 as a negative control was 229.5 ng/ml and AB7K7 had little killing effect on Raji-luc cells.

8.3 Evaluation of abilities of bispecific antibodies to activate T cells through reporter gene cell strains

Jurkat T cells containing NFAT RE reporter genes (BPS Bioscience, Cat. No. 60621) can overexpress luciferase in the presence of bispecific antibodies and CD20-positive Raji cells, and the degree of activation of the Jurkat T cells can be quantified by detecting the activity of the luciferase. A four-parameter curve was fitted using the concentration of bispecific antibody as the X-axis and the fluorescein signal as the Y-axis.

As shown in FIG. 7-3, AB2K can specifically activate Jurkat NFATRE Luc cells, wherein the $EC_{50}$ value was 0.2006 μg/ml and its concentration was proportional to signal intensity, while AB7K7 as a negative control had little ability to activate T cells.

8.4 NPG mouse model of transplanted tumor constructed by subcutaneously co-inoculating human CIK cells and human Burkkit's lymphoma Raji cells

CD20-positive human Burkkit's lymphoma Raji cells were selected to study the inhibiting effect of bispecific antibodies on tumor growth in vivo in an NPG mouse model of transplanted tumor constructed by subcutaneously co-inoculating human CIK cells and human Burkkit's lymphoma cells Raji.

CIK cells were prepared in the method as described in Example 3.1. Female NPG mice (purchased from Beijing Vitalstar Biotechnology Co., Ltd.) at the age of seven to eight weeks were selected, and Raji cells in the logarithmic growth stage were collected. $4 \times 10^6$ Raji cells and $8 \times 10^5$ CIK cells were mixed and inoculated subcutaneously on the right back of each NPG mouse. One hour after inoculation, the mice were randomly divided into five groups with six mice in each group according to their weights and intraperitoneally administered with corresponding drugs. Specifically, all treated groups were administered with Rituxan (from Roche) and bispecific antibody AB2K, respectively, at doses of 1 mg/kg and 0.1 mg/kg, twice a week. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 7-4, on Day 24 of administration, the average tumor volume of the PBS control group was $1766.84 \pm 155.62$ $mm^3$; the average tumor volume of the treated group administrated with Rituxan at a dose of 1 mg/kg was $647.92 \pm 277.11$ $mm^3$, and TGI was 63.33%, which was significantly different from that of the control group ($P < 0.01$); the average tumor volume of the treated group administrated with Rituxan at a dose of 0.1 mg/kg was $1893.81 \pm 186.99$ $mm^3$, and Rituxan herein exhibited no efficacy; the average tumor volume of the treated group administrated with AB2K at a dose of 1 mg/kg was $116.18 \pm 39.50$ $mm^3$, and TGI was 93.42%, which was significantly different from that of the control group ($P < 0.01$); the average tumor volume of the treated group administrated with AB2K at a dose of 0.1 mg/kg was 1226.03 ± 340.05 $mm^3$, and TGI was 30.61%, which was not significantly different from that of the control group. The results show that the bispecific antibody AB2K could inhibit the growth of tumor cells by activating human immune cells in animals; and at the same dose, the efficacy of the bispecific antibody was better than the efficacy of the monoclonal antibody Rituxan, and the bispecific antibody exhibited great anti-tumor effects.

8.5 NPG mouse model of transplanted tumor constructed by subcutaneously co-inoculating human CIK cells and human Burkkit's lymphoma Daudi cells

CD20-positive human Burkkit's lymphoma Daudi cells were selected to study the inhibiting effect of bispecific antibodies on tumor growth *in vivo* in an NPG mouse model of transplanted tumor constructed by subcutaneously co-inoculating human CIK cells and human Burkkit's lymphoma Daudi cells.

CIK cells were prepared in the method as described in Example 3.1. Female NPG mice at the age of seven to eight weeks were selected, and Daudi cells in the logarithmic growth stage were collected. $4 \times 10^6$ Daudi cells and $8 \times 10^5$ CIK cells were mixed and inoculated subcutaneously on the right back of each NPG mouse. One hour later, the mice were randomly divided into five groups with six mice in each group according to their weights and intraperitoneally administered with corresponding drugs. All treated groups were administrated twice a week. Rituxan and bispecific antibody AB2K were both administered at doses of 1 mg/kg and 0.1 mg/kg, respectively. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 7-5, on Day 30 of administration, the average tumor volume of the PBS control group was 889.68 ± 192.13 $mm^3$; the average tumor volume of the treated group administrated with Rituxan at a dose of 1 mg/kg was 241.51 ± 44.91 $mm^3$, and TGI was 72.85%, which was significantly different from that of the control group ($P < 0.01$); the average tumor volume of the treated group administrated with Rituxan at a dose of 0.1 mg/kg was 746.11 ± 299.71 $mm^3$, which was not significantly different from that of the control group; the average tumor volume of the treated group administrated with AB2K at a dose of 1 mg/kg was 72.05 ± 11.89 $mm^3$, and TGI was 91.9%, which was significantly different from that of the control group ($P < 0.01$); the average tumor volume of the treated group administrated with AB2K at a dose of 0.1 mg/kg was 75.36 ± 11.81 $mm^3$, and TGI was 91.53%, which was significantly different from that of the control group ($P < 0.01$). The results show that the bispecific antibody AB2K could inhibit the growth of tumor cells by activating human immune cells in animals; and at the same dose, the efficacy of the bispecific antibody was better than the efficacy of the monoclonal antibody Rituxan, and AB2K exhibited good anti-tumor effects even at a low dose.

Example 9 Evaluation of the safety of Anti-CD20xCD3 bispecific antibodies

The toxicity of AB2K was evaluated to determine appropriate dose ranges and observation indicators for subsequent toxicity tests. Adult Female cynomolgus monkeys (purchased from Guangzhou Xiangguan Biotechnology Co., Ltd.) at the age of 3-4 years and with the weight of 3-4 kg were divided into two groups with one mouse in each group, wherein the two groups were a vehicle control group and an AB2K treated group. The groups were administrated via intravenous drip by a peristaltic pump for 1 hour. The dose amount and volume administered are shown in Table 7. The groups were administrated on Day 0 (D0), Day 7 (D7), Day 21 (D21), and Day 28 (D28), respectively, for a total of four doses, and the drug dose was gradually escalated each time. The monkeys were weighed weekly.

Table 7 Dosing schedule for cynomolgus monkey acute toxicity evaluation

| Group | To-be-tested drugs name | Dose volume | Dose amount |
|---|---|---|---|
| G1 | Vehicle control group | D0: 5 mL/kg<br>D7: 5 mL/kg<br>D21: 10 mL/kg<br>D28: 10 mL/kg | N/A |
| G2 | AB2K | D0: 5 mL/kg<br>D7: 5 mL/kg<br>D21: 10 mL/kg<br>D28: 10 mL/kg | D0: 0.06 mg/kg<br>D7: 0.3 mg/kg<br>D21: 1.5 mg/kg<br>D28: 3 mg/kg |

During the test, animals were periodically monitored for clinical symptoms, body weight, food consumption, body temperature, electrocardiogram, blood pressure, clinicopathological indexes (blood cell count, coagulation function measure, and blood biochemistry), lymphocyte subsets, cytokines, drug plasma concentration measure, and toxicokinetics analyses. After administration of AB2K, the physical signs of cynomolgus monkeys exhibited no abnormal reaction, the body weight was relatively stable, the body temperature fluctuation was similar to the body temperature fluctuation of the vehicle control group, and no death or impending death was observed among animals during the administration period. As shown in FIG. 8, after administration, the white blood cell changes of cynomolgus monkeys in the AB2K group were similar to the white blood cell changes in the control group; the first administration of AB2K at a dose of 0.06 mg/kg had little effect on lymphocytes; 1 hour to 6 hours after the second administration, the number of lymphocytes in the animals of the treated group decreased sharply and recovered to normal after 24 hours; as the number of administrations increased, the effect of AB2K on the decrease in the number of lymphocytes was weaker and weaker despite increasing doses. In addition, after the first administration of AB2K, the release of IL-2, IL-6 and TNF-$\alpha$ factors was promoted and the release of IL-5 was slightly stimulated, but the release of IFN-$\gamma$ was not stimulated; as the number of administrations increased, the release-promoting effect of AB2K on cytokines became less and less significant, indicating that the body had already been adapted to the stimulation by bispecific antibodies.

Example 10 Pharmacokinetics evaluation of Anti-CD20xCD3 bispecific antibodies Female cynomolgus monkeys with the weight of 3-4 kg were divided into two groups with one in one monkey in each group. The first group was a blank control group, and the second group was an AB2K treated group administrated at a dose of 0.3 mg/kg. The blood sampling time points were Minute 15, Hour 1, Hour 3, Hour 6, Hour 10, Hour 24, Hour 30, Hour 48, Hour 54, Hour 72, Hour 96, and Hour 144, respectively, a total of 13 time points. Serum was collected from blood and frozen at -80°C.

The drug concentration of AB2K in serum was determined by ELISA. The pharmacokinetics parameters were calculated using software PKSolver. Specific parameters are shown in Table 8. The results show that $T_{1/2}$ of AB2K in normal cynomolgus monkeys was about 8.5 hours.

Table 8 Pharmacokinetics parameters of bispecific antibody AB2K in cynomolgus monkeys

| AB2K | $t_{1/2}$ (h) | AUC 0-inf_obs ($\mu$g/mL*h) | Vz_obs ($\mu$g/kg)/ ($\mu$g/mL) | Cl_obs ($\mu$g/kg)/ ($\mu$g/mL)/h |
|---|---|---|---|---|
| Pharmacokinetic s parameter | 8.45 | 168.63 | 21.68 | 1.78 |

Example 11 Evaluation of *in vitro* biological functions of Anti-CD19×CD3 bispecific antibodies

11.1 Detection of binding activities of bispecific antibodies to effector cells and target cells (FACS)

a) Detection of binding activities of bispecific antibodies to CD19-positive tumor Raji cells by flow cytometry

CD19-positive tumor cells Raji cells were cultured and collected by centrifugation. The collected cells were resuspended with 1% PBSB, placed in 96-well plates after the cell density was adjusted to ($2 \times 10^6$) cells/ml, 100 $\mu$l ($2 \times 10^5$ cells) per well, and blocked for 0.5 hours at 4 °C. The blocked cells were centrifuged to discard the supernatant, and then diluted bispecific antibodies AB1K2 with a series of concentrations and isotype CD19 bispecific antibodies AB23P8, AB23P9 and AB23P10 were added and incubated for 1 hour at 4 °C. The cells were centrifuged to discard the supernatant and then washed three times using PBSB with 1% BSA. Diluted AF647-labeled goat anti-human IgG antibodies were added to the cells, and the cells were incubated for 1 hour at 4 °C in the dark. The obtained cells were centrifuged to discard the supernatant and washed twice with 1% PBSB, and cells in each well were resuspended with 100 $\mu$l of 1% PF. The signal intensity was detected by flow cytometry. The analysis was performed with the average fluorescence intensity as the Y-axis and the antibody concentration as the X-axis through software GraphPad to calculate the $EC_{50}$ value for the binding of bispecific antibodies to tumor cells Raji.

The results show that bispecific antibodies with different structures had a good binding activity to tumor cells over-expressing CD19. FIG. 9-1 shows binding curves of bispecific antibodies with different structures to tumor cells Raji. As shown in Table 9-1, $EC_{50}$ for the binding of each of four bispecific antibodies to tumor cells Raji was at the nM level.

Table 9-1 Detection of abilities of Anti-CD19×CD3 bispecific antibodies to bind to tumor cells Raji

| | AB1K2 | AB23P8 | AB23P9 | AB23P10 |
|---|---|---|---|---|
| $EC_{50}$ (nM) | 1.393 | 1.924 | 2.600 | 2.678 |

b) Detection of binding activities of bispecific antibodies to human T cells by FACS

PBMCs were prepared from fresh human blood by density gradient centrifugation. The prepared PBMCs were resuspended in a 1640 medium containing 10% heat-inactivated FBS, added with 2 μg/ml of CD3 antibody for activation for 24 h, then added with 250 IU/ml of IL-2 for amplification for 7 days, to prepare expanded T cells which were detected by flow cytometry to be positive for CD3 expression on the surface. The to-be-detected sample was prepared and detected in the same manner as in a) of Example 11.1. Cells resuspended with 1% PF were detected on a machine and, with the average fluorescence intensity, analyzed by software GraphPad to calculate $EC_{50}$ value for the binding of each bispecific antibody to human T cells.

The results in FIG. 9-2 show that each bispecific antibody had a good binding activity to CIK. As shown in Table 9-2, the $EC_{50}$ of AB1K2 was about 16 nM, which was roughly equal to the $EC_{50}$ of AB23P8, and the $EC_{50}$ of AB23P9 and AB23P10 were about 50 nM and 30 nM, respectively.

Table 9-2 Detection of abilities of Anti-CD19×CD3 bispecific antibodies to bind to effector cells CIK

| | AB1K2 | AB23P8 | AB23P9 | AB23P10 |
|---|---|---|---|---|
| $EC_{50}$ (nM) | 15.69 | 16.69 | 49.52 | 32.41 |

c) Detection of cross-reactivity of bispecific antibodies with CD3 on the surface of cynomolgus monkey CIK cell membrane by FACS

PBMCs were prepared from fresh cynomolgus monkey blood by density gradient centrifugation. The prepared PBMCs were resuspended in a 1640 medium containing 10% heat-inactivated FBS, added with 2 μg/ml of OKT3 for activation for 24 h, then added with 250 IU/ml of IL-2 for amplification for 7 days to prepare cynomolgus monkey CIK cells for use. Human CIK cells and cynomolgus monkey CIK cells were collected by centrifugation. The to-be-detected sample was prepared and detected in the same manner as in a) of Example 11.1. Cells resuspended with 1% paraformaldehyde solution were detected on a machine and, with the average fluorescence intensity, analyzed by software GraphPad to calculate the $EC_{50}$ values for the binding of bispecific antibodies to human CIK cells and the $EC_{50}$ values for the binding of bispecific antibodies to cynomolgus monkey CIK cells.

As shown in FIG. 9-3, there was no difference between the binding ability of the bispecific antibody AB1K2 to cynomolgus monkey T cells and the ability of the bispecific antibody AB23P10 to cynomolgus monkey T cells, the $EC_{50}$ for the binding of each of both bispecific antibodies to cynomolgus monkey T cells was approximately 5.5 nM as detected by flow cytometry, and the ability of the both bispecific antibodies to cynomolgus monkey T cells was stronger than the ability of the both bispecific antibodies to human T cells.

11.2 Detection of abilities of bispecific antibodies to bind to antigens The binding of bispecific antibodies to soluble CD3 and CD19 was detected by double antigen sandwich ELISA.

CD19 proteins (ACRO Biosystems, Cat. No. CD9-H5251) were diluted with PBS to a concentration of 1 μg/ml and added to 96-well plates, 100 μl per well. The plates were coated at 4 °C overnight. The plates were then blocked with 1% skimmed milk powder for 1 hour at room temperature. Each bispecific antibody was diluted with a 5-fold gradient for a total of 10 concentration gradients. The 96-well plates were then washed with PBST, and then the diluted bispecific antibodies were added. Control wells without antibodies were set. Incubated for 2 hours at room temperature. Unbound bispecific antibodies were washed away with PBST. Biotinylated CD3E&CD3D (ACRO Biosystem, Cat. No. CDD-H82W1) were mixed at 50 ng/ml with streptavdin HRP (BD, Cat. No. 554066), added in 96-well plates, 100 μl per well, and incubated for 1 hour at room temperature. 96-well plates were washed with PBST, and TMB was added to the plates, 100 μl per wells. Color development was performed at room temperature for 15 minutes, and then 0.2 M $H_2SO_4$ was added to stop the color development reaction. The light absorbance values at A450-620 nm were measured by a microplate reader. Analysis was performed by software GraphPa, and the $EC_{50}$ values for the binding of bispecific antibodies to two antigens were calculated.

The results show that each bispecific antibody bound specifically to both CD3 and CD19 molecules and exhibited good dose-dependence as the concentration of the antibodies changed (FIG. 9-4). The abilities of several bispecific antibodies to bind to soluble CD3 and CD19 are shown in Table 9-3, with $EC_{50}$ values ranging from 0.19 nM to 0.47 nM, and there was little difference between binding activities at both ends.

Table 9-3 Detection of abilities of Anti-CD19×CD3 bispecific antibodies to bind to CD3 and CD19 molecules

| | AB1K2 | AB23P8 | AB23P9 | AB23P10 |
|---|---|---|---|---|
| $EC_{50}$ (nM) | 0.2185 | 0.1925 | 0.2211 | 0.4704 |

11.3 Evaluation of abilities of bispecific antibodies to activate T cells through reporter gene cell strains

Jurkat T cells containing NFAT RE reporter genes can overexpress luciferase in the presence of bispecific antibodies and target cells Raji, and the degree of activation of the Jurkat T cells can be quantified by detecting the activity of the luciferase. A four-parameter curve was fitted using the concentration of bispecific antibody as the X-axis and the fluorescein signal as the Y-axis.

The test results in FIGS. 9-5 and 9-6 show that Jurkat T cells can hardly be activated in the absence of target cells overexpressing CD19, and T cells can be activated only in the presence of both the bispecific antibody and the target cells at both ends. The ability of each bispecific antibody to activate Jurkat T cells is shown in Table 9-4, and the ability of each bispecific antibody to activate Jurkat T cells was almost equivalent to each other.

Table 9-4 Detection of abilities of Anti-CD19×CD3 bispecific antibodies to activate a reporter gene cell strain that are Jurkat T cells

| | AB1K2 | AB23P8 | AB23P9 | AB23P10 | Blincyto |
|---|---|---|---|---|---|
| $EC_{50}$ (nM) | 1.080 | 1.123 | 0.8527 | 0.7093 | 2.714 |

11.4 Abilities of bispecific antibodies to mediate T cells to kill tumor cells

Normally cultured tumor cell lines, including Raji-Luc, NALM6 and Reh cells (all purchased from the cell bank of Chinese Academy of Sciences, Shanghai) were used as target cells, and cell suspensions were collected and centrifuged, added to 96-well cell culture plates after the cell density was adjusted to $2 \times 10^5$ cells/ml, 100 μl per well, and cultured overnight. The antibodies were diluted according to the test design, and added to the cells, 50 μl per well, while wells without the addition of antibodies were supplemented with the same volume of the medium. Effector cells (human PBMCs or expanded CIK cells) whose number was five times larger than the number of target cells, were then added, 100 μl per well. Control wells were set, and wells without the addition of effector cells were supplemented with the same volume of the medium. After 48 hours of culture, Raji-Luc cells were detected by Steady-Glo Luciferase Assay System (Promega) and other cells were detected by CytoTox96 Non-Radio Cytotoxicity Assay (Promega). The analysis was performed with the detection results as the Y-axis and the bispecific antibody concentration as the X-axis through software GraphPad to calculate and compare the ability of each bispecific antibody to mediate the killing on Raji-luc cells.

The $EC_{50}$ values of each bispecific antibody to mediate effector cells to kill tumor cells are shown in Tables 9-5 to 9-7. The results show that each bispecific antibody exhibited a very significant killing effect on tumor cells with high expression of CD19 in a dose-dependent manner, wherein $EC_{50}$ of each bispecific antibody reached the pM level.

Table 9-5 $EC_{50}$ values of bispecific antibodies to mediate CIK to kill tumor cells

| $EC_{50}$ (pM) | AB1K2 | AB23P8 | AB23P9 | AB23P10 | Blincyto |
|---|---|---|---|---|---|
| Raji-LUC | 0.6988 | 0.5861 | 0.1480 | 0.1280 | 0.5952 |
| | 0.2024 | - | - | 0.4834 | 5.654 |
| Note: - means that no detection is performed. | | | | | |

Table 9-6 $EC_{50}$ values of bispecific antibodies to mediate PBMCs to kill tumor cells

| $EC_{50}$ (pM) | AB1K2 | AB23P8 | AB23P9 | AB23P10 | Blincyto |
|---|---|---|---|---|---|
| Raji-LUC | 1.225 | 1.025 | 1.014 | 0.9462 | 5.452 |
| | 1.254 | - | - | 1.254 | 21.22 |
| | - | - | - | 4.176 | 22.58 |
| Note: - means that no detection is performed. | | | | | |

Table 9-7 $EC_{50}$ values of bispecific antibodies to mediate CIK to kill different tumor cells

| $EC_{50}$ (pM) | AB1K2 | AB23P10 | Blincyto |
|---|---|---|---|
| NALM6 | - | 4.402 | 77.29 |
| Reh | 1.709 | 1.640 | 11.87 |
| Note: - means that no detection is performed. | | | |

Example 12 Evaluation of *in vitro* biological functions of Anti-Mucin1×CD3 bispecific antibodies

12.1 Binding activities of AB11K to tumor cells over-expressing Mucin1 and to human or cynomolgus monkey primary T cells

Human breast cancer cells MCF-7, BT-549, HCC70, T-47D and HCC1954, human ovarian cancer cell SK-OV-3, human cervical cancer cell Hela and human colon cancer cell HT-29 were cultured, wherein MCF-7, BT-549, T-47D, HCC1954, SK-OV-3, Hela and HT-29 cells were purchase from the cell bank of Chinese Academy of Sciences, and HCC70 cells were purchase from Nanjing Cobioer Biotechnology Co., Ltd. Each kind of the above cells was digested with trypsin, collected by centrifugation, resuspended with 1% PBSB, placed in 96-well plates after the cell density of each kind of cells was adjusted to $5 \times 10^5$ cells/ml, 100 μl per well, and blocked for 30 minutes at 4 °C. Human or cynomolgus monkey primary T cells were collected by centrifugation, resuspended with 1% PBSB, placed in 96-well plates after the cell density of each kind of cells was adjusted to $5 \times 10^5$ cells/ml, 100 μl per well, and blocked for 30 minutes at 4 °C. The cells were washed once with 1% PBSB. Diluted AB11K with a series of concentrations was added at 100 μl per well and incubated for 1 hour at 4 °C. The cells were centrifuged to discard the supernatant and then washed twice with 1% PBSB. Diluted AF647 goat anti human IgG (H+L) antibodies (Jackson Immuno Research Inc., diluted at 1 : 250) were added, 100 μl per well, and then the cells were incubated for 1 hour at 4 °C in the dark. The cells were centrifuged to discard the supernatant. The plates were washed, and after that, 4% PFA was added, 150 μl per well, to resuspend the cells. The signal intensity was detected by flow cytometry. The analysis was performed with the average fluorescence intensity as the Y-axis and the antibody molar concentration as the X-axis through software GraphPad Prism 6 to calculate the $EC_{50}$ values for the binding of AB11K to the above tumor cells and human or cynomolgus monkey primary T cells.

As shown in FIG. 10-1 and Table 10-1, at the cellular level, AB11K bound to the above tumor cells and human or cynomolgus monkey primary T cells, and the signal intensity was proportional to the antibody concentration, and $EC_{50}$ calculated for the binding of AB11K to the above tumor cells ranged from 5 nM to 300 nM, wherein the binding to T-47D and Hela was strongest, followed by the binding to HCC70, HCC1954, SKOV-3 and BT-549, while the binding to MCF-7 and HT-29 was the weakest, which did not reach the upper platform. The $EC_{50}$ values for the binding of AB7K to human or cynomolgus monkey T cells were 13.43 nM and 9.996 nM, respectively, and the ability of AB11K to bind to cynomolgus monkey T cells was roughly equivalent to the ability of AB11K to bind to human T cells.

Table 10-1 $EC_{50}$ results for the binding of AB11K to tumor cells over-expressing Mucin1 and to human or cynomolgus monkey primary T cells

| Cell name | $EC_{50}$ (nM) |
|---|---|
| MCF-7 | / |
| BT-549 | 287.2 |
| HCC70 | 58.98 |
| T-47D | 5.053 |
| HCC1954 | 81.24 |
| Hela | 5.515 |
| SK-OV-3 | 93.72 |
| HT-29 | / |
| Human T cells | 13.43 |
| cynomolgus monkey T cells | 9.996 |

12.2 Ability of AB11K to mediate T cells to kill tumor cells

Normally cultured cells MCF-7, BT-549, HCC70, T-47D, HCC1954, SK-OV-3, Hela and HT-29 were used as target cells, respectively. Each kind of cells was digested with trypsin, placed in 96-well cell culture plates after the cell density of each kind of cells was adjusted to 2 $\times$ $10^5$ cells/ml, 100 $\mu$l per well, and cultured overnight at 37 °C with 5% $CO_2$. Effector cells (expanded T cells) whose number was five times larger than the number of corresponding target cells were added as T cell group, and effector cells (PBMCs from healthy volunteers) whose number was ten times larger than the number of corresponding target cells were added as PBMC groups, 100 $\mu$l per well. Blank wells and wells without the addition of effector cells were set. AB11K was diluted to 50 $\mu$g/mL with a medium, after the 4-fold dilution, added to 96-well plates, 50 $\mu$l per well, and incubated for 48 hours at 37 °C with 5% $CO_2$. The cell culture plates were washed three times with PBS and the suspended cells were removed. A medium containing 10% CCK-8 was added, 100 $\mu$l per well, and incubated for 4 hours at 37 °C with 5% $CO_2$. Readings at 450 nm and 620 nm were obtained. The specific killing rates of the antibodies were calculated according to values at [OD450-OD620] using the formula as follows:

$$\text{Antibody specific killing rate (\%)} = \frac{[\text{OD450} - \text{OD620}](\text{effector cells} + \text{target cells}) - [\text{OD450} - \text{OD620}](\text{antibody teated group})}{[\text{OD450} - \text{OD620}](\text{effector cells} + \text{target cells}) - [\text{OD450} - \text{OD620}]\ (\text{blank group})} \times 100\%$$

The analysis was performed with the specific killing rate (%) as the Y-axis and the antibody molar concentration as the X-axis through software GraphPad Prism 6 to calculate the $EC_{50}$ value for AB11K to mediate the killing on tumor target cells.

As shown in FIGS. 10-2 and 10-3 and Table 10-2, the bispecific antibody AB11K exhibited very significant killing effects on tumor cells highly expressing Mucin1 through its mediation on effector cells. When expanded T cells were used as effector cells, the maximum specific killing of AB11K reached 99% or more, wherein the specific killing effects on MCF-7, BT-549, HCC70 and T-47D were the best with $EC_{50}$ ranging from 100 pM to 200 pM, followed by the specific killing effects on Hela, HCC1954 and SK-OV-3, while the specific killing effect on HT-29 was the weakest with a relatively large $EC_{50}$, about 1577 pM. When PBMCs were used as effector cells, the specific killing effects of AB11K on MCF-7 and BT-549 were the best with the maximum specific killing of 95% or more and $EC_{50}$ of 131.2 pM and 955.9 pM, respectively, followed by the specific killing effects on HCC1954 and HCC70, and $EC_{50}$ for specifically killing Hela and HT-29 were relatively large, which were 4810 pM and 9550 pM, respectively.

Table 10-2 $EC_{50}$ results of AB11K to mediate effector cells to kill tumor cells

| Cell name | T cell killing $EC_{50}$ (pM) | PBMC killing $EC_{50}$ (pM) |
|---|---|---|
| MCF-7 | 152.7 | 131.2 |
| BT-549 | 140.9 | 955.9 |
| HCC70 | 185.4 | 595.2 |
| T-47D | 84.53 | / |
| HCC1954 | 280.9 | 1893 |
| Hela | 278.2 | 4810 |
| SK-OV-3 | 689.4 | / |
| HT-29 | 1577 | 9550 |

12.3 Evaluation of abilities of bispecific antibodies to activate T cells

Jurkat T cells containing NFAT RE reporter genes (purchased from BPS Bioscience) can overexpress luciferase in the presence of bispecific antibodies and Mucin1-positive cells, and the degree of activation of the Jurkat T cells can be quantified by detecting the activity of the luciferase.

Specifically, cells MCF-7, BT-549, HCC70, T-47D, HCC1954, SK-OV-3, Hela and HT-29 were digested with trypsin, placed in 96-well cell culture plates after the cell density of each kind of cells was adjusted to 2 $\times$ $10^5$ cells/ml, 50 $\mu$l per well, and cultured overnight at 37 °C with 5% $CO_2$. The cell density of Jurkat-NFAT cells was adjusted to 2.5 $\times$ $10^6$ cells/ml, 40 $\mu$l per well. AB11K was diluted to 400 $\mu$g/mL with a medium, after the 4-fold dilution, added to 96-

well plates, 10 μl per well, and incubated for 48 hours at 37 °C with 5% $CO_2$ in an incubator. Steady-Glo® Luciferase was added, 100 μl per well and reacted for 5 minutes. After that, the luminescence value was measured by a microplate reader. The analysis was performed with the fluorescein intensity as the Y-axis and the antibody molar concentration as the X-axis through software GraphPad Prism 6 to calculate the $EC_{50}$ for bispecific antibodies to activate T cells.

As shown in FIG. 10-4 and Table 10-3, AB11K specifically activated Jurkat-NFAT cells, wherein the $EC_{50}$ value was at the nM level and its concentration was proportional to signal intensity.

Table 10-3 $EC_{50}$ results of the ability of AB11K to activate T cells

| Cell name | T cell activation $EC_{50}$ (nM) |
|---|---|
| MCF-7 | 14.22 |
| BT-549 | 10.49 |
| HCC70 | 3.016 |
| T-47D | 0.6294 |
| HCC1954 | 5.599 |
| Hela | 7.241 |
| SK-OV-3 | 10.37 |
| HT-29 | 6.711 |

Example 13 Pharmacodynamics study of Anti-EGFR×CD3 bispecific antibodies in a mouse transplanted tumor model

A mouse transplanted tumor model of human skin cancer A431 cells that highly expressed EGFR was selected to perform the pharmacodynamics study of Anti-EGFR×CD3 bispecific antibodies AB8K, AB2K and Erbitux (from Merck KGaA) on the *in vivo* inhibition of tumor growth.

CIK cells were prepared in the method as described in Example 3.1. A431 cells in the logarithmic growth stage were collected. Female NPG mice at the age of seven to eight weeks were selected, and $3 \times 10^6$ A431 cells and $1 \times 10^6$ CIK cells were mixed and inoculated subcutaneously on the right back of each NPG mouse. One hour later, the mice were randomly divided into five groups with six mice in each group according to their weights and intraperitoneally administered with corresponding drugs. All treated groups and the PBS control group were administrated twice a week, wherein AB2K and Erbitux were administrated at a dose of 1 mg/kg. AB8K was administrated at doses of 1 mg/kg and 0.1 mg/kg. The day of administration was recorded as Day 0. The maximum diameter (D) and the minimum diameter (d) of the tumor were measured weekly. The volume ($mm^3$) of the tumor of each group and the tumor growth inhibition rate (TGI) (%) of each treated group were calculated using the formulas as shown in Example 3.1.

As shown in FIG. 11, on Day 17 of administration, the average tumor volume of the PBS control group was $1370.76 \pm 216.35$ $mm^3$; the average tumor volume of the treated group administrated with Erbitux at a dose of 1 mg/kg was $1060.35 \pm 115.86$ $mm^3$, which was not significantly different from that of the control group; the average tumor volume of the treated group administrated with AB2K at a dose of 1 mg/kg was $877.76 \pm 120.38$ $mm^3$, which was not significantly different from that of the control group; the average tumor volumes of the treated groups administrated with AB8K at doses of 0.1 mg/kg and 1 mg/kg were $233.30 \pm 135.51$ $mm^3$ and $8.14 \pm 8.14$ $mm^3$, respectively, and TGIs were 82.98% and 98.36%, respectively, which were significantly different from that of the control group ($P < 0.01$), wherein the tumors in five of six mice of the treated group administrated with AB8K at a dose of 1 mg/kg exhibited complete regression. AB2K was an isotype control of AB8K. A431 cells did not express CD20. AB2K exhibited no pharmacological effect in this model, indicating that the structure of the bispecific antibody is relatively safe and does not cause non-specific killing. More than 90% of CIK cells were activated T cells. AB8K inhibited and killed tumor cells by activating human immune cells in animals, and completely inhibited tumor growth at a dose of 1 mg/kg and exhibited a good anti-tumor effect even at a dose of 0.1 mg/kg.

All the publications mentioned in the present invention are incorporated herein by reference as if each publication is separately incorporated herein by reference. In addition, it should be understood that those skilled in the art, who have read the disclosure, can make various changes or modifications on the present disclosure, and these equivalent forms fall within the scope of the appended claims.

## Claims

1. A bispecific antibody, which is a tetravalent homodimer formed by two identical polypeptide chains that bind to each other by a covalent bond, wherein each of the polypeptide chains comprises a first single-chain Fv that specifically binds to an tumor-associated antigen, a second single-chain Fv that specifically bind to effector cell antigen CD3, and an Fc fragment in sequence from N-terminus to C-terminus; wherein the first single-chain Fv is linked to the second single-chain Fv by a linker peptide, the second single-chain Fv is linked to the Fc fragment directly or by a linker peptide, and the Fc fragment has no effector functions comprising CDC, ADCC, and ADCP.

2. The bispecific antibody according to claim 1, wherein the first single-chain Fv comprises a VH domain and a VL domain that are linked by a linker peptide, which has an amino acid sequence of $(GGGGX)_n$, wherein X comprises Ser or Ala, preferably Ser, and n is a natural number of 1 to 5, preferably 3.

3. The bispecific antibody according to claim 1, wherein the tumor-associated antigen comprises, but is not limited to, CD19, CD20, CD22, CD25, CD30, CD33, CD38, CD39, CD40, CD47, CD52, CD73, CD74, CD123, CD133, CD138, BCMA, CA125, CEA, CS1, DLL3, DLL4, EGFR, EpCAM, FLT3, gpA33, GPC-3, Her2, MEGE-A3, NYESO1, PSMA, TAG-72, CIX, folate-binding protein, GD2, GD3, GM2, VEGF, VEGFR2, VEGFR3, Cadherin, Integrin, Mesothelin, Claudin18, $\alpha V\beta 3$, $\alpha 5\beta 1$, ERBB3, c-MET, IGF1R, EPHA3, TRAILR1, TRAILR2, RANKL, B7 protein family, Mucin family, FAP, and Tenascin; preferably, the tumor-associated antigen is CD19, CD20, CD22, CD30, CD38, BCMA, CS1, EpCAM, CEA, Her2, EGFR, CA125, Mucin1, GPC-3, and Mesothelin.

4. The bispecific antibody according to claim 1, wherein the first single-chain Fv specifically binds to CD19 and is selected from the group consisting of:

(i) a VH domain comprising HCDR1, HCDR2, and HCDR3 as shown in SEQ ID NOs: 9, 10, and 11, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 9, 10, and 11; and a VL domain comprising LCDR1, LCDR2, and LCDR3 as shown in SEQ ID NOs: 12, 13, and 14, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 12, 13, and 14;
(ii) a VH domain comprising HCDR1, HCDR2, and HCDR3 as shown in SEQ ID NOs: 17, 18, and 19, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 17, 18, and 19; and a VL domain comprising LCDR1, LCDR2, and LCDR3 as shown in SEQ ID NOs: 20, 21, and 22, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 20, 21, and 22;

(iii) a VH domain comprising HCDR1, HCDR2, and HCDR3 as shown in SEQ ID NOs: 25, 26, and 27, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 25, 26, and 27; and a VL domain comprising LCDR1, LCDR2, and LCDR3 as shown in SEQ ID NOs: 28, 29, and 30, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 28, 29, and 30; and

(iv) a VH domain comprising HCDR1, HCDR2, and HCDR3 as shown in SEQ ID NOs: 33, 34, and 35, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 33, 34, and 35; and a VL domain comprising LCDR1, LCDR2, and LCDR3 as shown in SEQ ID NOs: 36, 37, and 38, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 36, 37, and 38.

**5.** The bispecific antibody according to claim 1, wherein the first single-chain Fv specifically binds to CD20 and is selected from the group consisting of:

(i) a VH domain comprising HCDR1, HCDR2, and HCDR3 as shown in SEQ ID NOs: 41, 42, and 43, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 41, 42, and 43; and a VL domain comprising LCDR1, LCDR2, and LCDR3 as shown in SEQ ID NOs: 44, 45, and 46, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 44, 45, and 46;

(ii) a VH domain comprising HCDR1, HCDR2, and HCDR3 as shown in SEQ ID NOs: 49, 50, and 51, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 49, 50, and 51; and a VL domain comprising LCDR1, LCDR2, and LCDR3 as shown in SEQ ID NOs: 52, 53, and 54, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 52, 53, and 54;

(iii) a VH domain comprising HCDR1, HCDR2, and HCDR3 as shown in SEQ ID NOs: 57, 58, and 59, respectively or having sequences that are substantially identical (for example, are at least 80%, 85%, 90%, 92%, 95%, 97%, 98%, 99% or more similar to or having one or more amino acid substitutions (for example, conservative substitutions) than) to any of SEQ ID NOs: 57, 58, and 59; and a VL domain comprising LCDR1, LCDR2, and LCDR3 as shown in SEQ ID NOs: 60, 61, and 62, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 60, 61, and 62; and

(iv) a VH domain comprising HCDR1, HCDR2, and HCDR3 as shown in SEQ ID NOs: 65, 66, and 67, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 65, 66, and 67; and a VL domain comprising LCDR1, LCDR2, and LCDR3 as shown in SEQ ID NOs: 68, 69, and 70, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 68, 69, and 70.

**6.** The bispecific antibody according to claim 1, wherein the first single-chain Fv specifically binds to CD22 and is selected from the group consisting of:

(i) a VH domain comprising HCDR1, HCDR2, and HCDR3 as shown in SEQ ID NOs: 73, 74, and 75, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 73, 74, and 75; and a VL domain comprising LCDR1, LCDR2, and LCDR3 as shown in SEQ ID NOs: 76, 77, and 78, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 76, 77, and 78; and
(ii) a VH domain comprising HCDR1, HCDR2, and HCDR3 as shown in SEQ ID NOs: 81, 82, and 83, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 81, 82, and 83; and a VL domain comprising LCDR1, LCDR2, and LCDR3 as shown in SEQ ID NOs: 84, 85, and 86, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 84, 85, and 86.

**7.** The bispecific antibody according to claim 1, wherein the first single-chain Fv specifically binds to CD30 and is selected from the group consisting of:

(i) a VH domain comprising HCDR1, HCDR2, and HCDR3 as shown in SEQ ID NOs: 89, 90, and 91, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 89, 90, and 91; and a VL domain comprising LCDR1, LCDR2, and LCDR3 as shown in SEQ ID NOs: 92, 93, and 94, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 92, 93, and 94; and
(ii) a VH domain comprising HCDR1, HCDR2, and HCDR3 as shown in SEQ ID NOs: 97, 98, and 99, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 97, 98, and 99; and a VL domain comprising LCDR1, LCDR2, and LCDR3 as shown in SEQ ID NOs: 100, 101, and 102, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 100, 101, and 102.

**8.** The bispecific antibody according to claim 1, wherein the first single-chain Fv specifically binds to EpCAM and is selected from the group consisting of:

(i) a VH domain comprising HCDR1, HCDR2, and HCDR3 as shown in SEQ ID NOs: 105, 106, and 107, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 105, 106, and 107; and a VL domain comprising LCDR1, LCDR2, and LCDR3 as shown in SEQ ID NOs: 108, 109, and 110, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 108, 109, and 110; and
(ii) a VH domain comprising HCDR1, HCDR2, and HCDR3 as shown in SEQ ID NOs: 113, 114, and 115, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 113, 114, and 115; and a VL domain comprising LCDR1, LCDR2, and LCDR3 as shown in SEQ ID NOs: 116, 117, and 118, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 116, 117, and 118.

9. The bispecific antibody according to claim 1, wherein the first single-chain Fv specifically binds to CEA and is selected from the group consisting of:

(i) a VH domain comprising HCDR1, HCDR2, and HCDR3 as shown in SEQ ID NOs: 121, 122, and 123, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 121, 122, and 123; and a VL domain comprising LCDR1, LCDR2, and LCDR3 as shown in SEQ ID NOs: 124, 125, and 126, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 124, 125, and 126;
(ii) a VH domain comprising HCDR1, HCDR2, and HCDR3 as shown in SEQ ID NOs: 129, 130, and 131, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 129, 130, and 131; and a VL domain comprising LCDR1, LCDR2, and LCDR3 as shown in SEQ ID NOs: 132, 133, and 134, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 132, 133, and 134; and
(iii) a VH domain comprising HCDR1, HCDR2, and HCDR3 as shown in SEQ ID NOs: 137, 138, and 139, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 137, 138, and 139; and a VL domain comprising LCDR1, LCDR2, and LCDR3 as shown in SEQ ID NOs: 140, 141, and 142, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 140, 141, and 142.

10. The bispecific antibody according to claim 1, wherein the first single-chain Fv specifically binds to Her2 and is selected from the group consisting of:

(i) a VH domain comprising HCDR1, HCDR2, and HCDR3 as shown in SEQ ID NOs: 145, 146, and 147, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 145, 146, and 147; and a VL domain comprising LCDR1, LCDR2, and LCDR3 as shown in SEQ ID NOs: 148, 149, and 150, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 148, 149, and 150;
(ii) a VH domain comprising HCDR1, HCDR2, and HCDR3 as shown in SEQ ID NOs: 153, 154, and 155, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 153, 154, and 155; and a VL domain comprising LCDR1, LCDR2, and LCDR3 as shown in SEQ ID NOs: 156, 157, and 158, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 156, 157, and 158; and
(iii) a VH domain comprising HCDR1, HCDR2, and HCDR3 as shown in SEQ ID NOs: 161, 162, and 163, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 161, 162, and 163; and a VL domain comprising LCDR1, LCDR2, and LCDR3 as shown in SEQ ID NOs: 164, 165, and 166, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 164, 165, and 166.

**11.** The bispecific antibody according to claim 1, wherein the first single-chain Fv specifically binds to EGFR and is selected from the group consisting of:

(i) a VH domain comprising HCDR1, HCDR2, and HCDR3 as shown in SEQ ID NOs: 169, 170, and 171, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 169, 170, and 171; and a VL domain comprising LCDR1, LCDR2, and LCDR3 as shown in SEQ ID NOs: 172, 173, and 174, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 172, 173, and 174;
(ii) a VH domain comprising HCDR1, HCDR2, and HCDR3 as shown in SEQ ID NOs: 177, 178, and 179, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 177, 178, and 179; and a VL domain comprising LCDR1, LCDR2, and LCDR3 as shown in SEQ ID NOs: 180, 181, and 182, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 180, 181, and 182; and
(iii) a VH domain comprising HCDR1, HCDR2, and HCDR3 as shown in SEQ ID NOs: 185, 186, and 187, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 185, 186, and 187; and a VL domain comprising LCDR1, LCDR2, and LCDR3 as shown in SEQ ID NOs: 188, 189, and 190, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 188, 189, and 190.

**12.** The bispecific antibody according to claim 1, wherein the first single-chain Fv specifically binds to GPC-3; the VH domain of the first single-chain Fv comprises HCDR1, HCDR2, and HCDR3 as shown in SEQ ID NOs: 193, 194, and 195, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 193, 194, and 195; and the VL domain of the first single-chain Fv comprises LCDR1, LCDR2, and LCDR3 as shown in SEQ ID NOs: 196, 197, and 198, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 196, 197, and 198.

**13.** The bispecific antibody according to claim 1, wherein the first single-chain Fv specifically binds to Mesothelin; the VH domain of the first single-chain Fv comprises HCDR1, HCDR2, and HCDR3 as shown in SEQ ID NOs: 201, 202, and 203, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 201, 202, and 203; and the VL domain of the first single-chain Fv comprises LCDR1, LCDR2, and LCDR3 as shown in SEQ ID NOs: 204, 205, and 206, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 204, 205, and 206.

**14.** The bispecific antibody according to claim 1, wherein the first single-chain Fv specifically binds to Mucin1 and is selected from the group consisting of:

(i) a VH domain comprising HCDR1, HCDR2, and HCDR3 as shown in SEQ ID NOs: 209, 210, and 211, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 209, 210, and 211; and a VL domain comprising LCDR1, LCDR2, and LCDR3 as shown in SEQ ID NOs: 212, 213, and 214, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 212, 213, and 214; and

(ii) a VH domain comprising HCDR1, HCDR2, and HCDR3 as shown in SEQ ID NOs: 217, 218, and 219, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 217, 218, and 219; and a VL domain comprising LCDR1, LCDR2, and LCDR3 as shown in SEQ ID NOs: 220, 221, and 222, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 220, 221, and 222.

**15.** The bispecific antibody according to claim 1, wherein the first single-chain Fv specifically binds to CA125; the VH domain of the first single-chain Fv comprises HCDR1, HCDR2, and HCDR3 as shown in SEQ ID NOs: 225, 226, and 227, respectively or having sequences that are substantially identical to (for example, are at least 80%, 85%, 90%, 92%, 95%, 97%, 98%, 99% or more similar to or have one or more amino acid substitutions (for example, conservative substitutions) than) any of SEQ ID NOs: 225, 226, and 227; and the VL domain of the first single-chain Fv comprises LCDR1, LCDR2, and LCDR3 as shown in SEQ ID NOs: 228, 229, and 230, respectively or having sequences that are substantially identical the (for example, are at least 80%, 85%, 90%, 92%, 95%, 97%, 98%, 99% or more similar to or have one or more amino acid substitutions (for example, conservative substitutions) than) to any of SEQ ID NOs: 228, 229, and 230.

**16.** The bispecific antibody according to claim 1 or 4, wherein the first single-chain Fv specifically binds to CD19 and is selected from the group consisting of:

(i) a VH domain comprising an amino acid sequence as shown in SEQ ID NO: 15 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 15; and a VL domain comprising an amino acid sequence as shown in SEQ ID NO: 16 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 16;

(ii) a VH domain comprising an amino acid sequence as shown in SEQ ID NO: 23 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 23; and a VL domain comprising an amino acid sequence as shown in SEQ ID NO: 24 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 24;

(iii) a VH domain comprising an amino acid sequence as shown in SEQ ID NO: 31 or having a sequence that is substantially identical (for example, is at least 80%, 85%, 90%, 92%, 95%, 97%, 98%, 99% or more similar to or has one or more amino acid substitutions (for example, conservative substitutions) than)to SEQ ID NO: 31; and a VL domain comprising an amino acid sequence as shown in SEQ ID NO: 32 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 32; and

(iv) a VH domain comprising an amino acid sequence as shown in SEQ ID NO: 39 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 39; and a VL domain comprising an amino acid sequence as shown in SEQ ID NO: 40 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 40.

**17.** The bispecific antibody according to claim 1 or 5, wherein the first single-chain Fv specifically binds to CD20 and is selected from the group consisting of:

(i) a VH domain comprising an amino acid sequence as shown in SEQ ID NO: 47 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 47; and a VL domain comprising an amino acid sequence as shown in SEQ ID NO: 48 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 48;

(ii) a VH domain comprising an amino acid sequence as shown in SEQ ID NO: 55 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 55; and a VL domain comprising an amino acid sequence as shown in SEQ ID NO: 56 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 56;
(iii) a VH domain comprising an amino acid sequence as shown in SEQ ID NO: 63 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 63; and a VL domain comprising an amino acid sequence as shown in SEQ ID NO: 64 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 64; and
(iv) a VH domain comprising an amino acid sequence as shown in SEQ ID NO: 71 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 71; and a VL domain comprising an amino acid sequence as shown in SEQ ID NO: 72 or a sequence substantially identical to (for example, is at least 80%, 85%, 90%, 92%, 95%, 97%, 98%, 99% or more similar to or has one or more amino acid substitutions (for example, conservative substitutions) than) SEQ ID NO: 72.

**18.** The bispecific antibody according to claim 1 or 6, wherein the first single-chain Fv specifically binds to CD22 and is selected from the group consisting of:

(i) a VH domain comprising an amino acid sequence as shown in SEQ ID NO: 79 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 79; and a VL domain comprising an amino acid sequence as shown in SEQ ID NO: 80 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 80; and
(ii) a VH domain comprising an amino acid sequence as shown in SEQ ID NO: 87 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 87; and a VL domain comprising an amino acid sequence as shown in SEQ ID NO: 88 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 88.

**19.** The bispecific antibody according to claim 1 or 7, wherein the first single-chain Fv specifically binds to CD30 and is selected from the group consisting of:

(i) a VH domain comprising an amino acid sequence as shown in SEQ ID NO: 95 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 95; and a VL domain comprising an amino acid sequence as shown in SEQ ID NO: 96 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 96; and
(ii) a VH domain comprising an amino acid sequence as shown in SEQ ID NO: 103 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 103; and a VL domain comprising an amino acid sequence as shown in SEQ ID NO: 104 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 104.

**20.** The bispecific antibody according to claim 1 or 8, wherein more preferably, the first single-chain Fv specifically binds to EpCAM and is selected from the group consisting of:

(i) a VH domain comprising an amino acid sequence as shown in SEQ ID NO: 111 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 111; and a VL domain comprising an amino acid sequence as shown in SEQ ID NO: 112 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 112; and

(ii) a VH domain comprising an amino acid sequence as shown in SEQ ID NO: 119 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 119; and a VL domain comprising an amino acid sequence as shown in SEQ ID NO: 120 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 120.

**21.** The bispecific antibody according to claim 1 or 9, wherein the first single-chain Fv specifically binds to CEA and is selected from the group consisting of:

(i) a VH domain comprising an amino acid sequence as shown in SEQ ID NO: 127 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 127,; and a VL domain comprising an amino acid sequence as shown in SEQ ID NO: 128 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 128;
(ii) a VH domain comprising an amino acid sequence as shown in SEQ ID NO: 135 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 135; and a VL domain comprising an amino acid sequence as shown in SEQ ID NO: 136 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 136; and
(iii) a VH domain comprising an amino acid sequence as shown in SEQ ID NO: 143 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 143; and a VL domain comprising an amino acid sequence as shown in SEQ ID NO: 144 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 144.

**22.** The bispecific antibody according to claim 1 or 10, wherein the first single-chain Fv specifically binds to Her2 and is selected from the group consisting of:

(i) a VH domain comprising an amino acid sequence as shown in SEQ ID NO: 151 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 151; and a VL domain comprising an amino acid sequence as shown in SEQ ID NO: 152 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 152;
(ii) a VH domain comprising an amino acid sequence as shown in SEQ ID NO: 159 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 159; and a VL domain comprising an amino acid sequence as shown in SEQ ID NO: 160 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 160; and
(iii) a VH domain comprising an amino acid sequence as shown in SEQ ID NO: 167 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 167; and a VL domain comprising an amino acid sequence as shown in SEQ ID NO: 168 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 168.

**23.** The bispecific antibody according to claim 1 or 11, wherein the first single-chain Fv specifically binds to EGFR and is selected from the group consisting of:

(i) a VH domain comprising an amino acid sequence as shown in SEQ ID NO: 175 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 175; and a VL domain comprising an amino acid sequence as shown in SEQ ID NO: 176 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 176;
(ii) a VH domain comprising an amino acid sequence as shown in SEQ ID NO: 183 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 183; and a VL domain comprising an amino acid sequence as shown in SEQ ID NO: 184 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 184; and
(iii) a VH domain comprising an amino acid sequence as shown in SEQ ID NO: 191 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 191; and a VL domain comprising an amino acid sequence as shown in SEQ ID NO: 192 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 192.

**24.** The bispecific antibody according to claim 1 or 12, wherein the first single-chain Fv specifically binds to GPC-3; the VH domain of the first single-chain Fv comprises an amino acid sequence as shown in SEQ ID NO: 199 or having a sequence substantially identical to (for example, is at least 80%, 85%, 90%, 92%, 95%, 97%, 98%, 99% or more similar to or has one or more amino acid substitutions (for example, conservative substitutions) than) SEQ ID NO: 199; and the VL domain of the first single-chain Fv comprises an amino acid sequence as shown in SEQ ID NO: 200 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 200.

**25.** The bispecific antibody according to claim 1 or 13, wherein the first single-chain Fv specifically binds to Mesothelin; the VH domain of the first single-chain Fv comprises an amino acid sequence as shown in SEQ ID NO: 207 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 207; and the VL domain of the first single-chain Fv comprises an amino acid sequence as shown in SEQ ID NO: 208 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 208.

**26.** The bispecific antibody according to claim 1 or 14, wherein the first single-chain Fv specifically binds to Mucin1 and is selected from the group consisting of:

(i) a VH domain comprising an amino acid sequence as shown in SEQ ID NO: 215 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 215; and a VL domain comprising an amino acid sequence as shown in SEQ ID NO: 216 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 216; and
(ii) a VH domain comprising an amino acid sequence as shown in SEQ ID NO: 223 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 223; and a VL domain comprising an amino acid sequence as shown in SEQ ID NO: 224 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 224.

**27.** The bispecific antibody according to claim 1 or 15, wherein the first single-chain Fv specifically binds to CA125; the VH domain of the first single-chain Fv comprises an amino acid sequence as shown in SEQ ID NO: 231 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 231; and the VL domain of the first single-chain Fv comprises an amino acid sequence as shown in SEQ ID NO: 232 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 232.

**28.** The bispecific antibody according to claim 1, wherein the second single-chain Fv comprises a VH domain and a VL domain that are linked by a linker peptide which has an amino acid sequence of $(GGGGX)_n$, wherein X comprises Ser or Ala, preferably Ser, and n is a natural number of 1 to 5, preferably 3.

**29.** The bispecific antibody according to claim 1 or 28, wherein the second single-chain Fv binds to an effector cell at an $EC_{50}$ value greater than about 50 nM, or greater than 100 nM, or greater than 300 nM, or greater than 500 nM in an *in vitro* binding affinity assay; more preferably, the second single-chain Fv of the bispecific antibody not only binds to human CD3, but also specifically binds to CD3 of a cynomolgus monkey or a rhesus monkey.

**30.** The bispecific antibody according to claim 29, wherein the second single-chain Fv comprises a VH domain comprising HCDR1, HCDR2, and HCDR3 as shown in SEQ ID NOs: 241, 242, and 243, respectively or having sequences that are at least 80%, 85%, 90%, 92%, 95%, 97%, 98%, 99% or more similar to or have one or more amino acid substitutions than SEQ ID NOs: 241, 242, and 243; and a VL domain comprising LCDR1, LCDR2, and LCDR3 as shown in SEQ ID NOs: 244, 245, and 246, respectively or having sequences that are at least 80%, 85%, 90%, 92%, 95%, 97%, 98%, 99% or more similar to or have one or more amino acid substitutions than SEQ ID NOs: 244, 245, and 246.

**31.** The bispecific antibody according to claim 29, wherein the second single-chain Fv comprises a VH domain comprising HCDR1, HCDR2, and HCDR3 as shown in SEQ ID NOs: 249, 250, and 251, respectively or having sequences that are at least 80%, 85%, 90%, 92%, 95%, 97%, 98%, 99% or more similar to or have one or more amino acid substitutions than SEQ ID NOs: 249, 250, and 251; and a VL domain comprising LCDR1, LCDR2, and LCDR3 as shown in SEQ ID NOs: 252, 253, and 254, respectively or having sequences that are at least 80%, 85%, 90%, 92%, 95%, 97%, 98%, 99% or more similar to or have one or more amino acid substitutions than SEQ ID NOs: 252, 253, and 254.

**32.** The bispecific antibody according to claim 30, wherein the second single-chain Fv specifically binds to CD3; the VH domain of the second single-chain Fv comprises an amino acid sequence as shown in SEQ ID NO: 247 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 247; and the VL domain of the second single-chain Fv comprises an amino acid sequence as shown in SEQ ID NO: 248 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 248.

**33.** The bispecific antibody according to claim 31, wherein the second single-chain Fv specifically binds to CD3; the VH domain of the second single-chain Fv comprises an amino acid sequence as shown in SEQ ID NO: 255 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 255; and the VL domain of the second single-chain Fv comprises an amino acid sequence as shown in SEQ ID NO: 256 or having a sequence that is substantially identical to (for example, is at least 80%, 85%, 90%, 92%, 95%, 97%, 98%, 99% or more similar to or has one or more amino acid substitutions (for example, conservative substitutions) than) SEQ ID NO: 256.

**34.** The bispecific antibody according to claim 31, wherein the linker peptide that links the first single-chain Fv to the second single-chain Fv consists of a flexible peptide and a rigid peptide; wherein the flexible peptide comprises two or more amino acids, and preferably selected from the following amino acids: Gly(G), Ser(S), Ala(A), and Thr(T); more preferably, the flexible peptide comprises G and S residues; most preferably, an amino acid composition structure of the flexible peptide has a general formula of $G_xS_y(GGGGS)_z$, wherein x, y, and z are integers greater than or equal to 0, and $x + y + z \geq 1$; the rigid peptide is derived from a full-length sequence consisting of amino acids at positions 118 to 145 at carboxyl terminus of the natural human chorionic gonadotropin beta-subunit, or a truncated fragment thereof; preferably, the rigid peptide comprises SSSSKAPPPS.

**35.** The bispecific antibody according to claim 34, wherein the linker peptide comprises an amino acid sequence as shown in SEQ ID NO: 258.

**36.** The bispecific antibody according to claim 1, wherein the linker peptide that links the Fc fragment to the second single-chain Fv comprises 1 to 20 amino acids, and preferably selected from the following amino acids: Gly(G), Ser(S), Ala(A), and Thr(T); more preferably Gly(G) and Ser(S); further preferably, the linker peptide consists of $(GGGGS)_n$, wherein n = 1, 2, 3 or 4.

**37.** The bispecific antibody according to claim 1 or 36, wherein the Fc fragment comprises a hinge region, a CH2 domain, and a CH3 domain from a human immunoglobulin heavy chain constant region; preferably, the Fc fragment is selected from heavy chain constant regions of human IgG1, IgG2, IgG3, IgG4, IgM, IgA1, IgA2, IgD, and IgE; more preferably, the Fc fragment is selected from heavy chain constant regions of human IgG1, IgG2, IgG3, and IgG4; further preferably, the Fc fragment is selected from heavy chain constant region of human IgG1 or IgG4; and the Fc fragment has one or more amino acid substitutions, deletions or additions than a natural sequence from which the Fc fragment is derived.

**38.** The bispecific antibody according to claim 37, wherein the Fc fragment comprises amino acid substitutions, deletions or additions with reduced or eliminated effector functions (ADCP, ADCC, and CDC effects).

**39.** The bispecific antibody according to claim 38, wherein the Fc fragment comprises amino acid substitutions of L234A/L235A/P331 S that are determined according to the EU numbering system.

**40.** The bispecific antibody according to claim 38 or 39, wherein the Fc fragment further comprises amino acid substitutions, deletions or additions with one or more of the following properties:

(i) enhanced binding affinity for a neonatal receptor (FcRn);
(ii) reduced or eliminated glycosylation; and
(iii) reduced or eliminated charge heterogeneity.

**41.** The bispecific antibody according to claim 40, wherein the Fc fragment further comprises one or more of amino acid substitutions, deletions or additions as follows:

(i) amino acid substitutions M428L, T250Q/M428L, M428L/N434S or M252Y/S254T/T256E determined according to the EU numbering system;
(ii) an amino acid substitution N297A determined according to the EU numbering system; and
(iii) an amino acid deletion K447 determined according to the EU numbering system.

**42.** The bispecific antibody according to claim 40, wherein the Fc fragment has an amino acid sequence as shown in SEQ ID NO: 263 that has six amino acid substitutions or replacements L234A/L235A/N297A/P331S/T250Q/M428L determined according to the EU numbering system and a deleted or removed K447 determined according to the EU numbering system compared to the natural sequence from which the Fc fragment is derived.

**43.** The bispecific antibody according to claim 1, wherein the bispecific antibody binds to human CD19 and CD3 and has an amino acid sequence as follows:

(i) a sequence as shown in SEQ ID NO: 264;
(ii) a sequence with one or more substitutions, deletions or additions (such as 1, 2, 3, 4 or 5 substitutions, deletions or additions) compared to the sequence as shown in SEQ ID NO: 264; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 264.

**44.** The bispecific antibody according to claim 1, wherein the bispecific antibody binds to human CD19 and CD3 and has an amino acid sequence as follows:

(i) a sequence as shown in SEQ ID NO: 283;
(ii) a sequence with one or more substitutions, deletions or additions (such as 1, 2, 3, 4 or 5 substitutions, deletions or additions) compared to the sequence as shown in SEQ ID NO: 283; or

(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 283.

**45.** The bispecific antibody according to claim 1, wherein the bispecific antibody binds to human CD20 and CD3 and has an amino acid sequence as follows:

(i) a sequence as shown in SEQ ID NO: 266;
(ii) a sequence with one or more substitutions, deletions or additions (such as 1, 2, 3, 4 or 5 substitutions, deletions or additions) compared to the sequence as shown in SEQ ID NO: 266; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 266.

**46.** The bispecific antibody according to claim 1, wherein the bispecific antibody binds to human CD22 and CD3 and has an amino acid sequence as follows:

(i) a sequence as shown in SEQ ID NO: 268;
(ii) a sequence with one or more substitutions, deletions or additions (such as 1, 2, 3, 4 or 5 substitutions, deletions or additions) compared to the sequence as shown in SEQ ID NO: 268; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 268.

**47.** The bispecific antibody according to claim 1, wherein the bispecific antibody binds to human CD30 and CD3 and has an amino acid sequence as follows:

(i) a sequence as shown in SEQ ID NO: 270;
(ii) a sequence with one or more substitutions, deletions or additions (such as 1, 2, 3, 4 or 5 substitutions, deletions or additions) compared to the sequence as shown in SEQ ID NO: 270; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 270.

**48.** The bispecific antibody according to claim 1, wherein the bispecific antibody binds to human EpCAM and CD3 and has an amino acid sequence as follows:

(i) a sequence as shown in SEQ ID NO: 272;
(ii) a sequence with one or more substitutions, deletions or additions (such as 1, 2, 3, 4 or 5 substitutions, deletions or additions) compared to the sequence as shown in SEQ ID NO: 272; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 272.

**49.** The bispecific antibody according to claim 1, wherein the bispecific antibody binds to human CEA and CD3 and has an amino acid sequence as follows:

(i) a sequence as shown in SEQ ID NO: 274;
(ii) a sequence with one or more substitutions, deletions or additions (such as 1, 2, 3, 4 or 5 substitutions, deletions or additions) compared to the sequence as shown in SEQ ID NO: 274; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 274.

**50.** The bispecific antibody according to claim 1, wherein the bispecific antibody binds to human Her2 and CD3 and has an amino acid sequence as follows:

(i) a sequence as shown in SEQ ID NO: 8;

(ii) a sequence with one or more substitutions, deletions or additions (such as 1, 2, 3, 4 or 5 substitutions, deletions or additions) compared to the sequence as shown in SEQ ID NO: 8; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 8.

**51.** The bispecific antibody according to claim 1, wherein the bispecific antibody binds to human EGFR and CD3 and has an amino acid sequence as follows:

(i) a sequence as shown in SEQ ID NO: 277;
(ii) a sequence with one or more substitutions, deletions or additions (such as 1, 2, 3, 4 or 5 substitutions, deletions or additions) compared to the sequence as shown in SEQ ID NO: 277; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 277.

**52.** The bispecific antibody according to claim 1, wherein the bispecific antibody binds to human GPC-3 and CD3 and has an amino acid sequence as follows:

(i) a sequence as shown in SEQ ID NO: 279;
(ii) a sequence with one or more substitutions, deletions or additions (such as 1, 2, 3, 4 or 5 substitutions, deletions or additions) compared to the sequence as shown in SEQ ID NO: 279; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 279.

**53.** The bispecific antibody according to claim 1, wherein the bispecific antibody binds to human Mesothelin and CD3 and has an amino acid sequence as follows:

(i) a sequence as shown in SEQ ID NO: 281;
(ii) a sequence with one or more substitutions, deletions or additions (such as 1, 2, 3, 4 or 5 substitutions, deletions or additions) compared to the sequence as shown in SEQ ID NO: 281; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 281.

**54.** The bispecific antibody according to claim 1, wherein the bispecific antibody binds to human Mucin1 and CD3 and has an amino acid sequence as follows:

(i) a sequence as shown in SEQ ID NO: 285;
(ii) a sequence with one or more substitutions, deletions or additions (such as 1, 2, 3, 4 or 5 substitutions, deletions or additions) compared to the sequence as shown in SEQ ID NO: 285; or
(iii) a sequence with at least 80%, at least 85%, at least 90%, at least 91%, at least 92%, at least 93%, at least 94%, at least 95%, at least 96%, at least 97%, at least 98%, at least 99% or 100% sequence identity to the sequence as shown in SEQ ID NO: 285.

**55.** A DNA molecule encoding the bispecific antibody according to any one of claims 1 to 54.

**56.** The DNA molecule according to claim 55, wherein the DNA molecule has a nucleotide sequence as shown in SEQ ID NO: 265, 267, 269, 271, 273, 275, 276, 278, 280, 282, 284 or 286.

**57.** A vector, comprising the DNA molecule according to claim 55 or 56.

**58.** A host cell, comprising the vector according to claim 57, wherein the host cell comprises a prokaryotic cell, a yeast or a mammalian cell, preferably a CHO cell.

**59.** A pharmaceutical composition, comprising the bispecific antibody according to any one of claims 1 to 54 and a pharmaceutically acceptable excipient, carrier or diluent.

**60.** A method for preparing the bispecific antibody according to any one of claims 1 to 54, comprising: (a) obtaining a fusion gene of the bispecific antibody to construct an expression vector of the bispecific antibody; (b) transfecting the expression vector into a host cell by a genetic engineering method; (c) culturing the host cell under conditions that allow the bispecific antibody to be generated; and (d) separating and purifying the generated bispecific antibody;

wherein the expression vector in step (a) is one or more selected from plasmids, bacteria, and viruses, and preferably the expression vector is a pCDNA3.4 vector;
wherein the host cell into which the constructed vector is transfected by the genetic engineering method in step (b) comprises a prokaryotic cell, a yeast or a mammalian cell, such as a CHO cell, an NS0 cell or another mammalian cell, preferably a CHO cell; and
wherein the bispecific antibody is separated and purified in step (d) by a conventional immunoglobulin purification method comprising protein A affinity chromatography and ion exchange, hydrophobic chromatography or molecular sieve.

**61.** Use of the bispecific antibody according to any one of claims 1 to 54 in the preparation of a medicament for the treatment, prevention or alleviation of cancer/tumor, wherein examples of the cancer comprise, but are not limited to, mesothelioma, squamous cell carcinoma, myeloma, osteosarcoma, glioblastoma, neuroglioma, malignant epithelial tumours, adenocarcinoma, melanoma, sarcoma, acute and chronic leukemia, lymphoma and meningioma, Hodgkin’s disease, Sezary syndrome, multiple myeloma, lung cancer, non-small cell lung cancer, small cell lung cancer, laryngeal cancer, breast cancer, head and neck cancer, bladder cancer, uterine cancer, skin cancer, prostate cancer, cervical cancer, vaginal cancer, gastric cancer, renal cell carcinoma, renal carcinoma, pancreatic cancer, colorectal cancer, endometrial carcinoma, esophageal carcinoma, hepatobiliary cancer, bone cancer, skin cancer and blood cancer, and carcinoma of nasal cavity and sinus, nasopharyngeal carcinoma, oral cancer, oropharyngeal cancer, laryngeal cancer, sublaryngeal cancer, salivary cancer, mediastinal cancer, stomach cancer, small intestine cancer, colon cancer, cancer of rectum and anal regions, ureter cancer, urethral cancer, penile cancer, testicular cancer, vulva cancer, cancer of endocrine system, cancer of central nervous system, and plasmocytoma.

**62.** The bispecific antibody according to any one of claims 1 to 54 for use in a method for enhancing or stimulating an immune response or function, comprising administering to a patient/subject individual a therapeutically effective amount of the bispecific antibody.

**63.** The bispecific antibody according to any one of claims 1 to 54 for use in a method for treating, delaying development, or reducing/inhibiting recurrence of a tumor, comprising: giving or administering an effective amount of the bispecific antibody to an individual suffering from the foregoing disease or discorder, wherein examples of the cancer comprise, but are not limited to, mesothelioma, squamous cell carcinoma, myeloma, osteosarcoma, glioblastoma, neuroglioma, malignant epithelial tumours, adenocarcinoma, melanoma, sarcoma, acute and chronic leukemia, lymphoma and meningioma, Hodgkin’s lymphoma, Sezary syndrome, multiple myeloma, lung cancer, non-small cell lung cancer, small cell lung cancer, laryngeal cancer, breast cancer, head and neck cancer, bladder cancer, uterine cancer, skin cancer, prostate cancer, cervical cancer, vaginal cancer, gastric cancer, renal cell carcinoma, renal carcinoma, pancreatic cancer, colorectal cancer, endometrial carcinoma, esophageal carcinoma, hepatobiliary cancer, bone cancer, skin cancer and blood cancer, and carcinoma of nasal cavity and sinus, nasopharyngeal carcinoma, oral cancer, oropharyngeal cancer, laryngeal cancer, sublaryngeal cancer, salivary cancer, mediastinal cancer, cervical cancer, small intestine cancer, colon cancer, cancer of rectum and anal regions, ureter cancer, urethral cancer, penile cancer, testicular cancer, vulva cancer, cancer of endocrine system, cancer of central nervous system, and plasmocytoma.

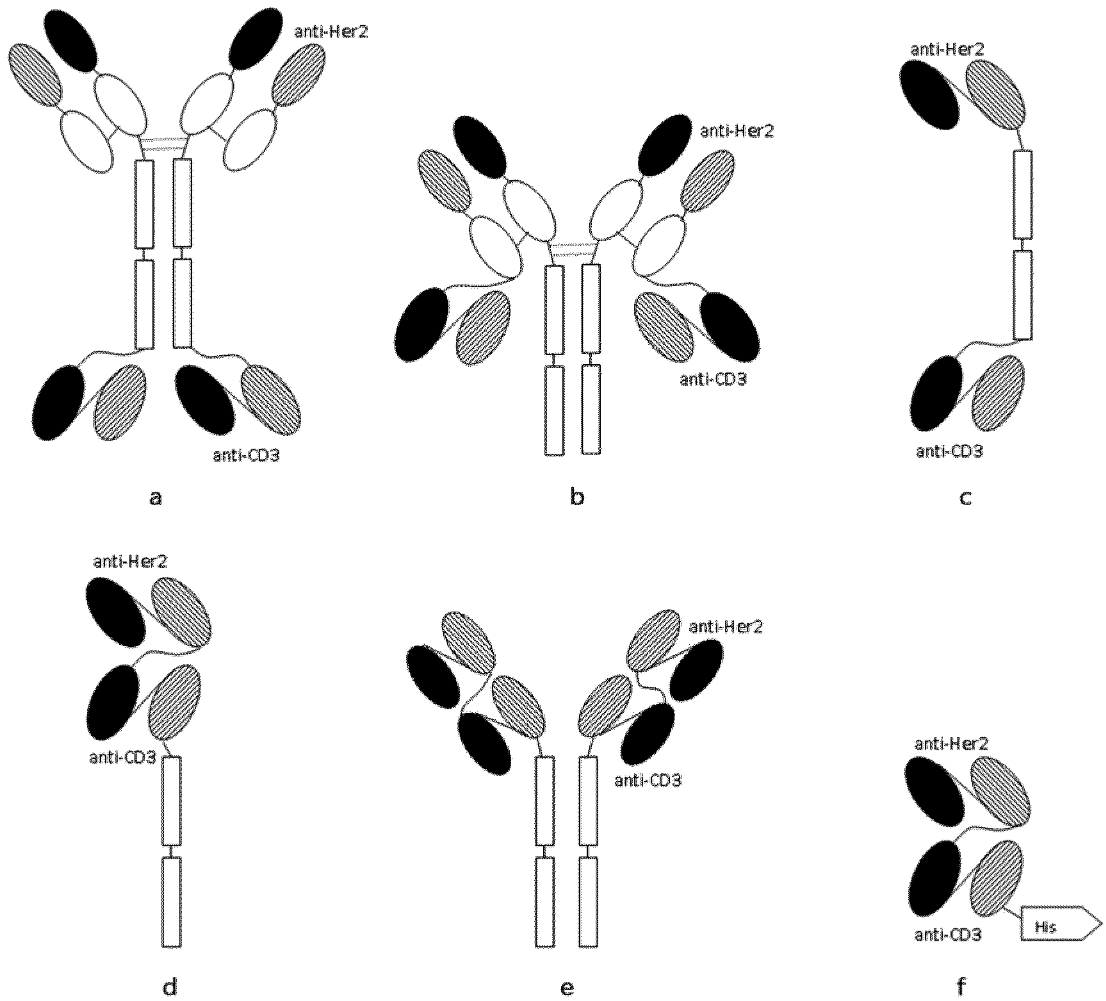
anti-Her2
anti-CD3
a
anti-Her2
anti-CD3
b
anti-Her2
anti-CD3
c
anti-Her2
anti-CD3
d
anti-Her2
anti-CD3
e
anti-Her2
anti-CD3
His
f

Figure 1-1

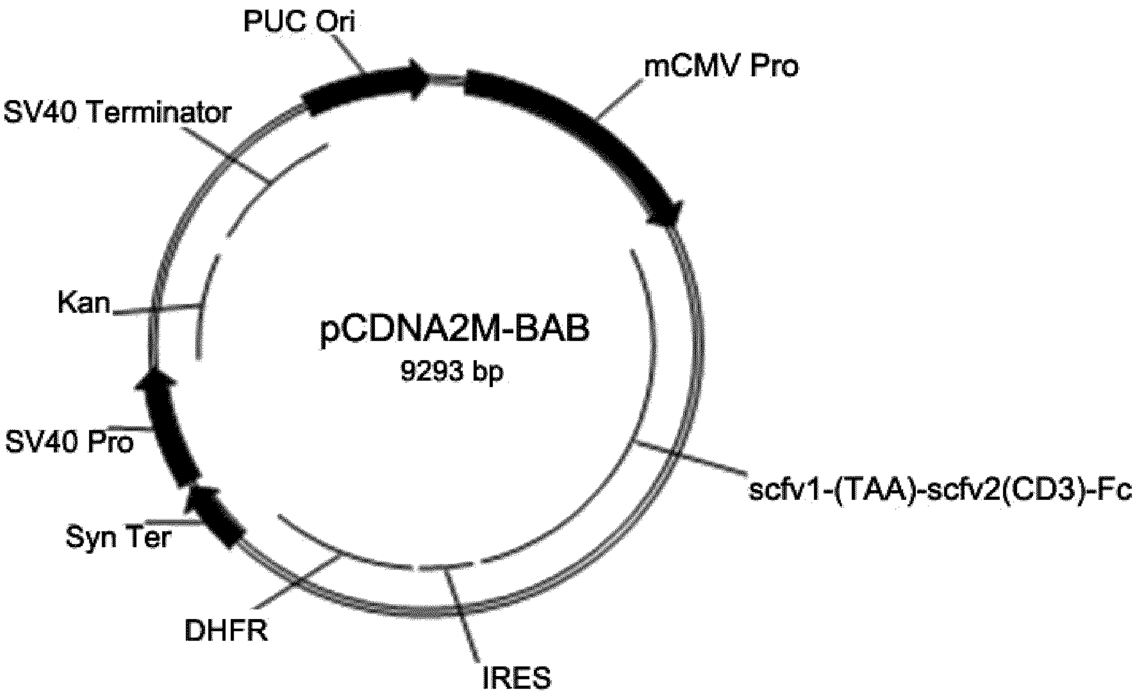


Figure 1-2

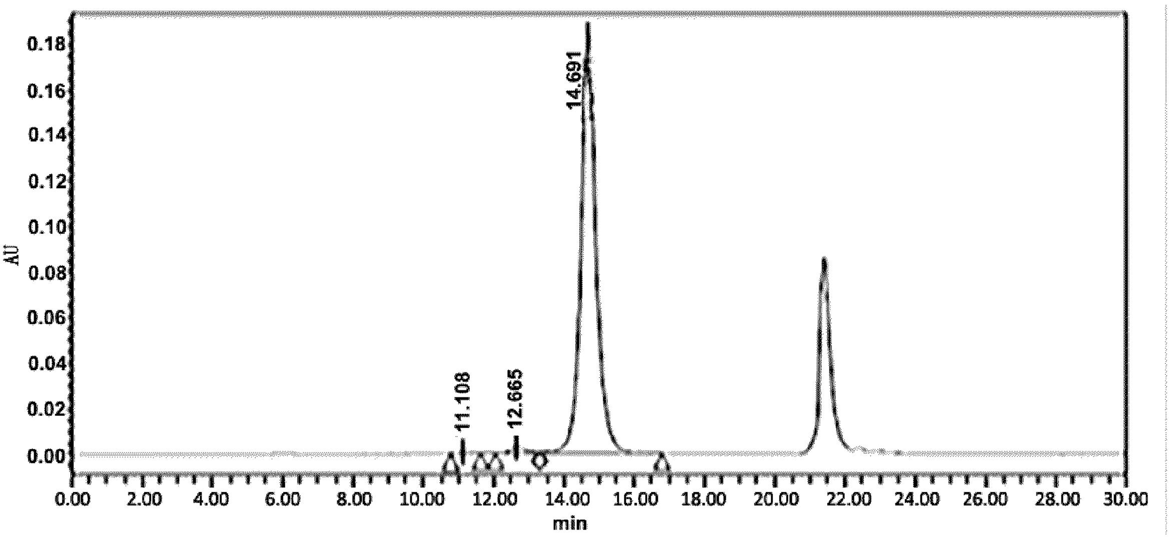


Peak results

| | Name | Retention time (min) | Start time (min) | End time (min) | Area (µV*sec) | Height (µV) | % Area | Integration | USP resolution | Symmetry factor | USP tailing | Theoretical plate number |
|---|---|---|---|---|---|---|---|---|---|---|---|---|
| 1 | | 11.108 | 10.778 | 11.628 | 10526 | 539 | 0.18 | BB | | 1.252 | 1.252 | 6507.3 |
| 2 | | 12.665 | 12.065 | 13.305 | 95949 | 2775 | 1.69 | BV | 2.108 | | | 3106.9 |
| 3 | | 14.691 | 13.305 | 16.792 | 5586292 | 183734 | 98.13 | VB | 2.350 | 1.109 | 1.109 | 6302.4 |

Figure 1-3

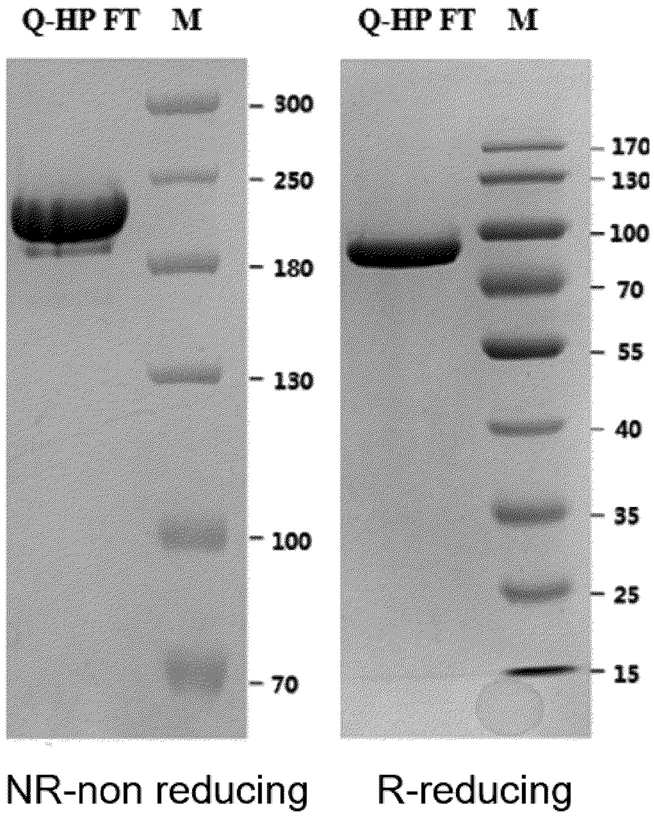


Figure 1-4

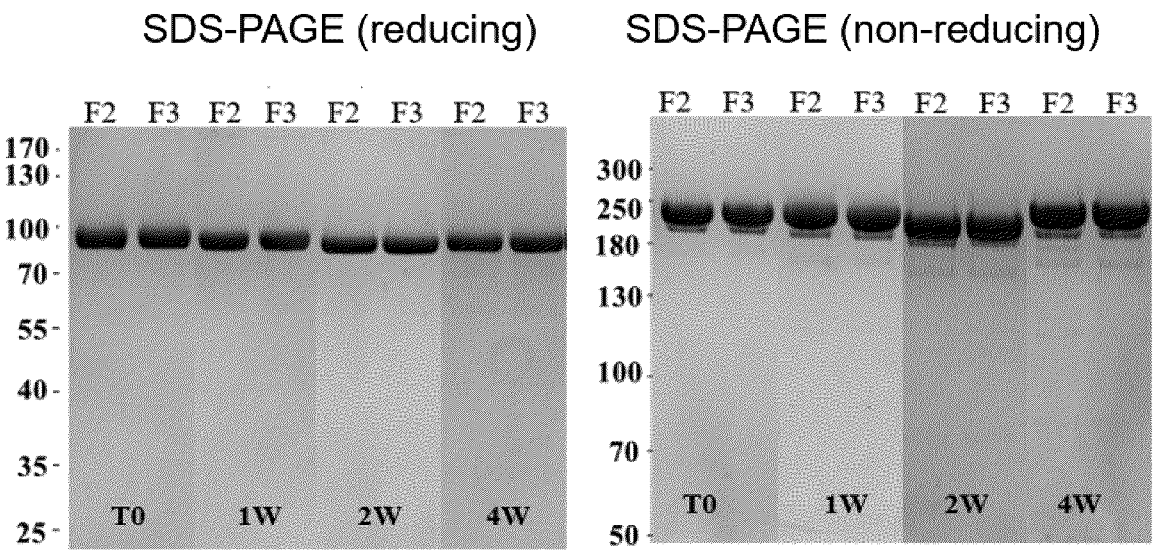


Figure 1-5

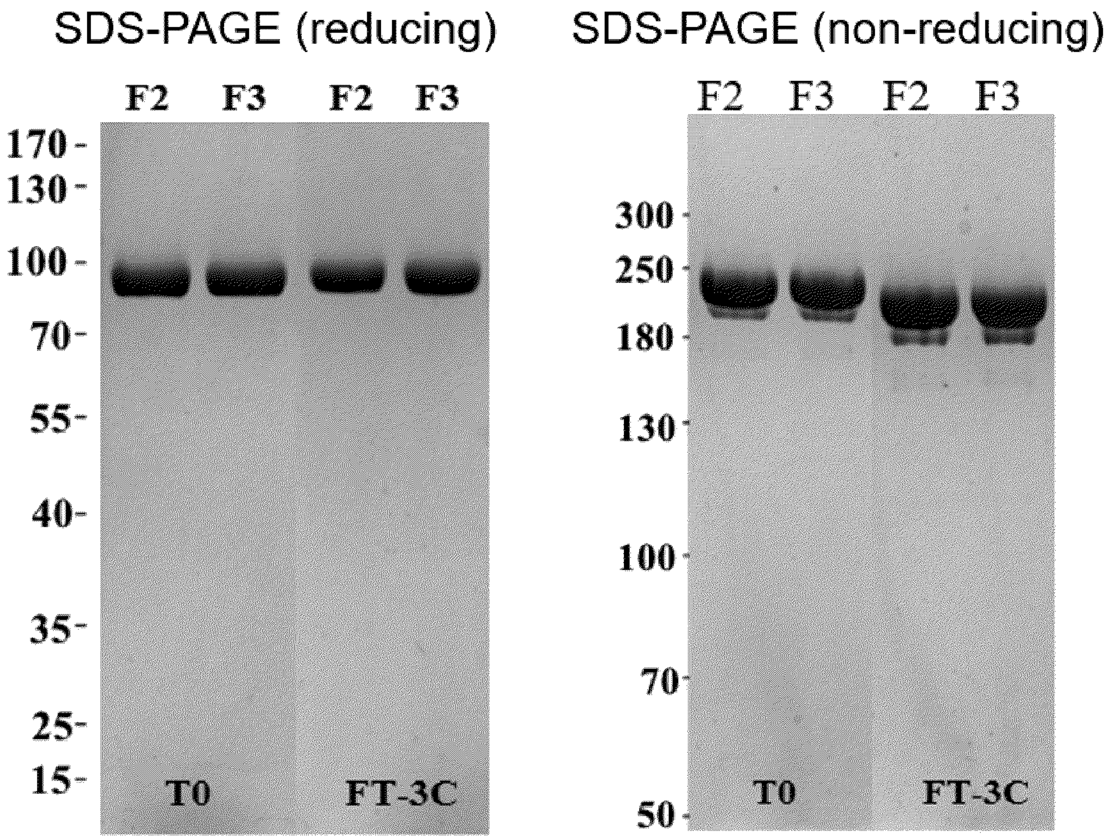


Figure 1-6

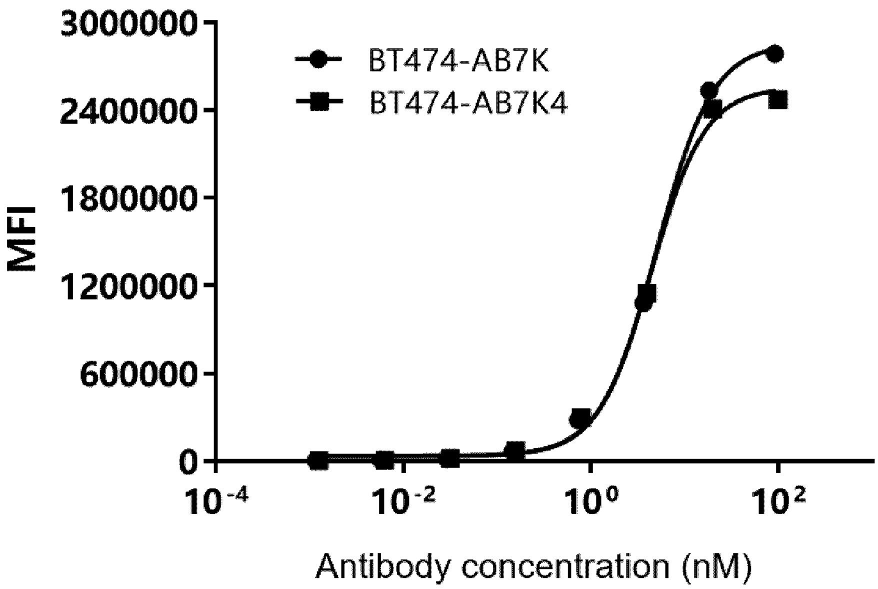


Figure 2-1

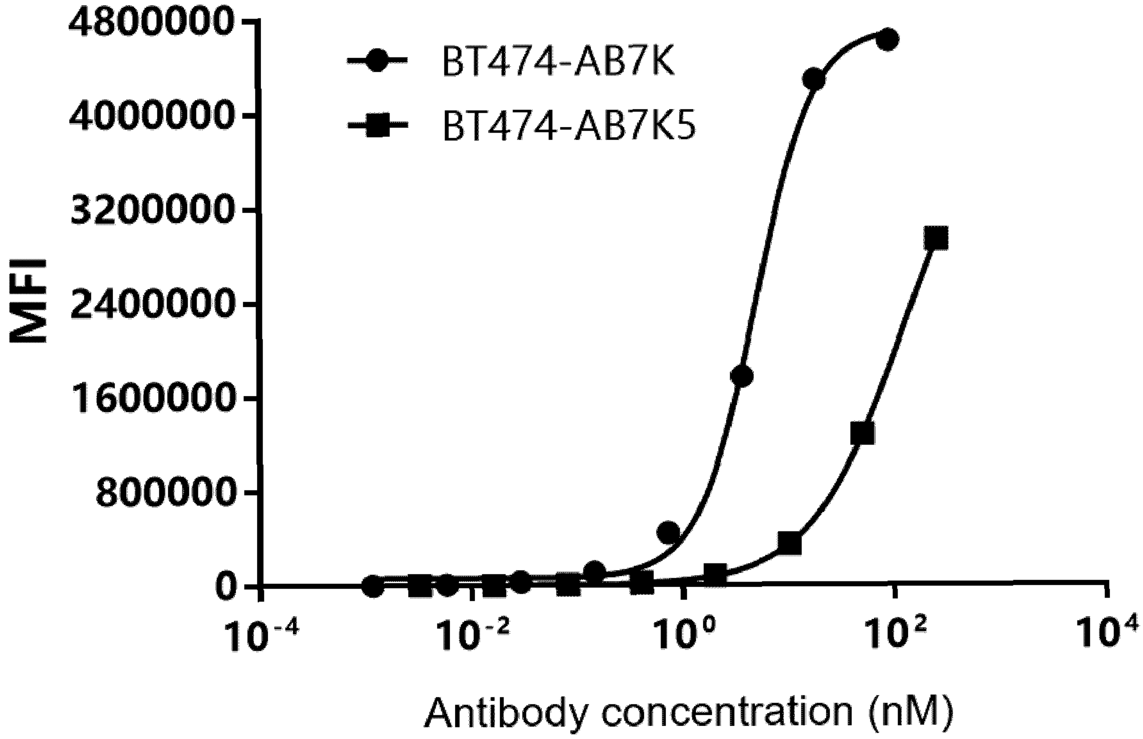


Figure 2-2

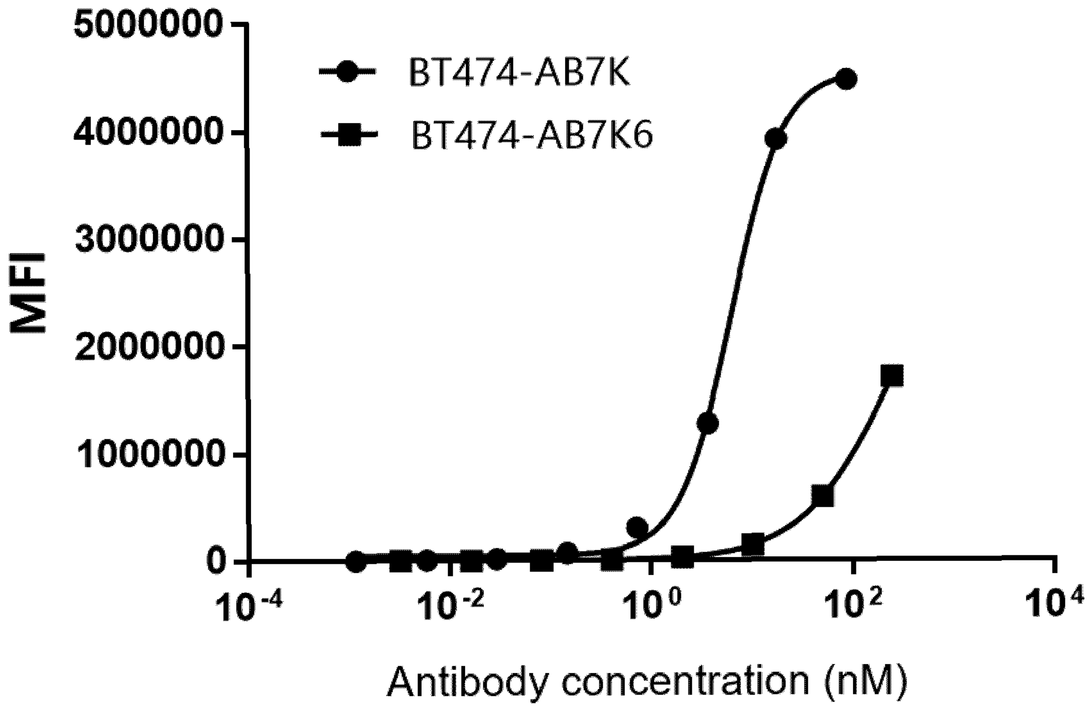


Figure 2-3

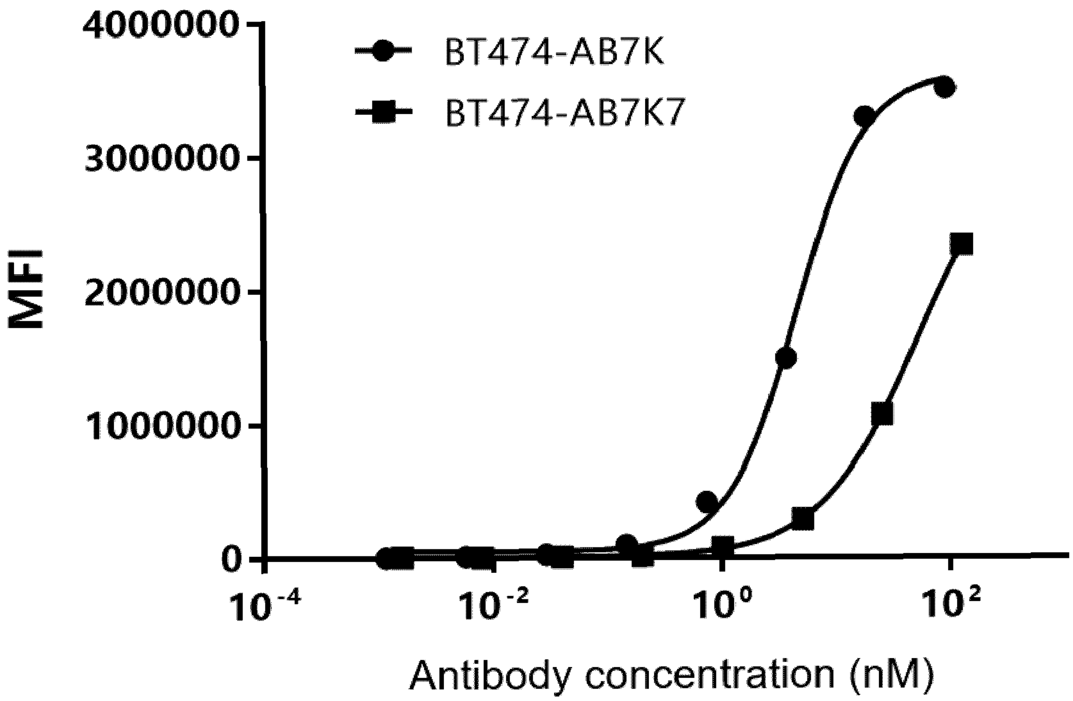


Figure 2-4

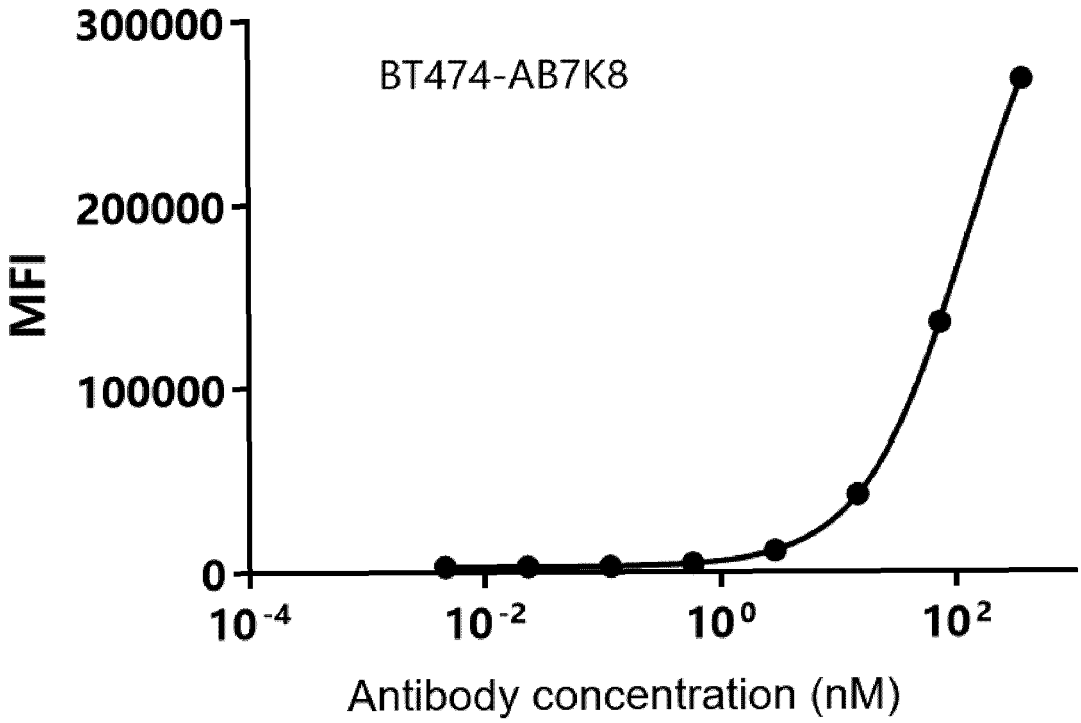


Figure 2-5

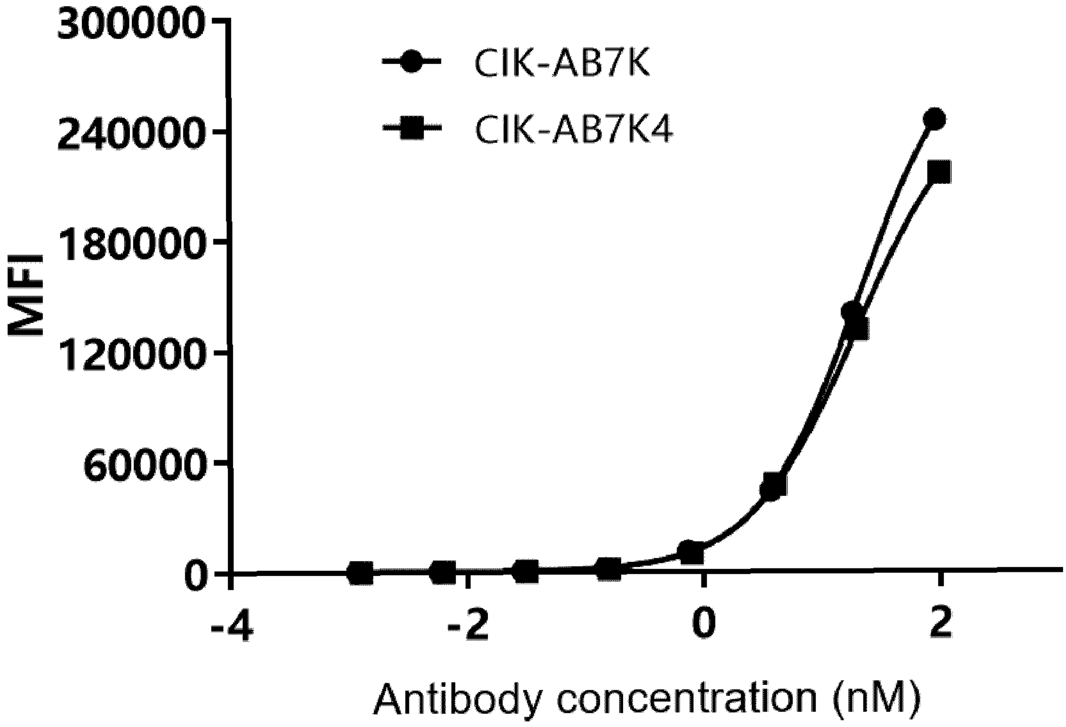


Figure 2-6

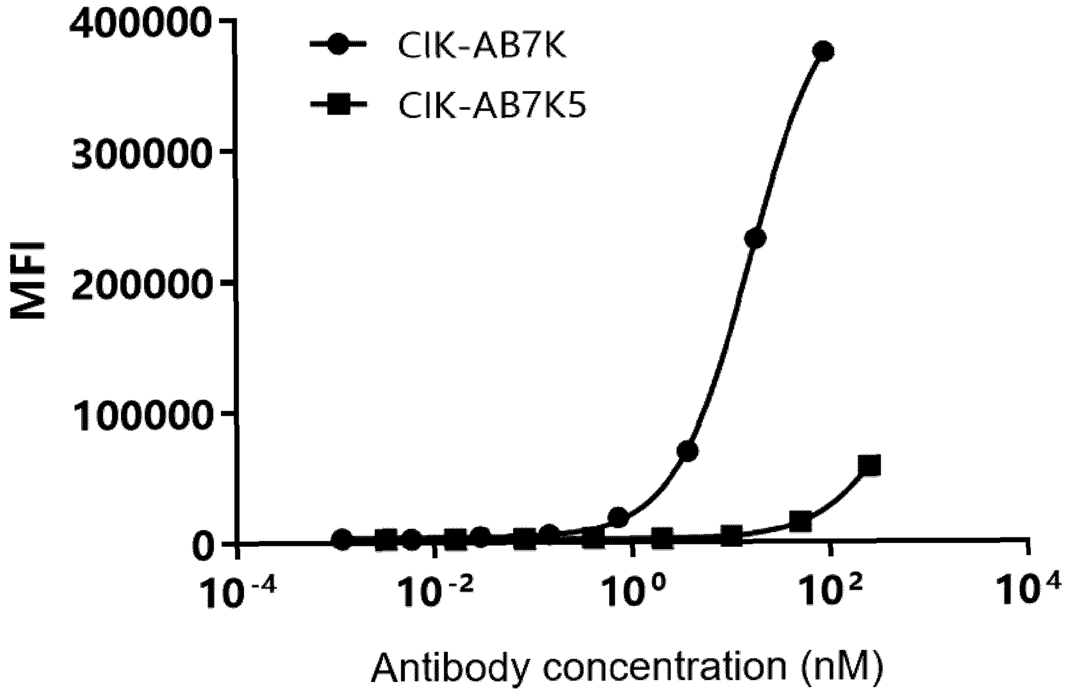


Figure 2-7

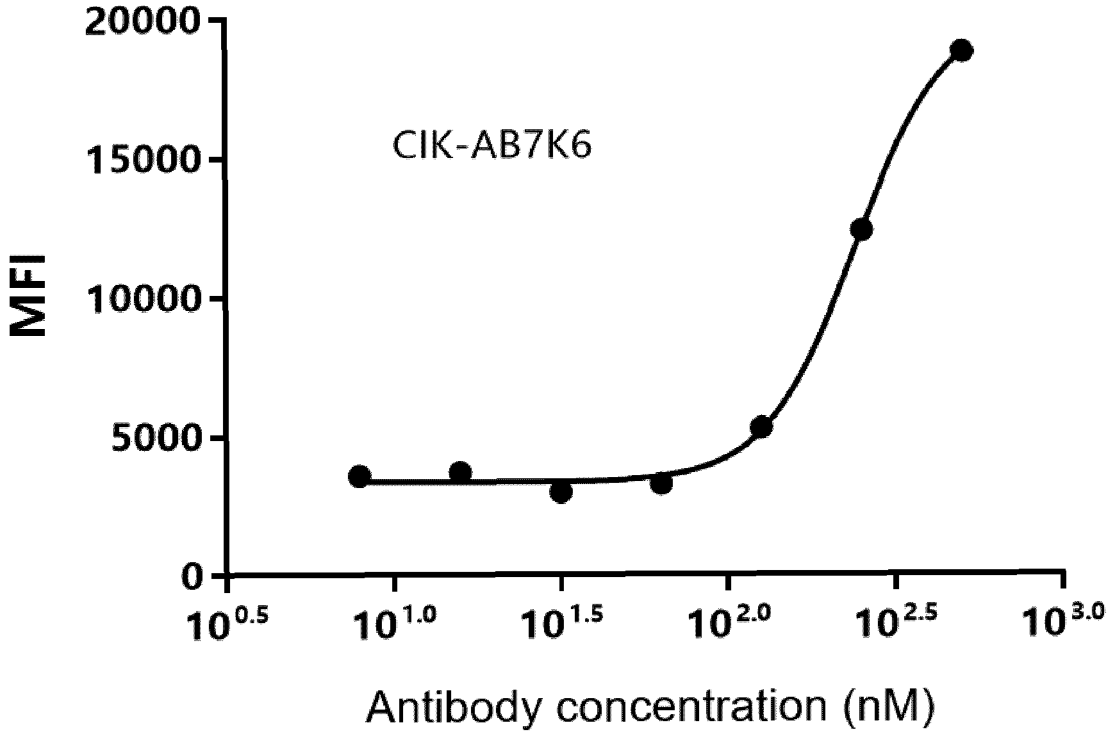


Figure 2-8

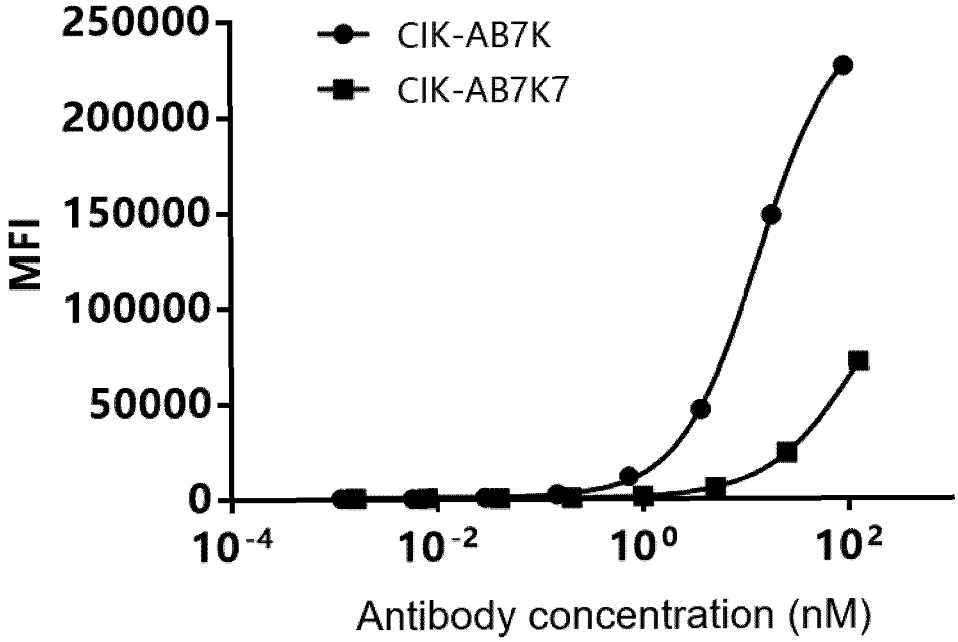


Figure 2-9

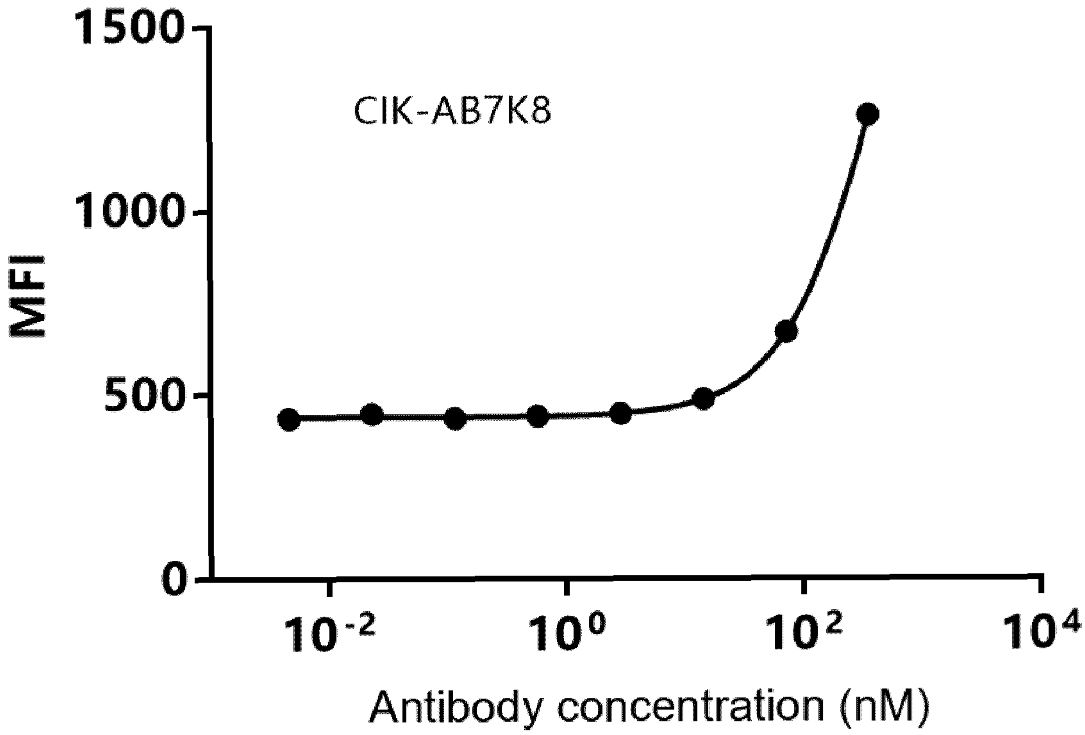


Figure 2-10

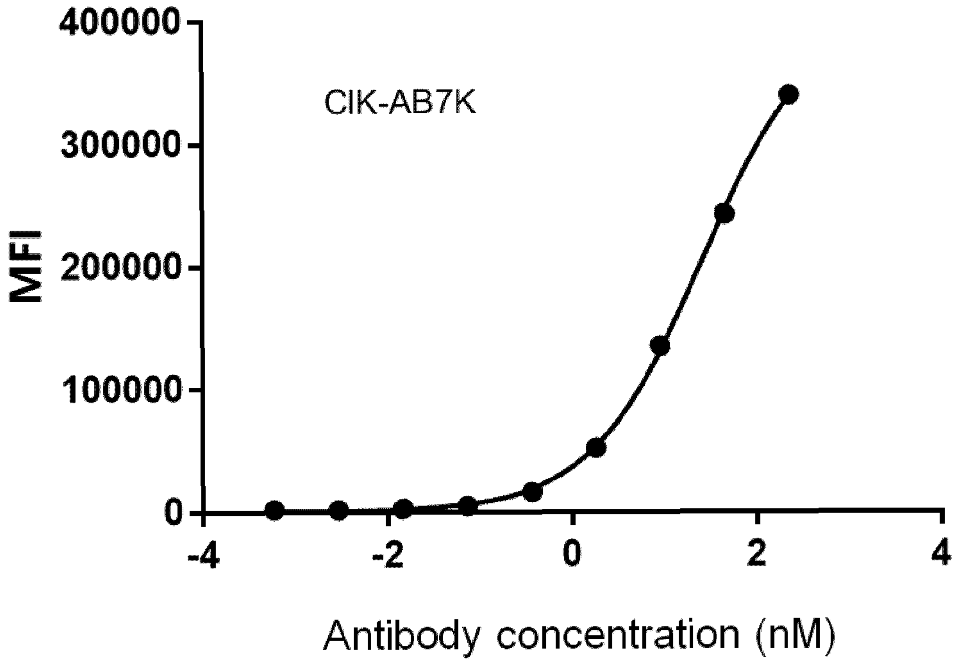


Figure 2-11

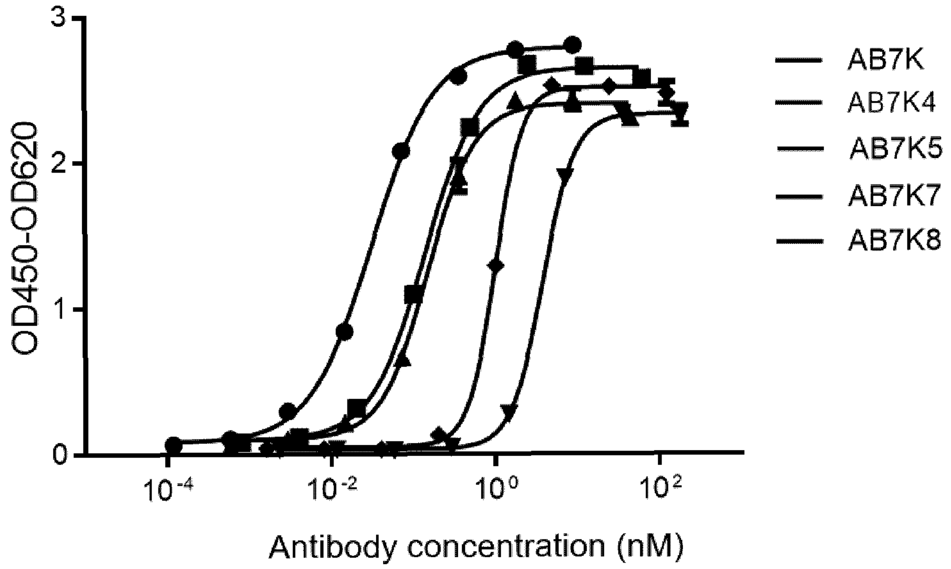


Figure 2-12

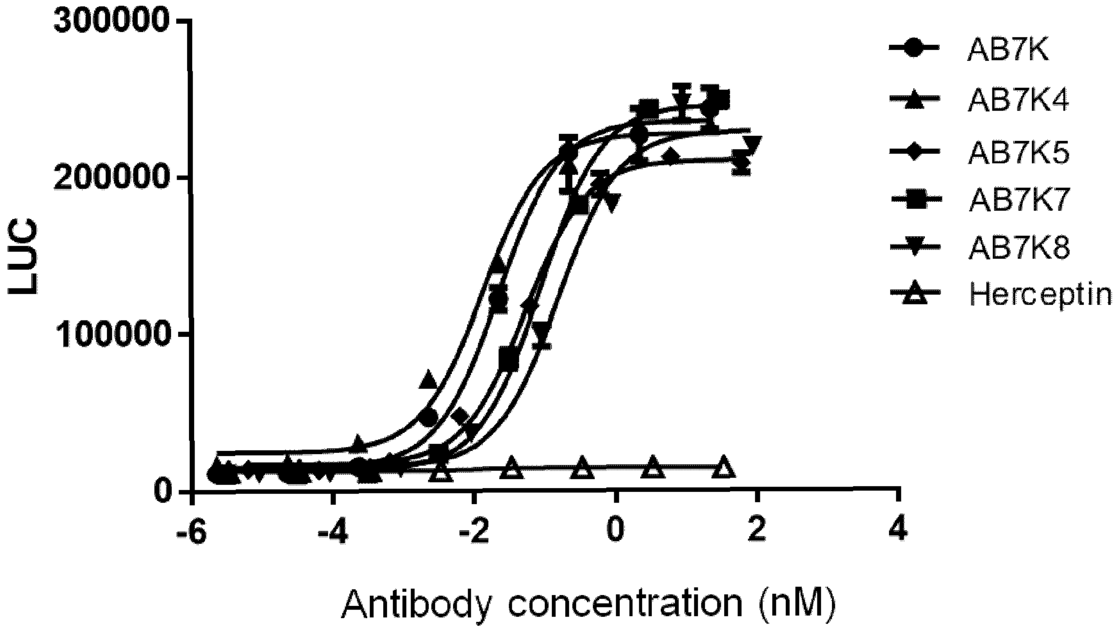


Figure 2-13

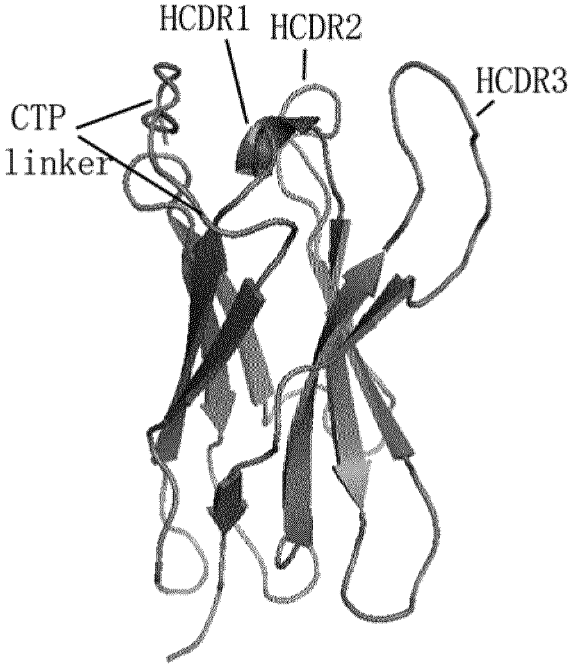


Figure 2-14

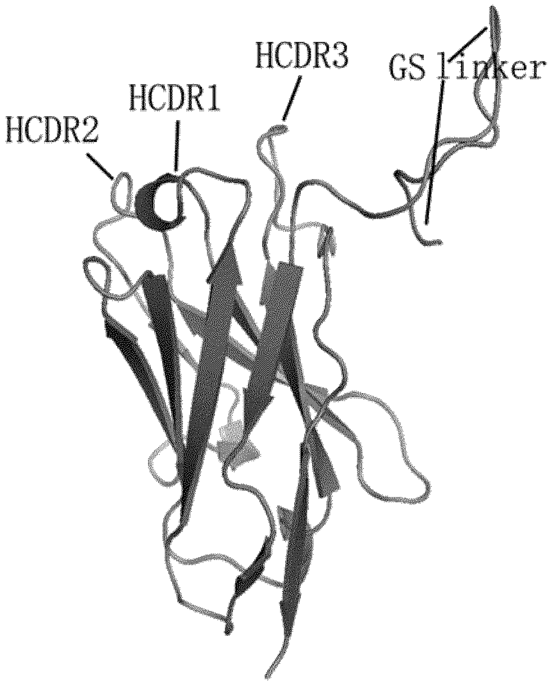


Figure 2-15

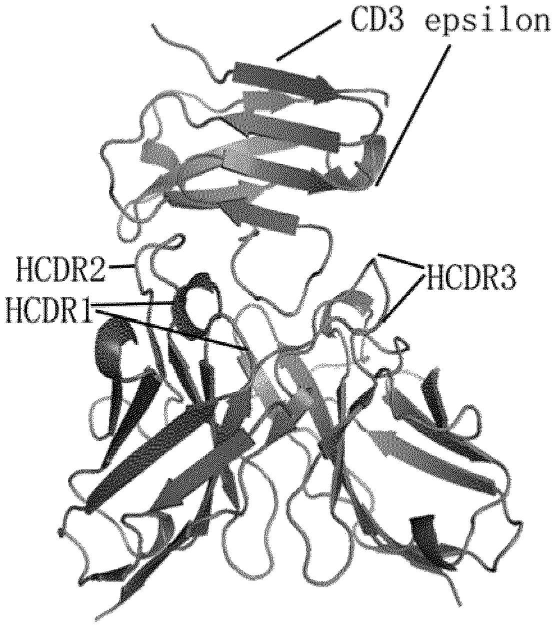


Figure 2-16

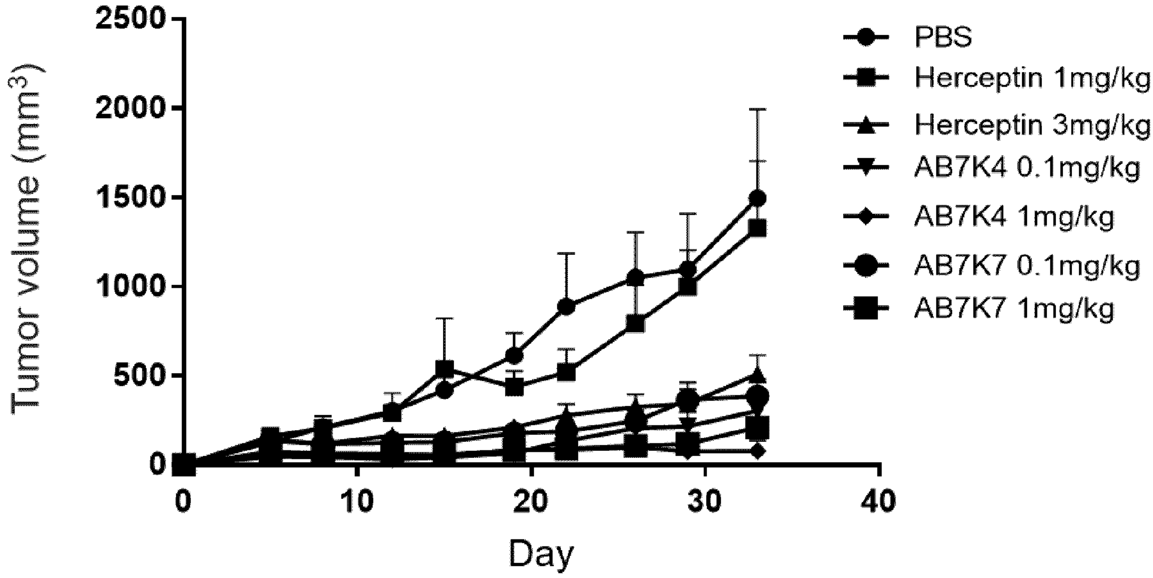


Figure 3-1

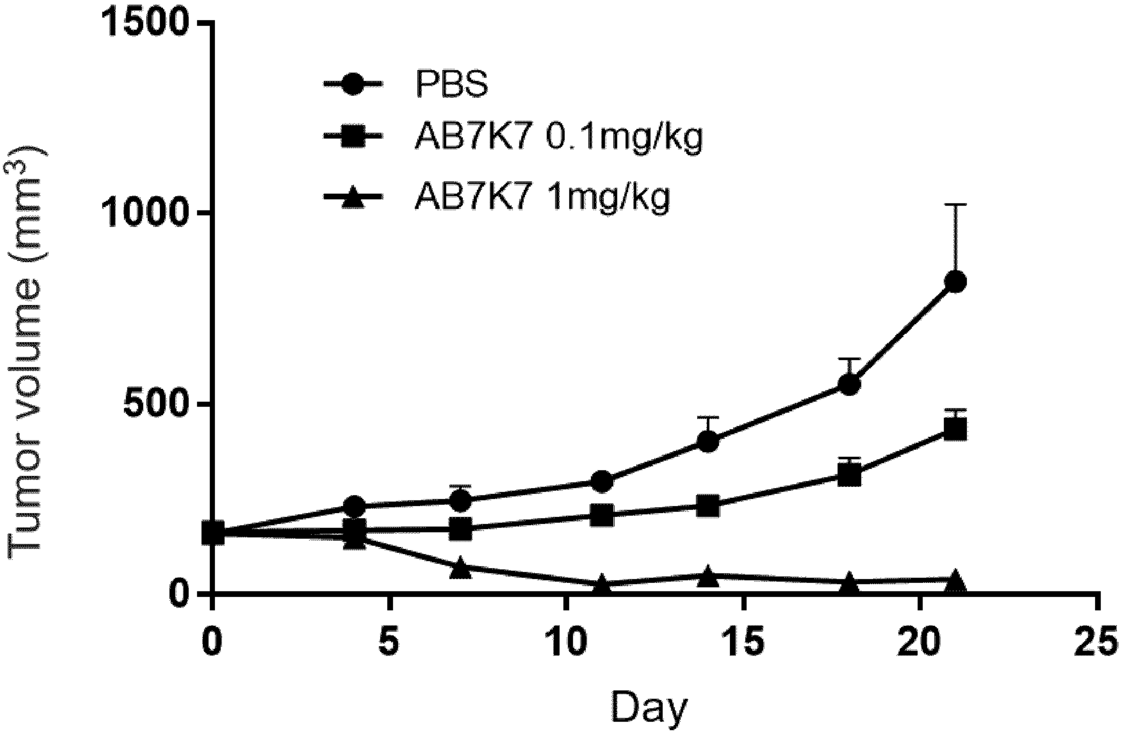


Figure 3-2

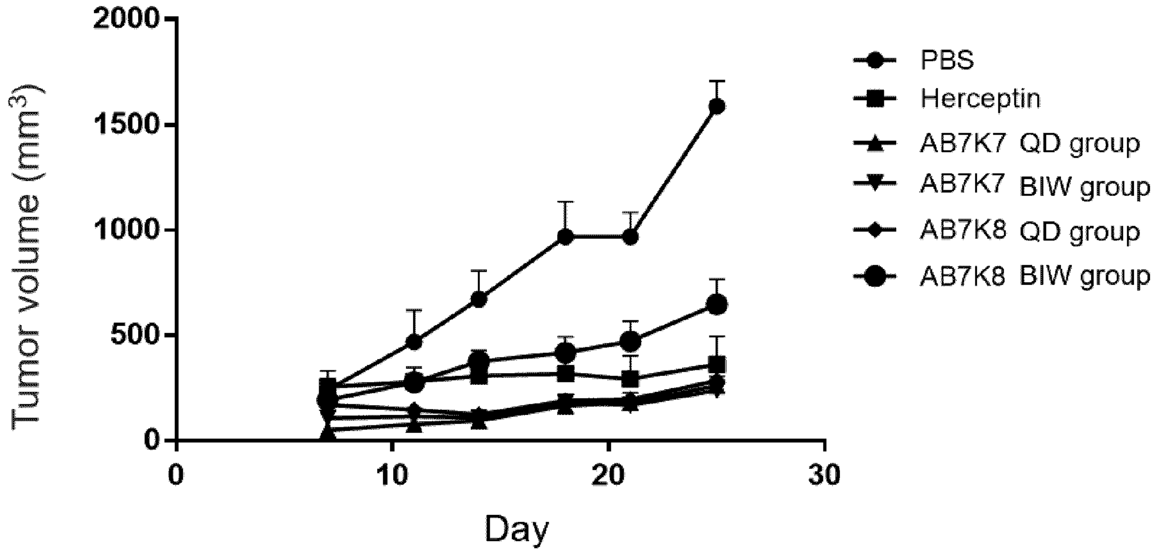


Figure 3-3

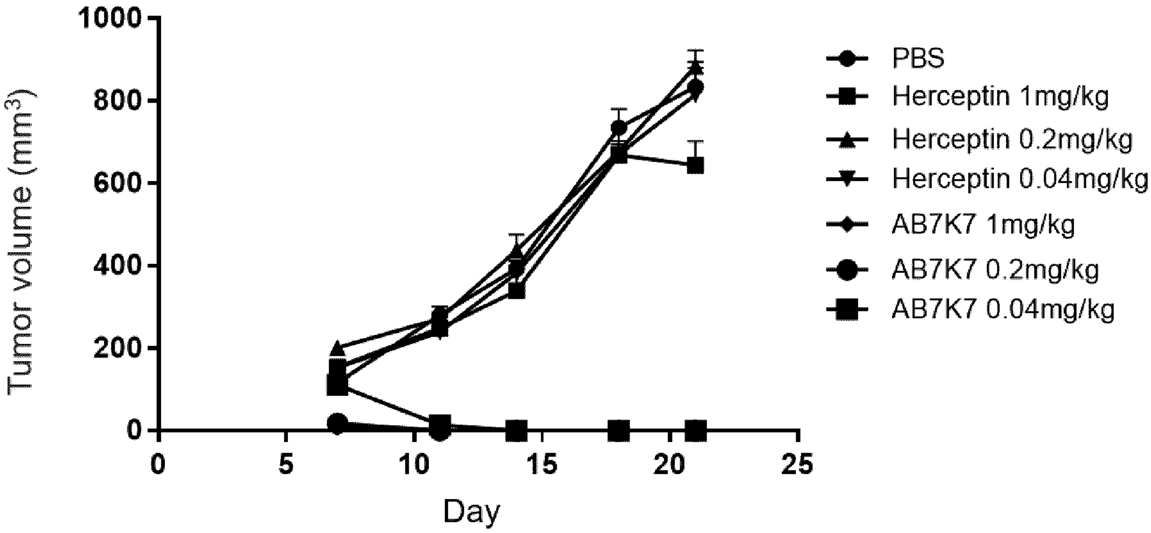


Figure 3-4

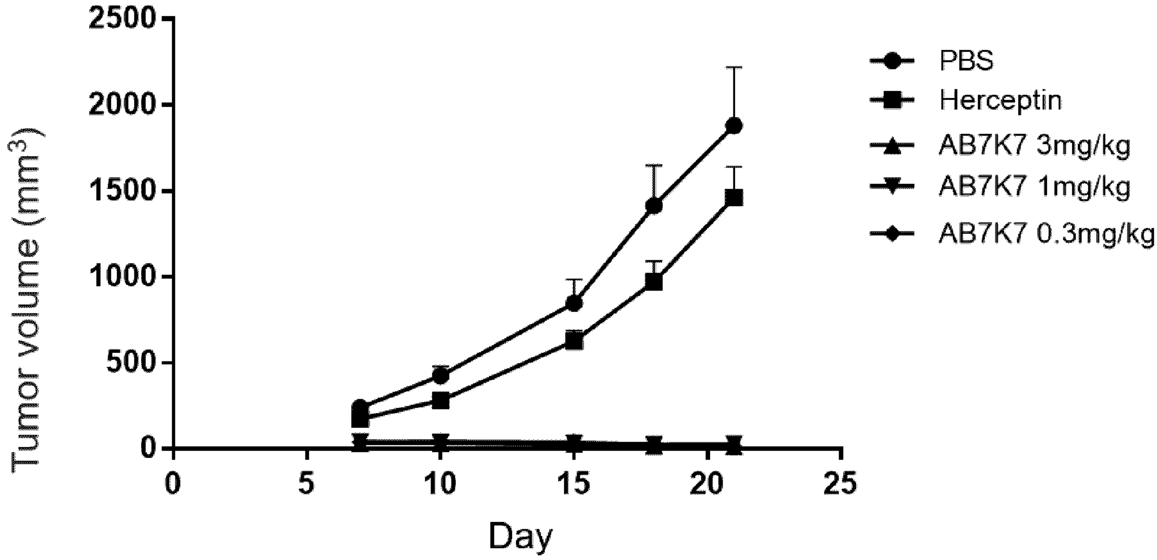


Figure 3-5

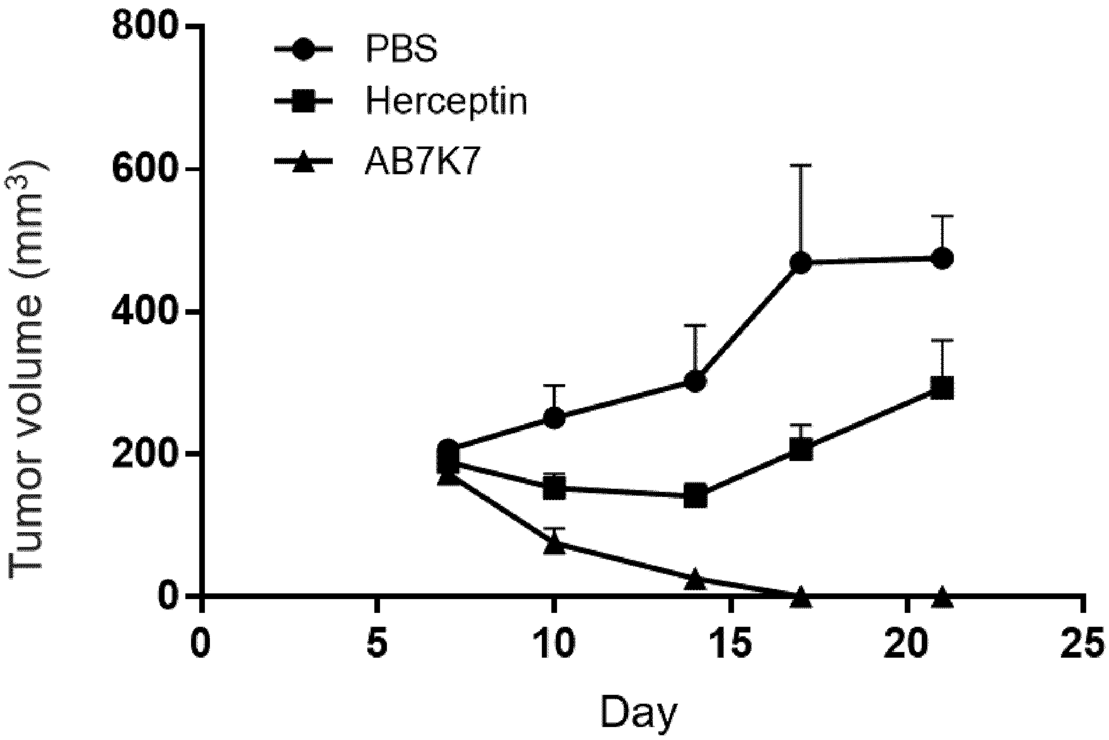


Figure 3-6

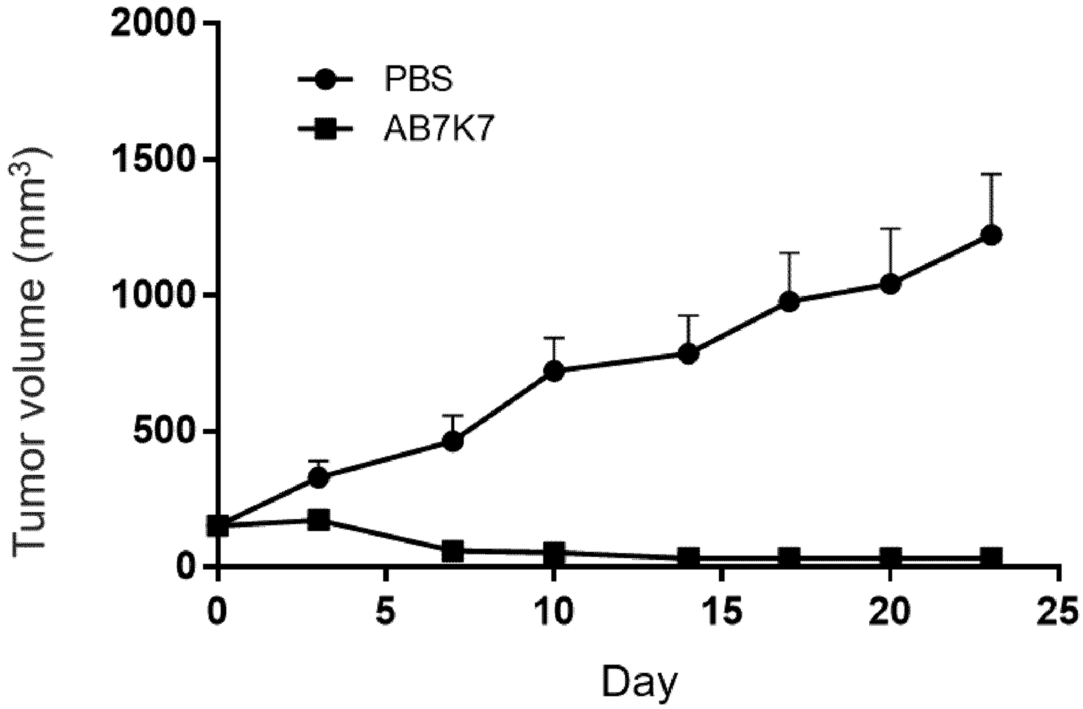


Figure 3-7

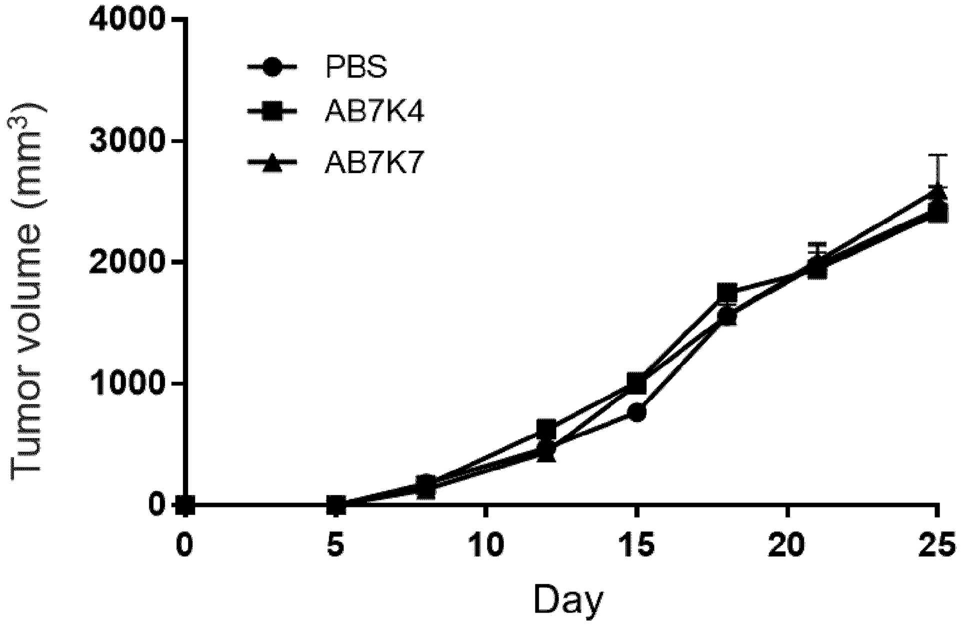


Figure 4-1

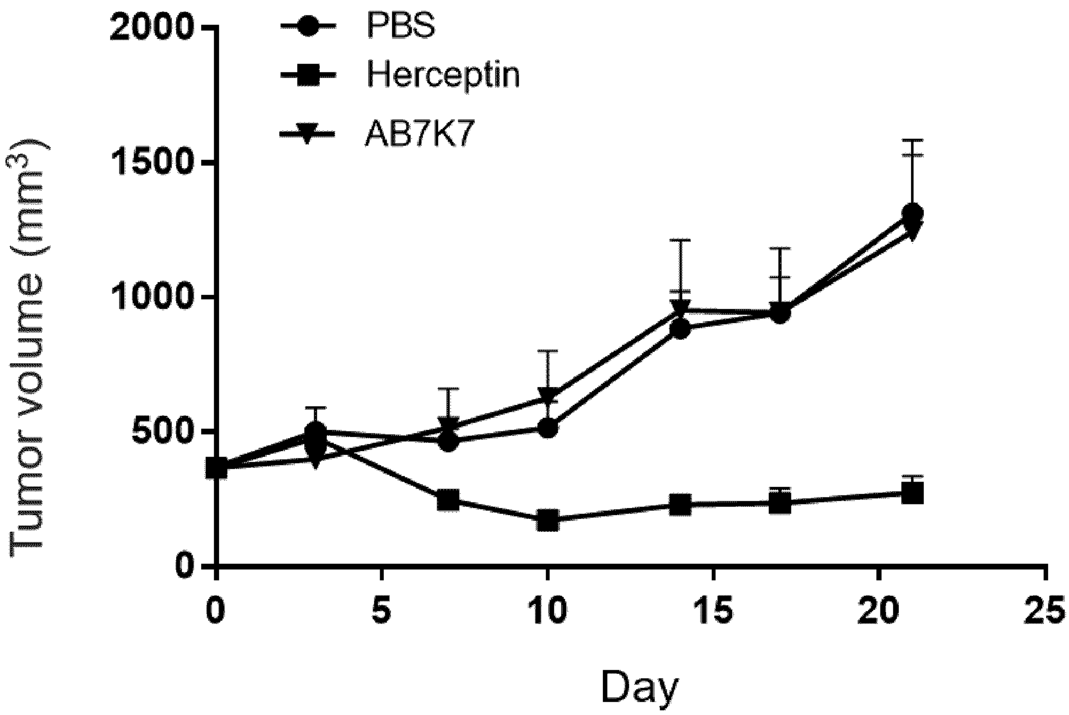


Figure 4-2

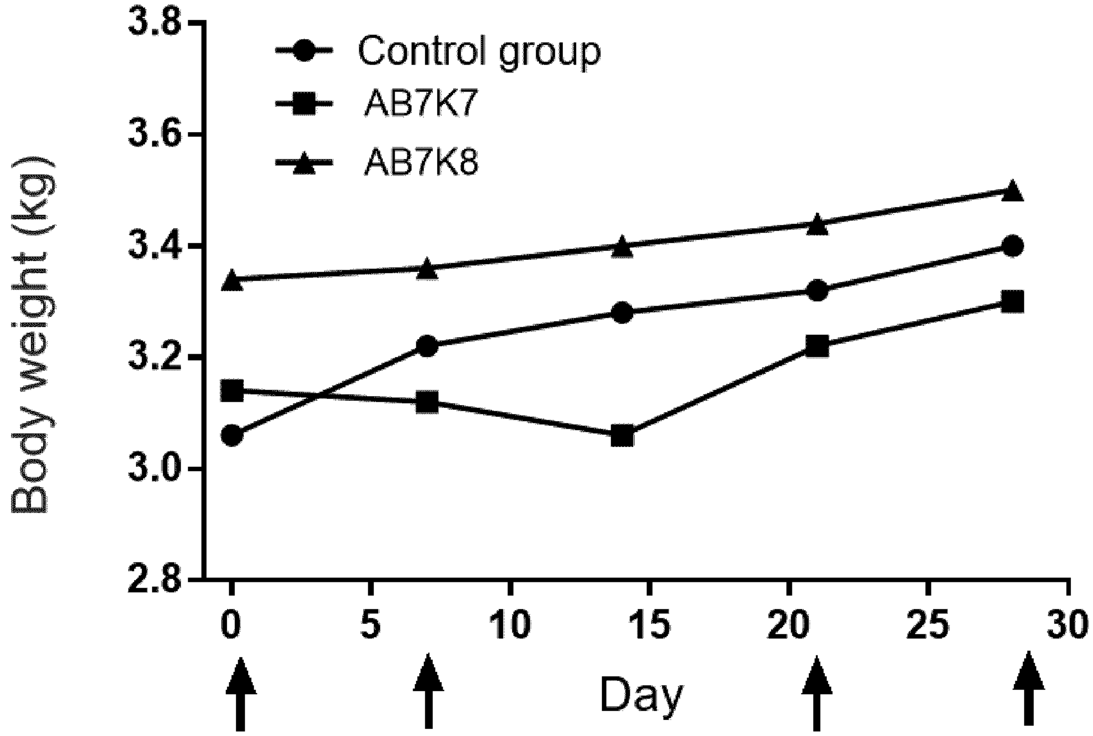


Figure 4-3

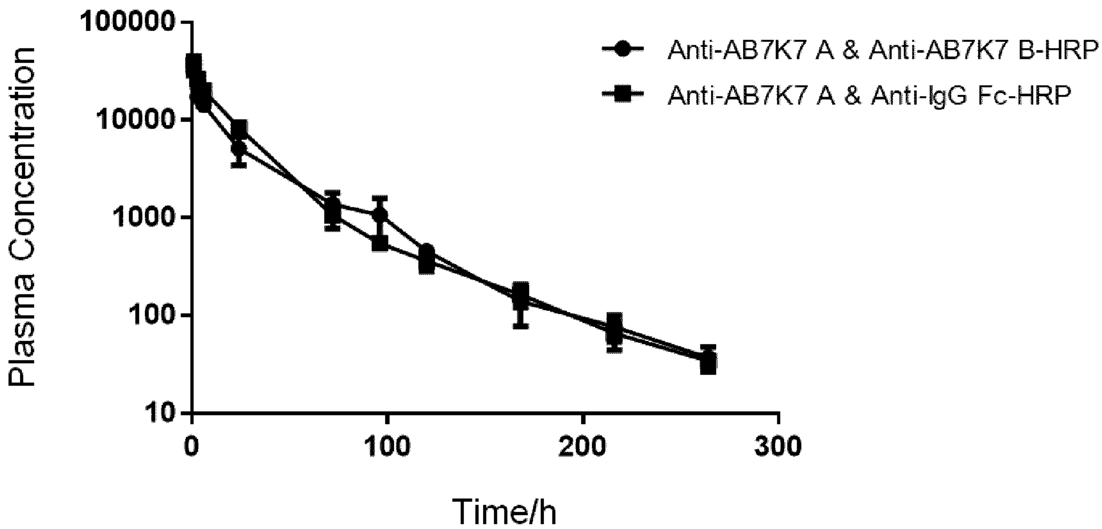


Figure 5-1

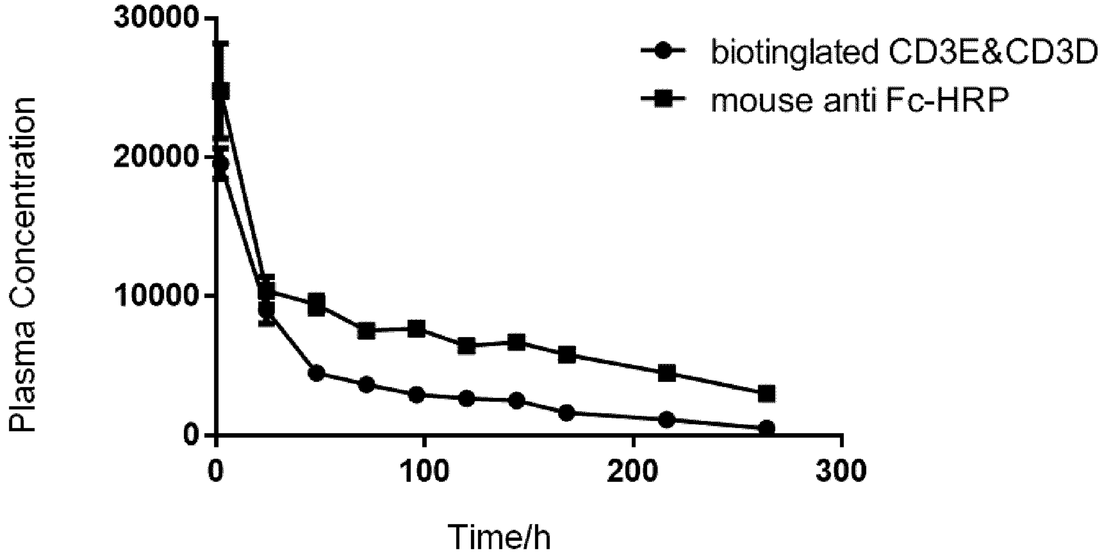


Figure 5-2

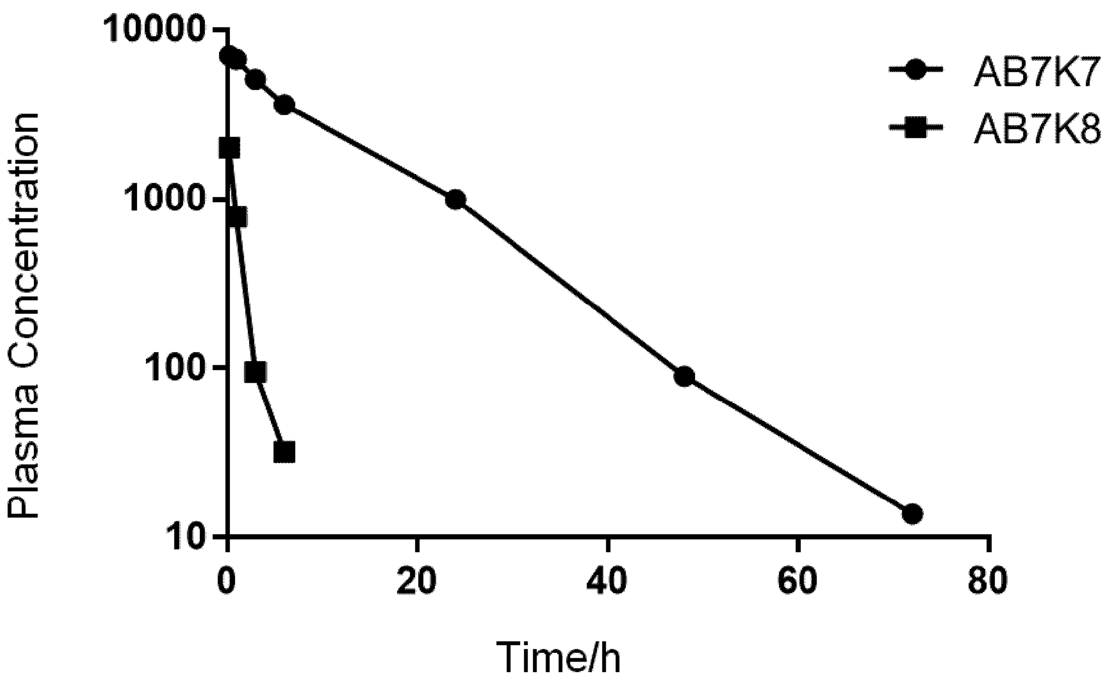


Figure 5-3

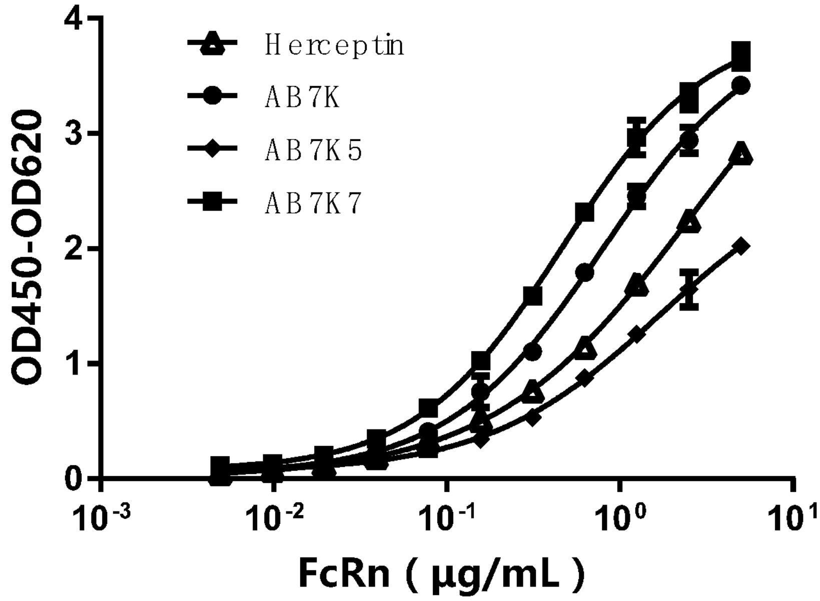


Figure 5-4

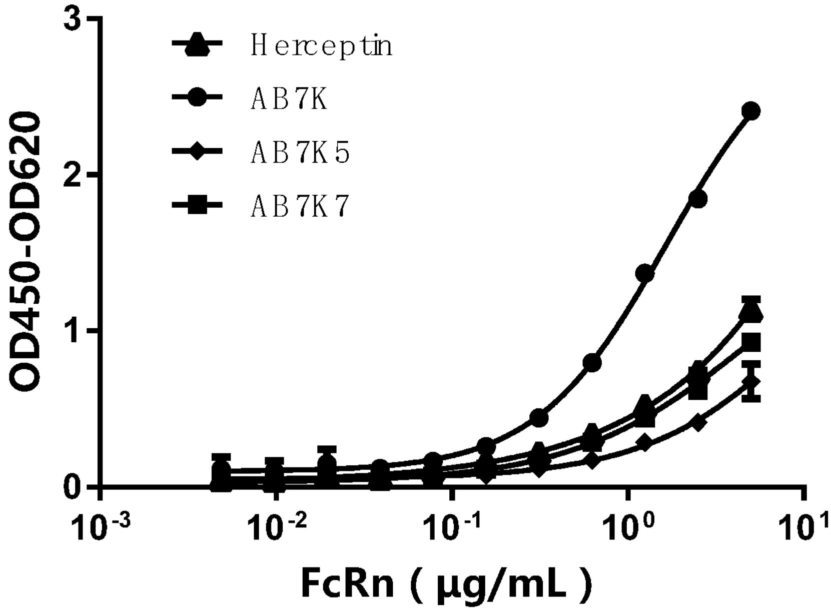


Figure 5-5

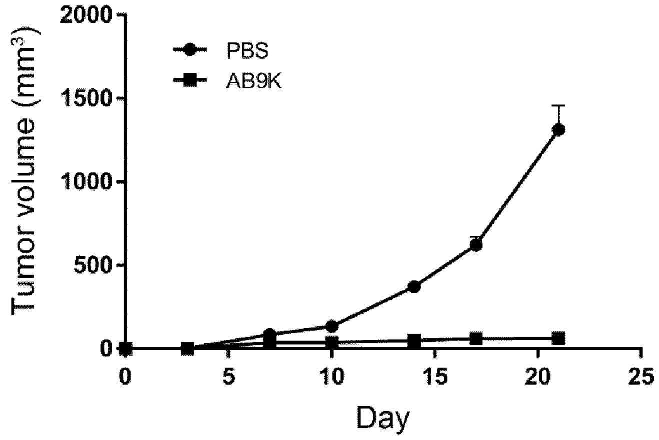


Figure 6-1

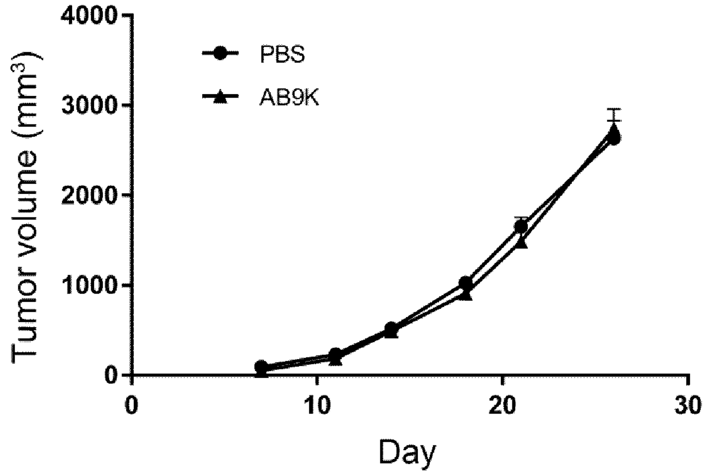


Figure 6-2

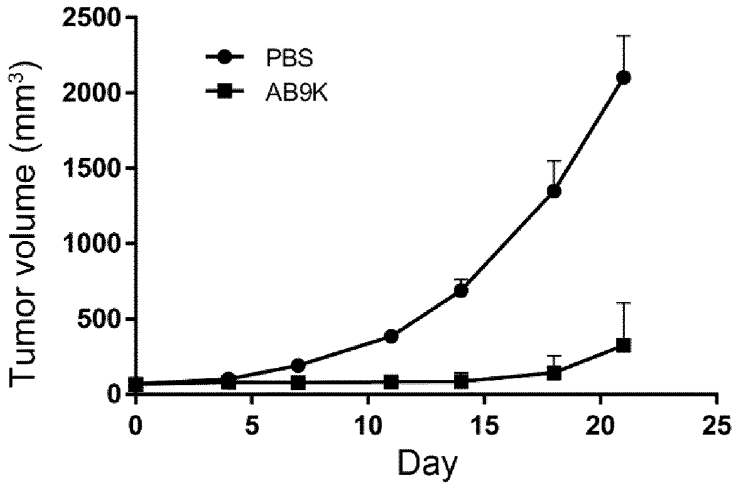


Figure 6-3

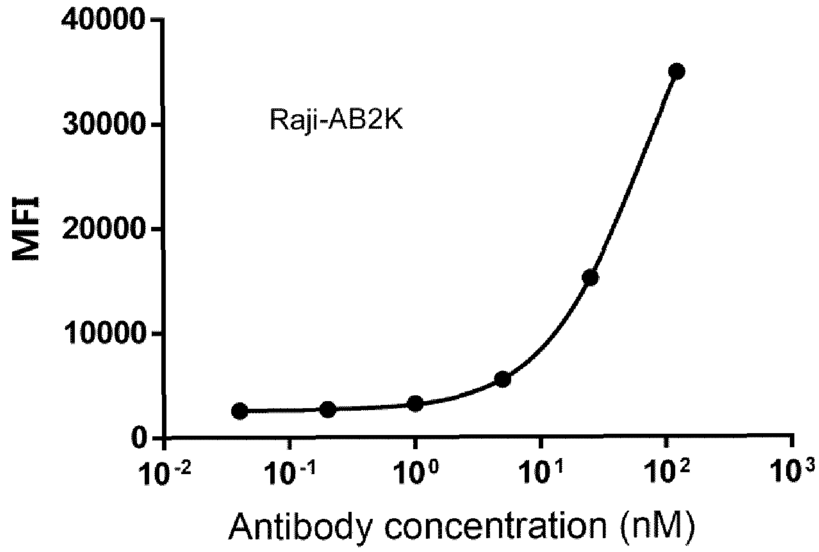


Figure 7-1

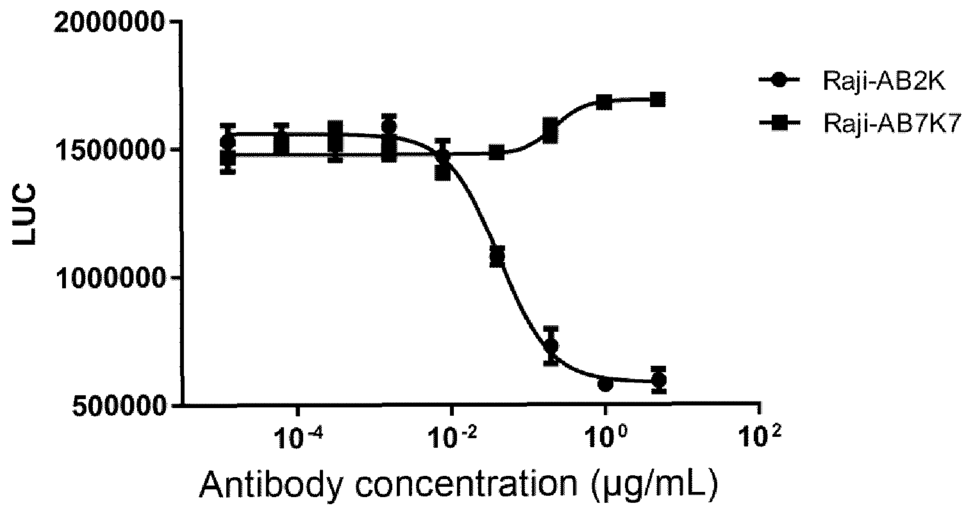


Figure 7-2

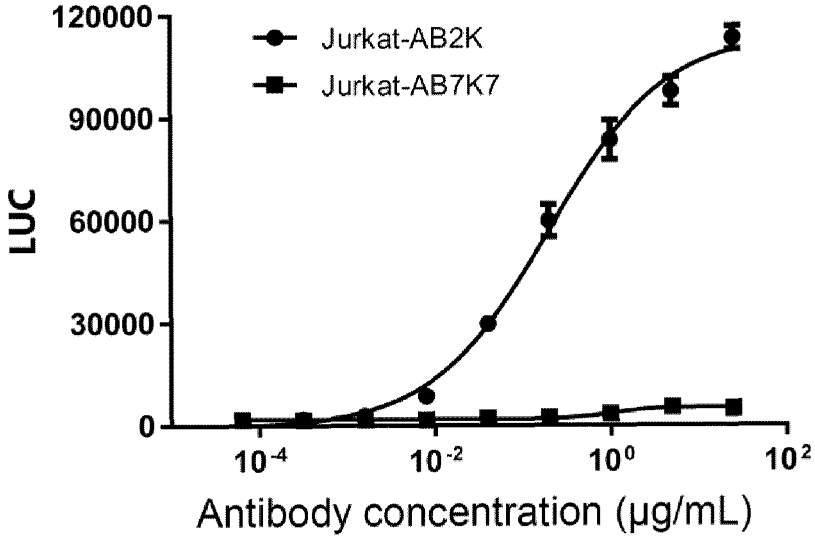


Figure 7-3

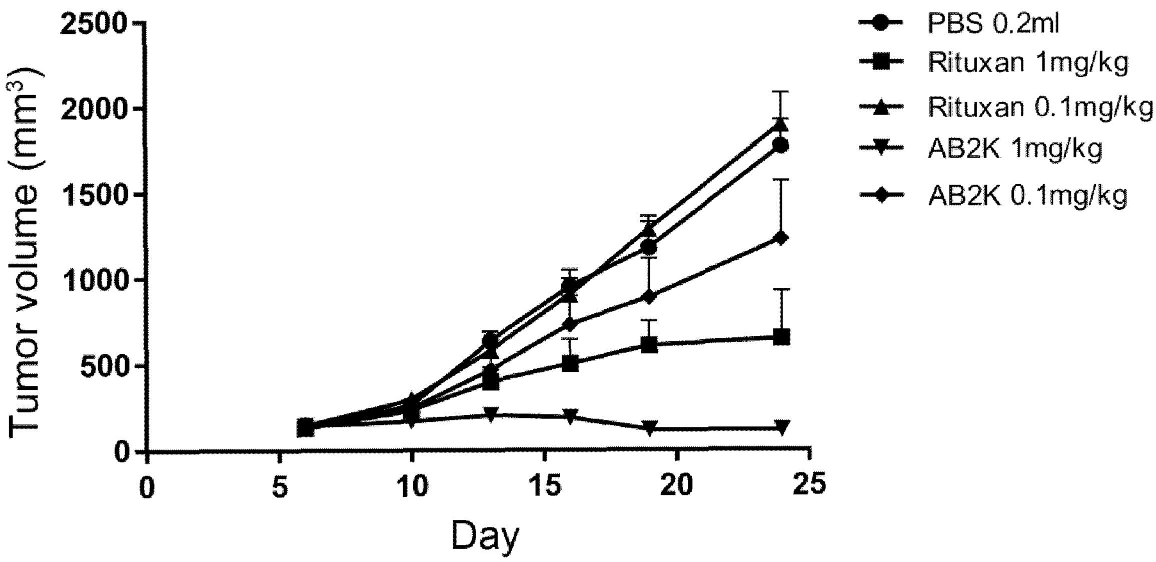


Figure 7-4

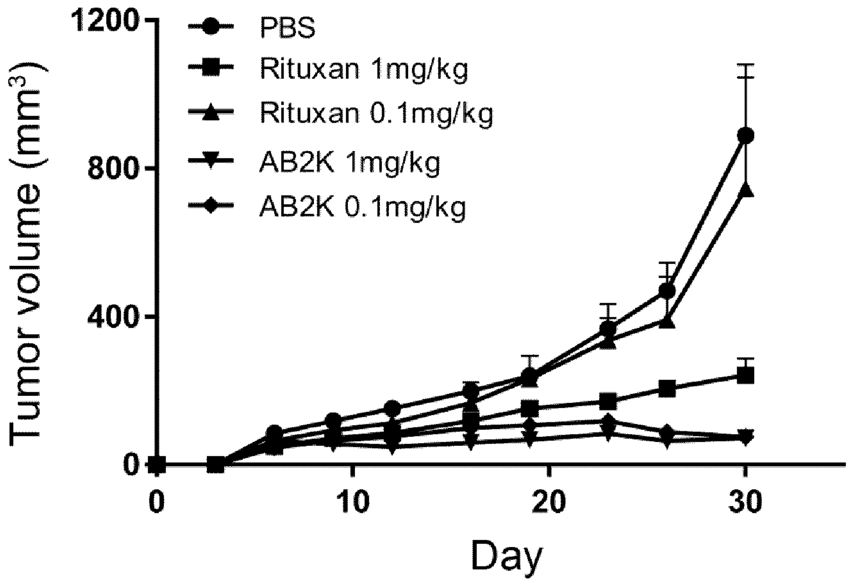


Figure 7-5

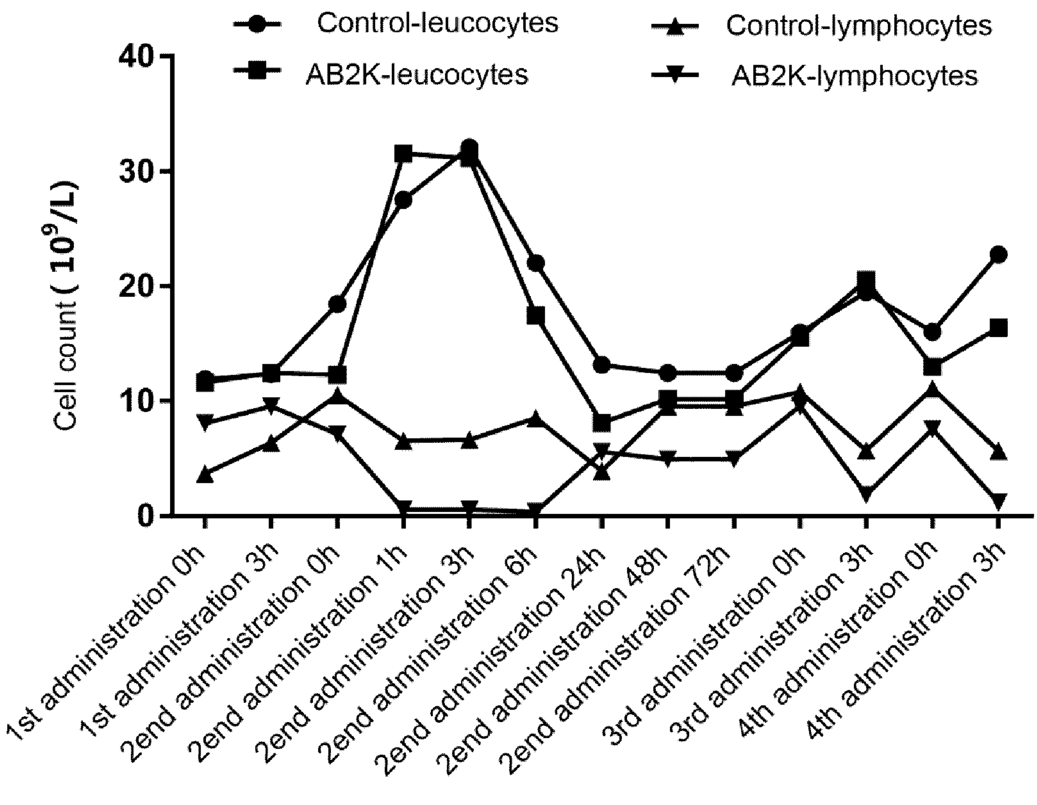


Figure 8

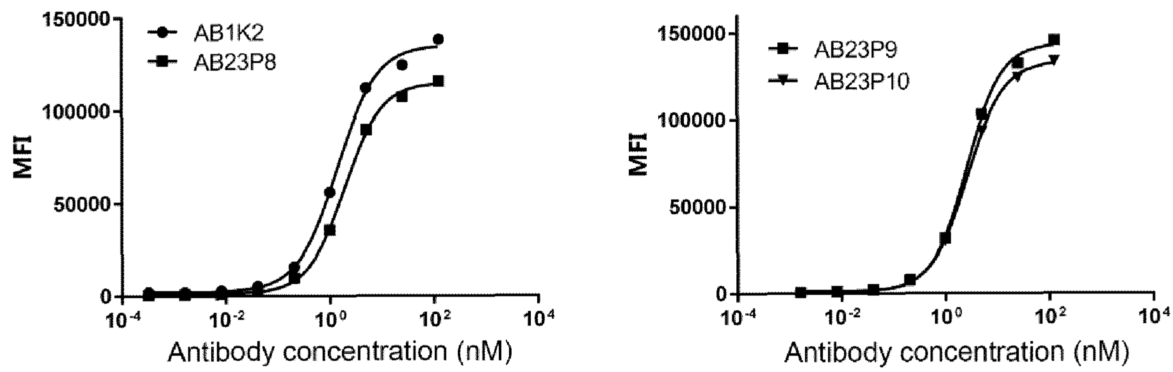


Figure 9-1

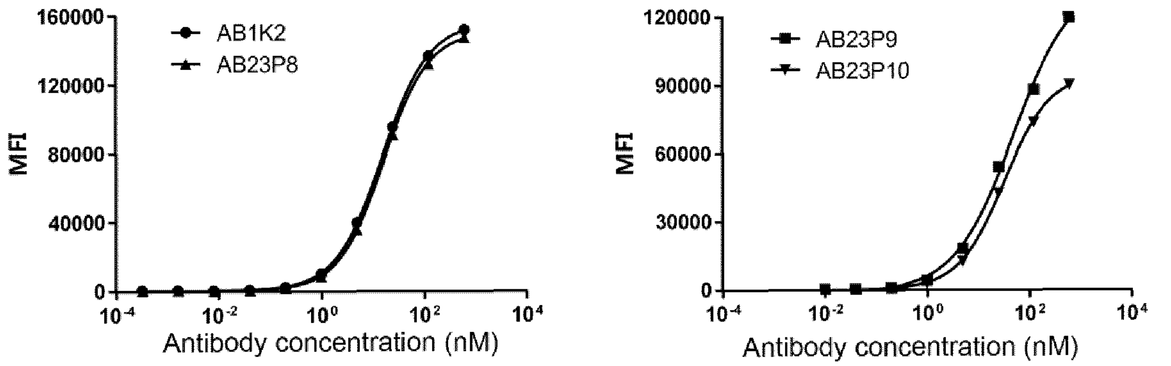


Figure 9-2

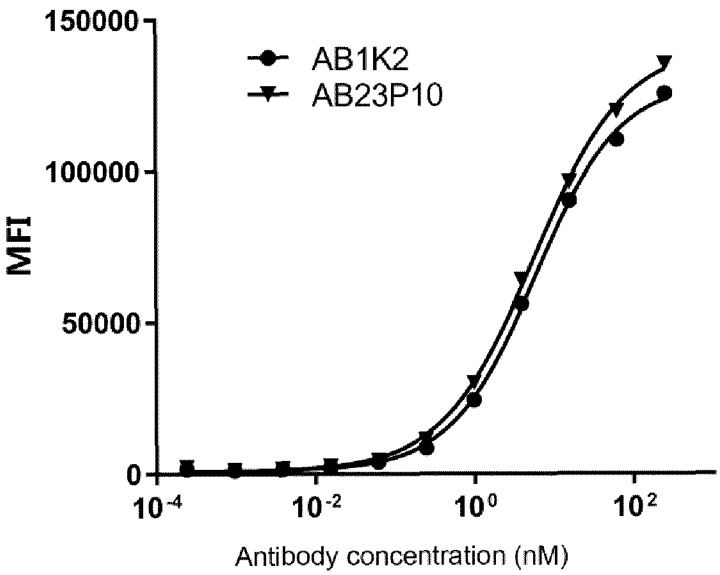


Figure 9-3

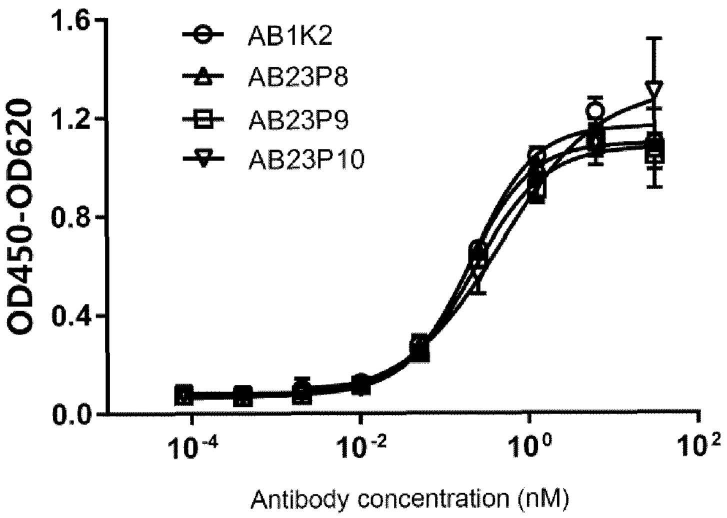


Figure 9-4

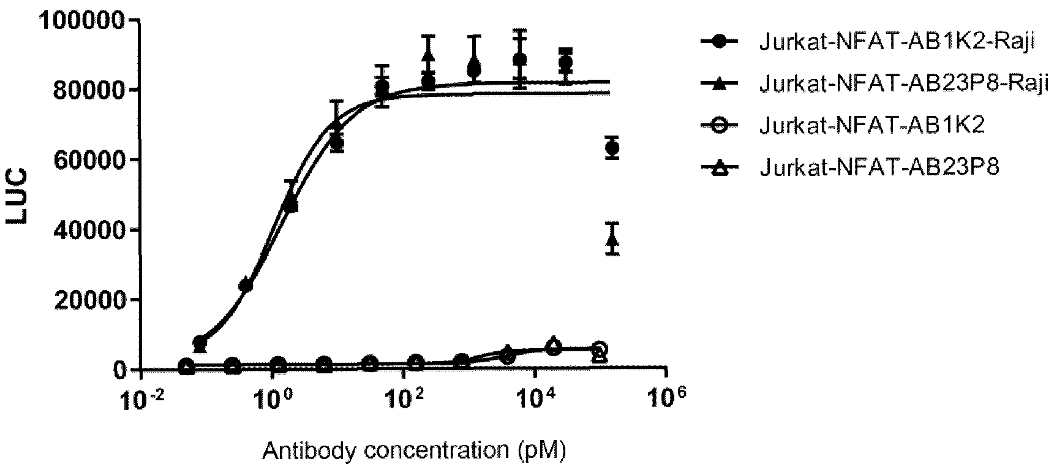


Figure 9-5

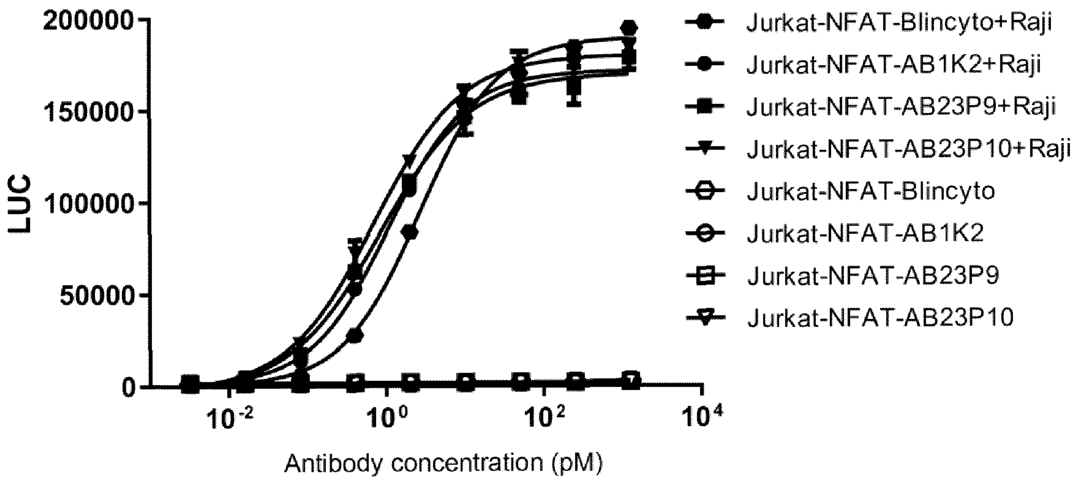

Figure 9-6

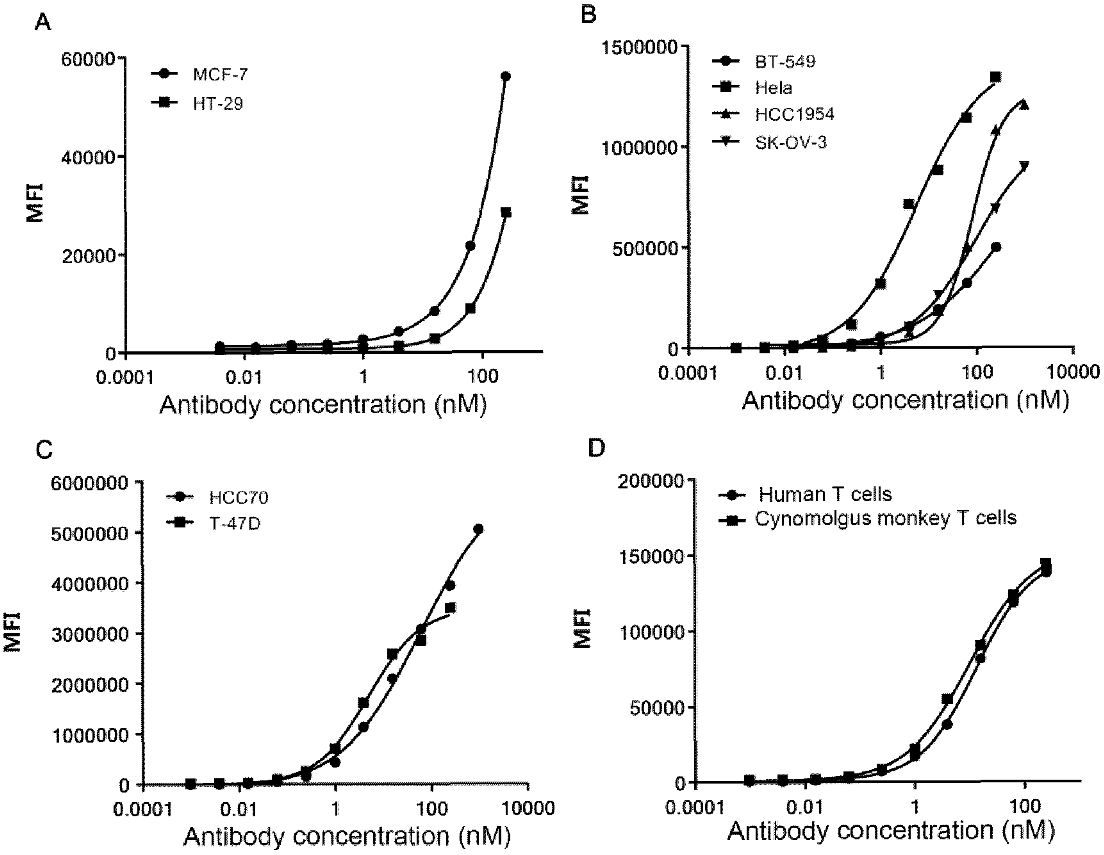


Figure 10-1

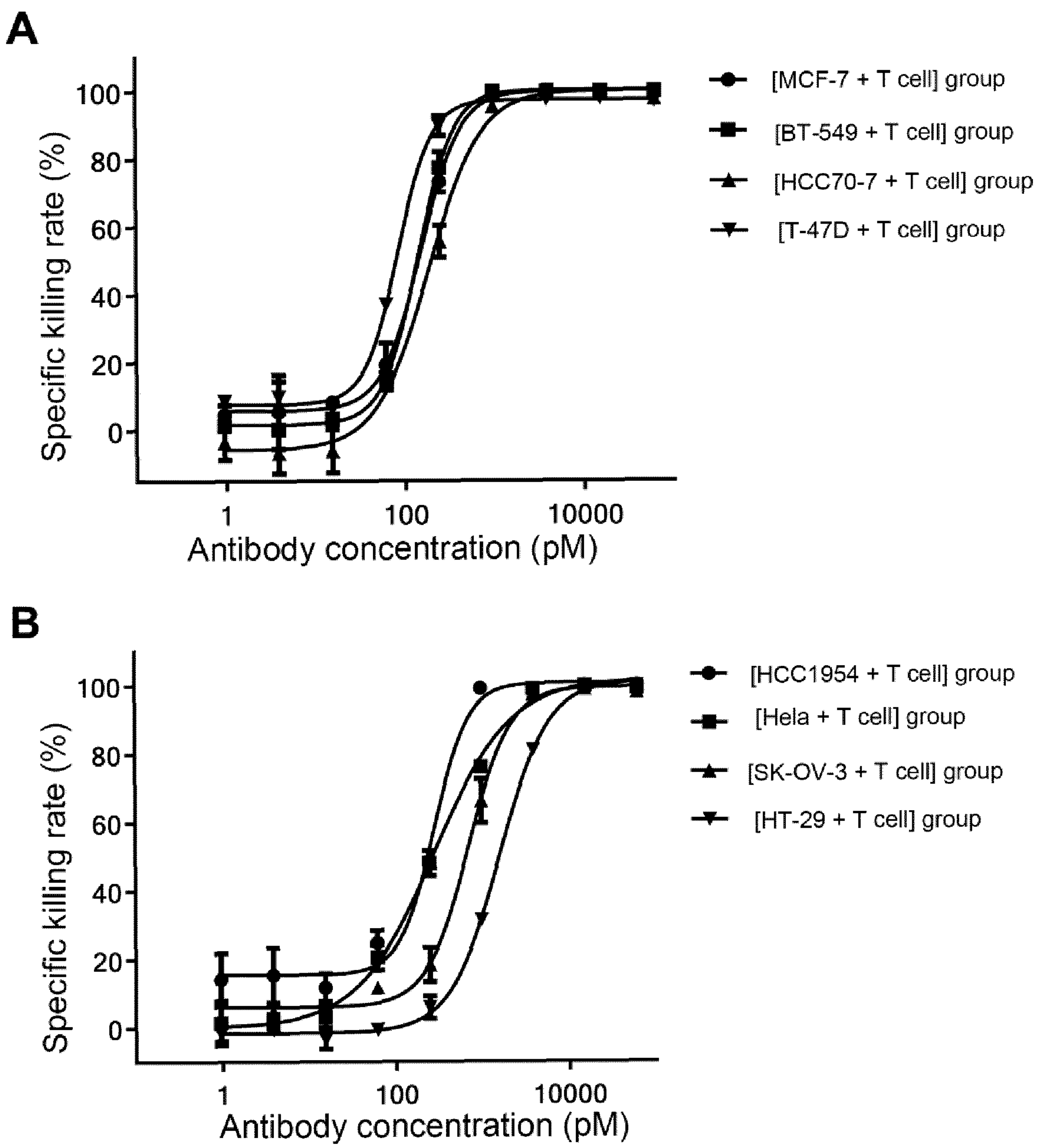
A
Specific killing rate (%)
100
80
60
40
20
0
1
100
10000
Antibody concentration (pM)
[MCF-7 + T cell] group
[BT-549 + T cell] group
[HCC70-7 + T cell] group
[T-47D + T cell] group
B
Specific killing rate (%)
100
80
60
40
20
0
1
100
10000
Antibody concentration (pM)
[HCC1954 + T cell] group
[Hela + T cell] group
[SK-OV-3 + T cell] group
[HT-29 + T cell] group

Figure 10-2

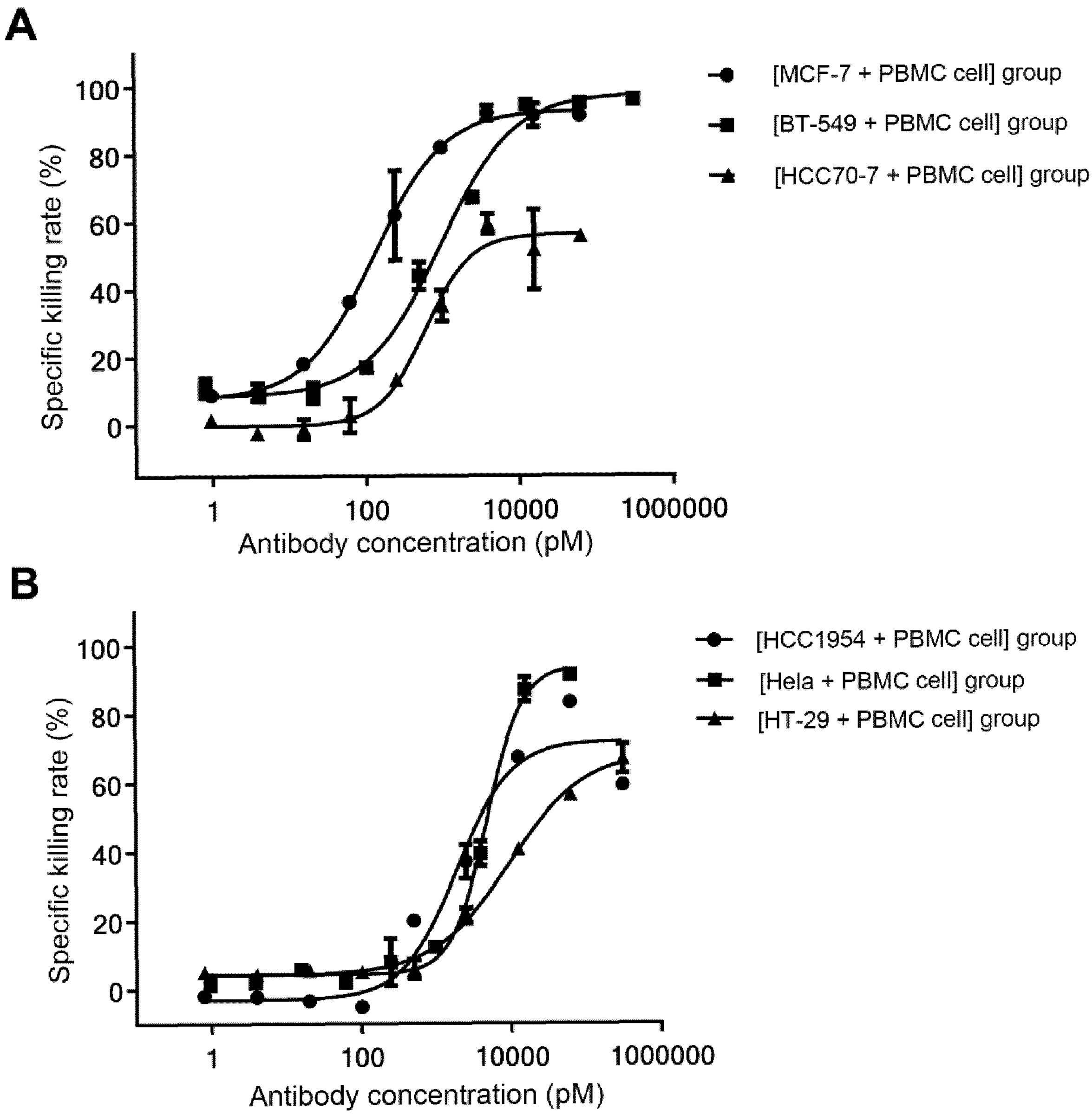
A
100
80
60
40
20
0
Specific killing rate (%)
1
100
10000
1000000
Antibody concentration (pM)
[MCF-7 + PBMC cell] group
[BT-549 + PBMC cell] group
[HCC70-7 + PBMC cell] group
B
100
80
60
40
20
0
Specific killing rate (%)
1
100
10000
1000000
Antibody concentration (pM)
[HCC1954 + PBMC cell] group
[Hela + PBMC cell] group
[HT-29 + PBMC cell] group

Figure 10-3

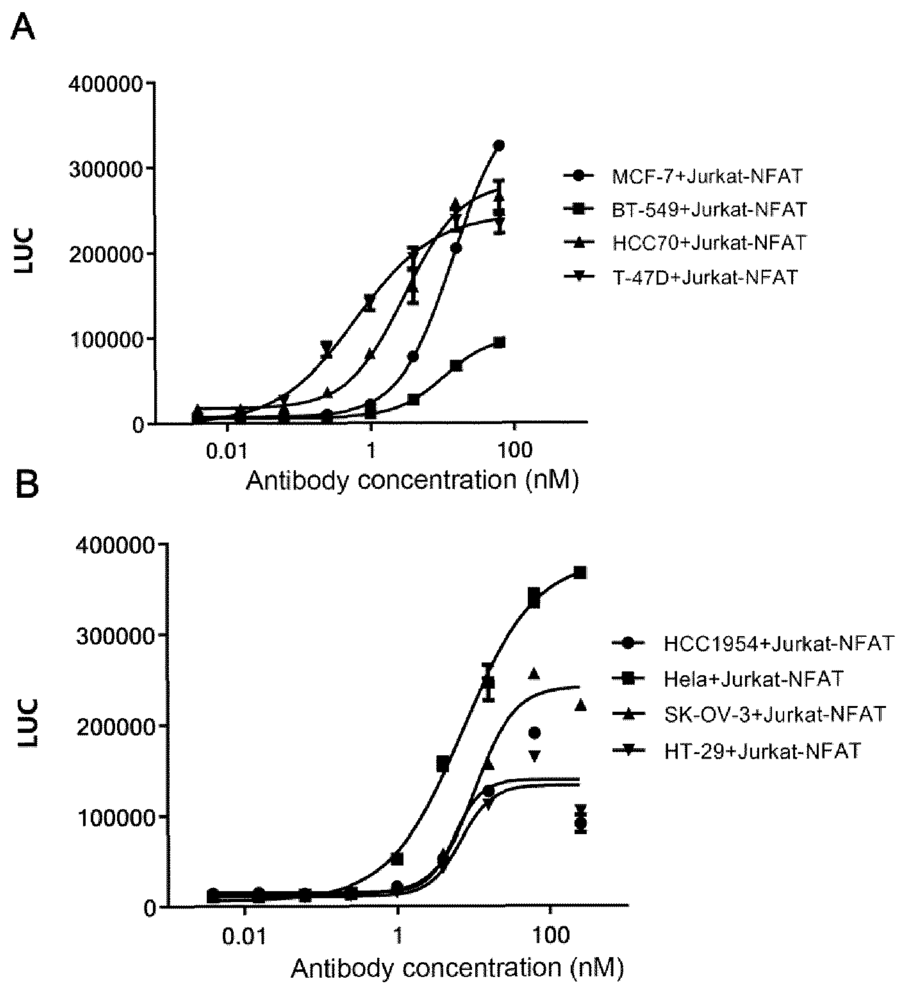


Figure 10-4

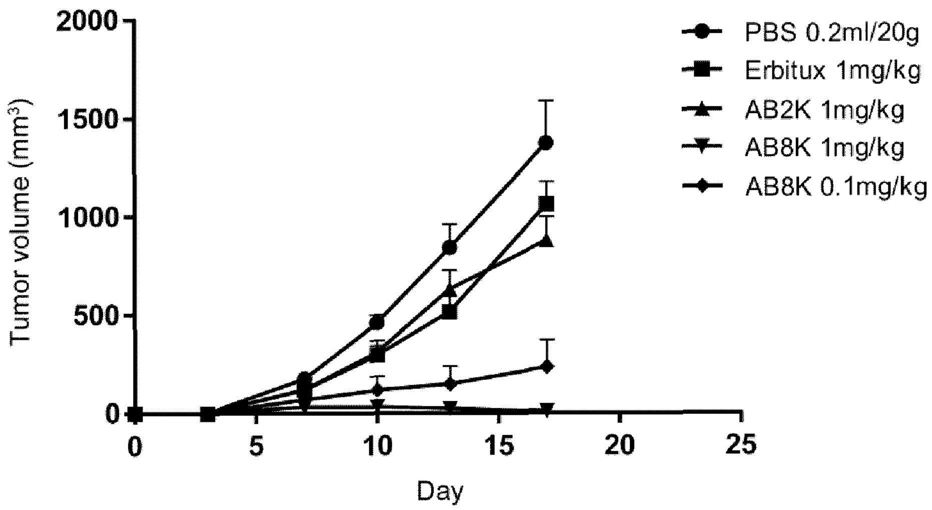


Figure 11

## INTERNATIONAL SEARCH REPORT

International application No.
**PCT/CN2019/114818**

**A. CLASSIFICATION OF SUBJECT MATTER**

C07K 16/46(2006.01)i; C12N 15/13(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

TWTXT; GBTXT; EPTXT; USTXT; WOTXT; CATXT; KRTXT; JPTXT; CNTXT; VEN; CNKI; ISI WEB OF SCIENCE; PUBMED: T250Q, M428L, BiTE, BiAb, Bispecific antibody, M252Y, L235A, CD3, K447, single chain variable fragment, P331S, FC, SCFV, L234A, T256E, SCFV2-FC, SCFV-SCFV-FC, S254T, N297A, N434S, SSSSKAPPPS, single chain fragment variable, single chain fv, tumor, cancer, FV, 单链抗体, 双特异抗体, 肿瘤, 癌

**C. DOCUMENTS CONSIDERED TO BE RELEVANT**

| Category* | Citation of document, with indication, where appropriate, of the relevant passages | Relevant to claim No. |
|---|---|---|
| X | ZOU, Jianxuan et. al. "Immunotherapy based on Bispecific T-cell Engager with hIgG1 Fc Sequence As a New Therapeutic Strategy in Multiple Myeloma" *Cancer Science,* 31 December 2015 (2015-12-31), the abstract, figures S1-S5, table S1, and pages 2, left column and page 9, right column | 1-63 |
| X | ASANO, R. et. al. "Cytotoxic Enhancement of a Bispecific Diabody by Format Conversion to Tandem Single-chain Variable Fragment (taFv) the Case of The hEx3 Diabody" *The Journal of Biological Chemistry,* Vol. 286, No. 3, 19 November 2010 (2010-11-19), the abstract, and pp. 1813-1817 | 1-63 |
| A | NATSUME, A. et. al. "Fucose Removal from Complex-type Oligosaccharide Enhances the Antibody-dependent Cellular Cytotoxicity of Single-gene-encoded Bispecific Antibody Comprising of Two Single-chain Antibodies Linked to The Antibody Constant Region" *The Journal of Biochemistry,* Vol. 140, No. 3, 31 December 2006 (2006-12-31), pp. 359-368 | 1-63 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2019** | **01 February 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No. **PCT/CN2019/114818**

**C. DOCUMENTS CONSIDERED TO BE RELEVANT**

| Category* | Citation of document, with indication, where appropriate, of the relevant passages | Relevant to claim No. |
|---|---|---|
| A | 王栋 (WANG, Dong). "多价抗前列腺癌/抗人CD3双特异性单链抗体的构建与表达 (Construction and Expression of Multivalent Anti-CD3×anti-prostate-cancer Bispecific Single-Chain Antibody)" *中国优秀硕士学位论文全文数据库医药卫生科技辑 (Chinese Master's Theses Full-text Database, Medical and Health Sciences),* No. 02, 15 December 2002 (2002-12-15), pp. E072-358 | 1-63 |
| A | EP 3363818 A1 (AJOU UNIVERSITY INDUSTRY-ACADEMIC COOPERATION FOUNDATION) 22 August 2018 (2018-08-22) entire document | 1-63 |
| A | CN 102918057 A (CHUGAI PHARMACEUTICAL CO., LTD.) 06 February 2013 (2013-02-06) entire document | 1-63 |
| A | US 2017274072 A1 (TOHOKU UNIVERSITY) 28 September 2017 (2017-09-28) entire document | 1-63 |
| A | TW 201731874 A (AMGEN RESEARCH (MUNICH) GMBH et al.) 16 September 2017 (2017-09-16) entire document | 1-63 |
| A | CN 106117370 A (ANYUAN PHARMACEUTICAL TECHNOLOGY (SHANGHAI) CO., LTD.) 16 November 2016 (2016-11-16) entire document | 1-63 |
| A | CN 103476795 A (ROCHE GLYCART AG) 25 December 2013 (2013-12-25) entire document | 1-63 |

Form PCT/ISA/210 (second sheet) (January 2015)

**INTERNATIONAL SEARCH REPORT**

International application No.
**PCT/CN2019/114818**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2019/114818**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **62-63**
because they relate to subject matter not required to be searched by this Authority, namely:

[1] Claims 62 and 63 relate to a treatment method for diseases, and do not meet the requirement of PCT Rule 39.1(iv). However, the search is still carried out on the basis of the corresponding pharmaceutical application of the treatment method.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/114818**

| Patent document cited in search report | | | Publication date (day/month/year) | Patent family member(s) | | | Publication date (day/month/year) |
|---|---|---|---|---|---|---|---|
| EP | 3363818 | A1 | 22 August 2018 | KR | 101851380 | B1 | 23 April 2018 |
| | | | | KR | 20170042943 | A | 20 April 2017 |
| | | | | EP | 3363818 | A4 | 25 September 2019 |
| | | | | US | 2018346600 | A1 | 06 December 2018 |
| | | | | WO | 2017065484 | A1 | 20 April 2017 |
| CN | 102918057 | A | 06 February 2013 | SG | 10201703798 Y | A | 29 June 2017 |
| | | | | SI | 2552955 | T1 | 31 August 2017 |
| | | | | JP | 2013528354 | A | 11 July 2013 |
| | | | | US | 2016244526 | A1 | 25 August 2016 |
| | | | | KR | 20190047111 | A | 07 May 2019 |
| | | | | EP | 3181581 | A1 | 21 June 2017 |
| | | | | LT | 2552955 | T | 25 July 2017 |
| | | | | TW | 201202419 | A | 16 January 2012 |
| | | | | EP | 2552955 | B1 | 03 May 2017 |
| | | | | WO | 2011122011 | A2 | 06 October 2011 |
| | | | | WO | 2011122011 | A3 | 05 January 2012 |
| | | | | JP | 2016145222 | A | 12 August 2016 |
| | | | | JP | 2017036318 | A | 16 February 2017 |
| | | | | US | 2017002080 | A1 | 05 January 2017 |
| | | | | KR | 20130010002 | A | 24 January 2013 |
| | | | | JP | 5415624 | B2 | 12 February 2014 |
| | | | | JP | 2019178134 | A | 17 October 2019 |
| | | | | AU | 2011233390 | B2 | 20 August 2015 |
| | | | | US | 2013131319 | A1 | 23 May 2013 |
| | | | | KR | 101974794 | B1 | 02 May 2019 |
| | | | | HR | P20171080 | T1 | 06 October 2017 |
| | | | | JP | 5913243 | B2 | 27 April 2016 |
| | | | | EP | 2552955 | A2 | 06 February 2013 |
| | | | | CA | 2794860 | A1 | 06 October 2011 |
| | | | | KR | 20180019764 | A | 26 February 2018 |
| | | | | JP | 6029252 | B2 | 24 November 2016 |
| | | | | PL | 2552955 | T3 | 29 December 2017 |
| | | | | ES | 2633597 | T3 | 22 September 2017 |
| | | | | US | 2017002066 | A1 | 05 January 2017 |
| | | | | TW | 201700499 | A | 01 January 2017 |
| | | | | US | 2017226206 | A1 | 10 August 2017 |
| | | | | SG | 184163 | A1 | 30 October 2012 |
| | | | | TW | I667257 | B | 01 August 2019 |
| | | | | RU | 2012146098 | A | 10 May 2014 |
| | | | | PT | 2552955 | T | 12 July 2017 |
| | | | | JP | 2014065708 | A | 17 April 2014 |
| | | | | KR | 101831464 | B1 | 22 February 2018 |
| | | | | TW | I667346 | B | 01 August 2019 |
| | | | | JP | 6518222 | B2 | 22 May 2019 |
| | | | | AU | 2011233390 | A1 | 08 November 2012 |
| | | | | HU | E033812 | T2 | 29 January 2018 |
| | | | | DK | 2552955 | T3 | 21 August 2017 |
| | | | | MX | 2012011338 | A | 30 November 2012 |
| US | 2017274072 | A1 | 28 September 2017 | WO | 2015146438 | A1 | 01 October 2015 |
| | | | | JP | WO2015146438 | A1 | 13 April 2017 |

Form PCT/ISA/210 (patent family annex) (January 2015)

**INTERNATIONAL SEARCH REPORT**
**Information on patent family members**

International application No.
**PCT/CN2019/114818**

| Patent document cited in search report | Publication date (day/month/year) | Patent family member(s) | Publication date (day/month/year) |
|---|---|---|---|
| TW 201731874 A | 16 September 2017 | WO 2017134134 A1 | 10 August 2017 |
| | | US 10301391 B2 | 28 May 2019 |
| | | CN 109219617 A | 15 January 2019 |
| | | PE 15362018 A1 | 26 September 2018 |
| | | CO 2018007552 A2 | 31 July 2018 |
| | | UY 37106 A | 31 August 2017 |
| | | JP 2017186301 A | 12 October 2017 |
| | | AU 2017216230 A1 | 19 July 2018 |
| | | PH 12018501536 A1 | 15 May 2019 |
| | | KR 20180103084 A | 18 September 2018 |
| | | BR 112018015715 A2 | 05 February 2019 |
| | | EP 3411402 A1 | 12 December 2018 |
| | | EA 201891755 A1 | 28 February 2019 |
| | | CR 20180420 A | 05 December 2018 |
| | | US 2017218077 A1 | 03 August 2017 |
| | | CA 3010704 A1 | 10 August 2017 |
| | | CL 2018002057 A1 | 15 February 2019 |
| | | SG 11201805770 U A | 30 August 2018 |
| | | PE 20181536 A1 | 26 September 2018 |
| | | AR 107521 A1 | 09 May 2018 |
| | | MX 2018009108 A | 11 March 2019 |
| CN 106117370 A | 16 November 2016 | CN 106117370 B | 17 May 2017 |
| CN 103476795 A | 25 December 2013 | IL 228002 A | 30 August 2018 |
| | | AR 085741 A1 | 23 October 2013 |
| | | RU 2607014 C2 | 10 January 2017 |
| | | SG 193554 A1 | 29 November 2013 |
| | | CN 105949313 A | 21 September 2016 |
| | | US 8969526 B2 | 03 March 2015 |
| | | JP 2014514287 A | 19 June 2014 |
| | | LT 2691417 T | 25 October 2018 |
| | | TR 201815420 T4 | 21 November 2018 |
| | | CN 103476795 B | 06 July 2016 |
| | | US 2017137530 A1 | 18 May 2017 |
| | | IL 228002 D0 | 30 September 2013 |
| | | HK 1193109 A1 | 21 July 2017 |
| | | AU 2012234335 B2 | 01 September 2016 |
| | | ES 2692268 T3 | 03 December 2018 |
| | | RS 57895 B1 | 31 January 2019 |
| | | NZ 614658 A | 31 July 2015 |
| | | US 2015239981 A1 | 27 August 2015 |
| | | EP 2691417 B1 | 01 August 2018 |
| | | PT 2691417 T | 31 October 2018 |
| | | KR 20130139342 A | 20 December 2013 |
| | | DK 2691417 T3 | 19 November 2018 |
| | | HU E041335 T2 | 28 May 2019 |
| | | WO 2012130831 A1 | 04 October 2012 |
| | | JP 5926791 B2 | 25 May 2016 |
| | | SI 2691417 T1 | 30 November 2018 |
| | | BR 112013024574 A2 | 19 September 2017 |
| | | MY 163539 A | 15 September 2017 |

Form PCT/ISA/210 (patent family annex) (January 2015)

**INTERNATIONAL SEARCH REPORT**
**Information on patent family members**

International application No.
**PCT/CN2019/114818**

| Patent document cited in search report | Publication date (day/month/year) | Patent family member(s) | | | Publication date (day/month/year) |
|---|---|---|---|---|---|
| | | HR | P20181690 | T1 | 28 December 2018 |
| | | PL | 2691417 | T3 | 31 January 2019 |
| | | RU | 2013145623 | A | 10 May 2015 |
| | | KR | 20160044598 | A | 25 April 2016 |
| | | CA | 2828289 | A1 | 04 October 2012 |
| | | MX | 336740 | B | 29 January 2016 |
| | | SG | 10201602394 Q | A | 30 May 2016 |
| | | US | 2012251531 | A1 | 04 October 2012 |
| | | KR | 101614195 | B1 | 20 April 2016 |
| | | MX | 354359 | B | 28 February 2018 |
| | | AU | 2012234335 | A1 | 05 September 2013 |
| | | MX | 2013009981 | A | 26 September 2013 |
| | | EP | 2691417 | A1 | 05 February 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201811294887
- EP 388151 A1
- US 5648260 A
- US 5624821 A
- CN 201280066663
- US 20050014934 A1
- WO 9743316 A
- US 5869046 A
- US 574703 A
- WO 9632478 A
- US 7994289 B
- US 6750325 B
- US 6706265 B
- US 5968509 A
- US 8076459 B
- US 7728114 B
- US 20100183615 A
- WO 2016130726 A
- US 20050176028 A
- WO 2007042261 A
- WO 2008119565 A
- WO 8801649 A
- US 4946778 A
- US 5260203 A
- KR 101027427
- KR 20100099179
- US 7083784 B
- US 20100234575
- US 7670600 B
- US 2012070146 W
- WO 2013096221 A
- US 4399216 A
- US 4818679 A


### Non-patent literature cited in the description


- **STEARZ UD et al.** *Nature,* 1985, vol. 314, 628-631
- **KUNG P et al.** *Science,* 1979, vol. 206, 347-349
- **HIRSCH R et al.** *J. Immunol.,* 1989, vol. 142, 737-743
- **KUHN C et al.** *Immunotherapy,* 2016, vol. 8, 889-906
- **Z WU et al.** *Pharmacology and Therapeutics,* 2018, vol. 182, 161-175
- **NATSUME A et al.** *Cancer Res,* 2008, vol. 68, 3863-3872
- **IDUSOGIE EE et al.** *J. Immunol.,* 2001, vol. 166, 2571-2575
- **LAZAR GA et al.** *PNAS,* 2006, vol. 103, 4005-4010
- **SHIELDS RL et al.** *JBC,* 2001, vol. 276, 6591-6604
- **STAVENHAGEN JB et al.** *Cancer Res.,* 2007, vol. 67, 8882-8890
- **STAVENHAGEN JB et al.** *Advan. Enzyme. Regul.,* 2008, vol. 48, 152-164
- **ALEGRE ML et al.** *J. Immunol.,* 1992, vol. 148, 3461-3468
- **KANEKO E et al.** *Biodrugs,* 2011, vol. 25, 1-11
- **SAMBROOK.** Molecular Cloning: A Laboratory Manual. Cold Spring Harbor Laboratory, 1989
- **KABAT et al.** Sequences of Proteins of Immunological Interest. Public Health Service, National Institutes of Health, 1991
- **CHOTHIA ; LESKL.** *J. Mol Biol,* 1987, vol. 196, 901-917
- **PLUCKTHUN.** The Pharmacology of Monoclonal Antibodies. Springer-Verlag, 1994, vol. 113, 269-315
- **NEEDLEMAN et al.** *Mol. Biol.,* vol. 48, 443-453
- **E. MEYERS ; W. MILLER.** *Comput. Appl Biosci.,* vol. 4, 11-17
- **NEEDLEMAN ; WUNSCH.** *J. Mol. Biol.,* vol. 48, 444-453
- *M. Annu. Rev. Immunol.,* 1997, vol. 15, 203-234
- **GHETIE V et al.** *Immunol Today,* 1997, vol. 18, 592-8
- **GHETIE V et al.** *Nature Biotechnology,* 1997, vol. 15, 637-40
- **GUYER RL et al.** *J. Immunol.,* 1976, vol. 117, 587
- **KIM YJ et al.** *J. Immunol.,* 1994, vol. 24, 249
- **MALMQVIST M et al.** *Nature,* 1993, vol. 361, 186-187
- **DAVIES et al.** *Annual Rev Biochem.,* 1990, vol. 59, 439-473

- **CHAMES P et al.** *Curr. Opin. Drug Disc. Dev.,* 2009, vol. 12, 276
- **SPIESS C et al.** *Mol. Immunol.,* 2015, vol. 67, 95-106
- **FARES F A et al.** *Proc Natl Acad. Sci. USA,* 1992, vol. 89, 4304-4308
- **ROOPENIAN et al.** *Nat. Rev. Immunol.,* 2007, vol. 7, 715-725
- **HINTON P.R. et al.** *J. Immunol.,* 2006, vol. 176, 346-356
- **DATTA-MANNAN A et al.** MAbs. Taylor&Francis, 2012, vol. 4, 267-273
- **XU D et al.** *Cellular Immunol.,* 2000, vol. 200, 16-26
- **STAERZ UD et al.** *Nature,* 1985, vol. 314, 628-31
- **MILSTEIN C et al.** *Nature,* 1983, vol. 305, 537-540
- **KARPOVSKY B et al.** *J. Exp. Med.,* 1984, vol. 160, 1686-1701
- **CHAMES ; BATY.** *Curr. Opin. Drug. Discov. Devel.,* 2009, vol. 12, 276-83
- **CHAMES ; BATY.** *mAbs,* vol. 1, 539-47
- **SENSI M et al.** *Clin. Cancer Res.,* 2006, vol. 12, 5023-32
- **PARMIANI et al.** *J. Immunol.,* 2007, vol. 178, 1975-79
- **NOVELLINO L et al.** *Cancer Immunol. Immunother.,* 2005, vol. 54, 187-207